# EUROPEAN PATENT SPECIFICATION

(11) **EP 4 436 667 B1**
(45) Date of publication and mention of the grant of the patent: **21.01.2026**
(21) Application number: 22809370.4
(22) Date of filing: 24.10.2022
(51) Int. Cl.: A61P 31/04, C07D 401/14, C07D 403/14, C07D 413/14, C07D 471/10, C07D 487/04, C07D 487/10, C07D 498/04, C07D 498/10

(54) **PIPERAZINYLSULFONYLARYL COMPOUNDS FOR TREATMENT OF BACTERIAL INFECTIONS**
PIPERAZINYLSULFONYLARYLVERBINDUNGEN ZUR BEHANDLUNG VON BAKTERIELLEN INFEKTIONEN
COMPOSÉS DE PIPÉRAZINYLSULFONYLARYLE POUR LE TRAITEMENT D'INFECTIONS BACTÉRIENNES

(30) Priority: 26.10.2021 WO PCT/CN2021/126369
(43) Date of publication of application: 02.10.2024
(73) Proprietor: F. Hoffmann-La Roche AG, 4070 Basel (CH)
(72) Inventor: DEY, Fabian, 4070 Basel (CH); DING, Xiao, Shanghai 201203 (CN); SHI, Houguang, Shanghai 201203 (CN); TAN, Xuefei, Shanghai 201203 (CN); WU, Jun, Shanghai 201203 (CN); ZHENG, Jiamin, Shanghai 201203 (CN); ZHOU, Mingwei, Shanghai 201203 (CN)
(74) Representative: Vitra, Hermeto
(86) International application number: PCT/EP2022/079506
(87) International publication number: WO 2023/072794

(56) References cited:
- WO-A2-03/032962
- KUMAR G. V. ET AL: "DESIGN, SYNTHESIS AND BIOLOGICAL EVALUATION OF NOVEL BROMO-PYRIMIDINE ANALOGS AS ANTICANCER AND ANTIMICROBIAL AGENTS", IJPCBS, vol. 9, no. 1, 1 January 2019 (2019-01-01), pages 27 - 41, XP093016039

## Description

The present invention relates to organic compounds useful for the treatment and/or prevention of bacterial infections in a mammal. Specifically these molecules can inhibit the LPS synthesis pathway, in particular to inhibit LpxH, and are useful for treating bacterial infections.

### BACKGROUND OF THE INVENTION

The intensive use of antibiotics has exerted a selective evolutionary pressure on microorganisms to produce genetically based resistance mechanisms. Modern medicine and socio-economic behaviour exacerbate the problem of resistance development by creating slow growth situations for pathogenic microbes, e.g. in artificial joints, and by supporting long-term host reservoirs, e.g. in immune-compromised patients. In hospital settings, an increasing number of strains of *Staphylococcus aureus, Streptococcus pneumoniae, Enterococcus spp., Enterobacteriaceae* such as *Klebsiella pneumonia, Acinetobacter baumannii* and *Pseudomonas aeruginosa,* major sources of infections, are becoming multi-drug resistant and therefore difficult to treat. This is particularly the case for Gram-negative organisms where the situation is getting worrisome since no novel agents with a differentiated mechanism of action have been approved for decades. Therefore, there is an important medical need for new antibacterial compounds addressing Gram-negative resistant bacteria, in particular third generation cephalosporins- and carbapenem-resistant *Enterobacteriaceae* and multi-drug-resistant *Pseudomonas aeruginosa* and *Acinetobacter baumannii.* One way to tackle the problem of cross-resistance to established classes of antibiotics is to inhibit an essential protein or function not targeted by current antibiotics. WO 03/032962 already disclosed antimicrobial agents effective against a variety of multi-drug resistant bacteria.

Gram-negative bacteria are unique in that their outer membrane contains Lipopolysaccharide (LPS), which is crucial for maintaining membrane integrity, and is essential for bacterial viability (reviewed in Ann. Rev. Biochem 76: 295-329, 2007). The major lipid component of LPS is Lipid A, and inhibition of Lipid A biosynthesis is lethal to bacteria. Lipid A is synthesized on the cytoplasmic surface of the bacterial inner membrane via a pathway that consists of nine different enzymes. These enzymes are highly conserved in most Gram-negative bacteria. LpxH, a calcineurin-like phosphatase (CLP), catalyzes the hydrolysis of UDP-2,3-diacyl-glucosamine (UDP-DAGn) to yield Lipid X and UMP (22, 24, 25). LpxH has no mammalian homologue, making it a good target for the development of novel antibiotics targeting Gram-negative bacteria.

### SUMMARY OF THE INVENTION

The scope of the invention is defined by the claims. Any references in the description to methods of treatment refer to the compounds, pharmaceutical compositions and medicaments of the present invention for use in a method for treatment of the human (or animal) body by therapy (or for diagnosis). The present invention relates to novel compounds of formula (I), 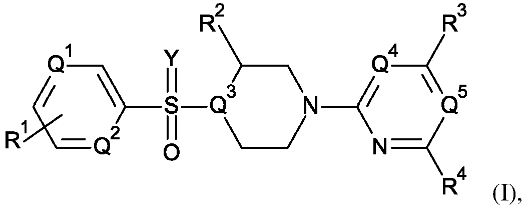 wherein
R¹ is 2-oxo-1-oxa-3,8-diazaspiro[4.5]decanyl unsubstituted or substituted by aminoC₁₋₆alkyl,
   2-oxo-3a,4,5,6,7,7a-hexahydrooxazolo[4.5-c]pyridinyl,
   2-oxo-3a,4,5,6,7,7a-hexahydrooxazolo[5.4-c]pyridinyl,
   2-oxo-3a,4,6,7,8,8a-hexahydrooxazolo[4.5-c]azepinyl substituted by aminoC₁₋₆alkyl or C₁₋₆alkylaminoC₁₋₆alkyl,
   2-oxo-3a,5,6,6a-tetrahydrofuro[2,3-d]oxazolyl substituted twice by substituents independently selected from hydroxy and hydroxyC₁₋₆alkyl,
   2-oxo-4,5,6,6a-tetrahydro-3aH-pyrrolo[3.4-d]oxazolyl,
   2-oxo-4,5,6,7,8,8a-hexahydro-3aH-oxazolo[4,5-c]azepinyl,
   2-oxo-5,6,7,7a-tetrahydro-3aH-pyrano[2,3-d]oxazolyl substituted three times by substituents independently selected from hydroxy and hydroxyC₁₋₆alkyl,
   3-oxo-2,6,9-triazaspiro[4.5]decanyl,
   3-oxo-2,6-diazaspiro[4.5]decanyl,
   3-oxo-2,8-diazaspiro[4.5]decanyl,
   5-oxo-1,2,3,3a,6,6a-hexahydropyrrolo[3,2-b]pyrrolyl,
   6-oxo-5-oxa-2,7-diazaspiro[3.4]octanyl,
   7-oxo-2,6-diazaspiro[3.4]octanyl,
   oxoazetidinyl substituted by amino,
   oxooxazolidinyl substituted once or twice by substituents independently selected from ((aminoC₁₋₆alkyl)azetidinyl)C₁₋₆alkyl, (C₁₋₆alkyl)₂aminoC₁₋₆alkyl, (dihydroxypyrrolidinyl)C₁₋₆alkyl, (pyrrolidinylamino)C₁₋₆alkyl, 2,6-diazaspiro[3.3]heptanylC₁₋₆alkyl, dihydroxy(C₁₋₆alkoxy)tetrahydrofuranyl, aminoazetidinylC₁₋₆alkyl, aminoC₁₋₆alkyl, C₁₋₆alkyl, C₁₋₆alkylaminoC₁₋₆alkyl, C₁₋₆alkylcarbonylaminoC₁₋₆alkyl, hydroxyC₁₋₆alkyl and piperazinylC₁₋₆alkyl, or
   oxopyrrolidinyl substituted once, twice or three times by substituents independently selected from (((C₁₋₆alkyl)₂aminoC₁₋₆alkyl)azetidinyl)C₁₋₆alkyl, ((aminoC₁₋₆alkyl)azetidinyl)C₁₋₆alkyl, ((aminoC₃₋₇cycloalkyl)azetidinyl)C₁₋₆alkyl, ((C₁₋₆alkylamino)azetidinyl)C₁₋₆alkyl, (aminoazetidinyl)C₁₋₆alkyl, (aminoC₁₋₆alkyl)azetidinyl, (azetidinylC₁₋₆alkylamino)C₁₋₆alkyl, (C₁₋₆alkylpiperazinyl)C₁₋₆alkyl, (hydroxyC₁₋₆alkyl)piperazinyl, 2,5-diazabicyclo[2.2.1]heptanyl, 2,6-diazaspiro[3.3]heptanyl, amino, amino(hydroxy)piperidinyl, amino(hydroxy)pyrrolidinyl, aminoazetidinyl, aminoC₁₋₆alkyl, (aminoC₁₋₆alkyl)amino, aminopyrrolidinyl, azetidinylamino, C₁₋₆alkyl, C₁₋₆alkylamino, dihydroxypyrrolidinyl, (hydroxyC₁₋₆alkyl)amino, piperazinyl, piperazinylC₁₋₆alkyl, piperidinylamino and pyrrolidinylamino;
R² is H or cyano;
R³ is benzyl,
   C₃₋₇cycloalkylsulfonyl,
   cyanoC₃₋₇cycloalkyl,
   phenoxy,
   phenylC₃₋₇cycloalkyl,
   tetrahydropyranylsulfonyl, or
   C₁₋₆alkyl substituted twice or three times by substituents independently selected from halogen, (aminoC₁₋₆alkyl)C₃₋₇cycloalkyl, (hydroxyC₁₋₆alkyl)C₃₋₇cycloalkyl, 1,2,3,6-tetrahydropyridinyl, 1,4-dioxanyl, 3,6-dihydro-2H-pyranyl, 3-azabicyclo[3.1.0]hexanyl, 3-oxabicyclo[3.1.0]hexanyl, 8-oxabicyclo[3.2.1]octanyl, azetidinyl, C₁₋₆alkylhalopyrazinyl, C₁₋₆alkylmorpholinyl, C₁₋₆alkylpyrazinyl, carbamoylC₃₋₇cycloalkyl, hydroxyC₃₋₇cycloalkyl, morpholinyl, oxetanyl, piperidinyl, pyrazinyl, pyridinyl, pyrimidinyl, tetrahydrofuranyl, tetrahydropyranyl, phenyl and pyridinyl;
R⁴ is halogen, C₁₋₆alkyl, C₃₋₇cycloalkyl, C₁₋₆alkoxy or C₂₋₆alkynyl;
Q¹ is N or CR^{a}, wherein R^{a} is H or halogen;
Q² is N or CR^{b}, wherein R^{b} is H or halogen;
Q³ is N or CH;
Q⁴ is N or CH;
Q⁵ is N or CH;
Y is O or NH;
or a pharmaceutically acceptable salt thereof.

### DETAILED DESCRIPTION OF THE INVENTION

### DEFINITIONS

The term "C₁₋₆alkyl" denotes a saturated, linear or branched chain alkyl group containing 1 to 6, particularly 1 to 4 carbon atoms, for example methyl, ethyl, *n*-propyl, isopropyl, *n*-butyl, isobutyl, *ter*t-butyl and the like. Particular "C₁₋₆alkyl" groups are methyl, ethyl and *n*-propyl.

The term "halogen" and "halo" are used interchangeably herein and denote fluoro, chloro, bromo, or iodo.

The term "halopyrazinyl" denotes a pyrazinyl group wherein at least one of the hydrogen atoms of the pyrazinyl group has been replaced by same or different halogen atoms, particularly fluoro atoms. Examples of halopiperidinyl include fluoropyrazinyl and difluoropyrazinyl.

The term "C₂₋₆alkynyl" denotes a monovalent linear or branched saturated hydrocarbon group of 2 to 6 carbon atoms comprising one, two or three triple bonds. In particular embodiments alkynyl has from 2 to 4 carbon atoms comprising one or two triple bonds. Examples of C₂₋₆alkynyl include ethynyl, propynyl, prop-2-ynyl, isopropynyl, and n-butynyl.

The term "haloC₁₋₆alkyl" denotes a C₁₋₆alkyl group wherein at least one of the hydrogen atoms of the C₁₋₆alkyl group has been replaced by same or different halogen atoms, particularly fluoro atoms. Examples of haloC₁₋₆alkyl include monofluoro-, difluoro- or trifluoro-methyl, - ethyl or -propyl, for example 3,3,3-trifluoropropyl, 2-fluoroethyl, trifluoroethyl, fluoromethyl, difluoromethyl, difluoroethyl or trifluoromethyl.

The term "C₃₋₇cycloalkyl" denotes a monovalent saturated monocyclic or bicyclic hydrocarbon group of 3 to 7 ring carbon atoms. Bicyclic means consisting of two saturated carbocycles having one or more carbon atoms in common. Examples for monocyclic cycloalkyl are cyclopropyl, cyclobutyl, cyclopentyl, cyclohexyl or cycloheptyl. Examples for bicyclic cycloalkyl are bicyclo[1.1.0]butyl, bicyclo[2.2.1]heptanyl, or bicyclo[2.2.2]octanyl.

The term "oxo" denotes a divalent oxygen atom =O.
The term "*cis*-isomers" and *"trans-*isomers*"* denote the relative stereochemistry of the molecule or moiety. For example: *tert-*butyl *cis*-1-[(2,6-dichloro-4-pyridyl)-difluoro-methyl]-3-azabicyclo[3.1.0]hexane-3-carboxylate (compound 116a) 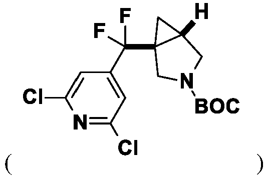 in Example 116 as the "cis-isomers" refers to a mixture of 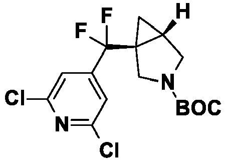 and 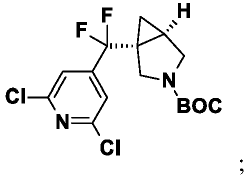 similarly, *trans*-ethyl 2-[(2,6-dichloro-4-pyridyl)-difluoromethyl]cyclopropanecarboxylate ( compound 117c) 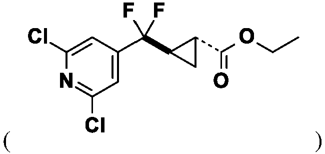 in Example 117 as the *"trans-*isomers*"* refers to a mixture of 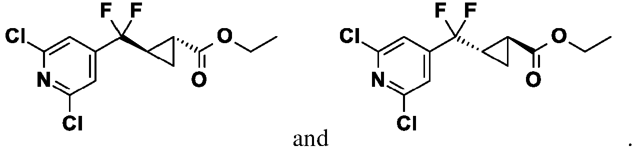 The way of showing relative stereochemistry also applies to the final compound.

The term "pharmaceutically acceptable salts" denotes salts which are not biologically or otherwise undesirable. Pharmaceutically acceptable salts include both acid and base addition salts.

The term "pharmaceutically acceptable acid addition salt" denotes those pharmaceutically acceptable salts formed with inorganic acids such as hydrochloric acid, hydrobromic acid, sulfuric acid, nitric acid, carbonic acid, phosphoric acid, and organic acids selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, maloneic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, *p*-toluenesulfonic acid, and salicyclic acid.

The term "pharmaceutically acceptable base addition salt" denotes those pharmaceutically acceptable salts formed with an organic or inorganic base. Examples of acceptable inorganic bases include sodium, potassium, ammonium, calcium, magnesium, iron, zinc, copper, manganese, and aluminum salts. Salts derived from pharmaceutically acceptable organic nontoxic bases includes salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, *N*-ethylpiperidine, and polyamine resins.

The term "A pharmaceutically active metabolite" denotes a pharmacologically active product produced through metabolism in the body of a specified compound or salt thereof. After entry into the body, most drugs are substrates for chemical reactions that may change their physical properties and biologic effects. These metabolic conversions, which usually affect the polarity of the compounds of the invention, alter the way in which drugs are distributed in and excreted from the body. However, in some cases, metabolism of a drug is required for therapeutic effect.

The term "therapeutically effective amount" denotes an amount of a compound or molecule of the present invention that, when administered to a subject, (i) treats or prevents the particular disease, condition or disorder, (ii) attenuates, ameliorates or eliminates one or more symptoms of the particular disease, condition, or disorder, or (iii) prevents or delays the onset of one or more symptoms of the particular disease, condition or disorder described herein. The therapeutically effective amount will vary depending on the compound, the disease state being treated, the severity of the disease treated, the age and relative health of the subject, the route and form of administration, the judgement of the attending medical or veterinary practitioner, and other factors.

The term "pharmaceutical composition" denotes a mixture or solution comprising a therapeutically effective amount of an active pharmaceutical ingredient together with pharmaceutically acceptable excipients to be administered to a mammal, e.g., a human in need thereof.

### INHIBITOR OF UDP-2,3-DIACYLGLUCOSAMINE HYDROLASE (LpxH)

The present invention relates to (i) a compound of formula (I), 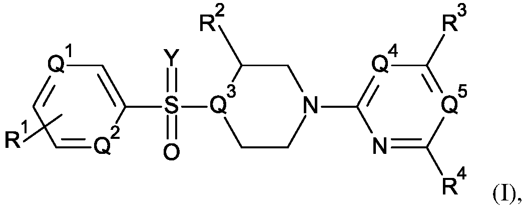 wherein
R¹ is 2-oxo-1-oxa-3,8-diazaspiro[4.5]decanyl unsubstituted or substituted by aminoC₁₋₆alkyl,
   2-oxo-3a,4,5,6,7,7a-hexahydrooxazolo[4.5-c]pyridinyl,
   2-oxo-3a,4,5,6,7,7a-hexahydrooxazolo[5.4-c]pyridinyl,
   2-oxo-3a,4,6,7,8,8a-hexahydrooxazolo[4.5-c]azepinyl substituted by aminoC₁₋₆alkyl or C₁₋₆alkylaminoC₁₋₆alkyl,
   2-oxo-3a,5,6,6a-tetrahydrofuro[2,3-d]oxazolyl substituted twice by substituents independently selected from hydroxy and hydroxyC₁₋₆alkyl,
   2-oxo-4,5,6,6a-tetrahydro-3aH-pyrrolo[3.4-d]oxazolyl,
   2-oxo-4,5,6,7,8,8a-hexahydro-3aH-oxazolo[4,5-c]azepinyl,
   2-oxo-5,6,7,7a-tetrahydro-3aH-pyrano[2,3-d]oxazolyl substituted three times by substituents independently selected from hydroxy and hydroxyC₁₋₆alkyl,
   3-oxo-2,6,9-triazaspiro[4.5]decanyl,
   3-oxo-2,6-diazaspiro[4.5]decanyl,
   3-oxo-2,8-diazaspiro[4.5]decanyl,
   5-oxo-1,2,3,3a,6,6a-hexahydropyrrolo[3,2-b]pyrrolyl,
   6-oxo-5-oxa-2,7-diazaspiro[3.4]octanyl,
   7-oxo-2,6-diazaspiro[3.4]octanyl,
   oxoazetidinyl substituted by amino,
   oxooxazolidinyl substituted once or twice by substituents independently selected from ((aminoC₁₋₆alkyl)azetidinyl)C₁₋₆alkyl, (C₁₋₆alkyl)₂aminoC₁₋₆alkyl, (dihydroxypyrrolidinyl)C₁₋₆alkyl, (pyrrolidinylamino)C₁₋₆alkyl, 2,6-diazaspiro[3.3]heptanylC₁₋₆alkyl, dihydroxy(C₁₋₆alkoxy)tetrahydrofuranyl, aminoazetidinylC₁₋₆alkyl, aminoC₁₋₆alkyl, C₁₋₆alkyl, C₁₋₆alkylaminoC₁₋₆alkyl, C₁₋₆alkylcarbonylaminoC₁₋₆alkyl, hydroxyC₁₋₆alkyl and piperazinylC₁₋₆alkyl, or
   oxopyrrolidinyl substituted once, twice or three times by substituents independently selected from (((C₁₋₆alkyl)₂aminoC₁₋₆alkyl)azetidinyl)C₁₋₆alkyl, ((aminoC₁₋₆alkyl)azetidinyl)C₁₋₆alkyl, ((aminoC₃₋₇cycloalkyl)azetidinyl)C₁₋₆alkyl, ((C₁₋₆alkylamino)azetidinyl)C₁₋₆alkyl, (aminoazetidinyl)C₁₋₆alkyl, (aminoC₁₋₆alkyl)azetidinyl, (azetidinylC₁₋₆alkylamino)C₁₋₆alkyl, (C₁₋₆alkylpiperazinyl)C₁₋₆alkyl, (hydroxyC₁₋₆alkyl)piperazinyl, 2,5-diazabicyclo[2.2.1]heptanyl, 2,6-diazaspiro[3.3]heptanyl, amino, amino(hydroxy)piperidinyl, amino(hydroxy)pyrrolidinyl, aminoazetidinyl, aminoC₁₋₆alkyl, (aminoC₁₋₆alkyl)amino, aminopyrrolidinyl, azetidinylamino, C₁₋₆alkyl, C₁₋₆alkylamino, dihydroxypyrrolidinyl, (hydroxyC₁₋₆alkyl)amino, piperazinyl, piperazinylC₁₋₆alkyl, piperidinylamino and pyrrolidinylamino;
R² is H or cyano;
R³ is benzyl,
   C₃₋₇cycloalkylsulfonyl,
   cyanoC₃₋₇cycloalkyl,
   phenoxy,
   phenylC₃₋₇cycloalkyl,
   tetrahydropyranylsulfonyl, or
   C₁₋₆alkyl substituted twice or three times by substituents independently selected from halogen, (aminoC₁₋₆alkyl)C₃₋₇cycloalkyl, (hydroxyC₁₋₆alkyl)C₃₋₇cycloalkyl, 1,2,3,6-tetrahydropyridinyl, 1,4-dioxanyl, 3,6-dihydro-2H-pyranyl, 3-azabicyclo[3.1.0]hexanyl, 3-oxabicyclo[3.1.0]hexanyl, 8-oxabicyclo[3.2.1]octanyl, azetidinyl, C₁₋₆alkylhalopyrazinyl, C₁₋₆alkylmorpholinyl, C₁₋₆alkylpyrazinyl, carbamoylC₃₋₇cycloalkyl, hydroxyC₃₋₇cycloalkyl, morpholinyl, oxetanyl, piperidinyl, pyrazinyl, pyridinyl, pyrimidinyl, tetrahydrofuranyl, tetrahydropyranyl, phenyl and pyridinyl;
R⁴ is halogen, C₁₋₆alkyl, C₃₋₇cycloalkyl, C₁₋₆alkoxy or C₂₋₆alkynyl;
Q¹ is N or CR^{a}, wherein R^{a} is H or halogen;
Q² is N or CR^{b}, wherein R^{b} is H or halogen;
Q³ is N or CH;
Q⁴ is N or CH;
Q⁵ is N or CH;
Y is O or NH;
or a pharmaceutically acceptable salt thereof.

Another embodiment of present invention is (ii) a compound of formula (Ia), 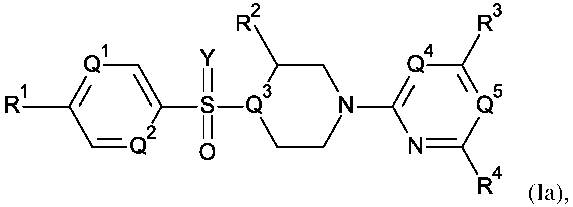 wherein
R¹ is 2-oxo-1-oxa-3,8-diazaspiro[4.5]decanyl unsubstituted or substituted by aminoC₁₋₆alkyl,
   2-oxo-3a,4,5,6,7,7a-hexahydrooxazolo[4.5-c]pyridinyl,
   2-oxo-3a,4,5,6,7,7a-hexahydrooxazolo[5.4-c]pyridinyl,
   2-oxo-3a,4,6,7,8,8a-hexahydrooxazolo[4.5-c]azepinyl substituted by aminoC₁₋₆alkyl or C₁₋₆alkylaminoC₁₋₆alkyl,
   2-oxo-3a,5,6,6a-tetrahydrofuro[2,3-d]oxazolyl substituted twice by substituents independently selected from hydroxy and hydroxyC₁₋₆alkyl,
   2-oxo-4,5,6,6a-tetrahydro-3aH-pyrrolo[3.4-d]oxazolyl,
   2-oxo-4,5,6,7,8,8a-hexahydro-3aH-oxazolo[4,5-c]azepinyl,
   2-oxo-5,6,7,7a-tetrahydro-3aH-pyrano[2,3-d]oxazolyl substituted three times by substituents independently selected from hydroxy and hydroxyC₁₋₆alkyl,
   3-oxo-2,6,9-triazaspiro[4.5]decanyl,
   3-oxo-2,6-diazaspiro[4.5]decanyl,
   3-oxo-2,8-diazaspiro[4.5]decanyl,
   5-oxo-1,2,3,3a,6,6a-hexahydropyrrolo[3,2-b]pyrrolyl,
   6-oxo-5-oxa-2,7-diazaspiro[3.4]octanyl,
   7-oxo-2,6-diazaspiro[3.4]octanyl,
   oxoazetidinyl substituted by amino,
   oxooxazolidinyl substituted once or twice by substituents independently selected from ((aminoC₁₋₆alkyl)azetidinyl)C₁₋₆alkyl, (C₁₋₆alkyl)₂aminoC₁₋₆alkyl, (dihydroxypyrrolidinyl)C₁₋₆alkyl, (pyrrolidinylamino)C₁₋₆alkyl, 2,6-diazaspiro[3.3]heptanylC₁₋₆alkyl, dihydroxy(C₁₋₆alkoxy)tetrahydrofuranyl, aminoazetidinylC₁₋₆alkyl, aminoC₁₋₆alkyl, C₁₋₆alkyl, C₁₋₆alkylaminoC₁₋₆alkyl, C₁₋₆alkylcarbonylaminoC₁₋₆alkyl, hydroxyC₁₋₆alkyl and piperazinylC₁₋₆alkyl, or
   oxopyrrolidinyl substituted once, twice or three times by substituents independently selected from (((C₁₋₆alkyl)₂aminoC₁₋₆alkyl)azetidinyl)C₁₋₆alkyl, ((aminoC₁₋₆alkyl)azetidinyl)C₁₋₆alkyl, ((aminoC₃₋₇cycloalkyl)azetidinyl)C₁₋₆alkyl, ((C₁₋₆alkylamino)azetidinyl)C₁₋₆alkyl, (aminoazetidinyl)C₁₋₆alkyl, (aminoC₁₋₆alkyl)azetidinyl, (azetidinylC₁₋₆alkylamino)C₁₋₆alkyl, (C₁₋₆alkylpiperazinyl)C₁₋₆alkyl, (hydroxyC₁₋₆alkyl)piperazinyl, 2,5-diazabicyclo[2.2.1]heptanyl, 2,6-diazaspiro[3.3]heptanyl, amino, amino(hydroxy)piperidinyl, amino(hydroxy)pyrrolidinyl, aminoazetidinyl, aminoC₁₋₆alkyl, (aminoC₁₋₆alkyl)amino, aminopyrrolidinyl, azetidinylamino, C₁₋₆alkyl, C₁₋₆alkylamino, dihydroxypyrrolidinyl, (hydroxyC₁₋₆alkyl)amino, piperazinyl, piperazinylC₁₋₆alkyl, piperidinylamino and pyrrolidinylamino;
R² is H or cyano;
R³ is benzyl,
   C₃₋₇cycloalkylsulfonyl,
   cyanoC₃₋₇cycloalkyl,
   phenoxy,
   phenylC₃₋₇cycloalkyl,
   tetrahydropyranylsulfonyl, or
   C₁₋₆alkyl substituted twice or three times by substituents independently selected from halogen, (aminoC₁₋₆alkyl)C₃₋₇cycloalkyl, (hydroxyC₁₋₆alkyl)C₃₋₇cycloalkyl, 1,2,3,6-tetrahydropyridinyl, 1,4-dioxanyl, 3,6-dihydro-2H-pyranyl, 3-azabicyclo[3.1.0]hexanyl, 3-oxabicyclo[3.1.0]hexanyl, 8-oxabicyclo[3.2.1]octanyl, azetidinyl, C₁₋₆alkylhalopyrazinyl, C₁₋₆alkylmorpholinyl, C₁₋₆alkylpyrazinyl, carbamoylC₃₋₇cycloalkyl, hydroxyC₃₋₇cycloalkyl, morpholinyl, oxetanyl, piperidinyl, pyrazinyl, pyridinyl, pyrimidinyl, tetrahydrofuranyl, tetrahydropyranyl, phenyl and pyridinyl;
R⁴ is halogen, C₁₋₆alkyl, C₃₋₇cycloalkyl, C₁₋₆alkoxy or C₂₋₆alkynyl;
Q¹ is N or CR^{a}, wherein R^{a} is H or halogen;
Q² is CR^{b}, wherein R^{b} is H or halogen;
Q³ is N;
Q⁴ is N or CH;
Q⁵ is N or CH;
Y is O;
or a pharmaceutically acceptable salt thereof.

A further embodiment of present invention is (iii) a compound of formula (I) or (Ia) according to (i) or (ii), or a pharmaceutically acceptable salt thereof, wherein
R¹ is 2-oxo-1-oxa-3,8-diazaspiro[4.5]decanyl,
   2-oxo-3a,4,5,6,7,7a-hexahydrooxazolo[4.5-c]pyridinyl,
   2-oxo-3a,4,5,6,7,7a-hexahydrooxazolo[5.4-c]pyridinyl,
   2-oxo-3a,5,6,6a-tetrahydrofuro[2,3-d]oxazolyl substituted twice by substituents independently selected from hydroxy and hydroxyC₁₋₆alkyl,
   2-oxo-4,5,6,6a-tetrahydro-3aH-pyrrolo[3.4-d]oxazolyl,
   2-oxo-4,5,6,7,8,8a-hexahydro-3aH-oxazolo[4,5-c]azepinyl,
   3-oxo-2,6-diazaspiro[4.5]decanyl,
   5-oxo-1,2,3,3a,6,6a-hexahydropyrrolo[3,2-b]pyrrolyl,
   6-oxo-5-oxa-2,7-diazaspiro[3.4]octanyl,
   oxooxazolidinyl substituted once or twice by substituents independently selected from ((aminoC₁₋₆alkyl)azetidinyl)C₁₋₆alkyl, (C₁₋₆alkyl)₂aminoC₁₋₆alkyl, dihydroxy(C₁₋₆alkoxy)tetrahydrofuranyl, aminoC₁₋₆alkyl, C₁₋₆alkyl, C₁₋₆alkylaminoC₁₋₆alkyl, hydroxyC₁₋₆alkyl and piperazinylC₁₋₆alkyl, or
   oxopyrrolidinyl substituted by substituent selected from ((aminoC₁₋₆alkyl)azetidinyl)C₁₋₆alkyl, ((aminoC₃₋₇cycloalkyl)azetidinyl)C₁₋₆alkyl, (aminoazetidinyl)C₁₋₆alkyl, (C₁₋₆alkylpiperazinyl)C₁₋₆alkyl, (hydroxyC₁₋₆alkyl)piperazinyl, amino, amino(hydroxy)piperidinyl, amino(hydroxy)pyrrolidinyl, aminoC₁₋₆alkyl, aminopyrrolidinyl, C₁₋₆alkylamino, dihydroxypyrrolidinyl, (hydroxyC₁₋₆alkyl)amino, piperazinyl, piperazinylC₁₋₆alkyl and piperidinylamino.

A further embodiment of present invention is (iv) a compound of formula (I) or (Ia), according to any one of (i) to (iii), or a pharmaceutically acceptable salt thereof, wherein
R¹ is 2-oxo-1-oxa-3,8-diazaspiro[4.5]decanyl,
   2-oxo-3a,4,5,6,7,7a-hexahydrooxazolo[4.5-c]pyridinyl,
   2-oxo-3a,4,5,6,7,7a-hexahydrooxazolo[5.4-c]pyridinyl,
   2-oxo-3a,5,6,6a-tetrahydrofuro[2,3-d]oxazolyl substituted twice by substituents independently selected from hydroxy and hydroxymethyl,
   2-oxo-4,5,6,6a-tetrahydro-3aH-pyrrolo[3.4-d]oxazolyl,
   2-oxo-4,5,6,7,8,8a-hexahydro-3aH-oxazolo[4,5-c]azepinyl,
   3-oxo-2,6-diazaspiro[4.5]decanyl,
   5-oxo-1,2,3,3a,6,6a-hexahydropyrrolo[3,2-b]pyrrolyl,
   6-oxo-5-oxa-2,7-diazaspiro[3.4]octanyl,
   oxooxazolidinyl substituted once or twice by substituents independently selected from (3-(aminomethyl)azetidin-1-yl)methyl, (dimethylamino)methyl, 3,4-dihydroxy-5-methoxy-tetrahydrofuran-2-yl, aminomethyl, aminomethyl, hydroxymethyl, methyl, (methylamino)methyl and piperazin-1-ylmethyl, or
   oxopyrrolidinyl substituted by substituent selected from (3-(1-aminocyclopropyl)azetidin-1-yl)methyl, (3-(aminomethyl)azetidin-1-yl)methyl, (3-aminoazetidin-1-yl)methyl, (4-methylpiperazin-1-yl)methyl, 3-(hydroxymethyl)piperazin-1-yl, 3,4-dihydroxypyrrolidin-1-yl, 3-amino-4-hydroxypiperidin-1-yl, 3-amino-4-hydroxy-pyrrolidin-1-yl, 3-aminopyrrolidin-1-yl, 4-piperidinylamino, amino, aminomethyl, (hydroxyethyl)amino, methylamino, piperazin-1-yl and piperazin-1-ylmethyl.

A further embodiment of present invention is (v) a compound of formula (I) or (Ia) according to any one of (i) to (iv), wherein R¹ is 2-oxo-1-oxa-3,8-diazaspiro[4.5]decan-3-yl, 2-oxo-3a,4,5,6,7,7a-hexahydrooxazolo[4,5-c]pyridin-3-yl, 2-oxo-3a,4,5,6,7,7a-hexahydrooxazolo[5,4-c]pyridin-1-yl, 2-oxo-4,5,6,6a-tetrahydro-3aH-pyrrolo[3,4-d]oxazol-3-yl, 2-oxo-4,5,6,7,8,8a-hexahydro-3aH-oxazolo[4,5-c]azepin-3-yl, 3-oxo-2,6-diazaspiro[4.5]decan-2-yl, 4-((3-(1-aminocyclopropyl)azetidin-1-yl)methyl)-2-oxo-pyrrolidin-1-yl, 4-((3-aminoazetidin-1-yl)methyl)-2-oxo-pyrrolidin-1-yl, 4-((4-methylpiperazin-1-yl)methyl)-2-oxo-pyrrolidin-1-yl, 4-(2-hydroxyethylamino)-2-oxo-pyrrolidin-1-yl, 4-(3-(aminomethyl)azetidin-1-yl)methyl)-2-oxo-pyrrolidin-1-yl, 4-(3-(hydroxymethyl)piperazin-1-yl)-2-oxo-pyrrolidin-1-yl, 4-(3,4-dihydroxypyrrolidin-1-yl)-2-oxo-pyrrolidin-1-yl, 4-(3-amino-4-hydroxypiperidin-1-yl)-2-oxopyrrolidin-1-yl, 4-(3-amino-4-hydroxy-pyrrolidin-1-yl)-2-oxo-pyrrolidin-1-yl, 4-(3-aminopyrrolidin-1-yl)-2-oxo-pyrrolidin-1-yl, 4-(4-piperidinylamino)-2-oxo-pyrrolidin-1-yl, 4-(methylamino)-2-oxo-pyrrolidin-1-yl, 4-(piperazin-1-yl)-2-oxo-pyrrolidin-1-yl, 4-(piperazin-1-ylmethyl)-2-oxo-pyrrolidin-1-yl, 4-amino-2-oxo-pyrrolidin-1-yl, 4-aminomethyl-2-oxo-oxazolidin-3-yl, 4-aminomethyl-2-oxo-pyrrolidin-1-yl, 4-methyl-2-oxo-5-(3,4-dihydroxy-5-methoxy-tetrahydrofuran-2-yl)oxazolidin-3-yl, 5-((3-(aminomethyl)azetidin-1-yl)methyl)-2-oxo-oxazolidin-3-yl, 5-((dimethylamino)methyl)-2-oxo-oxazolidin-3-yl, 5-(2-aminoethyl)-2-oxo-oxazolidin-3-yl, 5-(methylaminomethyl)-2-oxo-oxazolidin-3-yl, 5-(piperazin-1-ylmethyl)-2-oxo-oxazolidin-3-yl, 5-aminomethyl-2-oxo-oxazolidin-3-yl, 5-hydroxymethyl-2-oxo-oxazolidin-3-yl, 5-oxo-1,2,3,3a,6,6a-hexahydropyrrolo[3,2-b]pyrrol-4-yl, 6-hydroxy-5-hydroxymethyl-2-oxo-3a,5,6,6a-tetrahydrofuro[2,3-d]oxazol-3-yl or 6-oxo-5-oxa-2,7-diazaspiro[3.4]octan-7-yl.

A further embodiment of present invention is (vi) a compound of formula (I) or (Ia), or a pharmaceutically acceptable salt thereof, according to any one of (i) to (v), wherein R¹ is oxopyrrolidinyl substituted by substituent selected from ((aminoC₁₋₆alkyl)azetidinyl)C₁₋₆alkyl, ((aminoC₃₋₇cycloalkyl)azetidinyl)C₁₋₆alkyl, (aminoazetidinyl)C₁₋₆alkyl, (C₁₋₆alkylpiperazinyl)C₁₋₆alkyl, (hydroxyC₁₋₆alkyl)piperazinyl, amino, amino(hydroxy)piperidinyl, amino(hydroxy)pyrrolidinyl, aminoC₁₋₆alkyl, aminopyrrolidinyl, C₁₋₆alkylamino, dihydroxypyrrolidinyl, (hydroxyC₁₋₆alkyl)amino, piperazinyl, piperazinylC₁₋₆alkyl and piperidinylamino.

A further embodiment of present invention is (vii) a compound of formula (I) or (Ia), or a pharmaceutically acceptable salt thereof, according to any one of (i) to (vi), wherein R¹ is 4-((3-(1-aminocyclopropyl)azetidin-1-yl)methyl)-2-oxo-pyrrolidin-1-yl, 4-((3-aminoazetidin-1-yl)methyl)-2-oxo-pyrrolidin-1-yl, 4-((4-methylpiperazin-1-yl)methyl)-2-oxo-pyrrolidin-1-yl, 4-(2-hydroxyethylamino)-2-oxo-pyrrolidin-1-yl, 4-(3-(aminomethyl)azetidin-1-yl)methyl)-2-oxopyrrolidin-1-yl, 4-(3-(hydroxymethyl)piperazin-1-yl)-2-oxo-pyrrolidin-1-yl, 4-(3,4-dihydroxypyrrolidin-1-yl)-2-oxo-pyrrolidin-1-yl, 4-(3-amino-4-hydroxypiperidin-1-yl)-2-oxopyrrolidin-1-yl, 4-(3-amino-4-hydroxy-pyrrolidin-1-yl)-2-oxo-pyrrolidin-1-yl, 4-(3-aminopyrrolidin-1-yl)-2-oxo-pyrrolidin-1-yl, 4-(4-piperidinylamino)-2-oxo-pyrrolidin-1-yl, 4-(methylamino)-2-oxo-pyrrolidin-1-yl, 4-(piperazin-1-yl)-2-oxo-pyrrolidin-1-yl, 4-(piperazin-1-ylmethyl)-2-oxo-pyrrolidin-1-yl, 4-amino-2-oxo-pyrrolidin-1-yl or 4-aminomethyl-2-oxopyrrolidin-1-yl.

A further embodiment of present invention is (viii) a compound of formula (I) or (Ia), or a pharmaceutically acceptable salt thereof, according to any one of (i) to (vii), wherein R³ is benzyl, phenylC₃₋₇cycloalkyl, or C₁₋₆alkyl substituted three times by substituents independently selected from halogen, (hydroxyC₁₋₆alkyl)C₃₋₇cycloalkyl, 1,2,3,6-tetrahydropyridinyl, 1,4-dioxanyl, 3,6-dihydro-2H-pyranyl, 3-azabicyclo[3.1.0]hexanyl, 3-oxabicyclo[3.1.0]hexanyl, 8-oxabicyclo[3.2.1]octanyl, C₁₋₆alkylhalopyrazinyl, C₁₋₆alkylpyrazinyl, carbamoylC₃₋₇cycloalkyl, hydroxyC₃₋₇cycloalkyl, morpholinyl, oxetanyl, pyrazinyl, pyridinyl, tetrahydropyranyl and phenyl.

A further embodiment of present invention is (ix) a compound of formula (I) or (Ia), or a pharmaceutically acceptable salt thereof, according to any one of (i) to (viii), wherein R³ is C₁₋₆alkyl substituted three times by substituents independently selected from halogen, (hydroxyC₁₋₆alkyl)C₃₋₇cycloalkyl, 1,4-dioxanyl, 3-azabicyclo[3.1.0]hexanyl, 3-oxabicyclo[3.1.0]hexanyl, C₁₋₆alkylhalopyrazinyl, C₁₋₆alkylpyrazinyl, hydroxyC₃₋₇cycloalkyl, morpholinyl, oxetanyl, phenyl, pyridinyl and tetrahydropyranyl.

A further embodiment of present invention is (x) a compound of formula (I) or (Ia), or a pharmaceutically acceptable salt thereof, according to any one of (i) to (ix), wherein R³ is difluoro(1,4-dioxan-2-yl)methyl, difluoro(2-(hydroxymethyl)cyclopropyl)methyl, difluoro(2-pyridinyl)methyl, difluoro(3-hydroxycyclobutyl)methyl, difluoro(3-oxabicyclo[3.1.0]hexan-6-yl)methyl, difluoro(5-chloro-6-methyl-pyrazin-2-yl)methyl, difluoro(6-methylpyrazin-2-yl)methyl, difluoro(morpholin-2-yl)methyl, difluoro(oxetan-3-yl)methyl, difluoro(phenyl)methyl, difluoro(tetrahydropyran-4-yl)methyl or difluoro[3-azabicyclo[3.1.0]hexan-1-yl]methyl.

A further embodiment of present invention is (xi) a compound of formula (I) or (Ia), or a pharmaceutically acceptable salt thereof, according to any one of (i) to (x), wherein R² is H.

A further embodiment of present invention is (xii) a compound of formula (I) or (Ia), or a pharmaceutically acceptable salt thereof, according to any one of (i) to (xi), wherein R⁴ is halogen or C₁₋₆alkyl.

A further embodiment of present invention is (xiii) a compound of formula (I) or (Ia), or a pharmaceutically acceptable salt thereof, according to any one of (i) to (xii), wherein R⁴ is chloro or methyl.

A further embodiment of present invention is (xiv) a compound of formula (I) or (Ia), or a pharmaceutically acceptable salt thereof, according to any one of (i) to (xiii), wherein Q¹ is CR^{a}, wherein R^{a} is H or halogen.

A further embodiment of present invention is (xv) a compound of formula (I) or (Ia), or a pharmaceutically acceptable salt thereof, according to any one of (i) to (xiv), wherein Q¹ is CR^{a}, wherein R^{a} is H or fluoro.

A further embodiment of present invention is (xvi) a compound of formula (I) or (Ia), or a pharmaceutically acceptable salt thereof, according to any one of (i) to (xv), wherein Q² is CH.

A further embodiment of present invention is (xvii) a compound of formula (I) or (Ia) according to any one of (i) to (xvi), wherein
R¹ is oxopyrrolidinyl substituted by amino or aminoC₁₋₆alkyl;
R² is H;
R³ is C₁₋₆alkyl substituted three times by substituents independently selected from halogen, (hydroxyC₁₋₆alkyl)C₃₋₇cycloalkyl, 1,4-dioxanyl, 3-azabicyclo[3.1.0]hexanyl, 3-oxabicyclo[3.1.0]hexanyl, C₁₋₆alkylhalopyrazinyl, C₁₋₆alkylpyrazinyl, hydroxyC₃₋₇cycloalkyl, morpholinyl, oxetanyl, phenyl, pyridinyl and tetrahydropyranyl;
R⁴ is halogen or C₁₋₆alkyl;
Q¹ is CR^{a}, wherein R^{a} is H or halogen;
Q² is CH;
Q³ is N;
Q⁴ is N or CH;
Q⁵ is N or CH;
Y is O;
   or a pharmaceutically acceptable salt thereof.

A further embodiment of present invention is (xviii) a compound of formula (I) or (Ia) according to any one of (i) to (xvii), wherein
R¹ is oxopyrrolidinyl substituted by amino or aminomethyl;
R² is H;
R³ is difluoro(1,4-dioxan-2-yl)methyl, difluoro(2-(hydroxymethyl)cyclopropyl)methyl, difluoro(2-pyridinyl)methyl, difluoro(3-hydroxycyclobutyl)methyl, difluoro(3-oxabicyclo[3.1.0]hexan-6-yl)methyl, difluoro(5-chloro-6-methyl-pyrazin-2-yl)methyl, difluoro(6-methylpyrazin-2-yl)methyl, difluoro(morpholin-2-yl)methyl, difluoro(oxetan-3-yl)methyl, difluoro(phenyl)methyl, difluoro(tetrahydropyran-4-yl)methyl or difluoro[3-azabicyclo[3.1.0]hexan-1-yl]methyl;
R⁴ is chloro or methyl;
Q¹ is CR^{a}, wherein R^{a} is H or fluoro;
Q² is CH;
Q³ is N;
Q⁴ is N or CH;
Q⁵ is N or CH;
Y is O;
or a pharmaceutically acceptable salt thereof.

Another embodiment of present invention is a compound of formula (I) or (Ia) selected from the following:
4-Amino-1-[4-[4-[6-chloro-4-(trifluoromethyl)-2-pyridyl]piperazin-1-yl]sulfonylphenyl] pyrrolidin-2-one;
4-(Aminomethyl)-1-[4-[4-[6-chloro-4-(trifluoromethyl)-2-pyridyl]piperazin-1-yl]sulfonylphenyl]pyrrolidin-2-one;
5-(Aminomethyl)-3-[4-[4-[6-chloro-4-(trifluoromethyl)-2-pyridyl]piperazin-1-yl]sulfonylphenyl]oxazolidin-2-one;
*N*-[[3-[4-[4-[6-chloro-4-(trifluoromethyl)-2-pyridyl]piperazin-1-yl]sulfonylphenyl]-2-oxo-oxazolidin-5-yl]methyl]acetamide;
4-Amino-1-[4-[4-[6-chloro-4-(trifluoromethyl)-2-pyridyl]piperazin-1-yl]sulfonylphenyl]-3,3-dimethyl-pyrrolidin-2-one;
4-(Aminomethyl)-3-[4-[4-[6-chloro-4-(trifluoromethyl)-2-pyridyl]piperazin-1-yl]sulfonylphenyl]oxazolidin-2-one;
(3*S*)-3-amino-1-[4-[4-[6-chloro-4-(trifluoromethyl)-2-pyridyl]piperazin-1-yl]sulfonylphenyl] azetidin-2-one;
3-Amino-1-[4-[4-[6-chloro-4-(trifluoromethyl)-2-pyridyl]piperazin-1-yl]sulfonylphenyl] pyrrolidin-2-one;
5-(2-Aminoethyl)-3-[4-[4-[6-chloro-4-(trifluoromethyl)-2-pyridyl]piperazin-1-yl]sulfonylphenyl]oxazolidin-2-one;
2-[4-[4-[6-Chloro-4-(trifluoromethyl)-2-pyridyl]piperazin-1-yl]sulfonylphenyl]-2,6-diazaspiro[4.5]decan-3-one;
2-[4-[4-[6-Chloro-4-(trifluoromethyl)-2-pyridyl]piperazin-1-yl]sulfonylphenyl]-2,8-diazaspiro[4.5]decan-3-one;
6-[4-[4-[6-Chloro-4-(trifluoromethyl)-2-pyridyl]piperazin-1-yl]sulfonylphenyl]-2,6-diazaspiro[3.4]octan-7-one;
7-[4-[4-[6-Chloro-4-(trifluoromethyl)-2-pyridyl]piperazin-1-yl]sulfonylphenyl]-5-oxa-2,7-diazaspiro[3.4]octan-6-one;
3-[4-[4-[6-Chloro-4-(trifluoromethyl)-2-pyridyl]piperazin-1-yl]sulfonylphenyl]-1-oxa-3,8-diazaspiro[4.5]decan-2-one;
(3a*R*,6a*S*)-3-[4-[4-[6-chloro-4-(trifluoromethyl)-2-pyridyl]piperazin-1-yl]sulfonylphenyl]-4,5,6,6a-tetrahydro-3a*H*-pyrrolo[3,4-d]oxazol-2-one;
(3a*R*,7a*S*)-3-[4-[4-[6-chloro-4-(trifluoromethyl)-2-pyridyl]piperazin-1-yl]sulfonylphenyl]-3a,4,5,6,7,7a-hexahydrooxazolo[4,5-c]pyridin-2-one;
(3a*R*,7a*S*)-1-[4-[4-[6-chloro-4-(trifluoromethyl)-2-pyridyl]piperazin-1-yl]sulfonylphenyl]-3a,4,5,6,7,7a-hexahydrooxazolo[5,4-c]pyridin-2-one;
(3a*S*,7a*R*)-3-[4-[4-[6-chloro-4-(trifluoromethyl)-2-pyridyl]piperazin-1-yl]sulfonylphenyl]-3a,4,5,6,7,7a-hexahydrooxazolo[4,5-c]pyridin-2-one;
(3a*S*,8a*R*)-3-[4-[4-[6-chloro-4-(trifluoromethyl)-2-pyridyl]piperazin-1-yl]sulfonylphenyl]-4,5,6,7, 8,8a-hexahydro-3 aH-oxazolo [4,5-c] azepin-2-one;
4-[4-[4-[6-Chloro-4-(trifluoromethyl)-2-pyridyl]piperazin-1-yl]sulfonylphenyl]-1,2,3,3a,6,6a-hexahydropyrrolo[3,2-b]pyrrol-5-one;
3-[4-[4-[6-Chloro-4-(trifluoromethyl)-2-pyridyl]piperazin-1-yl]sulfonylphenyl]-6-hydroxy-5-(hydroxymethyl)-3a,5,6,6a-tetrahydrofuro[2,3-d]oxazol-2-one;
3-[4-[4-[6-Chloro-4-(trifluoromethyl)-2-pyridyl] piperazin-1-yl]sulfonylphenyl]-5-[(3*S*,4*R*,5*R*)-3,4-dihydroxy-5-methoxy-tetrahydrofuran-2-yl]-4-methyl-oxazolidin-2-one;
(3a*S*,5*S*,6*S*,7*S*,7a*R*)-3-[4-[4-[6-chloro-4-(trifluoromethyl)-2-pyridyl]piperazin-l-yl]sulfonyl phenyl]-5,7-dihydroxy-6-(hydroxymethyl)-5,6,7,7a-tetrahydro-3aH-pyrano[2,3-d]oxazol-2-one;
8-(2-Aminoethyl)-3-[4-[4-[6-chloro-4-(trifluoromethyl)-2-pyridyl]piperazin-1-yl]sulfonylphenyl]-1-oxa-3,8-diazaspiro[4.5]decan-2-one;
(3a*S*,8a*R*)-5-(2-aminoethyl)-3-[4-[4-[6-chloro-4-(trifluoromethyl)-2-pyridyl]piperazin-l-yl] sulfonylphenyl] -3a,4,6,7,8,8a-hexahydrooxazolo[4,5-c] azepin-2-one;
(3a*S*,8a*R*)-3-[4-[4-[6-chloro-4-(trifluoromethyl)-2-pyridyl]piperazin-1-yl]sulfonylphenyl]-5-[2-(methylamino)ethyl]-3a,4,6,7,8,8a-hexahydrooxazolo[4,5-c]azepin-2-one;
3-[4-[4-[6-Chloro-4-(trifluoromethyl)-2-pyridyl]piperazin-1-yl] sulfonylphenyl]-5-(methylaminomethyl)oxazolidin-2-one;
3-[4-[4-[6-Chloro-4-(trifluoromethyl)-2-pyridyl] piperazin-1-yl]sulfonylphenyl]-5-[(dimethylamino)methyl]oxazolidin-2-one;
3-[4-[4-[6-Chloro-4-(trifluoromethyl)-2-pyridyl] piperazin-1-yl]sulfonylphenyl]-5-(piperazin-1-ylmethyl)oxazolidin-2-one;
3-[4-[4-[6-Chloro-4-(trifluoromethyl)-2-pyridyl]piperazin-1-yl]sulfonylphenyl]-5-[[cis-3,4-dihydroxypyrrolidin-1-yl]methyl]oxazolidin-2-one;
5-[(3-Aminoazetidin-1-yl)methyl]-3-[4-[4-[6-chloro-4-(trifluoromethyl)-2-pyridyl]piperazin-1-yl]sulfonylphenyl]oxazolidin-2-one;
3-[4-[4-[6-Chloro-4-(trifluoromethyl)-2-pyridyl]piperazin-1-yl]sulfonylphenyl]-5-(2,6-diazaspiro[3.3]heptan-2-ylmethyl)oxazolidin-2-one;
5-[[3-(Aminomethyl)azetidin-1-yl]methyl]-3-[4-[4-[6-chloro-4-(trifluoromethyl)-2-pyridyl]piperazin-1-yl]sulfonylphenyl]oxazolidin-2-one;
3-[4-[4-[6-Chloro-4-(trifluoromethyl)-2-pyridyl]piperazin-1-yl]sulfonylphenyl]-5-[(pyrrolidin-3-ylamino)methyl]oxazolidin-2-one;
4-[[3-(Aminomethyl)azetidin-1-yl]methyl]-1-[4-[4-[6-chloro-4-(trifluoromethyl)-2-pyridyl]piperazin-1-yl] sulfonylphenyl]pyrrolidin-2-one;
1-[4-[4-[6-Chloro-4-(trifluoromethyl)-2-pyridyl] piperazin-1-yl]sulfonylphenyl]-4-[(4-methylpiperazin-1-yl)methyl]pyrrolidin-2-one;
1-[4-[4-[6-Chloro-4-(trifluoromethyl)-2-pyridyl]piperazin-1-yl]sulfonylphenyl]-4-[[3-[(dimethylamino)methyl]azetidin-1-yl]methyl]pyrrolidin-2-one;
4-[(Azetidin-3-ylmethylamino)methyl]-1-[4-[4-[6-chloro-4-(trifluoromethyl)-2-pyridyl]piperazin-1-yl] sulfonylphenyl]pyrrolidin-2-one;
4-[[3-(1-Aminocyclopropyl)azetidin-1-yl]methyl]-1-[4-[4-[6-chloro-4-(trifluoromethyl)-2-pyridyl]piperazin-1-yl] sulfonylphenyl]pyrrolidin-2-one;
4-[(3-Aminoazetidin-1-yl)methyl]-1-[4-[4-[6-chloro-4-(trifluoromethyl)-2-pyridyl] piperazin-1-yl]sulfonylphenyl]pyrrolidin-2-one;
1-[4-[4-[6-Chloro-4-(trifluoromethyl)-2-pyridyl] piperazin-1-yl]sulfonylphenyl]-4-(piperazin-1-ylmethyl)pyrrolidin-2-one;
1-[4-[4-[6-Chloro-4-(trifluoromethyl)-2-pyridyl]piperazin-1-yl]sulfonylphenyl]-4-[[3-(dimethylamino)azetidin-1-yl]methyl]pyrrolidin-2-one;
1-[4-[4-[6-Chloro-4-(trifluoromethyl)-2-pyridyl] piperazin-1-yl]sulfonylphenyl]-4-[[3-(methylamino)azetidin-1-yl]methyl]pyrrolidin-2-one;
1-[4-[4-[6-chloro-4-(trifluoromethyl)-2-pyridyl]piperazin-1-yl]sulfonylphenyl]-4-(methylamino)pyrrolidin-2-one;
4-(2-Aminoethylamino)-1-[4-[4-[6-chloro-4-(trifluoromethyl)-2-pyridyl]piperazin-1-yl] sulfonylphenyl]pyrrolidin-2-one;
1-[4-[4-[6-Chloro-4-(trifluoromethyl)-2-pyridyl] piperazin-1-yl]sulfonylphenyl]-4-(2-hydroxyethylamino)pyrrolidin-2-one;
1-[4-[4-[6-Chloro-4-(trifluoromethyl)-2-pyridyl]piperazin-1-yl]sulfonylphenyl]-4-piperazin-1-yl-pyrrolidin-2-one;
1-[4-[4-[6-Chloro-4-(trifluoromethyl)-2-pyridyl] piperazin-1-yl]sulfonylphenyl]-4-[(**3S,4R**)-3,4-dihydroxypyrrolidin-1-yl]pyrrolidin-2-one;
4-[3-(Aminomethyl)azetidin-1-yl]-1-[4-[4-[6-chloro-4-(trifluoromethyl)-2-pyridyl]piperazin-1-yl] sulfonylphenyl]pyrrolidin-2-one;
4-(3-Aminopyrrolidin-1-yl)-1-[4-[4-[6-chloro-4-(trifluoromethyl)-2-pyridyl]piperazin-1-yl]sulfonylphenyl]pyrrolidin-2-one;
4-(3-Aminoazetidin-1-yl)-1-[4-[4-[6-chloro-4-(trifluoromethyl)-2-pyridyl]piperazin-1-yl]sulfonylphenyl]pyrrolidin-2-one;
1-[4-[4-[6-Chloro-4-(trifluoromethyl)-2-pyridyl] piperazin-1-yl]sulfonylphenyl]-4-(2,6-diazaspiro[3.3]heptan-2-yl)pyrrolidin-2-one;
1-[4-[4-[6-Chloro-4-(trifluoromethyl)-2-pyridyl]piperazin-1-yl]sulfonylphenyl]-4-[(3*S*)-3-(hydroxymethyl)piperazin-1-yl]pyrrolidin-2-one;
1-[4-[4-[6-Chloro-4-(trifluoromethyl)-2-pyridyl]piperazin-1-yl]sulfonylphenyl]-4-[(3*R*)-3-(hydroxymethyl)piperazin-1-yl]pyrrolidin-2-one;
1-[4-[4-[6-Chloro-4-(trifluoromethyl)-2-pyridyl]piperazin-1-yl]sulfonylphenyl]-4-[(1S,4S)-2,5-diazabicyclo[2.2.1]heptan-2-yl]pyrrolidin-2-one;
4-[(3*R*,4*R*)-3-amino-4-hydroxy-pyrrolidin-1-yl]-1-[4-[4-[6-chloro-4-(trifluoromethyl)-2-pyridyl]piperazin-1-yl]sulfonylphenyl]pyrrolidin-2-one;
4-((3*R*,4*R*)-3-amino-4-hydroxypiperidin-1-yl)-1-(4-((4-(6-chloro-4-(trifluoromethyl)pyridin-2-yl)piperazin-1-yl)sulfonyl)phenyl)pyrrolidin-2-one;
4-(Azetidin-3-ylamino)-1-[4-[4-[6-chloro-4-(trifluoromethyl)-2-pyridyl]piperazin-1-yl]sulfonylphenyl]pyrrolidin-2-one;
1-[4-[4-[6-Chloro-4-(trifluoromethyl)-2-pyridyl] piperazin-1-yl]sulfonylphenyl]-4-(pyrrolidin-3-ylamino)pyrrolidin-2-one;
1-[4-[4-[6-Chloro-4-(trifluoromethyl)-2-pyridyl] piperazin-1-yl]sulfonylphenyl]-4-(4-piperidylamino)pyrrolidin-2-one;
4-Amino- 1-[4-[4-[6-chloro-4-(trifluoromethyl)-2-pyridyl]piperazin-1-yl]sulfonylphenyl]-5-methyl-pyrrolidin-2-one;
4-Amino- 1-[5-[4-[6-chloro-4-(trifluoromethyl)-2-pyridyl]piperazin-1-yl]sulfonyl-2-pyridyl]pyrrolidin-2-one;
5-(Aminomethyl)-3-[4-[4-[6-chloro-4-(trifluoromethyl)-2-pyridyl]piperazin-1-yl]sulfonyl-2-fluoro-phenyl]oxazolidin-2-one;
5-(Aminomethyl)-3-[4-[4-[6-chloro-4-(trifluoromethyl)-2-pyridyl]piperazin-1-yl]sulfonyl-3-fluoro-phenyl]oxazolidin-2-one;
4-Amino-1-[4-[4-[4-methyl-6-(trifluoromethyl)pyrimidin-2-yl]piperazin-1-yl]sulfonylphenyl]pyrrolidin-2-one;
4-Amino-1-[4-[4-[4-(trideuteriomethyl)-6-(trifluoromethyl)pyrimidin-2-yl]piperazin-1-yl] sulfonylphenyl]pyrrolidin-2-one;
1-[4-(4-Amino-2-oxo-pyrrolidin-1-yl)phenyl]sulfonyl-4-[6-chloro-4-(trifluoromethyl)-2-pyridyl]piperazine-2-carbonitrile;
4-Amino-1-[4-[4-[6-chloro-4-(difluoromethyl)-2-pyridyl]piperazin-1-yl]sulfonylphenyl] pyrrolidin-2-one;
4-Amino-1-[4-[4-(6-chloro-4-cyclopropylsulfonyl-2-pyridyl)piperazin-1-yl] sulfonylphenyl] pyrrolidin-2-one;
4-Amino-1-[4-[4-(6-chloro-4-tetrahydropyran-4-ylsulfonyl-2-pyridyl)piperazin-1-yl] sulfonylphenyl]pyrrolidin-2-one;
1-[2-[4-[4-(4-Amino-2-oxo-pyrrolidin-1-yl)phenyl] sulfonylpiperazin-1-yl]-6-chloro-4-pyridyl]cyclopropanecarbonitrile;
4-Amino- 1-[4-[4-(6-chloro-4-phenoxy-2-pyridyl)piperazin- 1-yl]sulfonylphenyl]pyrrolidin-2-one;
4-Amino-1-[4-[4-[6-chloro-4-[difluoro(phenyl)methyl]-2-pyridyl]piperazin-1-yl]sulfonylphenyl] pyrrolidin-2-one;
4-Amino-1-[4-[4-(4-benzyl-6-chloro-2-pyridyl)piperazin-1-yl]sulfonylphenyl]pyrrolidin-2-one;
4-Amino-1-[4-[4-[6-chloro-4-(1-phenylcyclopropyl)-2-pyridyl]piperazin-1-yl]sulfonylphenyl] pyrrolidin-2-one;
4-Amino-1-[4-[4-[4-[difluoro(phenyl)methyl]-6-methyl-pyrimidin-2-yl]piperazin-1-yl] sulfonylphenyl]pyrrolidin-2-one;
4-Amino-1-[4-[4-[2-chloro-6-[difluoro(phenyl)methyl]pyrimidin-4-yl]piperazin-1-yl] sulfonylphenyl]pyrrolidin-2-one;
4-Amino-1-[4-[4-[4-chloro-6-[difluoro(phenyl)methyl]pyrimidin-2-yl]piperazin-1-yl] sulfonylphenyl]pyrrolidin-2-one;
4-Amino-1-[4-[4-[2-cyclopropyl-6-[difluoro(phenyl)methyl]pyrimidin-4-yl]piperazin-1-yl] sulfonylphenyl]pyrrolidin-2-one;
4-Amino-1-[4-[4-[4-cyclopropyl-6-[difluoro(phenyl)methyl]pyrimidin-2-yl]piperazin-1-yl] sulfonylphenyl]pyrrolidin-2-one;
4-Amino-1-[4-[4-[6-[difluoro(phenyl)methyl]-2-methoxy-pyrimidin-4-yl]piperazin-1-yl] sulfonylphenyl]pyrrolidin-2-one;
4-Amino-1-[4-[4-[4-[difluoro(phenyl)methyl]-6-methoxy-pyrimidin-2-yl]piperazin-1-yl]sulfonylphenyl]pyrrolidin-2-one;
(4*R*)-4-Amino-1-[4-[4-[6-[difluoro(phenyl)methyl]-2-methyl-pyrimidin-4-yl]piperazin-1-yl]sulfonylphenyl]pyrrolidin-2-one;
4-Amino-1-[4-[4-[6-[difluoro(phenyl)methyl]-2-vinyl-pyrimidin-4-yl]piperazin-1-yl]sulfonylphenyl]pyrrolidin-2-one;
2-[4-[4-[6-Chloro-4-[difluoro(phenyl)methyl]-2-pyridyl]piperazin-1-yl]sulfonylphenyl]-2,6,9-triazaspiro[4.5]decan-3-one;
5-(Aminomethyl)-1-[4-[4-[6-chloro-4-[difluoro(phenyl)methyl]-2-pyridyl]piperazin-1-yl]sulfonylphenyl]pyrrolidin-2-one;
(3a*R*,7a*S*)-3-[4-[4-[6-chloro-4-[difluoro(phenyl)methyl]-2-pyridyl]piperazin-1-yl]sulfonyl phenyl]-3a,4,5,6,7,7a-hexahydrooxazolo[4,5-c]pyridin-2-one;
5-(Aminomethyl)-3-[4-[4-[6-chloro-4-[difluoro(phenyl)methyl]-2-pyridyl]piperazin-1-yl]sulfonylphenyl]oxazolidin-2-one;
4-Amino-1-[4-[4-[6-chloro-4-[difluoro(2-pyridyl)methyl]-2-pyridyl]piperazin-1-yl] sulfonylphenyl] pyrrolidin-2-one;
4-Amino-1-[4-[4-[6-chloro-4-[difluoro(3-pyridyl)methyl]-2-pyridyl]piperazin-1-yl]sulfonylphenyl] pyrrolidin-2-one;
4-Amino-1-[4-[4-[6-chloro-4-[difluoro(4-pyridyl)methyl]-2-pyridyl]piperazin-1-yl] sulfonylphenyl]pyrrolidin-2-one;
4-Amino-1-[4-[4-[6-chloro-4-[difluoro(pyrimidin-5-yl)methyl]-2-pyridyl]piperazin-1-yl]sulfonylphenyl]pyrrolidin-2-one;
4-Amino-1-[4-[4-[6-chloro-4-[difluoro(pyrazin-2-yl)methyl]-2-pyridyl]piperazin-1-yl]sulfonylphenyl]pyrrolidin-2-one;
4-Amino-1-[4-[4-[6-chloro-4-[difluoro(pyrimidin-2-yl)methyl]-2-pyridyl]piperazin-1-yl]sulfonylphenyl]pyrrolidin-2-one;
4-Amino-1-[4-[4-[6-chloro-4-[difluoro(tetrahydropyran-4-yl)methyl]-2-pyridyl]piperazin-1-yl]sulfonylphenyl]pyrrolidin-2-one;
4-Amino-1-[4-[4-[6-chloro-4-[difluoro(tetrahydrofuran-3-yl)methyl]-2-pyridyl]piperazin-1-yl]sulfonylphenyl]pyrrolidin-2-one;
4-Amino-1-[4-[4-[6-chloro-4-[difluoro(oxetan-3-yl)methyl]-2-pyridyl]piperazin-1-yl]sulfonylphenyl] pyrrolidin-2-one;
4-Amino-1-[4-[4-[6-chloro-4-[3,6-dihydro-2H-pyran-4-yl(difluoro)methyl]-2-pyridyl] piperazin-1-yl]sulfonylphenyl]pyrrolidin-2-one;
4-Amino-1-[4-[4-[6-chloro-4-[difluoro(tetrahydropyran-3-yl)methyl]-2-pyridyl]piperazin-1-yl]sulfonylphenyl]pyrrolidin-2-one;
4-Amino-1-[4-[4-[6-chloro-4-[difluoro(8-oxabicyclo[3.2.1]octan-3-yl)methyl]-2-pyridyl] piperazin-1-yl]sulfonylphenyl]pyrrolidin-2-one;
(4*R*)-4-amino-1-[4-[4-[6-chloro-4-[difluoro(3-oxabicyclo[3.1.0]hexan-6-yl)methyl]-2-pyridyl]piperazin-1-yl]sulfonylphenyl]pyrrolidin-2-one;
(4*R*)-4-amino-1-[4-[4-[6-chloro-4-[[(25)-1,4-dioxan-2-yl]-difluoro-methyl]-2-pyridyl] piperazin-1-yl]sulfonylphenyl]pyrrolidin-2-one;
(4*R*)-4-amino-1-[4-[4-[6-chloro-4-[[(2*R*)- 1,4-dioxan-2-yl]-difluoro-methyl]-2-pyridyl] piperazin-1-yl]sulfonylphenyl]pyrrolidin-2-one;
(4*R*)-4-amino-1-[4-[4-[6-chloro-4-[difluoro-[(2*S*)-morpholin-2-yl]methyl]-2-pyridyl] piperazin-1-yl]sulfonylphenyl]pyrrolidin-2-one;
(4*R*)-4-amino-1-[4-[4-[6-chloro-4-[difluoro-[(2*R*)-morpholin-2-yl]methyl]-2-pyridyl] piperazin-1-yl]sulfonylphenyl]pyrrolidin-2-one;
(4*R*)-4-amino-1-[4-[4-[6-chloro-4-[difluoro(1,2,3,6-tetrahydropyridin-4-yl)methyl]-2-pyridyl]piperazin-1-yl]sulfonylphenyl]pyrrolidin-2-one;
(4*R*)-4-amino-1-[4-[4-[6-chloro-4-[difluoro-[(3*R*)-3-piperidyl]methyl]-2-pyridyl]piperazin-1-yl]sulfonylphenyl]pyrrolidin-2-one;
(4*R*)-4-amino-1-[4-[4-[6-chloro-4-[difluoro-[(3*S*)-3-piperidyl]methyl]-2-pyridyl]piperazin-1-yl]sulfonylphenyl]pyrrolidin-2-one;
(4*R*)-4-amino-1-[4-[4-[4-[azetidin-3-yl(difluoro)methyl]-6-chloro-2-pyridyl]piperazin-1-yl]sulfonylphenyl]pyrrolidin-2-one;
3-[4-[4-[6-Chloro-4-[difluoro-[(2*S*)-morpholin-2-yl]methyl]-2-pyridyl]piperazin-1-yl]sulfonylphenyl] -5-(hydroxymethyl)oxazolidin-2-one;
(4*R*)-4-amino-1-[4-[4-[6-chloro-4-[difluoro-(5-methylpyrazin-2-yl)methyl]-2-pyridyl]piperazin-1-yl]sulfonylphenyl]pyrrolidin-2-one;
(4*R*)-4-amino-1-[4-[4-[6-chloro-4-[difluoro-(6-methylpyrazin-2-yl)methyl]-2-pyridyl]piperazin-1-yl]sulfonylphenyl]pyrrolidin-2-one;
(4*R*)-4-amino-1-[4-[4-[6-chloro-4-[(5-chloro-6-methyl-pyrazin-2-yl)-difluoro-methyl]-2-pyridyl]piperazin-1-yl]sulfonylphenyl]pyrrolidin-2-one;
(4*R*)-4-amino-1-[4-[4-[6-chloro-4-[difluoro-[cis-6-methylmorpholin-2-yl]methyl]-2-pyridyl]piperazin-1-yl]sulfonylphenyl]pyrrolidin-2-one;
((4*R*)-4-amino-1-[4-[4-[6-chloro-4-[difluoro-(5-methylmorpholin-2-yl)methyl]-2-pyridyl]piperazin-1-yl]sulfonylphenyl]pyrrolidin-2-one;
(4*R*)-4-amino-1-[4-[4-[4-[[*cis*-3-azabicyclo[3.1.0]hexan-1-yl]-difluoro-methyl]-6-chloro-2-pyridyl]piperazin-1-yl]sulfonylphenyl]pyrrolidin-2-one;
(4*R*)-4-amino-1-[4-[4-[6-chloro-4-[trans-difluoro-[2-(hydroxymethyl)cyclopropyl]methyl]-2-pyridyl]piperazin-1-yl]sulfonylphenyl]pyrrolidin-2-one;
(4*R*)-4-amino-1-[4-[4-[4-[[*trans*-4-(aminomethyl)cyclohexyl]-difluoro-methyl]-6-chloro-2-pyridyl]piperazin-1-yl]sulfonylphenyl]pyrrolidin-2-one;
(4*R*)-4-amino-1-[4-[4-[6-chloro-4-[*trans*-difluoro-(3-hydroxycyclobutyl)methyl]-2-pyridyl]piperazin-1-yl]sulfonylphenyl]pyrrolidin-2-one; and
*Trans*-4-[[2-[4-[4-[(4R)-4-amino-2-oxo-pyrrolidin-1-yl]phenyl]sulfonylpiperazin-1-yl]-6-chloro-4-pyridyl]-difluoro-methyl]cyclohexanecarboxamide;
or a pharmaceutically acceptable salt thereof.

### PHARMACEUTICAL COMPOSITIONS AND ADMINISTRATION

Another embodiment provides pharmaceutical compositions or medicaments containing the compounds of the invention and a therapeutically inert carrier, diluent or excipient, as well as methods of using the compounds of the invention to prepare such compositions and medicaments. In one example, compounds of formula (I) may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but preferably ranges anywhere from about 3 to about 8. In one example, a compound of formula (I) is formulated in an acetate buffer, at pH 5. In another embodiment, the compounds of formula (I) are sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "effective amount" of the compound to be administered will be governed by such considerations, and is the minimum amount necessary to reduced bacterial load or improve host survival through the inhibition of Lipid A biosynthesis by targeting LpxH enzyme. For example, such amount may be below the amount that is toxic to normal cells, or the mammal as a whole.

In one example, the pharmaceutically effective amount of the compound of the invention administered parenterally per dose will be in the range of about 0.1 to 1000 mg/kg, alternatively about 1 to 100 mg/kg of patient body weight per day, with the typical initial range of compound used being 0.3 to 15 mg/kg/day. In another embodiment, oral unit dosage forms, such as tablets and capsules, preferably contain from about 5 to about 5000 mg of the compound of the invention.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents.

A typical formulation is prepared by mixing a compound of the present invention and a carrier or excipient. Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

An example of a suitable oral dosage form is a tablet containing about 10 to 500 mg of the compound of the invention compounded with about 40 to 400mg anhydrous lactose, about 5 to 50 mg sodium croscarmellose, about 5 to 50 mg polyvinylpyrrolidone (PVP) K30, and about 1 to 10 mg magnesium stearate. The powdered ingredients are first mixed together and then mixed with a solution of the PVP. The resulting composition can be dried, granulated, mixed with the magnesium stearate and compressed to tablet form using conventional equipment. An example of an aerosol formulation can be prepared by dissolving the compound, for example 5 to 1000 mg) of the invention in a suitable buffer solution, e.g. a phosphate buffer, adding a tonicifier, e.g. a salt such sodium chloride, if desired. The solution may be filtered, e.g., using a 0.2 micron filter, to remove impurities and contaminants.

An embodiment, therefore, includes a pharmaceutical composition comprising a compound of Formula (I), or a stereoisomer or pharmaceutically acceptable salt thereof. In a further embodiment includes a pharmaceutical composition comprising a compound of Formula (I), or a stereoisomer or pharmaceutically acceptable salt thereof, together with a pharmaceutically acceptable carrier or excipient.

Another embodiment includes a pharmaceutical composition comprising a compound of formula (I) for use in the treatment and/or prevention of bacterial infections.

The following composition A and B illustrate typical compositions of the present invention, but serve merely as representative thereof.

### Composition A

A compound of the present invention can be used in a manner known per se as the active ingredient for the production of tablets of the following composition:

| | Per tablet |
|---|---|
| Active ingredient | 200 mg |
| Microcrystalline cellulose | 155 mg |
| Corn starch | 25 mg |
| Talc | 25 mg |
| Hydroxypropylmethylcellulose | 20 mg |
| | 425 mg |

### Composition B

A compound of the present invention can be used in a manner known per se as the active ingredient for the production of capsules of the following composition:

| | Per capsule |
|---|---|
| Active ingredient | 100.0 mg |
| Corn starch | 20.0 mg |
| Lactose | 95.0 mg |
| Talc | 4.5 mg |
| Magnesium stearate | 0.5 mg |
| | 220.0 mg |

### INDICATIONS AND METHODS OF TREATMENT

The compounds of the invention are inhibitors of the LpxH enzyme, a key enzyme of the LPS synthesis pathway that is essential in most gram-negative bacteria. Accordingly, the compounds of the invention can prevent bacterial growth of susceptible organisms and are useful for: preventing or treating a bacterial infection, preferably a Gram-negative bacterial infection (all claimed) e.g. nosocomial pneumonia, urinary tract infections, systemic infections (bacteraemia and sepsis), skin and soft tissue infections, surgical infections, eye infections, intraabdominal infections, lung infections and diabetic foot infections caused by Gram-negative bacteria e.g. third generation cephalosporins- and carbapenem- resistant *Enterobacteriaeceae* (e.g. *Klebsiella pneumoniae, Escherichia coli*) and multi-drug-resistant *Pseudomonas aeruginosa* and *Acinetobacter baumannii* or *Acinetobacter* spp., e.g. *Neisseria gonorrhoeae, Haemophilus influenzae, Helicobacter pylorus* e.g. *Bacteroides spp.* e.g. *Bacteroides fragilis, Bacteroides thetaiotaomicron, Bacteroides distasonis, Campylobacter jejuni, Campylobacter fetus* or *Campylobacter coil, Francisella tularensis* and *Providencia spp.* e.g. *Providencia stuartii, Providencia rettgeri* or *Providencia alcalifaciens* and *Pseudomonas spp.;* and for cleaning purposes e.g. to remove pathogenic microbes and bacteria from surgical instruments, catheters and artificial implants or to make a room or an area aseptic.

The products of the invention can be administered, for example, parenterally e.g. by injection, or administered orally, perorally, such as in the form of tablets, coated tablets, dragees, hard and soft gelatin capsules, solutions, emulsions or suspensions, or rectally, such as in the form of suppositories. Pharmaceutical compositions containing these compounds can be prepared using conventional procedures familiar to those skilled in the art, such as by combining the ingredients into a dosage form together with suitable, non-toxic, inert, therapeutically compatible solid or liquid carrier materials and, if desired, the usual pharmaceutical adjuvants. It is contemplated that the compounds are ultimately embodied into compositions of suitable oral, parenteral or topical dosage forms. The compositions of this invention can contain, as optional ingredients, any of the various adjuvants, which are used ordinarily in the production of pharmaceutical preparations. Thus, for example, in formulating the present compositions into the desired oral dosage forms, one may use, as optional ingredients, fillers, such as co-precipitated aluminum hydroxide-calcium carbonate, di-calcium phosphate or lactose; disintegrating agents such as maize starch; and lubricating agents, such as talc, calcium stearate, and the like. It should be fully understood, however, that the optional ingredients herein named are given by way of example only and that the invention is not restricted to the use hereof. Other such adjuvants, which are well known in the art, can be employed in carrying out this invention. Suitable as such carrier materials are not only inorganic, but also organic carrier materials. Thus, for tablets, coated tablets, dragees and hard gelatin capsules there can be used, for example, lactose, maize starch or derivatives thereof, talc, stearic acid or its salts. Suitable carriers for soft gelatin capsules are, for example, vegetable oils, waxes, fats and semi-solid and liquid polyols (depending on the nature of the active substance; no carriers are, however, required in the case of soft gelatin capsules). Suitable carrier materials for the preparation of solutions and syrups are, for example, water, polyols, saccharose, invert sugar and glucose. Suitable carrier materials for suppositories are, for example, natural or hardened oils, waxes, fats and semi-liquid or liquid polyols. As pharmaceutical adjuvants there are contemplated the usual preservatives, solubilizers, stabilizers, wetting agents, emulsifiers, sweeteners, colorants, flavorings, salts for varying the osmotic pressure, buffers, coating agents and antioxidants.

### SYNTHESIS

The compounds of the present invention can be prepared by any conventional means. Suitable processes for synthesizing these compounds as well as their starting materials are provided in the schemes below and in the examples. All substituents, in particular, R¹, R², R³, R⁴, Y, Q¹, Q², Q³, Q⁴ and Q⁵ are defined above. Furthermore, and unless explicitly otherwise stated, all reactions, reaction conditions, abbreviations and symbols have the meanings well known to a person of ordinary skill in organic chemistry.

General synthetic routes for preparing the compound of formula (I) are shown below.

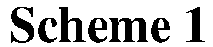

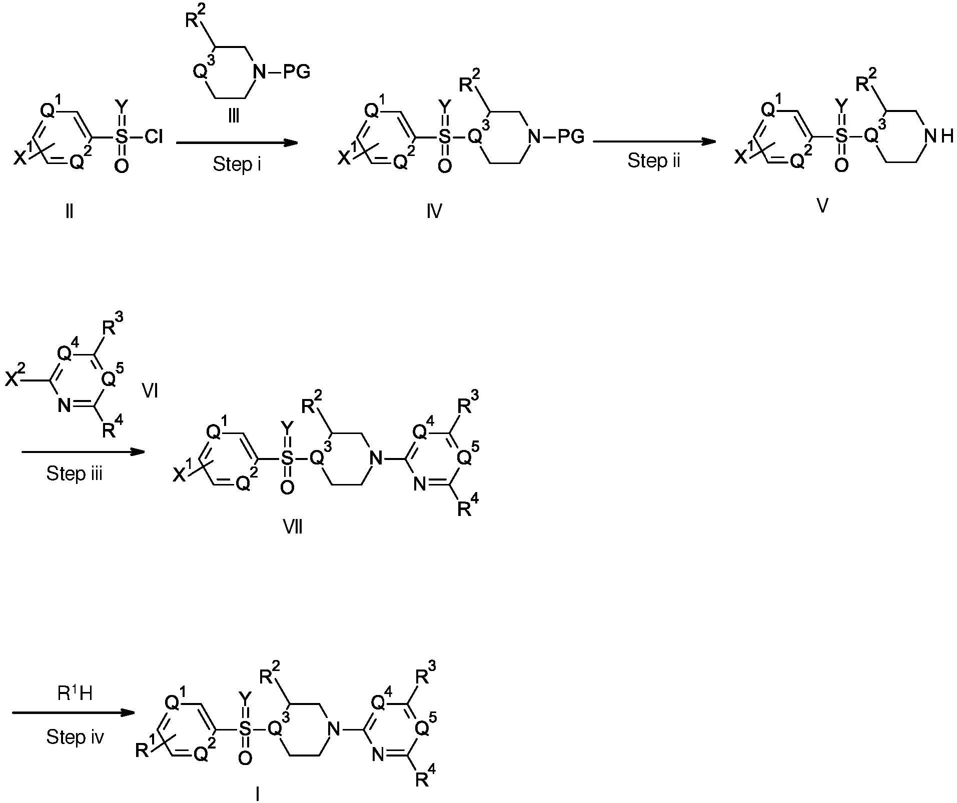

Wherein X¹ and X² are halogen, such as Cl, Br, I; PG is a protecting group such as *tert-*Boc or Cbz group.

Step (i) could be a sulfonated reaction between halogenated phenyl sulfonyl chlorides (II) and protected piperazine (III) under in basic conditions (for example, in the presence of a suitable base such as trimethylamine) to provide compound of formula (IV).

Compounds of formula (V) may be obtained in step (ii) by de-protection of compound of formula (IV) with a suitable acid such as trifluoroacetic acid or by a reducing agent, such as H₂ with palladium.

Step (iii) may be a nucleophilic substitution by reacting Intermediate (V) with halogenated compound of formula (VI) using a suitable base to provide compound of formula (VII).

The final compound of formula (I) could be obtained in step (iv) through the coupling reaction of compound of formula (VII)with an cyclic-amide such as lactam or carbamate, R¹H, using catalysts such as CuI in the presence of bases such as Cs₂CO₃.

Compounds of this invention can be obtained as mixtures of diastereomers or enantiomers, which can be separated by methods well known in the art, e.g. (chiral) HPLC or SFC.

This invention also relates to a process for the preparation of a compound of formula (I) comprising following step:
a) Coupling reaction between compound of formula (VII), 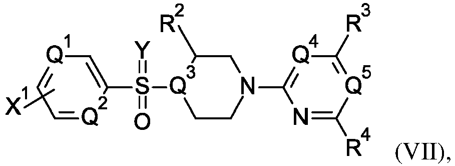 with lactam or carbamate, R¹H, in the presence of catalyst and base;
wherein
in step a), the catalyst can be CuI, the base can be Cs₂CO₃;

A compound of formula (I) when manufactured according to the above process is also an object of the invention.

### EXAMPLES

The invention will be more fully understood by reference to the following examples. They should not, however, be construed as limiting the scope of the invention.

### ABBREVIATIONS

The invention will be more fully understood by reference to the following examples. They should not, however, be construed as limiting the scope of the invention.

Abbreviations used herein are as follows:
- ACN: acetonitrile
- aq.: aqueous
- BAST: bis(2-methoxyethyl)aminosulfur Trifluoride
- DAST: diethylaminosulfur trifluoride
- DBU: 1,8-diazabicyclo[5.4.0]undec-7-ene
- DCM: dichloromethane
- DIPEA: *N,N*-diisopropylethylamine
- DMAP: 4-dimethylaminopyridine
- DMEDA: 1,2-dimethylethylenediamine
- DMF: dimethylformamide
- DMP: Dess-Martin periodinane
- DMSO: dimethyl sulfoxide
- EA: ethyl acetate
- FA: formic acid
- h(s) or hr(s): hour
- HATU: 1-[bis(dimethylamino)methylene]-1H-1,2,3-triazolo[4,5-b]pyridinium 3-oxid hexafluorophosphate
- HPLC: high performance liquid chromatography
- IPA: isopropyl alcohol
- m-CPBA: meta-chloroperoxybenzoic acid
- MIC: minimum inhibitory concentration
- NMP: *N*-methyl-2-pyrrolidone
- obsd.: observed
- PE: petroleum ether
- prep-HPLC: preparative high performance liquid chromatography
- rt: room temperature
- SFC: supercritical fluid chromatography
- TEA: triethylamine
- TFA: trifluoroacetic acid
- TLC: thin layer chromatography

### GENERAL EXPERIMENTAL CONDITIONS

Intermediates and final compounds were purified by flash chromatography using one of the following instruments: i) Biotage SP1 system and the Quad 12/25 Cartridge module. ii) ISCO combi-flash chromatography instrument. Silica gel brand and pore size: i) KP-SIL 60 Å, particle size: 40-60 µm; ii) CAS registry NO: Silica Gel: 63231-67-4, particle size: 47-60 micron silica gel; iii) ZCX from Qingdao Haiyang Chemical Co., Ltd, pore: 200-300 or 300-400.

Intermediates and final compounds were purified by preparative HPLC on reversed phase column using XBridge^{™} Prep-C18 (5 µm, OBDTM 30 × 100 mm) column, SunFire^{™} Prep-C18 (5 µm, OBD^{™} 30 × 100 mm) column, Phenomenex Synergi-C18 (10 µm, 25 × 150 mm) or Phenomenex Gemini-C18 (10 µm, 25 × 150 mm). Waters AutoP purification System (Sample Manager 2767, Pump 2525, Detector: Micromass ZQ and UV 2487, solvent system: acetonitrile and 0.1% ammonium hydroxide in water; acetonitrile and 0.1% FA in water or acetonitrile and 0.1% TFA in water). Or Gilson-281 purification System (Pump 322, Detector: UV 156, solvent system: acetonitrile and 0.05% ammonium hydroxide in water; acetonitrile and 0.225% FA in water; acetonitrile and 0.05% HCl in water; acetonitrile and 0.075% TFA in water; or acetonitrile and water).

For SFC chiral separation, intermediates were separated by chiral column (Daicel chiralpak IC, 5 µm, 30 × 250 mm), AS (10 µm, 30 × 250 mm) or AD (10 µm, 30 × 250 mm) using Mettler Toledo Multigram III system SFC, Waters 80Q preparative SFC or Thar 80 preparative SFC, solvent system: CO₂ and IPA (0.5% TEA in IPA) or CO₂ and MeOH (0.1% NH₃·H₂O in MeOH), back pressure 100bar, detection UV@ 254 or 220 nm.

LC/MS spectra of compounds were obtained using a LC/MS (Waters^{™} Alliance 2795-Micromass ZQ, Shimadzu Alliance 2020-Micromass ZQ or Agilent Alliance 6110-Micromass ZQ), LC/MS conditions were as follows (running time 3 or 1.5 mins):
Acidic condition I: A: 0.1% TFA in H₂O; B: 0.1% TFA in acetonitrile;
Acidic condition II: A: 0.0375% TFA in H₂O; B: 0.01875% TFA in acetonitrile;
Basic condition I: A: 0.1% NH₃·H₂O in H₂O; B: acetonitrile;
Basic condition II: A: 0.025% NH₃·H₂O in H₂O; B: acetonitrile;
Neutral condition: A: H₂O; B: acetonitrile.

Mass spectra (MS): generally only ions which indicate the parent mass are reported, and unless otherwise stated the mass ion quoted is the positive mass ion (MH)⁺.

NMR Spectra were obtained using Bruker Avance 400 MHz.

The microwave assisted reactions were carried out in a Biotage Initiator Sixty microwave synthesizer. All reactions involving air-sensitive reagents were performed under an argon or nitrogen atmosphere. Reagents were used as received from commercial suppliers without further purification unless otherwise noted.

### PREPARATIVE EXAMPLES

The following examples are intended to illustrate the meaning of the present invention but should by no means represent a limitation within the meaning of the present invention:

### Intermediate A 1-[6-Chloro-4-(trifluoromethyl)-2-pyridyl]-4-(4-iodophenyl)sulfonyl-piperazine

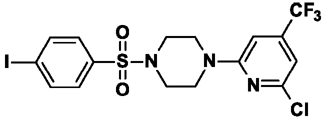

The title compound was prepared according to the following procedures:

### Step 1: 1-[6-chloro-4-(trifluoromethyl)-2-pyridyl]piperazine(intermediate A1)

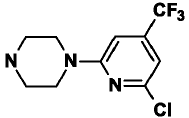

To a solution of piperazine (47.86 g, 555.58 mmol) in 1,4-dioxane (150 mL) were added 2,6-dichloro-4-(trifluoromethyl)pyridine (15.0 g, 69.45 mmol) slowly. The mixture was stirred at 60 °C for 1 h, then diluted with water (300 mL) and extracted with EA (200 mL) three times. The combined organic layer was washed with brine (300 mL) four times, dried over anhydrous Na₂SO₄, filtered and concentrated to give intermediate A1 (18 g, 67.76 mmol, 97.6% yield) as a white solid. MS obsd.: 265.8 (MH⁺).

### Step 2: 1-[6-chloro-4-(trifluoromethyl)-2-pyridyl]-4-(4-iodophenyl)sulfonyl-piperazine (intermediate A)

To a solution of intermediate A1 (7.9 g, 29.75 mmol) and TEA (12.44 mL, 89.25 mmol) of in THF (100 mL) was added 4-iodobenzenesulfonyl chloride (9.0 g, 29.75 mmol) and the mixture was stirred at 20 °C for 12 h. The mixture was diluted with EA (400 mL) and filtered. The filtrate was washed with brine (100 mL) three times, dried over anhydrous Na₂SO₄, filtered and concentrated. The crude was triturated with PE (150 mL) to give intermediate A (14.5 g, 27.27 mmol, 91.7% yield) as a white solid. MS obsd.: 531.8 (MH⁺).

### Intermediate B

### 1-[4-[4-[6-Chloro-4-(trifluoromethyl)-2-pyridyl]piperazin-1-yl]sulfonylphenyl]-2H-pyrrol-5-one

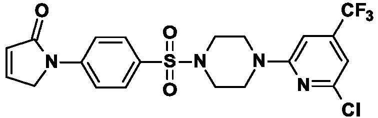

The title compound was prepared according to the following procedures:

### Step 1: 1-[4-[4-[6-chloro-4-(trifluoromethyl)-2-pyridyl]piperazin-1-yl]sulfonylphenyl]-4-hydroxy-pyrrolidin-2-one (intermediate B1)

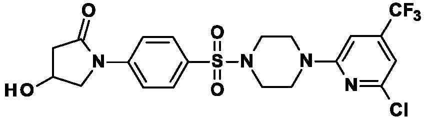

To a solution of intermediate A (1.0 g, 1.88 mmol) in DMF (10 mL) were added 4-hydroxy-2-pyrrolidone (209.15 mg, 2.07 mmol), Cs₂CO₃ (1.23 g, 3.76 mmol), DMEDA (33.16 mg, 0.380 mmol), CuI (0.01 mL, 0.380 mmol) in one portion. The reaction was stirred at 70 °C for 2 hrs under N₂. The mixture was diluted with water (50 mL), extracted with EA (30 mL) three times. The combined organic layer was washed with brine (50 mL), dried over anhydrous Na₂SO₄, filtered and concentrated. The crude was purified by column chromatography on silica gel (PE: EA=10:1 to 0:1) and prep-HPLC to give intermediate B1 (320 mg, 0.630 mmol, 33.7% yield) as a white solid.

### Step 2: 1-[4-[4-[6-chloro-4-(trifluoromethyl)-2-pyridyl]piperazin-1-yl]sulfonylphenyl]-2H-pyrrol-5-one (intermediate B)

To a solution of intermediate B1 (320.0 mg, 0.630 mmol) in DCM (4 mL) was added TEA (0.18 mL, 1.27 mmol) in one portion. After cooled to 0°C, methanesulfonyl chloride (0.07 mL, 0.950 mmol) was added and then the reaction was warmed to 20 °C for 1 h. The mixture was diluted with DCM (20 mL), washed with water (20 mL) and brine (20 mL). The organic layer was dried over anhydrous Na₂SO₄, filtered and concentrated to give intermediate B (290 mg, 0.60 mmol, 93.98% yield) was obtained as yellow oil.

### Intermediate C

### Tert-butyl N-[5-oxo-1-(4-piperazin-1-ylsulfonylphenyl)pyrrolidin-3-yl]carbamate

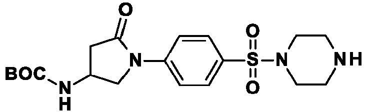

The title compound was prepared according to the following procedures:

### Step 1: benzyl 4-(4-iodophenyl) sulfonylpiperazine-1-carboxylate (intermediate C1)

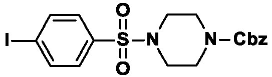

To a solution of 1-Cbz-piperazine (8.01 g, 36.4 mmol), TEA (6.45 mL, 46.3 mmol) in DCM (200 mL) was added 4-iodobenzenesulfonyl chloride (10.0 g, 33.06 mmol) at 0 °C and the mixture was stirred at 25 °C for 2 hrs.. The mixture was concentrated to give the residue which was triturated with petroleum ether and washed by water to give intermediate C1 (18.0 g, 37.0 mmol, 95.2% yield) as a white solid. MS obsd.: 487.0 (MH⁺). ¹H NMR (400 MHz, CDCl₃): δ ppm 7.94 (br d, *J =* 8.8 Hz, 2 H), 7.89 (m, 2 H), 7.43 (m, 1 H), 7.32 - 7.33 (m, 4 H), 5.01 (s, 2 H), 3.12 - 3.14 (m, 8 H).

### Step 2: benzyl 4-((4-(4-((tert-butoxycarbonyl)amino)-2-oxopyrrolidin-1-yl)phenyl)sulfonyl) piperazine-1-carboxylate (intermediate C2)

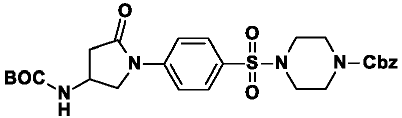

To a mixture of intermediate C1 (18.0 g, 37.0 mmol), *tert*-butyl *N*-(5-oxopyrrolidin-3-yl)carbamate (8.89 g, 44.4 mmol) and K₂CO₃ (15.4 g, 111 mmol) in NMP (180 mL) was added CuI (7.05 g, 37.0 mmol, 1.00 eq) and *N1,N2*-dimethylcyclohexane-1,2-diamine (5.26 g, 37.0 mmol). The mixture was stirred at 90 °C for 2 hrs under N₂. The mixture was diluted with EA (200 mL) and filtered. The filtrate was washed with NH₃ . H₂O (2% wt, 200 mL), aq CaCl₂ (150 mL, 2 N) two times and NaCl (150 mL) two times, dried over Na₂SO₄, filtered and concentrated to give yellow oil. The oil was purified by pre-HPLC to give intermediate C2 (13.5 g, 24.2 mmol, 65.3% yield) as a gray solid. MS obsd.: 559.3 (MH⁺).

### Step 3: tert-butyl N-[5-oxo-1-(4-piperazin-1-ylsulfonylphenyl)pyrrolidin-3-yl]carbamate (intermediate C)

To a solution of intermediate C2 (500.0 mg, 0.90 mmol) in methanol (10 mL) was added Pd/C (10% wt, 100.0 mg). The mixture was stirred under H₂ (15psi) at 25 °C for 2 hrs. The mixture was diluted with MeOH (100 mL), filtered through a pad of Celite and concentrated to give Intermediate C (300 mg, 0.710 mmol, 78.96% yield) as a grey solid. MS obsd.: 425.3 (MH⁺).

### Intermediate D

### Tert-butyl N-[(3R)-5-oxo-1-(4-piperazin-1-ylsulfonylphenyl)pyrrolidin-3-yl]carbamate

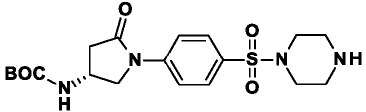

The title compound was prepared in analogy to the preparation of intermediate C by using *tert-*butyl *N*-[(3R)-5-oxopyrrolidin-3-yl]carbamate instead of *tert-*butyl *N*-(5-oxopyrrolidin-3-yl)carbamate. **Intermediate D** (700 mg) was obtained as a white solid. MS obsd.: 425.2 (MH⁺).

### Intermediate E and Intermediate F

### 2,6-Dichloro-4-[[(2S)-1,4-dioxan-2-yl]-difluoro-methyl]pyridine and 2,6-Dichloro-4-[[(2R)-1,4-dioxan-2-yl]-difluoro-methyl]pyridine

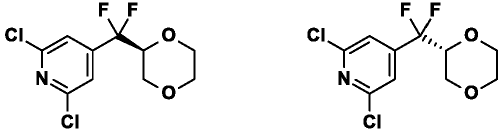

The title compounds were prepared according to the following procedures:

### Step 1: 1,4-dioxane-2-carbaldehyde (intermediate E1)

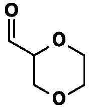

To a mixture of DMSO (15.75 g, 315.1 mmol) in DCM (450 mL) was added (COCl)₂ (19.84 g, 157.6 mmol) dropwise at -60 °C. After stirring for 1 h, (1,4-dioxan-2-yl)methanol (15.5 g, 131.3 mmol) was added and the mixture was stirred at -60 °C for 2 hrs. TEA (39.83 g, 393.9 mmol) was added at -60 °C and the mixture was warmed to 10 °C slowly. The suspension was filtered and the filtrate was concentrated to give the crude, which was distilled under vacuum pump and collected the 70 °C co-boiled to give intermediate E1 (21.5 g, yield: 70.7 %) as colorless liquid. ¹H NMR (400 MHz, CDCl₃): δ ppm 9.54 - 9.64 (m, 1 H), 3.97 - 4.09 (m, 1 H) 3.82 - 3.95 (m, 2 H) 3.52 - 3.79 (m, 4 H).

### Step 2: (2,6-dichloropyridin-4-yl)(1,4-dioxan-2-yl)methanol (intermediate E2)

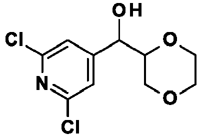

To a mixture of 2,6-dichloro-4-iodopyridine (21.46 g, 78.37 mmol) in THF (70 mL) was added CH₃MgCl/LiCl (64.92 mL, 84.4 mmol) under N₂ at -50 °C. The mixture was stirred at 25 °C for 2 hrs and then 1,4-dioxane-2-carbaldehyde (14.0 g, 60.28 mmol) in THF (10 mL) was added at -50 °C. The mixture was stirred at 25 °C for 1h. The reaction was then poured into saturated aq. NH₄Cl solution and extracted with EA (300 mL) three times. The organic layer were combined, washed with brine, dried over anhydrous Na₂SO₄, filtered and concentrated. The crude was purified by column chromatography on silica gel (PE: EA=3:2) to give intermediate E2 (8.3 g, 31.43 mmol, 52.13% yield) as yellow oil. MS obsd.: 264.1 (MH⁺).

### Step 3: (2,6-dichloropyridin-4-yl)(1,4-dioxan-2-yl)methanone(intermediate E3)

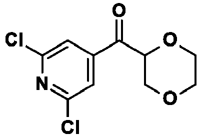

To a solution of intermediate E2 (6.4 g, 24.23 mmol) in DCM (60 mL) was added DMP (11.31 g, 26.66 mmol) at 0°C and the reaction was stirred at 20°C for 0.5 h. The mixture was poured into a mixture of aq. Na₂CO₃ (400 mL) and EA: THF=5:1 (500 mL). After stirring for 0.5h, the reaction was filtered and the organic layer was extracted with aq. Na₂CO₃ (300 mL) two times and dried over anhydrous Na₂SO₄, filtered and concentrated to give intermediate E3 (6 g, 22.89 mmol, 94.47% yield) as a light yellow solid.

### Step 4: 2,6-dichloro-4-[1,4-dioxan-2-yl(difluoro)methyl]pyridine (intermediate E4)

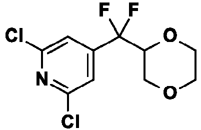

To a solution of intermediate E3 (7.0 g, 26.71 mmol) in DCM (150 mL) was added DAST (140.0 mL, 1060 mmol) and then the mixture was stirred at 20°C for 12 hrs. The reaction was extracted by EA (400 mL × 3) and the organic layer was washed with aq. NaHCO₃ (200 × 3 mL), brine (200 mL), dried over anhydrous Na₂SO₄, filtered and concentrated. The crude was purified by pre-HPLC (TFA as addictive) to give intermediate E4 (4 g, 14.08 mmol, 52.72% yield) as a white solid. MS obsd.: 284.0 (MH⁺). ¹H NMR (400 MHz, CDCl₃): δ ppm 7.35 (s, 2H ), 3.87 - 4.00 (m, 2H ), 3.79-3.81 (m, 1H), 3.62-3.71 (m, 2H), 3.53-3.60 (m, 2H).

### Step 5: 2,6-dichloro-4-[[(2S)-1,4-dioxan-2-yl]-difluoro-methyl]pyridine and 2,6-dichloro-4-[[(2R)-1,4-dioxan-2-yl]-difluoro-methyl]pyridine(intermediates E and F)

4.0 g of intermediate E4 was purified by SFC using the following condition to give intermediate E (1.48 g, 36.5% yield, 99% ee, faster eluting) and intermediate F (1.5 g, 37.5% yield, 99% ee, slower eluting) as white solids. SFC condition: Column: OJ (250mm×50mm, 10 um); 0.1% NH₃H₂O EtOH; begin B 15%, end B 15%; Flow Rate (ml/min): 180.

### Intermediate G

### Tert-butyl (2R)-2-[(2,6-dichloro-4-pyridyl)-difluoro-methyl]morpholine-4-carboxylate

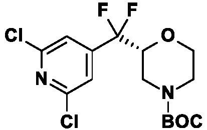

The title compound was prepared according to the following procedures:

### Step 1: tert-butyl (2R)-2-formylmorpholine-4-carboxylate (intermediate G1)

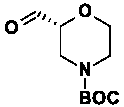

To a solution of (COCl)₂ (21.91 g, 172.6 mmol) in DCM (80 mL) was added DMSO (17.97 g, 230.14 mmol) at -60 °C and the mixture was stirred at -60 °C for 0.5 h under N₂. Then a solution of (*R*)-*N*-Boc-2-hydroxymethylmorpholine (25.0 g, 115.07 mmol) in DCM (50 mL) was added and the reaction was stirred at -60 °C for additional 1 h. DIPEA (59.49 g, 460.28 mmol) was added and the mixture was stirred at - 60 ~ -20 °C for 1 h and then at 0 °C for 1 h. The mixture was diluted with DCM (500 mL), washed with aq. CaCl₂ (100 mL) three times and brine (50 mL) two times, dried over anhydrous Na₂SO₄, filtered and concentrated to give the crude, which was purified by silica gel column (PE:EA=10:1 to 1:5) to give intermediate G1 (8 g, 37.17 mmol, 32.3% yield) as colorless oil. MS obsd.: 115.9 (MH⁺-100).

### Step 2: tert-butyl-(2R)-2-[(2,6-dichloro-4-pyridyl)-hydroxy-methyl]morpholine-4-carboxylate (intermediate G2)

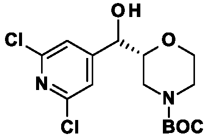

To a solution of 2,6-dichloro-4-iodopyridine (9.16 g, 33.45 mmol) in THF (30.0 ml) was added CH₃MgCl/LiCl in THF (28.3 mL, 36.79 mmol) at -40 °C under N₂ and the mixture was stirred at 25 °C for 1 h. Then intermediate G1 (7.2 g, 33.45 mmol) in THF (130 mL) was added at -40 °C and the mixture was stirred at 25 °C for 1 h. The mixture was poured into water (100 mL) and extracted with EA (600 mL). The organic layer was washed with brine (100 mL) two times, dried over anhydrous Na₂SO₄, filtered and concentrated. The crude was purified by column chromatography on silica gel (PE: EA = 100:1∼1:2) to give intermediate G2 (6 g, 16.52 mmol, 49.38% yield) as a white solid. MS obsd.: 363.3 (MH⁺).

### Step 3: tert-butyl-(2R)-2-(2,6-dichloropyridine-4-carbonyl)morpholine-4-carboxylate (intermediate G3)

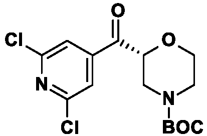

To a solution of intermediate G2 (5.5 g. 15.14 mmol) in THF (80 mL) was added DMP (7.06 g, 16.66 mmol) slowly at 0 °C under N₂ and then the mixture was stirred for 2 h. The mixture was poured into aq. K₂CO₃ (100 mL) at 0 °C and then extracted with EA (150 mL) three times. The organic layer was washed with aq. K₂CO₃ (50 mL) three times and brine (100 mL) two times, dried over anhydrous Na₂SO₄, filtered and concentrated to give intermediate G3 (5.3 g, 14.67 mmol, 96.9% yield) as a white solid. MS obsd.: 305.2 (MH⁺-56).

### Step 4: tert-butyl-(2R)-2-[(2,6-dichloro-4-pyridyl)-difluoro-methyl]morpholine-4-carboxylate (intermediate G)

A solution of intermediate G3 (5.0 g, 13.84 mmol) in DCM (8 mL) was added DAST (3.0 mL, 207.63 mmol) and stirred at 40°C for 3 hrs. The mixture was poured into aq. NaHCO₃ at 0 °C and then extracted with EA (200 mL) three times. The combined organic layer was washed with brine (100 mL) two times, dried over anhydrous Na₂SO₄, filtered and concentrated. The crude was purified by column chromatography on silica gel (PE: EA= 10:1~1:1) to give intermediate G (2.2 g, 5.74 mmol, 41.47% yield, 90% ee) as a white solid. MS obsd.: 327.3 (MH⁺-56).

### Intermediate H

### Tert-butyl-(2S)-2-[(2,6-dichloro-4-pyridyl)-difluoro-methyl]morpholine-4-carboxylate

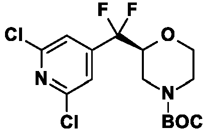

The title compound was prepared in analogy to the preparation of intermediate G by using (*S)-N*-Boc-2-hydroxymethylmorpholine instead of (*R*)-*N*-Boc-2-hydroxymethylmorpholine. Intermediate H (1.2 g, 95.5% ee) was obtained as a white solid. MS obsd.: 327.2 (MH⁺-56).

### Intermediate I

### Tert-butyl 4-[(2,6-dichloro-4-pyridyl)-difluoro-methyl]-3,6-dihydro-2H-pyridine-1-carboxylate

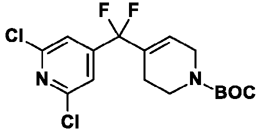

The title compound was prepared according to the following procedures:

### Step 1: tert-butyl 4-(methoxy(methyl)carbamoyl)-5,6-dihydropyridine-1(2H)-carboxylate (intermediate I1)

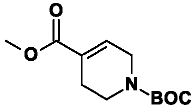

To a solution of 1-(*tert*-butoxycarbonyl)-1,2,3,6-tetrahydropyridine-4-carboxylic acid (4.5 g, 19.80 mmol), HATU (11.29 g, 29.70 mmol) and DIPEA (8.96 g, 69.30 mmol) in DMF (50 mL) was added N,O-dimethylhydroxylamine hydrochloride (4.83 g, 49.50 mmol). The reaction was stirred at 15 °C for 2 hrs. The mixture was poured into water (30 mL) and extracted by EA (40 mL) two times. The organic layer was washed by brine (50 mL) two times, dried over anhydrous Na₂SO₄, filtered and concentrated. The crude was purified by column chromatography on silica gel (PE: EA=1:0 to 5:1) to give intermediate I1 (4.7 g, yield: 87.80%) as a white solid. MS obsd.: 215.4 (MH⁺-56).

### Step 2: tert-butyl 4-(2,6-dichloropyridine-4-carbonyl)-3,6-dihydro-2H-pyridine-1-carboxylate (intermediate I2)

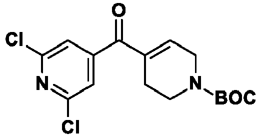

To a solution of 2,6-dichloro-4-iodopyridine (5.07 g, 18.5 mmol) in THF (15.0 ml) was added CH₃MgCl/LiCl in THF (14.23 mL, 18.5 mmol) at -40 °C under N₂. After stirred at 25 °C for 1 h, intermediate I1 (2.5 g, 9.25 mmol) in THF (25 mL) was added into the solution at -40 °C. The mixture was poured into water (100 mL) and extracted with EA (600 mL). The organic layer was washed with brine (100 mL) two times, dried over anhydrous Na₂SO₄, filtered and concentrated. The crude was purified by column chromatography on silica gel (PE: EA=100:1~1:1) to give intermediate I2 (1.5 g. 4.2 mmol, 45.4% yield) as light yellow oil. MS obsd.: 301.3 (MH⁺-56).

### Step 3: tert-butyl 4-[(2,6-dichloro-4-pyridyl)-difluoro-methyl]-3,6-dihydro-2H-pyridine-1-carboxylate(intermediate I)

A solution of intermediate I2 (1.5 g, 4.2 mmol) in DCM (3 mL) was added DAST (6.77 g, 41.99 mmol) and stirred at 40°C for 12 hrs. The mixture was poured into saturated NaHCO₃ (aq.) at 0 °C and then extracted with EA (100 mL) three times. The combined organic layer was washed with brine (500 mL) two times, dried over anhydrous Na₂SO₄, filtered and concentrated. The crude was purified by column chromatography on silica gel (PE: EA=10:1~1:1) to give intermediate I (1 g, 2.64 mmol, 62.8% yield) as a light yellow oil. MS obsd.: 323.2 (MH⁺-56).

### Intermediate J

### Tert-butyl 3-(2,6-dichloropyridine-4-carbonyl)piperidine-1-carboxylate

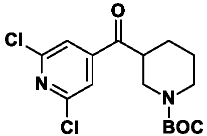

The title compound was prepared in analogy to the preparation of intermediate G3 by using *tert-*butyl 3-formylpiperidine-1-carboxylate instead of intermediate G1. Intermediate J (3.1 g) was obtained as yellow oil. MS obsd.: 359.0 (MH⁺).

### Intermediate K

### Tert-butyl 3-[(2,6-dichloro-4-pyridyl)-difluoro-methyl]azetidine-1-carboxylate

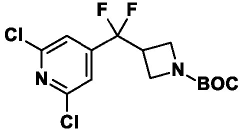

The title compound was prepared in analogy to the preparation of intermediate G by using *tert-*butyl 3-formylazetidine-1-carboxylate instead of intermediate G1. Intermediate K (1.1 g) was obtained as a yellow solid. MS obsd.: 353.1 (MH⁺).

### Example 1

### 4-Amino-1-[4-[4-[6-chloro-4-(trifluoromethyl)-2-pyridyl]piperazin-1-yl]sulfonylphenyl] pyrrolidin-2-one

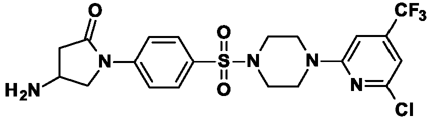

The title compound was prepared according to the following procedures:

### Step 1: tert-butyl N-[1-[4-[4-[6-chloro-4-(trifluoromethyl)-2-pyridyl]piperazin-1-yl]sulfonylphenyl]-5-oxo-pyrrolidin-3-yl]carbamate (compound 1a)

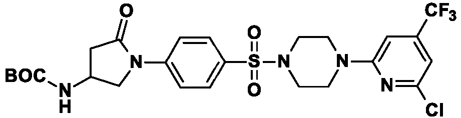

A mixture of intermediate A (100 mg, 188 µmol), *tert*-butyl (5-oxopyrrolidin-3-yl) carbamate (75.3 mg, 376 µmol), DMEDA (3.32 mg, 37.6 µmol), CuI (7.17 mg, 37.6 µmol) and Cs₂CO₃ (122 mg, 376 µmol) in DMF (5 mL) was heated at 75 °C for 1 h. The mixture was then poured into ice water, extracted with DCM (10 mL) three times, dried over anhydrous Na₂SO₄, filtered and concentrated. The crude was purified by column chromatography on silica gel (PE: EA= 5:1) to give compound 1a (97 mg, 85% yield) as a white solid. MS obsd.: 604.2 (MH⁺).

### Step 2: 4-Amino-1-[4-[4-[6-chloro-4-(trifluoromethyl)-2-pyridyl]piperazin-1-yllsulfonylphenyll pyrrolidin-2-one(Example 1)

A solution of compound 1a (100 mg, 166 µmol) in TFA (5 mL) was stirred at rt for 5 hrs. The mixture was concentrated and the crude was purified by prep-HPLC to give **Example 1** (10 mg, 12 % yield) as a white solid. MS obsd.: 504.2 (MH⁺). ¹H NMR (400 MHz, CD₃OD): δ ppm 7.78 - 7.89 (m, 2H), 7.64 - 7.75 (m, 2H), 6.81 (s, 1H), 6.72 (s, 1H), 4.29 (dd, *J* = 7.2, 11.2 Hz, 1H), 3.97 - 4.16 (m, 1H), 3.85 (dd, *J* = 2.4, 11.2 Hz, 1H), 3.56 - 3.70 (m, 4H), 3.09 (dd, *J =* 8.4, 18.0 Hz, 1H), 2.95 - 3.00 (m, 4H), 2.46 - 2.69 (m, 1H).

### Example 2

### 4-(Aminomethyl)-1-[4-[4-[6-chloro-4-(trifluoromethyl)-2-pyridyl]piperazin-1-yl]sulfonylphenyl]pyrrolidin-2-one

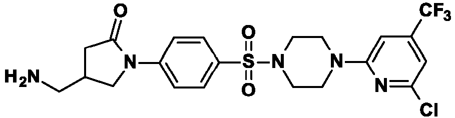

The title compound was prepared in analogy to the preparation of **Example 1** by using *tert-*butyl *N*-[(5-oxopyrrolidin-3-yl)methyl]carbamate instead of *tert-*butyl (5-oxopyrrolidin-3-yl)carbamate. **Example 2** (17.1 mg) was obtained as a white solid. MS obsd.: 518.0 (MH⁺). ¹H NMR (400 MHz, CD₃OD): δ ppm 7.75 - 7.790 (m, 2H), 7.68 - 7.73 (m, 2H), 6.81 (s, 1H), 6.73 (s, 1H), 4.02 (dd, *J =* 8.0, 9.6 Hz, 1H), 3.57 - 3.73 (m, 5H), 2.92 - 3.15 (m, 6H), 2.66 - 2.83 (m, 2H), 2.35 - 2.55 (m, 1H).

### Example 3

### 5-(Aminomethyl)-3-[4-[4-[6-chloro-4-(trifluoromethyl)-2-pyridyl]piperazin-1-yl]sulfonylphenyl]oxazolidin-2-one

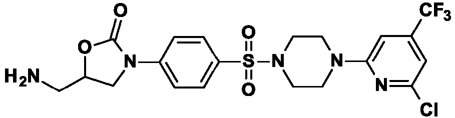

The title compound was prepared in analogy to the preparation of **Example 1** by using *tert-*butyl *N*-[(2-oxooxazolidin-5-yl)methyl]carbamate instead of *tert*-butyl (5-oxopyrrolidin-3-yl)carbamate. **Example 3** (30 mg) was obtained as a white solid. MS obsd.: 520.0 (MH⁺). ¹H NMR (400 MHz, DMSO-d₆): δ ppm 8.15 (brs, 3H), 7.53 - 7.96 (m, 4H), 7.10 (s, 1H), 6.98 (s, 1H), 4.81 - 5.06 (m, 1H), 4.24 (t, *J =* 9.2 Hz, 1H), 3.87 (dd, *J =* 6.4, 9.2 Hz, 1H), 3.63 - 3.77 (m, 4H), 3.27 (s, 2H), 2.96 (t, *J =* 4.8 Hz, 4H).

### Example 4

### N-[[3-[4-[4-[6-chloro-4-(trifluoromethyl)-2-pyridyl]piperazin-1-yl]sulfonylphenyl]-2-oxo-oxazolidin-5-yl]methyl]acetamide

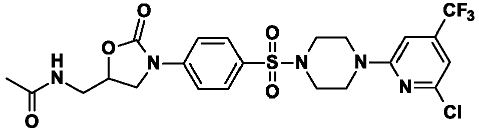

To a solution of **Example 3** (50 mg, 96.2 µmol) and DIPEA (62 mg, 481 µmol) in DCM (5 mL) was added acetyl chloride (15.1 mg, 192 µmol) dropwise at rt. The mixture was stirred at rt for 16 hrs. Solvent was evaporated and the crude was purified by prep-HPLC to give **Example 4** as a white solid (15 mg, 25 % yield). MS obsd.: 561.9 (MH⁺). ¹H NMR (400 MHz, DMSO-*d₆*): δ ppm 8.23 (t, *J =* 6.0 Hz, 1H), 7.78 (s, 4H), 7.09 (s, 1H), 6.97 (s, 1H), 4.58 - 4.86 (m, 1H), 4.16 (t, *J = 9.2* Hz, 1H), 3.78 (dd, *J =* 6.4, 9.12 Hz, 1H), 3.68 - 3.74 (m, 4H), 3.42 (t, *J =* 5.6 Hz, 2H), 2.96 (t, *J =* 4.8 Hz, 4H), 1.81 (s, 3H).

### Example 5

### 4-Amino-1-[4-[4-[6-chloro-4-(trifluoromethyl)-2-pyridyl]piperazin-1-yl]sulfonylphenyl]-3,3-dimethyl-pyrrolidin-2-one

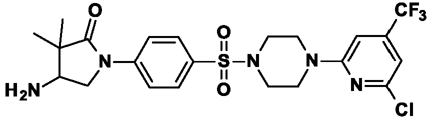

A mixture of intermediate A (0.060 g, 113 µmol), 4-amino-3,3-dimethylpyrrolidin-2-one (14.5 mg, 113 µmol), DMEDA (1.99 mg, 22.6 µmol), CuI (4.3 mg, 22.6 µmol) and K₃PO₄ (71.8 mg, 339 µmol) in DMF (1.5 mL) was heated at 80 °C for 1.5 hrs. The mixture was then poured into water, extracted with DCM. The organic layer was concentrated and the crude was purified by pre-HPLC to give **Example 5** as a white solid (40 mg, 64.4% yield). MS obsd.: 521.1 (MH⁺). ¹H NMR (400 MHz, DMSO-*d₆*): δ ppm 8.31(br s, 2H), 7.83 - 7.89 (m, 2H), 7.71 - 7.76 (m, 2H), 7.02 (s, 1H), 6.90 (s, 1H), 4.22 (dd, *J =* 6.4, 11.2 Hz, 1H), 3.61 - 3.80 (m, 6H), 2.90 (br t, *J* = 4.8 Hz, 4H), 1.16 (s, 3H), 1.11 (s, 3H).

### Example 6

### 4-(Aminomethyl)-3-[4-[4-[6-chloro-4-(trifluoromethyl)-2-pyridyl]piperazin-1-yl]sulfonylphenyl]oxazolidin-2-one

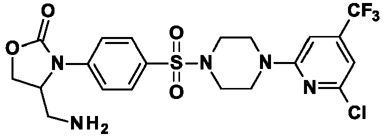

The title compound was prepared in analogy to the preparation of **Example 5** by using 4-(aminomethyl)oxazolidin-2-one instead of 4-amino-3,3-dimethylpyrrolidin-2-one. **Example 6** (10 mg) was obtained as a white solid. MS obsd.: 520.1 (MH⁺). ¹H NMR (400 MHz, CD₃OD): δ ppm 7.95 (br s, 3H), 7.84 - 7.90 (m, 2H), 7.78 - 7.83 (m, 2H), 7.10 (s, 1H), 6.98 (s, 1H), 4.81 - 4.98 (m, 1H), 4.53 - 4.60 (m, 1H), 4.45 - 4.51 (m, 1H), 3.66 - 3.68 (m, 4H), 3.11 - 3.22 (m, 1H), 2.91 -3.09 (m, 5H).

### Example 7

### (3S)-3-amino-1-[4-[4-[6-chloro-4-(trifluoromethyl)-2-pyridyl]piperazin-1-yl]sulfonylphenyl] azetidin-2-one

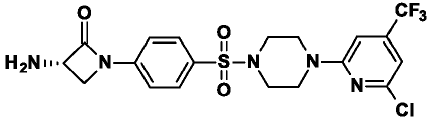

The title compound was prepared in analogy to the preparation of **Example 1** by using (3*S*)-3-aminoazetidin-2-one instead of *tert-*butyl (5-oxopyrrolidin-3-yl)carbamate. **Example 7** (10 mg) was obtained as a white solid. MS obsd.: 490.0 (MH⁺). ¹H NMR (400 MHz, CD₃OD): δ ppm 7.67 - 7.85 (m, 2H), 7.38 - 7.61 (m, 2H), 6.82 (s, 1H), 6.74 (s, 1H), 4.62 (dd, *J =* 2.8, 5.6 Hz, 1H), 4.07 (dd, *J =* 5.6, 7.2 Hz, 1H), 3.53 - 3.76 (m, 5H), 2.92 - 3.03 (m, 4H).

### Example 8

### 3-Amino-1-[4-[4-[6-chloro-4-(trifluoromethyl)-2-pyridyl]piperazin-1-yl]sulfonylphenyl] pyrrolidin-2-one

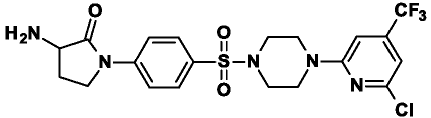

The title compound was prepared in analogy to the preparation of **Example 1** by using *tert-*butyl *N*-(2-oxopyrrolidin-3-yl)carbamate instead of *tert-butyl* (5-oxopyrrolidin-3-yl)carbamate. **Example 8** (20 mg) was obtained as a white solid. MS obsd.: 504.1 (MH⁺). ¹H NMR (400 MHz, CD₃OD): δ ppm 7.82 - 8.02 (m, 2H), 7.65 - 7.82 (m, 2H), 6.82 (s, 1H), 6.73 (s, 1H), 4.19 (dd, *J =* 8.8, 11.2 Hz, 1H), 3.82 - 4.04 (m, 2H), 3.59 - 3.68 (m, 4H), 2.91 - 3.04 (m, 4H), 2.51 - 2.69 (m, 1H), 1.95 - 2.19 (m, 1H).

### Example 9

### 5-(2-Aminoethyl)-3-[4-[4-[6-chloro-4-(trifluoromethyl)-2-pyridyl]piperazin-1-yl]sulfonylphenyl]oxazolidin-2-one

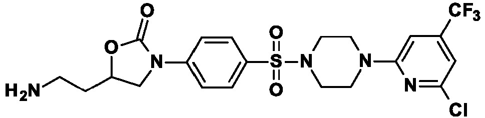

The title compound was prepared in analogy to the preparation of **Example *1*** by using *tert-*butyl *N*-[2-(2-oxooxazolidin-5-yl)ethyl]carbamate instead of *tert*-butyl (5-oxopyrrolidin-3-yl)carbamate. **Example 9** (66.4 mg) was obtained as a white solid. MS obsd.: 578.1 (MH⁺). ¹H NMR (400 MHz, DMSO-*d₆*): δ ppm 8.02 - 8.26 (m, 3 H), 7.74 - 7.84 (m, 4 H), 7.08 - 7.13 (m, 1 H), 6.95 - 7.00 (m, 1 H), 4.79 - 4.92 (m, 1 H), 4.18 - 4.29 (m, 1 H), 3.67 - 3.90 (m, 5 H), 2.86 - 3.07 (m, 6 H), 1.99 - 2.13 (m, 2 H).

### Example 10

### 2-[4-[4-[6-Chloro-4-(trifluoromethyl)-2-pyridyl]piperazin-1-yl]sulfonylphenyl]-2,6-diazaspiro[4.5]decan-3-one

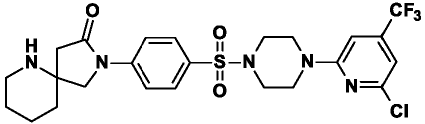

The title compound was prepared in analogy to the preparation of **Example 1** by using *tert-*butyl 3-oxo-2,6-diazaspiro[4.5]decane-6-carboxylate instead of *tert*-butyl (5-oxopyrrolidin-3-yl)carbamate. **Example 10** (10 mg) was obtained as a white solid. MS obsd.: 558.2 (MH⁺). ¹H NMR (400 MHz, CD₃OD): δ ppm 7.78 - 7.90 (m, 2H), 7.67 - 7.78 (m, 2H), 6.81 (s, 1H), 6.73 (s, 1H), 4.07 (s, 2H), 3.51 - 3.81 (m, 4H), 3.24 - 3.33 (m, 4H), 2.95 - 3.02 (m, 4H), 1.92 (s, 2H), 1.64 - 1.77 (m, 4H).

### Example 11

### 2-[4-[4-[6-Chloro-4-(trifluoromethyl)-2-pyridyl]piperazin-1-yl]sulfonylphenyl]-2,8-diazaspiro[4.5]decan-3-one

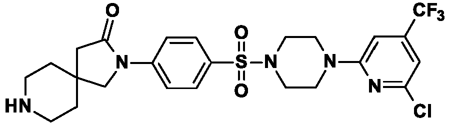

The title compound was prepared in analogy to the preparation of **Example 1** by using *tert-*butyl 3-oxo-2,8-diazaspiro[4.5]decane-8-carboxylate instead of *tert*-butyl (5-oxopyrrolidin-3-yl)carbamate. **Example 11** (15 mg) was obtained as a white solid. MS obsd.: 558.1 (MH⁺). ¹H NMR (400 MHz, CD₃OD): δ ppm 8.50 (br s, 2H), 7.93 (d, *J =* 8.8 Hz, 2H), 7.77 (d, *J =* 8.8 Hz, 2H), 7.09 (s, 1H), 6.97 (s, 1H), 3.79 (s, 2H), 3.66 - 3.75 (m, 4H), 3.03 - 3.22 (m, 4H), 2.96 (t, *J =* 4.8 Hz, 4H), 2.61 (s, 2H), 1.65 - 1.88 (m, 4H).

### Example 12

### 6-[4-[4-[6-Chloro-4-(trifluoromethyl)-2-pyridyl]piperazin-1-yl]sulfonylphenyl]-2,6-diazaspiro[3.4]octan-7-one

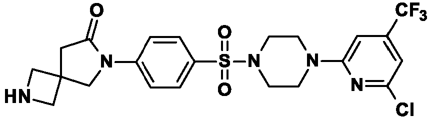

The title compound was prepared in analogy to the preparation of **Example 1** by using *tert-*butyl 6-oxo-2,7-diazaspiro[3.4]octane-2-carboxylate instead of *tert-*butyl (5-oxopyrrolidin-3-yl)carbamate. **Example 11** (10 mg) was obtained as a white solid. MS obsd.: 530.0 (MH⁺). ¹H NMR (400 MHz, CD₃OD): δ ppm 8.73 (br d, *J* = 1.6 Hz, 2H), 7.83 - 7.91 (m, 2H), 7.74 - 7.83 (m, 2H), 7.09 (s, 1H), 6.98 (s, 1H), 4.14 (s, 2H), 4.06 (td, *J* = 5.2, 10.8 Hz, 4H), 3.65 - 3.80 (m, 4H), 2.90 - 3.03 (m, 6H).

### Example 13

### 7-[4-[4-[6-Chloro-4-(trifluoromethyl)-2-pyridyl]piperazin-1-yl]sulfonylphenyl]-5-oxa-2,7-diazaspiro[3.4]octan-6-one

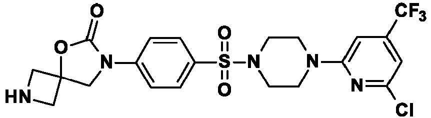

The title compound was prepared in analogy to the preparation of **Example 5** by using 5-oxa-2,7-diazaspiro[3.4]octan-6-one instead of 4-amino-3,3-dimethylpyrrolidin-2-one. **Example 13** (8 mg) was obtained as a white solid. MS obsd.: 532.2 (MH⁺). ¹H NMR (400 MHz, CD₃OD): δ ppm 7.80 - 7.87 (m, 2H), 7.71 - 7.79 (m, 2H), 7.10 (s, 1H), 6.98 (s, 1H), 4.19 - 4.45 (m, 6H), 3.71 (t, *J =* 5.2 Hz, 4H), 2.96 (br t, *J* = 4.8 Hz, 4H).

### Example 14

### 3-[4-[4-[6-Chloro-4-(trifluoromethyl)-2-pyridyl]piperazin-1-yl]sulfonylphenyl]-1-oxa-3,8-diazaspiro[4.5]decan-2-one

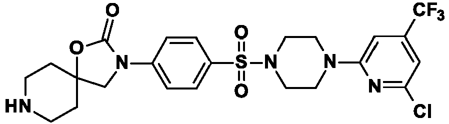

The title compound was prepared in analogy to the preparation of **Example 5** by using 1-oxa-3,8-diazaspiro[4.5]decan-2-one hydrochloride instead of 4-amino-3,3-dimethylpyrrolidin-2-one. **Example 14** (58 mg) was obtained as a white solid. MS obsd.: 560.1 (MH⁺). ¹H NMR (400 MHz, DMSO-*d₆*): δ ppm 7.66 - 7.80 (m, 4H), 6.97 - 7.04 (m, 1H), 6.84 - 6.93 (m, 1H), 3.81 - 3.87 (m, 2H), 3.62 - 3.66 (m, 4H), 2.86 - 2.91 (m, 4H), 2.72 - 2.81 (m, 2H), 2.59 - 2.67 (m, 2H), 1.59 - 1.80 (m, 4H).

### Example 15

### (3aR,6aS)-3-[4-[4-[6-chloro-4-(trifluoromethyl)-2-pyridyl]piperazin-1-yl]sulfonylphenyl]-4,5,6,6a-tetrahydro-3aH-pyrrolo[3,4-d]oxazol-2-one

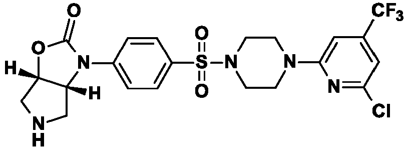

The title compound was prepared in analogy to the preparation of **Example 5** by using (3aR,6aS)-3,3a,4,5,6,6a-hexahydropyrrolo[3,4-d]oxazol-2-one instead of 4-amino-3,3-dimethylpyrrolidin-2-one. **Example 15** (10 mg) was obtained as a white solid. MS obsd.: 532.1 (MH⁺). ¹H NMR (400 MHz, CD₃OD): δ ppm 7.73 - 8.02 (m, 4H), 6.94 (s, 1H), 6.85 (s, 1H), 5.48 (dd, *J* = 4.8, 7.6 Hz, 1H), 5.36 - 5.43 (m, 1H), 3.90 (d, *J* = 13.2 Hz, 1H), 3.73 - 3.81 (m, 4H), 3.747 - 3.72 (m, 3H), 3.05 - 3.19 (m, 4H).

### Example 16

### (3aR,7aS)-3-[4-[4-[6-chloro-4-(trifluoromethyl)-2-pyridyl]piperazin-1-yl]sulfonylphenyl]-3a,4,5,6,7,7a-hexahydrooxazolo[4,5-c]pyridin-2-one

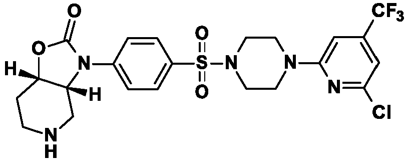

The title compound was prepared in analogy to the preparation of **Example 1** by using *tert-*butyl (3a*R*,7a*S*)-2-oxo-3,3a,4,6,7,7a-hexahydrooxazolo[4,5-c]pyridine-5-carboxylate instead of *tert*-butyl (5-oxopyrrolidin-3-yl)carbamate. **Example 16** (10 mg) was obtained as a white solid. MS obsd.: 546.1 (MH⁺). ¹H NMR (400 MHz, CD₃OD): δ ppm 7.74 (d, *J =* 2.4 Hz, 4H), 6.82 (s, 1H), 6.73 (s, 1H), 4.82 - 4.99 (m, 2H), 3.60 - 3.70 (m, 4H), 3.55 (dd, *J* = 4.4, 14.0 Hz, 1H), 3.10 - 3.31 (m, 3H), 2.91 - 3.04 (m, 4H), 2.16 - 2.30 (m, 2H).

### Example 17

### (3aR,7aS)-1-[4-[4-[6-chloro-4-(trifluoromethyl)-2-pyridyl]piperazin-1-yl]sulfonylphenyl]-3a,4,5,6,7,7a-hexahydrooxazolo[5,4-c]pyridin-2-one

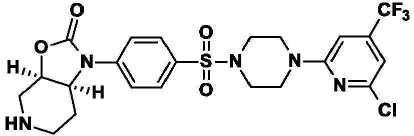

The title compound was prepared in analogy to the preparation of **Example** 1 by using *tert-*butyl (3aR,7aS)-2-oxo-1,3a,4,6,7,7a-hexahydrooxazolo[5,4-c]pyridine-5-carboxylate instead of *tert-*butyl (5-oxopyrrolidin-3-yl)carbamate. **Example 17** (33 mg) was obtained as a white solid. MS obsd.: 546.1(MH⁺). ¹H NMR (400 MHz, DMSO-*d₆*): δ ppm 9.09 - 9.67 (m, 2H), 7.83 - 7.89 (m, 2H), 7.76 - 7.82 (m, 2H), 7.09 (s, 1H), 6.97 (s, 1H), 4.98 (br d, *J* = 7.2 Hz, 1H), 4.78 - 4.89 (m, 1H), 3.61 - 3.677 (m, 5H), 3.39 - 3.45 (m. 1H), 3.06 - 3.14 (m, 1H), 2.98 (br t, *J* = 4.4 Hz, 5H), 2.30 - 2.41 (m, 1H), 1.66 - 1.78 (m, 1H).

### Example 18

### (3aS,7aR)-3-[4-[4-[6-chloro-4-(trifluoromethyl)-2-pyridyl]piperazin-1-yl]sulfonylphenyl]-3a,4,5,6,7,7a-hexahydrooxazolo[4,5-c]pyridin-2-one

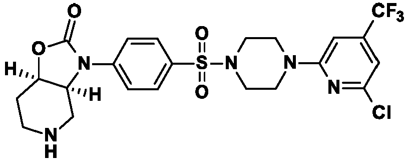

The title compound was prepared in analogy to the preparation of **Example 1** by using *tert-*butyl (3a*S*,7a*R*)-2-oxo-3,3a,4,6,7,7a-hexahydrooxazolo[4,5-c]pyridine-5-carboxylate instead of *tert-*butyl (5-oxopyrrolidin-3-yl)carbamate. **Example 18** (120 mg) was obtained as a white solid. MS obsd.: 546.0 (MH⁺). ¹H NMR (400 MHz, DMSO-*d₆*): δ ppm 9.19 - 9.31 (m, 2H), 7.80 (s, 4H), 7.10 (s, 1H), 6.98 (s, 1H), 4.09 - 5.02 (m, 2H), 3.68 - 3.78 (m, 4H), 3.49 - 3.56 (m, 1H), 3.11 - 3.22 (m, 2H), 3.02 - 3.09 (m, 1H), 2.99 (br s, 4H), 2.31 (br s, 1H), 2.09 - 2.020 (m, 1H).

### Example 19

### (3aS,8aR)-3-[4-[4-[6-chloro-4-(trifluoromethyl)-2-pyridyl]piperazin-1-yl]sulfonylphenyl]-4,5,6,7,8,8a-hexahydro-3aH-oxazolo[4,5-c]azepin-2-one

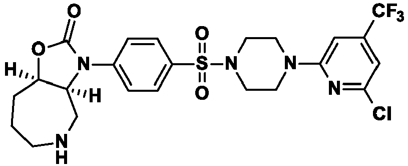

The title compound was prepared in analogy to the preparation of **Example 1** by using *tert-*butyl (3aS,8aR)-2-oxo-3a,4,6,7,8,8a-hexahydro-3H-oxazolo[4,5-c]azepine-5-carboxylate instead of *tert-*butyl (5-oxopyrrolidin-3-yl)carbamate. **Example 19** (10 mg) was obtained as a white solid. MS obsd.: 560.1(MH⁺). ¹H NMR (400 MHz, CD₃OD): δ ppm 7.67 - 7.85 (m, 4H), 6.82 (s, 1H), 6.74 (s, 1H), 4.97 - 5.12 (m, 2H), 3.59 - 3.72 (m, 4H), 3.41 - 3.454 (m, 1H), 3.24 - 3.30 (m, 1H), 3.13 - 3.19 (m, 2H), 2.92 - 3.07 (m, 4H), 2.20 (d, *J =* 4.4 Hz, 2H), 1.95 - 2.11 (m, 1H), 1.69 - 1.86 (m, 1H).

### Example 20

### 4-[4-[4-[6-Chloro-4-(trifluoromethyl)-2-pyridyl]piperazin-1-yl]sulfonylphenyl]-1,2,3,3a,6,6a-hexahydropyrrolo[3,2-b]pyrrol-5-one

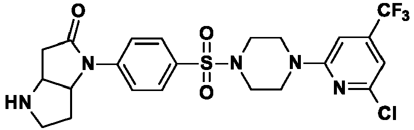

The title compound was prepared in analogy to the preparation of **Example 1** by using *tert-*butyl 5-oxo-2,3,3a,4,6,6a-hexahydropyrrolo[3,2-b]pyrrole-1-carboxylate instead of *tert*-butyl (5-oxopyrrolidin-3-yl)carbamate. **Example 20** (100 mg) was obtained as a white solid. MS obsd.: 530.2(MH⁺). ¹H NMR (400 MHz, DMSO-*d₆*): δ ppm 9.56 (br s, 2H), 7.84 - 7.93 (m, 2H), 7.74 - 7.84 (m, 2H), 7.09 (s, 1H), 6.92 - 7.01 (m, 1H), 5.02 - 5.16 (m, 1H), 4.32 - 4.47 (m, 1H), 3.68 - 3.73 (m, 4H), 3.20 - 3.29 (m, 2H), 2.99 (br d, *J =* 5.2 Hz, 6H), 2.29 (br s, 1H), 1.85 (br d, *J* = 7.2 Hz, 1H).

### Example 21

### 3-[4-[4-[6-Chloro-4-(trifluoromethyl)-2-pyridyl]piperazin-1-yl]sulfonylphenyl]-6-hydroxy-5-(hydroxymethyl)-3a,5,6,6a-tetrahydrofuro[2,3-d]oxazol-2-one

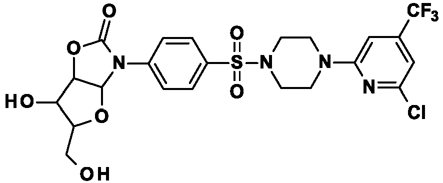

The title compound was prepared in analogy to the preparation of **Example 5** by using 6-hydroxy-5-(hydroxymethyl)-3a,5,6,6a-tetrahydro-3H-furo[2,3-d]oxazol-2-one instead of 4-amino-3,3-dimethylpyrrolidin-2-one. **Example 21** (5 mg) was obtained as a white solid. MS obsd.: 579.1 (MH⁺). ¹H NMR (400 MHz, CD₃OD): δ ppm 7.85 (d, *J* = 8.8 Hz, 2H), 7.71 (d, *J =* 8.8 Hz, 2H), 6.82 (s, 1H), 6.73 (s, 1H), 6.12 (d, *J =* 6.0 Hz, 1H), 4.84 (d, *J =* 5.2 Hz, 1H), 4.31 (d, *J =* 2.4 Hz, 1H), 4.02 (dt, *J =* 2.8, 5.2 Hz, 1H), 3.57 - 3.69 (m, 4H), 3.46 (d, *J* = 5.2 Hz, 2H), 2.91 - 3.05 (m, 4H).

### Example 22

### 3-[4-[4-[6-Chloro-4-(trifluoromethyl)-2-pyridyl]piperazin-1-yl]sulfonylphenyl]-5-[(3S,4R,5R)-3,4-dihydroxy-5-methoxy-tetrahydrofuran-2-yl]-4-methyl-oxazolidin-2-one

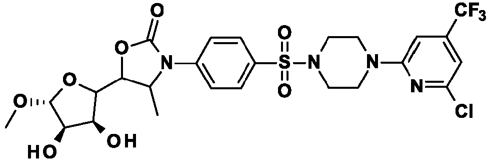

The title compound was prepared in analogy to the preparation of **Example 5** by using 5 [(3*S*,4*R*,5*R*)-3,4-dihydroxy-5-methoxy-tetrahydrofuran-2-yl]-4-methyl-oxazolidin-2-one instead of 4-amino-3,3-dimethylpyrrolidin-2-one. **Example 22** (20 mg) was obtained as a white solid. MS obsd.: 637.1(MH⁺). ¹H NMR (400 MHz, DMSO-d₆): δ ppm 7.92 (d, *J =* 8.8 Hz, 2H), 7.77 (d, *J =* 8.8 Hz, 2H), 7.09 (s, 1H), 6.97 (s, 1H), 4.94 - 5.38 (m, 1H), 4.76 - 4.89 (m, 2H), 4.74 (d, *J* = 1.2 Hz, 1H), 4.51 (dd, *J =* 7.32, 9.6 Hz, 1H), 4.17 (t, *J =* 5.2 Hz, 1H), 4.03 (dd, *J* = 5.2, 9.8 Hz, 1H), 3.83 (dd, *J* = 1.2, 4.8 Hz, 1H), 3.67 - 3.75 (m, 4H), 3.27 (s, 3H), 2.99 (t, *J* = 4.8 Hz, 4H), 1.26 (d, *J =* 6.4 Hz, 3H).

### Example 23

### (3aS,5S,6S,7S,7aR)-3-[4-[4-[6-chloro-4-(trifluoromethyl)-2-pyridyl]piperazin-1-yl]sulfonyl phenyl]-5,7-dihydroxy-6-(hydroxymethyl)-5,6,7,7a-tetrahydro-3aH-pyrano[2,3-d]oxazol-2-one

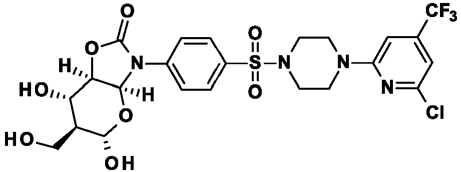

The title compound was prepared in analogy to the preparation of **Example 5** by using (3a*S*,5*S*,6*S*,7*S*,7a*R*)-5,7-dihydroxy-6-(hydroxymethyl)-3,3a,5,6,7,7a-hexahydropyrano[2,3-d] oxazol-2-one of 4-amino-3,3-dimethylpyrrolidin-2-one. **Example 23** (10 mg) was obtained as a white solid. MS obsd.: 609.2(MH⁺). ¹H NMR (400 MHz, DMSO-d₆): δ ppm 7.92 (d, *J =* 8.8 Hz, 2H), 7.78 (d, *J =* 8.8 Hz, 2H), 7.08 (s, 1H), 6.97 (s, 1H), 6.07 (d, *J =* 6.0 Hz, 1H), 5.61 (d, *J =* 5.2 Hz, 1H), 5.38 (d, *J* = 5.2 Hz, 1H), 4.81 (t, *J =* 5.6 Hz, 1H), 4.60 (dd, *J =* 4.4, 6.0 Hz, 1H), 3.80 - 3.87 (m, 1H), 3.67 - 3.75 (m, 4H), 3.57 - 3.63 (m, 2H), 3.44 - 3.54 (m, 2H), 2.93 - 3.04 (m, 4H).

### Example 24

### 8-(2-Aminoethyl)-3-[4-[4-[6-chloro-4-(trifluoromethyl)-2-pyridyl]piperazin-1-yl]sulfonylphenyl]-1-oxa-3,8-diazaspiro[4.5]decan-2-one

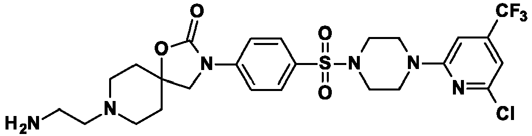

The title compound was prepared according to the following scheme:

### Step 1: tert-butyl N-[2-[3-[4-[4-[6-chloro-4-(trifluoromethyl)-2-pyridyl]piperazin-1-yl] sulfonylphenyl]-2-oxo-1-oxa-3,8-diazaspiro[4.5]decan-8-yl]ethyl]carbamate (compound 24a)

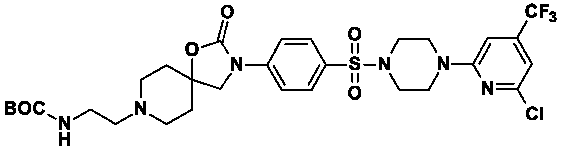

A mixture of **Example 11** (0.050 g, 89.3 µmol), *tert-butyl* (2-oxoethyl)carbamate (28.4 mg, 179 µmol) and NaBH(OAc)₃ (75.7 mg, 357 µmol) in DCM (1 mL) and was heated at 25 °C overnight. Then, the mixture was poured into water, extracted with DCM. The organic layer was concentrated to give the crude, which was used directly in next step. MS obsd.: 703.2 (MH⁺).

### Step 2: 8-(2-aminoethyl)-3-[4-[4-[6-chloro-4-(trifluoromethyl)-2-pyridyl]piperazin-1-yl]sulfonylphenyl]-1-oxa-3,8-diazaspiro[4.5]decan-2-one (Example 24)

A mixture of compound 24a (0.062 g, 88.2 µmol) and TFA (450 mg, 0.3mL, 3.95 mmol) in DCM (1 mL) was heated at 25 °C for 2 hrs. The mixture was concentrated and the crude was purified by HPLC to give **Example 24** (15 mg, 26.8% yield) as a white solid. MS obsd.: 603.1 (MH⁺). ¹H NMR (400 MHz, DMSO-*d₆*): δ ppm 7.77 - 7.84 (m, 4H), 7.06 - 7.11 (m, 1H), 6.93 - 7.00 (m, 1H), 3.97 - 4.04 (m, 2H), 3.70 - 3.74 (m, 4H), 3.50 - 3.56 (m, 4H), 3.07 - 3.22 (m, 4H), 2.94 - 2.99 (m, 4H), 1.90 - 2.23 (m, 4H).

### Example 25

### (3aS,8aR)-5-(2-aminoethyl)-3-[4-[4-[6-chloro-4-(trifluoromethyl)-2-pyridyl]piperazin-1-yl]sulfonylphenyl]-3a,4,6,7,8,8a-hexahydrooxazolo[4,5-c]azepin-2-one

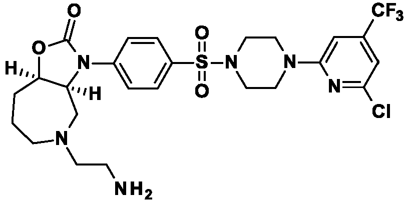

The title compound was prepared according to the following procedures:

### Step 1: tert-butyl N-[2-[(3aS,8aR)-3-[4-[4-[6-chloro-4-(trifluoromethyl)-2-pyridyl] piperazin-1-yl]sulfonylphenyl]-2-oxo-3a,4,6,7,8,8a-hexahydrooxazolo[4,5-c]azepin-5-yl]ethyl] carbamate (compound 25a)

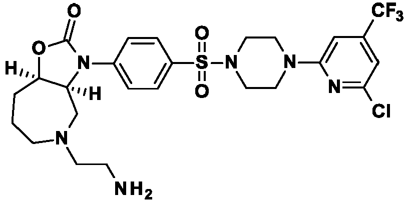

A mixture of **Example 19** (50mg, 74.2 µmol), *tert*-butyl (2-oxoethyl)carbamate (23.6 mg, 148 µmol) and NaBH(OAc)₃ (62.9 mg, 297 µmol) in DCM (2 mL) was stirred at 25 °C overnight. Then the mixture was poured into water, extracted with DCM. The organic layer was concentrated to give the crude, which was used directly in next step (52 mg, 100% yield). MS obsd.: 703.2 (MH⁺).

### Step 2: (3aS,8aR)-5-(2-aminoethyl)-3-[4-[4-[6-chloro-4-(trifluoromethyl)-2-pyridyl] piperazin-1-yl]sulfonylphenyl]-3a,4,6,7,8,8a-hexahydrooxazolo[4,5-clazepin-2-one (Example 25)

A solution of compound 24a (80 mg, 114 µmol) in TFA (5 mL) was stirred at rt for 2 hrs. The mixture was concentrated and the crude was purified by prep-HPLC to give **Example 25** (15 mg, 21.9 % yield). MS obsd.: 603.2 (MH⁺). ¹H NMR (400 MHz, CD₃OD): δ ppm 7.85 (s, 4H), 6.92 (s, 1H), 6.84 (s, 1H), 4.99 (td, *J =* 6.0, 8.8 Hz, 1H), 4.69 - 4.83 (m, 1H), 3.63 - 3.87 (m, 4H), 3.07 - 3.16 (m, 4H), 2.95 (dd, *J* = 6.8, 14.8 Hz, 1H), 2.55 - 2.84 (m, 7H), 2.08 - 2.20 (m, 2H), 1.90 - 2.04 (m, 1H), 1.54 - 1.72 (m, 1H).

### Example 26

### (3aS,8aR)-3-[4-[4-[6-chloro-4-(trifluoromethyl)-2-pyridyl]piperazin-1-yl]sulfonylphenyl]-5-[2-(methylamino)ethyl]-3a,4,6,7,8,8a-hexahydrooxazolo[4,5-c]azepin-2-one

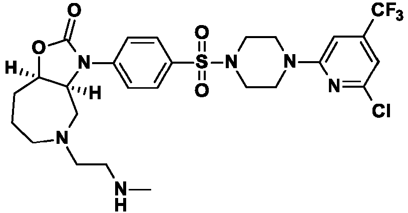

The title compound was prepared in analogy to the preparation of **Example 25** by using *tert-butyl* N-methyl-N-(2-oxoethyl)carbamate instead of *tert-butyl* (2-oxoethyl)carbamate. **Example 26** (19 mg) was obtained as a white solid. MS obsd.: 617.2 (MH⁺). ¹H NMR (400 MHz, CD₃OD): δ ppm 7.85 (s, 4H), 6.92 (s, 1H), 6.84 (s, 1H), 4.99 (td, *J =* 5.6, 8.8 Hz, 1H), 4.77 - 4.84 (m, 1H), 3.69 - 3.80 (m, 4H), 3.07 - 3.18 (m, 4H), 2.97 (dd, J = 6.6, 14.8 Hz, 1H), 2.73 - 2.82 (m, 3H), 2.65 - 2.72 (m, 4H), 2.60 (s, 3H), 2.08 - 2.19 (m, 2H), 1.90 - 2.04 (m, 1H), 1.58 - 1.70 (m, 1H).

### Example 27

### 3-[4-[4-[6-Chloro-4-(trifluoromethyl)-2-pyridyl]piperazin-1-yl]sulfonylphenyl]-5-(methylaminomethyl)oxazolidin-2-one

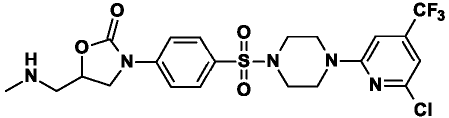

The title compound was prepared according to the following procedures:

### Step 1: 3-[4-[4-[6-chloro-4-(trifluoromethyl)-2-pyridyl]piperazin-1-yl]sulfonylphenyl]-5-(hydroxymethyl)oxazolidin-2-one (compound 27a)

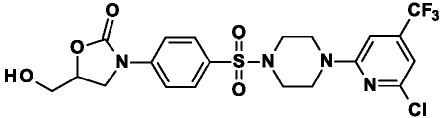

To a solution of intermediate A (3.4 g, 6.39 mmol), 5-(hydroxymethyl)oxazolidin-2-one (1.12 g, 9.59 mmol) and K₂CO₃ (2.65 g, 19.18 mmol) in NMP (42.5 mL) were added CuI (243.56 mg, 1.28 mmol) and trans-N,N'-dimethylcyclohexane-1,2-diamine (181.91 mg, 1.28 mmol) under N₂. The mixture was stirred at 80 °C for 5 hrs under N₂. The mixture was diluted with EA (400 mL) and filtered. The filtrate was washed with aq. CaCl₂ (100 mL) three times and brine (100 mL) twice, dried over anhydrous Na₂SO₄, filtered and concentrated. The crude was purified by column chromatography on silica gel (PE: EA= 2:1~1:5) to give compound 27a(2.7 g, 5.18 mmol, 81.1% yield) as a white solid. MS obsd.: 520.1 (MH⁺).

### Step 2: [3-[4-[4-[6-chloro-4-(trifluoromethyl)-2-pyridyl]piperazin-1-yl]sulfonylphenyl]-2-oxo-oxazolidin-5-yl]methyl 4-methylbenzenesulfonate (compound 27b)

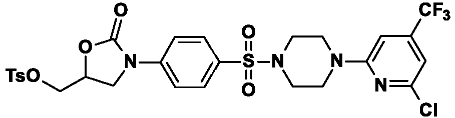

To a solution of compound 27a (1.4 g, 2.69 mmol), p-toluenesulfonic anhydride (1.75 g, 5.38 mmol) and DIPEA (1.04 g, 8.06 mmol) in THF (8 mL) were added DMAP (65.67 mg, 0.540 mmol) under nitrogen. The mixture was stirred at 45 °C for 12 hrs under N₂ and then diluted with EA (300 mL). The solution washed with aq. K₂CO₃ (100 mL), 1N aq. HCl (100 mL) twice and brine (100 mL) twice. The organic layer was dried over anhydrous Na₂SO₄, filtered and concentrated. The crude was purified by column chromatography on silica gel (PE: EA= 2:1-1:5) to give compound 27b (1.7 g, 2.52 mmol, 93.69% yield) as a white solid. MS obsd.: 675.2 (MH⁺).

### Step 3: 3-[4-[4-[6-chloro-4-(trifluoromethyl)-2-pyridyl]piperazin-1-yl]sulfonylphenyl]-5-(methylaminomethyl)oxazolidin-2-one (Example 27)

To a solution of compound 27b (120.0 mg, 0.18 mmol) and KI (88.5 mg, 0.53 mmol) in DMSO (1.5 mL) were added monomethylamine in THF (3.0 mL, 6 mmol) and 18-crown-6 (4.7 mg, 0.02 mmol) under N₂. The mixture was stirred at 60 °C for 3 hrs under N₂. The mixture was diluted with EA (200 mL) and filtered. The filtrate was washed with aq CaCl₂ (50 mL) three times and brine (100 mL) two times, dried over anhydrous Na₂SO₄, filtered and concentrated. The crude was purified by prep-HPLC to give **Example 27** (23.4 mg, 0.040 mmol, 22.64% yield) as a white solid. MS obsd.: 534.3 (MH⁺). ¹H NMR (400 MHz, DMSO-*d₆*): δ ppm 8.99 - 9.90 (m, 2 H), 7.75 - 7.84 (m, 4 H), 7.06 - 7.13 (m, 1 H), 6.94 - 6.99 (m, 1 H), 5.04 - 5.20 (m, 1 H), 4.20 - 4.34 (m, 1 H), 3.91 - 4.03 (m, 1 H), 3.61 - 3.79 (m, 4 H), 3.30 - 3.36 (m, 2 H), 2.86 - 3.06 (m, 4 H), 2.56 - 2.63 (m, 3 H).

### Example 28

### 3-[4-[4-[6-Chloro-4-(trifluoromethyl)-2-pyridyl]piperazin-1-yl]sulfonylphenyl]-5-[(dimethylamino)methyl]oxazolidin-2-one

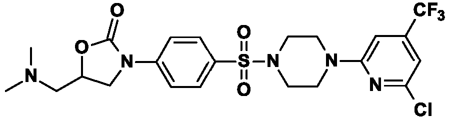

The title compound was prepared in analogy to the preparation of **Example 27** by using dimethylamine instead of monomethylamine. **Example 28** (17.1 mg) was obtained as a white solid. MS obsd.: 548.3 (MH⁺). ¹H NMR (400 MHz, DMSO-*d₆*): δ ppm 10.77 - 11.01 (m, 1 H), 7.73 - 7.86 (m, 4 H), 7.08 - 7.12 (m, 1 H), 6.95 - 7.00 (m, 1 H), 5.20 - 5.35 (m, 1 H), 4.21 - 4.35 (m, 1 H), 3.84 - 3.95 (m, 1 H), 3.50 - 3.75 (m, 6 H), 2.93 - 3.06 (m, 4 H), 2.80 - 2.90 (m, 6 H).

### Example 29

### 3-[4-[4-[6-Chloro-4-(trifluoromethyl)-2-pyridyl]piperazin-1-yl]sulfonylphenyl]-5-(piperazin-1-ylmethyl)oxazolidin-2-one

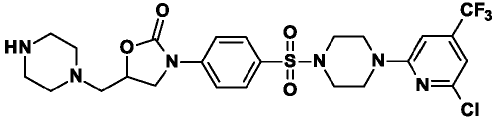

The title compound was prepared in analogy to the preparation of **Example 27** by using piperazine instead of monomethylamine. **Example 29** (49.9 mg) was obtained as a white solid. MS obsd.: 589.3 (MH⁺). ¹H NMR (400 MHz, DMSO-*d₆*): δ ppm 9.51 - 10.01 (m, 2 H), 7.72 - 7.90 (m, 4 H), 7.07 - 7.14 (m, 1 H), 6.93 - 7.00 (m, 1 H), 5.16 - 5.38 (m, 1 H), 4.24 - 4.34 (m, 1 H), 3.49 - 3.92 (m, 14 H), 2.90 - 3.02 (m, 4 H).

### Example 30

### 3-[4-[4-[6-Chloro-4-(trifluoromethyl)-2-pyridyl]piperazin-1-yl]sulfonylphenyl]-5-[[cis-3,4-dihydroxypyrrolidin-1-yl]methyl]oxazolidin-2-one

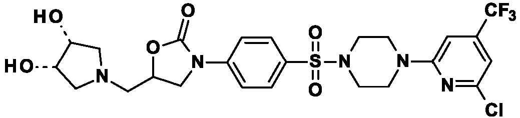

The title compound was prepared in analogy to the preparation of **Example 27** by using cis-pyrrolidine-3,4-diol hydrochloride instead of monomethylamine. **Example 30** (25.5 mg) was obtained as a white solid. MS obsd.: 606.2 (MH⁺). ¹H NMR (400 MHz, DMSO-*d₆*): δ ppm 10.69 - 11.44 (m, 1 H), 7.75 - 7.83 (m, 4 H), 7.08 - 7.13 (m, 1 H), 6.95 - 7.01 (m, 1 H), 5.09 - 5.71 (m, 3 H), 4.03 - 4.37 (m, 3 H), 3.59 - 3.94 (m, 8 H), 3.37 - 3.48 (m, 2 H), 2.92 - 3.18 (m, 5 H).

### Example 31

### 5-[(3-Aminoazetidin-1-yl)methyl]-3-[4-[4-[6-chloro-4-(trifluoromethyl)-2-pyridyl]piperazin-1-yl]sulfonylphenyl]oxazolidin-2-one

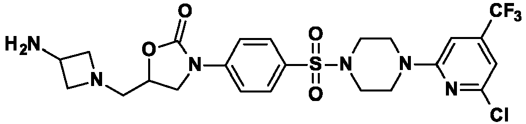

The title compound was prepared according to the following scheme:

### Step 1: tert-butyl N-[1-[[3-[4-[4-[6-chloro-4-(trifluoromethyl)-2-pyridyl]piperazin-1-yl]sulfonylphenyl]-2-oxo-oxazolidin-5-yl]methyl]azetidin-3-yl]carbamate(compound 31a)

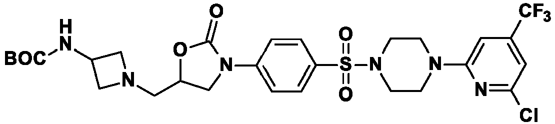

To a solution of compound 27b (220.0 mg, 0.330 mmol), 3-N-Boc-amino-azetidine (168.37 mg, 0.980 mmol) and KI (0.05 mL, 0.980 mmol) in ACN (5 mL) was added K₂CO₃ (135.11 mg, 0.980 mmol) under N₂. The mixture was stirred at 70 °C for 8 hrs. The mixture was diluted with EA (200 mL) and filtered. The filtrate was washed with brine (100 mL) two times, dried over anhydrous Na₂SO₄, filtered and concentrated. The crude was purified by prep-HPLC to give compound 31a (90 mg, 0.130 mmol, 40.91% yield) as a white solid. MS obsd.: 675.0 (MH⁺).

### Step 2: 5-[(3-aminoazetidin-1-yl)methyl]-3-[4-[4-[6-chloro-4-(trifluoromethyl)-2-pyridyl] piperazin-1-yl]sulfonylphenyl]oxazolidin-2-one (Example 31)

To a solution of compound 31a (90.0 mg, 0.130 mmol) in DCM (2 mL) was added TFA (1.0 mL, 12.98 mmol) and the reaction was stirred at 25°C for 2 hrs. The mixture was concentrated and the crude was purified by prep-HPLC to give **Example 31** (40 mg, 0.060 mmol, 43.06% yield) as a white solid. MS obsd.: 575.2 (MH⁺). ¹H NMR (400 MHz, DMSO-*d₆*): δ ppm 8.29 - 8.76 (m, 2 H), 7.74 - 7.86 (m, 4 H), 7.07 - 7.14 (m, 1 H), 6.94 - 7.02 (m, 1 H), 4.86 - 5.02 (m, 1 H), 3.83 - 4.31 (m, 9 H), 3.70 - 3.76 (m, 4 H), 2.89 - 3.02 (m, 4 H).

### Example 32

### 3-[4-[4-[6-Chloro-4-(trifluoromethyl)-2-pyridyl]piperazin-1-yl]sulfonylphenyl]-5-(2,6-diazaspiro[3.3]heptan-2-ylmethyl)oxazolidin-2-one

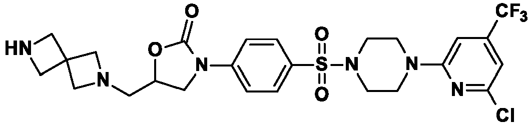

The title compound was prepared in analogy to the preparation of **Example 31** by using *tert-*butyl 2,6-diazaspiro[3.3]heptane-2-carboxylate instead of 3-*N*-Boc-amino-azetidine. **Example 32** (95.7 mg) was obtained as a white solid. MS obsd.: 601.2 (MH⁺). ¹H NMR (400 MHz, DMSO-*d₆*): δ ppm 8.84 - 9.07 (m, 2 H), 7.73 - 7.87 (m, 4 H), 7.07 - 7.12 (m, 1 H), 6.93 - 6.99 (m, 1 H), 4.89 - 5.03 (m, 1 H), 4.31 - 4.45 (m, 4 H), 4.11 - 4.28 (m, 5 H), 3.82 - 3.88 (m, 1 H), 3.70 - 3.73 (m, 4 H), 3.53 - 3.62 (m, 2 H), 2.89 - 3.03 (m, 4 H).

### Example 33

### 5-[[3-(Aminomethyl)azetidin-1-yl]methyl]-3-[4-[4-[6-chloro-4-(trifluoromethyl)-2-pyridyl]piperazin-1-yl]sulfonylphenyl]oxazolidin-2-one

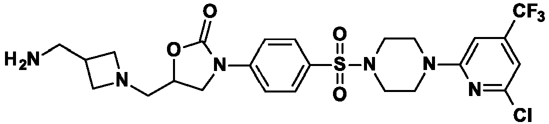

The title compound was prepared in analogy to the preparation of **Example 31** by using *tert-butyl* N-(azetidin-3-ylmethyl)carbamate instead of 3-*N*-Boc-amino-azetidine. **Example 33** (95.7 mg) was obtained as a white solid. MS obsd.: 589.2 (MH⁺). ⁺). ¹H NMR (400 MHz, DMSO-*d₆*): δ ppm 10.55 - 11.53 (m, 1 H), 7.96 - 8.22 (m, 3 H), 7.72 - 7.86 (m, 4 H), 7.06 - 7.14 (m, 1 H), 6.93 - 6.99 (m, 1 H), 4.89 - 5.07 (m. 1 H), 3.98 - 4.34 (m, 5 H), 3.82 - 3.90 (m, 1 H), 3.72 (br d, *J* =4.4 Hz, 4 H), 3.59 - 3.67 (m, 2 H), 2.90 - 3.26 (m, 7 H).

### Example 34

### 3-[4-[4-[6-Chloro-4-(trifluoromethyl)-2-pyridyl]piperazin-1-yl]sulfonylphenyl]-5-[(pyrrolidin-3-ylamino)methyl]oxazolidin-2-one

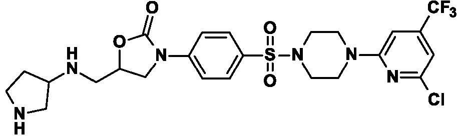

The title compound was prepared in analogy to the preparation of **Example 31** by using N-Boc-3-aminopyrrolidine instead of 3-*N*-Boc-amino-azetidine. **Example 34** (57.3 mg) was obtained as a white solid. MS obsd.: 589.2 (MH⁺). ¹H NMR (400 MHz, DMSO-*d₆*): δ ppm 8.96 - 9.61 (m, 2 H), 7.73 - 7.87 (m, 4 H), 7.06 - 7.14 (m, 1 H), 6.92 - 7.00 (m, 1 H), 4.93 - 5.11 (m, 1 H), 4.20 - 4.35 (m, 1 H), 3.85 - 3.96 (m, 2 H), 3.70 - 3.75 (m, 4 H), 3.56 (br dd, *J* =12.0, 7.6 Hz, 1 H), 3.22 - 3.48 (m, 5 H), 2.91 - 3.02 (m, 4 H).

### Example 35

### 4-[[3-(Aminomethyl)azetidin-1-yl]methyl]-1-[4-[4-[6-chloro-4-(trifluoromethyl)-2-pyridyl]piperazin-1-yl]sulfonylphenyl]pyrrolidin-2-one

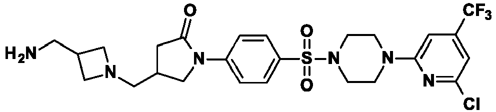

The title compound was prepared in analogy to the preparation of **Example 31** by using 4-(hydroxymethyl)pyrrolidin-2-one instead of 5-(hydroxymethyl)oxazolidin-2-one and *tert-butyl* N-(azetidin-3-ylmethyl)carbamate instead of 3-*N*-Boc-amino-azetidine. **Example 35** (15 mg) was obtained as a white solid. MS obsd.: 587.2 (MH⁺). ¹H NMR (400 MHz, DMSO-*d₆*): δ ppm 9.71 - 11.11 (m, 1H), 7.84 - 7.95 (m, 4H), 7.73 - 7.82 (m, 2H), 7.09 (s, 1H), 6.98 (s, 1H), 3.81 - 4.30 (m, 5H), 3.68 - 3.75 (m, 4H), 3.64 (dd, *J =* 6.4, 9.2 Hz, 1H), 3.16 - 3.23 (m, 1H), 3.01 - 3.15 (m, 2H), 2.96 (t, *J =* 4.8 Hz, 4H), 2.62 - 2.82 (m, 2H), 2.39 - 2.55 (m, 3H).

### Example 36

### 1-[4-[4-[6-Chloro-4-(trifluoromethyl)-2-pyridyl]piperazin-1-yl]sulfonylphenyl]-4-[(4-methylpiperazin-1-yl)methyl]pyrrolidin-2-one

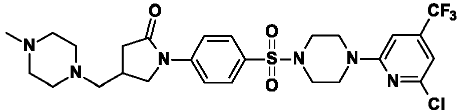

The title compound was prepared in analogy to the preparation of **Example 27** by using 4-(hydroxymethyl)pyrrolidin-2-one instead of 5-(hydroxymethyl)oxazolidin-2-one and 1-methylpiperazine instead of monomethylamine. **Example 36** (25 mg) was obtained as a white solid. MS obsd.: 600.9 (MH⁺). ¹H NMR (400 MHz, CD₃OD): δ ppm 7.88 - 7.93 (m, 2H), 7.75 - 7.83 (m, *J =* 9.2 Hz, 2H), 6.91 (s, 1H), 6.82 (s, 1H), 4.03 (dd, *J =* 7.6, 9.6 Hz, 1H), 3.67 - 3.77 (m, 5H), 3.01 - 3.0114 (m, 4H), 2.62 - 2.87 (m, 3H), 2.39 - 2.356 (m, 10H), 2.31 (s, 3H).

### Example 37

### 1-[4-[4-[6-Chloro-4-(trifluoromethyl)-2-pyridyl]piperazin-1-yl]sulfonylphenyl]-4-[[3-[(dimethylamino)methyl]azetidin-1-yl]methyl]pyrrolidin-2-one

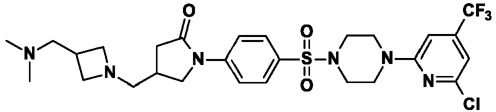

The title compound was prepared in analogy to the preparation of **Example 27** by using 4-(hydroxymethyl)pyrrolidin-2-one instead of 5-(hydroxymethyl)oxazolidin-2-one and 1-(azetidin-3-yl)-N,N-dimethyl-methanamine instead of monomethylamine. **Example 37** (25 mg) was obtained as a white solid. MS obsd.: 614.9 (MH⁺). ¹H NMR (400 MHz, CD₃OD): δ ppm 7.86 - 7.93 (m, 2H), 7.75 - 7.85 (m, 2H), 6.91 (s, 1H), 6.82 (s, 1H), 4.25 - 4.41 (m, 2H), 3.95 - 4.14 (m, 3H), 3.69 - 3.80 (m, 5H), 3.48 (dd, *J =* 1.6, 3.2 Hz, 2H), 3.34 - 3.42 (m, 2H), 3.11 - 3.16 (m, 1H), 3.03 - 3.10 (m, 4H), 2.88 (s, 6H), 2.72 - 2.86 (m, 2H), 2.42 - 2.55 (m, 1H).

### Example 38

### 4-[(Azetidin-3-ylmethylamino)methyl]-1-[4-[4-[6-chloro-4-(trifluoromethyl)-2-pyridyl]piperazin-1-yl]sulfonylphenyl]pyrrolidin-2-one

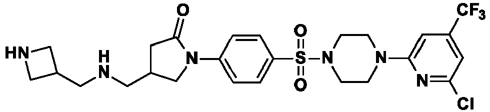

The title compound was prepared in analogy to the preparation of **Example 31** by using 4-(hydroxymethyl)pyrrolidin-2-one instead of 5-(hydroxymethyl)oxazolidin-2-one and *tert-butyl* 3-(aminomethyl)azetidine-1-carboxylate instead of 3-*N*-Boc-amino-azetidine. **Example 38** (23 mg) was obtained as a white solid. MS obsd.: 587.0 (MH⁺). ¹H NMR (400 MHz, CD₃OD): δ ppm 7.86 - 7.92 (m, 2H), 7.77 - 7.84 (m, 2H), 6.91 (s, 1H), 6.82 (s, 1H), 4.10 - 4.24 (m, 3H),3.99 - 4.07 (m, 2H), 3.67 - 3.83 (m, 5H), 3.37 - 3.39 (m, 3H), 3.16 - 3.27 (m, 2H), 3.04 - 3.11 (m, 4H), 2.81 - 2.94 (m, 2H), 2.47 - 2.60 (m, 1H).

### Example 39

### 4-[[3-(1-Aminocyclopropyl)azetidin-1-yl]methyl]-1-[4-[4-[6-chloro-4-(trifluoromethyl)-2-pyridyl]piperazin-1-yl]sulfonylphenyl]pyrrolidin-2-one

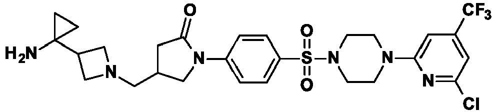

The title compound was prepared in analogy to the preparation of **Example 31** by using 4-(hydroxymethyl)pyrrolidin-2-one instead of 5-(hydroxymethyl)oxazolidin-2-one and *tert-butyl* N-[1-(azetidin-3-yl)cyclopropyl]carbamate instead of 3-*N*-Boc-amino-azetidine. **Example 39** (25 mg) was obtained as a white solid. MS obsd.: 612.9 (MH⁺). ¹H NMR (400 MHz, CD₃OD): δ ppm 7.87 - 7.94 (m, 2H), 7.76 - 7.82 (m, 2H), 6.91 (s, 1H), 6.82 (s, 1H), 4.00 (dd, *J* = 7.6, 9.8 Hz, 1H), 3.61 - 3.78 (m, 5H), 3.38 - 3.47 (m, 2H), 3.04 - 3.11 (m, 4H), 2.95 (t, *J =* 7.6 Hz, 2H), 2.72 (dd, *J* = 8.0, 16.8 Hz, 1H), 2.46 - 2.66 (m, 4H), 2.29 - 2.44 (m, 1H), 0.47 - 0.67 (m, 4H).

### Example 40

### 4-[(3-Aminoazetidin-1-yl)methyl]-1-[4-[4-[6-chloro-4-(trifluoromethyl)-2-pyridyl] piperazin-1-yl]sulfonylphenyl]pyrrolidin-2-one

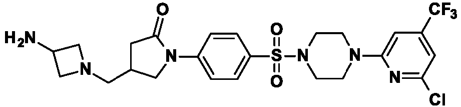

The title compound was prepared in analogy to the preparation of **Example 31** by using 4-(hydroxymethyl)pyrrolidin-2-one instead of 5-(hydroxymethyl)oxazolidin-2-one and *tert-butyl* N-(azetidin-3-yl)carbamate instead of 3-*N*-Boc-amino-azetidine. **Example 40** (25 mg) was obtained as a white solid. MS obsd.: 573.0 (MH⁺). ¹H NMR (400 MHz, CD₃OD): δ ppm 7.89 (d, *J* = 8.8 Hz, 2H), 7.74 - 7.83 (m, 2H), 6.91 (s, 1H), 6.82 (s, 1H), 4.00 (dd, *J* = 7.6, 9.6 Hz, 1H), 3.62 - 3.77 (m, 7H), 3.57 (quin, J = 6.8 Hz, 1H), 3.03 - 3.11 (m, 4H), 2.85 (dt, *J* = 4.0, 7.2 Hz, 2H), 2.50 - 2.77 (m, 4H), 2.33 - 2.45 (m, 1H).

### Example 41

### 1-[4-[4-[6-Chloro-4-(trifluoromethyl)-2-pyridyl]piperazin-1-yl]sulfonylphenyl]-4-(piperazin-1-ylmethyl)pyrrolidin-2-one

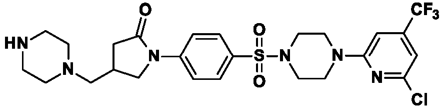

The title compound was prepared in analogy to the preparation of **Example 31** by using 4-(hydroxymethyl)pyrrolidin-2-one instead of 5-(hydroxymethyl)oxazolidin-2-one and *tert-butyl* piperazine-1-carboxylate instead of 3-*N*-Boc-amino-azetidine. **Example 41** (25 mg) was obtained as a white solid. MS obsd.: 587.0 (MH⁺). ¹H NMR (400 MHz, CD₃OD): δ ppm 7.88 - 7.94 (m, 2H), 7.80 (d, *J =* 8.8 Hz, 2H), 6.91 (s. 1H), 6.82 (s, 1H), 4.05 (dd, *J* = 7.6, 9.8 Hz, 1H), 3.68 - 3.77 (m, 5H), 3.23 (t, *J =* 5.2 Hz, 4H), 3.04 - 3.11 (m, 4H), 2.65 - 2.85 (m, 6H), 2.57 (d, *J* = 3.2 Hz, 2H), 2.38 - 2.50 (m, 1H).

### Example 42

### 1-[4-[4-[6-Chloro-4-(trifluoromethyl)-2-pyridyl]piperazin-1-yl]sulfonylphenyl]-4-[[3-(dimethylamino)azetidin-1-yl]methyl]pyrrolidin-2-one

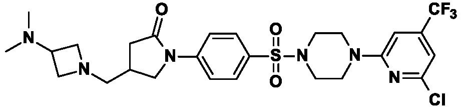

The title compound was prepared in analogy to the preparation of **Example 27** by using 4-(hydroxymethyl)pyrrolidin-2-one instead of 5-(hydroxymethyl)oxazolidin-2-one and *N,N-*dimethylazetidin-3-amine instead of monomethylamine. **Example 42** (25 mg) was obtained as a white solid. MS obsd.: 600.1 (MH⁺). ¹H NMR (400 MHz, CD₃OD): δ ppm 7.86 - 7.892 (m, 2H), 7.77 - 7.84 (m, 2H), 6.91 (s, 1H), 6.82 (s, 1H), 4.06 (t, *J =* 8.8 Hz, 3H), 3.82 (d, *J =* 2.8 Hz, 3H), 3.67 - 3.76 (m, 5H), 3.11 - 3.18 (m, 1H), 3.00 - 3.11 (m, 5H), 2.63 - 2.85 (m, 8H), 2.41 - 2.55 (m, 1H).

### Example 43

### 1-[4-[4-[6-Chloro-4-(trifluoromethyl)-2-pyridyl]piperazin-1-yl]sulfonylphenyl]-4-[[3-(methylamino)azetidin-1-yl]methyl]pyrrolidin-2-one

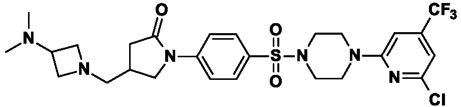

The title compound was prepared in analogy to the preparation of **Example 31** by using 4-(hydroxymethyl)pyrrolidin-2-one instead of 5-(hydroxymethyl)oxazolidin-2-one and *tert-butyl* N-(azetidin-3-yl)-N-methyl-carbamate instead of 3-*N*-Boc-amino-azetidine. **Example 43** (25 mg) was obtained as a white solid. MS obsd.: 587.0 (MH⁺). ¹H NMR (400 MHz, CD₃OD): δ ppm 7.86 - 7.93 (m, 2H), 7.75 - 7.84 (m, 2H), 6.91 (s, 1H), 6.82 (s, 1H), 3.98 - 4.15 (m, 4H), 3.80 - 3.97 (m, 2H), 3.68 - 3.78 (m, 5H), 2.98 - 3.12 (m, 6H), 2.74 - 2.85 (m, 1H), 2.70 (s, 4H), 2.41 - 2.53 (m, 1H).

### Example 44

### 1-[4-[4-[6-chloro-4-(trifluoromethyl)-2-pyridyl]piperazin-1-yl]sulfonylphenyl]-4-(methylamino)pyrrolidin-2-one

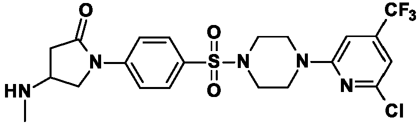

To a solution of intermediate B (100.0 mg, 0.210 mmol) in THF (3 mL) was added methylamine (1.03 mL, 2.05 mmol) and the mixture was heated to 90 °C for 1 h. The mixture was concentrated and the crude was purified by prep-HPLC to give **Example 44** (25 mg, 0.050 mmol, 23.2% yield) as a white solid. MS obsd.: 518.3 (MH⁺). ¹H NMR (400 MHz, DMSO-*d₆*): δ ppm 9.42 - 9.61 (m, 2H), 7.91 (s, 2H), 7.79 (d, *J =* 9.2 Hz, 2H), 7.09 (s, 1H), 6.97 (s, 1H), 4.21 - 4.28 (m, 1H), 4.04 - 4.10 (m, 1H), 3.95 - 4.03 (m, 1H), 3.71 (br s, 4H), 3.01 - 3.09 (m, 1H), 2.97 (br s, 4H), 2.80 - 2.88 (m, 1H), 2.61 (br s, 3H).

### Example 45

### 4-(2-Aminoethylamino)-1-[4-[4-[6-chloro-4-(trifluoromethyl)-2-pyridyl]piperazin-1-yl]sulfonylphenyl]pyrrolidin-2-one

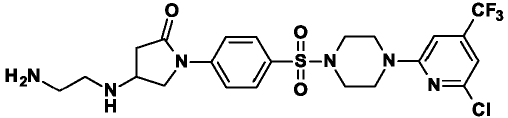

The title compound was prepared in analogy to the preparation of **Example 44** by using ethylenediamine instead of monomethylamine. **Example 45** (18 mg) was obtained as a white solid. MS obsd.: 487.0 (MH⁺). ¹H NMR (400 MHz, DMSO-*d₆*): δ ppm 10.21 - 10.97 (m, 1 H), 7.58 - 8.52 (m, 4 H), 6.73 - 7.31 (m, 2 H), 3.59 - 4.13 (m, 4 H), 2.91 - 3.27 (m, 3 H), 1.97 - 2.24 (m, 3 H).

### Example 46

### 1-[4-[4-[6-Chloro-4-(trifluoromethyl)-2-pyridyl]piperazin-1-yl]sulfonylphenyl]-4-(2-hydroxyethylamino)pyrrolidin-2-one

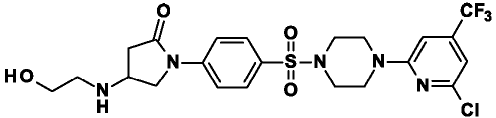

The title compound was prepared in analogy to the preparation of **Example 44** by using hydroxyethylamine instead of monomethylamine. **Example 46** (35 mg) was obtained as a white solid. MS obsd.: 548.2 (MH⁺). ¹H NMR (400 MHz, DMSO-*d₆*): δ ppm 9.31 - 9.47 (m, 2H), 7.90 (s, 2H), 7.81 (s, 2H), 7.09 (s, 1H), 6.97 (s, 1H), 5.27 - 5.38 (m, 1H), 4.21 - 4.29 (m, 1H), 4.03 - 4.15 (m, 2H), 3.66 - 3.74 (m, 6H), 3.08 - 3.13 (m, 2H), 3.01 - 3.07 (m, 1H), 2.97 (br t, *J =* 4.8 Hz, 4H), 2.82 - 2.90 (m, 1H).

### Example 47

### 1-[4-[4-[6-Chloro-4-(trifluoromethyl)-2-pyridyl]piperazin-1-yl]sulfonylphenyl]-4-piperazin-1-yl-pyrrolidin-2-one

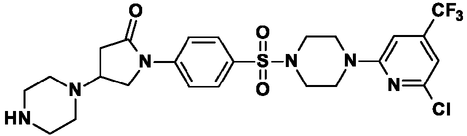

The title compound was prepared in analogy to the preparation of **Example 44** by using piperazine instead of monomethylamine. **Example 47** (11 mg) was obtained as a white solid. MS obsd.: 573.1 (MH⁺). ¹H NMR (400 MHz, DMSO-*d₆*): δ ppm 9.33 - 9.50 (m, 1H), 7.90 - 7.96 (m, 2H), 7.80 (s, 2H), 7.08 (s, 1H), 6.97 (s, 1H), 4.16 - 4.25 (m, 1H), 4.00 - 4.10 (m, 1H), 3.80 - 3.94 (m, 2H), 3.71 (br s, 4H), 3.29 - 3.35 (m, 4H), 3.08 - 3.24 (m, 4H), 3.00 - 3.01 (m, 1H), 2.95 - 2.99 (m, 4H), 2.73 - 2.92 (m, 2H).

### Example 48

### 1-[4-[4-[6-Chloro-4-(trifluoromethyl)-2-pyridyl]piperazin-1-yl]sulfonylphenyl]-4-[(3S,4R)-3,4-dihydroxypyrrolidin-1-yl]pyrrolidin-2-one

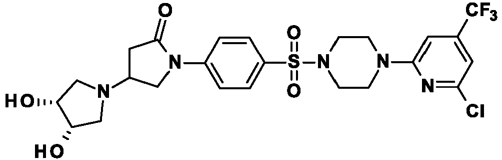

The title compound was prepared in analogy to the preparation of **Example 44** by using - **[(3S,4R)**-pyrrolidine-3,4-diol instead of monomethylamine. **Example 48** (45 mg) was obtained as a white solid. MS obsd.: 590.0 (MH⁺). ¹H NMR (400 MHz, DMSO-*d₆*): δ ppm 7.90 - 7.97 (m, 2H), 7.70 - 7.78 (m, 2H), 7.08 (s, 1H), 6.93 - 6.99 (m, 1H), 4.56 - 4.63 (m, 2H), 3.86 - 3.99 (m, 3H), 3.66 - 3.75 (m, 5H), 3.14 - 3.23 (m, 1H), 2.87 - 3.00 (m, 6H), 2.68 - 2.75 (m, 1H), 2.52 (br s, 1H), 2.33 - 2.40 (m, 2H).

### Example 49

### 4-[3-(Aminomethyl)azetidin-1-yl]-1-[4-[4-[6-chloro-4-(trifluoromethyl)-2-pyridyl]piperazin-1-yl]sulfonylphenyl]pyrrolidin-2-one

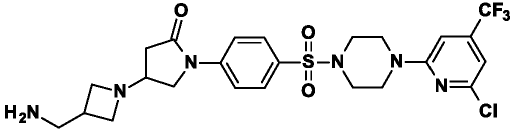

The title compound was prepared according to the following scheme:

### Step 1: tert-butyl N-[1-[1-[4-[4-[6-chloro-4-(trifluoromethyl)-2-pyridyl]piperazin-1-yl]sulfonylphenyl]-5-oxo-pyrrolidin-3-yl]azetidin-3-yl]carbamate (compound 49a)

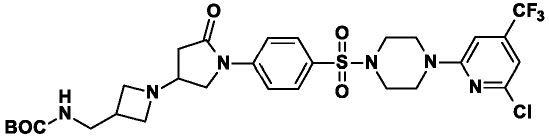

To a solution of intermediate B (80.0 mg. 0.16 mmol) in DMF (1 mL) was added *tert-butyl* N-(azetidin-3-ylmethyl)carbamate (84.89 mg, 0.49 mmol) and the mixture was heated to 90 °C for 1 h. The mixture was diluted with water (20 mL) and then extracted with EA (10 mL) three times. The combined organic layer was washed with brine (50 mL), dried over anhydrous Na₂SO₄, filtered, and concentrated. The crude was purified by prep-HPLC to give compound 49a (85 mg, 0.130 mmol, 78.49% yield) as a white solid. MS obsd.: 673.2 (MH⁺).

### Step 2: 4-[3-(aminomethyl)azetidin-1-yl]-1-[4-[4-[6-chloro-4-(trifluoromethyl)-2-pyridyl] piperazin-1-yl]sulfonylphenyl]pyrrolidin-2-one (Example 49)

To a solution of compound 49a (80.0 mg. 0.12 mmol) in DCM (1 mL) was added TFA (0.5 mL, 6.49 mmol) in one portion. The mixture was stirred at 20 °C for 1 h and then concentrated. The crude was purified by prep-HPLC to give **Example 49** (45 mg, 0.070 mmol, 58.2% yield) as a white solid. MS obsd.: 573.1 (MH⁺).¹H NMR (400 MHz, DMSO-*d₆*): δ ppm 8.11 - 8.16 (m, 1H), 7.89 - 7.94 (m, 2H), 7.73 - 7.77 (m, 2H), 7.08 (s, 1H), 6.95 - 6.98 (m, 1H), 3.90 - 4.24 (m, 2H), 3.69 - 3.73 (m, 4H), 3.58 - 3.66 (m, 1H). 3.37 - 3.42 (m, 4H), 3.01 - 3.07 (m, 2H), 2.94 - 2.97 (m, 4H), 2.51 - 2.53 (m, 2H).

### Example 50

### 4-(3-Aminopyrrolidin-1-yl)-1-[4-[4-[6-chloro-4-(trifluoromethyl)-2-pyridyl]piperazin-1-yl]sulfonylphenyl]pyrrolidin-2-one

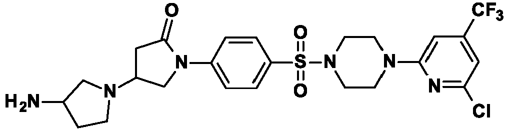

The title compound was prepared in analogy to the preparation of **Example 49** by using *tert-butyl N*-[pyrrolidin-3-yl]carbamate instead of *tert-butyl* N-(azetidin-3-ylmethyl)carbamate. **Example 50** (16 mg) was obtained as a white solid. MS obsd.: 573.3 (MH⁺). ¹H NMR (400 MHz, DMSO-*d₆*): δ ppm 8.49 - 8.73 (m, 3H), 8.48 - 8.49 (m, 1H), 7.91 - 7.97 (m, 2H), 7.77 - 7.82 (m, 2H), 7.05 - 7.12 (m, 1H), 6.95 - 6.99 (m, 1H). 4.25 - 4.45 (m, 2H), 4.12 - 4.24 (m, 1H), 3.79 - 4.10 (m, 2H), 3.71 (br s, 4H), 3.51 - 3.59 (m, 1H), 3.03 - 3.18 (m, 2H), 2.98 (br s, 4H), 2.33 (br d, *J =* 1.6 Hz, 2H).

### Example 51

### 4-(3-Aminoazetidin-1-yl)-1-[4-[4-[6-chloro-4-(trifluoromethyl)-2-pyridyl]piperazin-1-yl]sulfonylphenyl]pyrrolidin-2-one

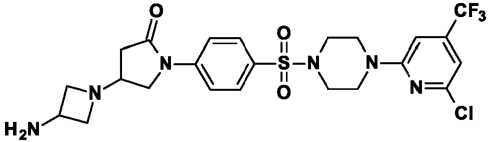

The title compound was prepared in analogy to the preparation of **Example 49** by using *tert-butyl N*-(azetidin-3-yl)carbamate instead of *tert-butyl* N-(azetidin-3-ylmethyl)carbamate. **Example 51** (21 mg) was obtained as a white solid. MS obsd.: 559.1 (MH⁺). ¹H NMR (400 MHz, DMSO-*d₆*): δ ppm 8.24 - 8.30 (m, 1H), 7.90 (s, 2H), 7.73 (d, *J* = 8.8 Hz, 2H), 7.08 (s, 1H), 6.96 (s, 1H), 3.89 (s, 1H), 3.69 - 3.72 (m, 4H), 3.50 - 3.52 (m, 4H), 3.05 - 3.08 (m, 1H), 2.96 (br t, *J =* 4.8 Hz, 4H), 2.67 - 2.75 (m, 3H), 2.16 - 2.22 (m, 1H).

### Example 52

### 1-[4-[4-[6-Chloro-4-(trifluoromethyl)-2-pyridyl]piperazin-1-yl]sulfonylphenyl]-4-(2,6-diazaspiro[3.3]heptan-2-yl)pyrrolidin-2-one

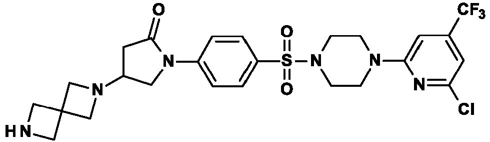

The title compound was prepared in analogy to the preparation of **Example 49** by using *tert-butyl* 2,6-diazaspiro[3.3]heptane-2-carboxylate instead of *tert-butyl* N-(azetidin-3-ylmethyl) carbamate. **Example 52** (26 mg) was obtained as a white solid. MS obsd.: 585.3 (MH⁺). ¹H NMR (400 MHz, DMSO-*d₆*): δ ppm 8.68 - 8.81 (m, 2H), 7.87 (s, 2H), 7.78 (d, *J =* 8.8 Hz, 2H), 7.09 (s, 1H), 6.97 (s, 1H), 4.16 - 4.40 (m, 4H), 4.12 (br s, 4H), 3.78 - 3.84 (m, 1H), 3.69 - 3.74 (m, 4H), 2.98 - 3.05 (m, 1H), 2.96 (br s, 4H), 2.52 - 2.61 (m, 2H).

### Example 53

### 1-[4-[4-[6-Chloro-4-(trifluoromethyl)-2-pyridyl]piperazin-1-yl]sulfonylphenyl]-4-[(3S)-3-(hydroxymethyl)piperazin-1-yl]pyrrolidin-2-one

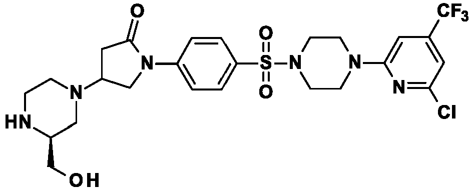

The title compound was prepared in analogy to the preparation of **Example 49** by using *tert-butyl* (2*S*)-2-(hydroxymethyl)piperazine-1-carboxylate instead of *tert-butyl N*-(azetidin-3-ylmethyl)carbamate. **Example 53** (35 mg) was obtained as a white solid. MS obsd.: 603.3 (MH⁺). ¹H NMR (400 MHz, DMSO-*d₆*): δ ppm 9.34 - 9.58 (m, 1H), 8.93 - 9.26 (m, 1H), 7.93 (d, *J* = 8.8 Hz, 2H), 7.78 (d, *J* = 8.8 Hz, 2H), 7.04 - 7.10 (m, 1H), 6.97 (s, 1H), 4.11 - 4.21 (m, 1H), 3.92 - 4.02 (m, 1H), 3.76 - 3.64 (m, 7H), 3.55 - 3.63 (m, 4H), 3.06 - 3.21 (m, 2H), 2.97 (br t, *J* = 4.4 Hz, 4H), 2.85 - 2.92 (m, 1H), 2.70 - 2.84 (m, 2H), 2.54 - 2.69 (m, 1H).

### Example 54

### 1-[4-[4-[6-Chloro-4-(trifluoromethyl)-2-pyridyl]piperazin-1-yl]sulfonylphenyl]-4-[(3R)-3-(hydroxymethyl)piperazin-1-yl]pyrrolidin-2-one

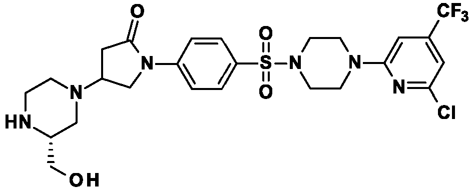

The title compound was prepared in analogy to the preparation of **Example 49** by using *tert-butyl* (2*R*)-2-(hydroxymethyl)piperazine-1-carboxylate instead of *tert-butyl N*-(azetidin-3-ylmethyl)carbamate. **Example 54** (65 mg) was obtained as a white solid. MS obsd.: 603.1 (MH⁺). ¹H NMR (400 MHz, DMSO-*d₆*): δ ppm 9.57 - 9.69 (m, 1H), 9.18 - 9.38 (m, 1H), 7.93 (s, 2H), 7.79 (d, *J* = 8.8 Hz, 2H), 7.09 (s, 1H), 6.97 (s. 1H), 4.18 (br s, 1H), 3.99 - 4.06 (m, 1H), 3.67 - 3.77 (m, 7H), 3.58 - 3.66 (m, 4H), 3.14 - 3.25 (m, 2H), 2.98 (br t, *J =* 4.4 Hz, 4H), 2.70 - 2.94 (m, 4H).

### Example 55

### 1-[4-[4-[6-Chloro-4-(trifluoromethyl)-2-pyridyl]piperazin-1-yl]sulfonylphenyl]-4-[(1S,4S)-2,5-diazabicyclo[2.2.1]heptan-2-yl]pyrrolidin-2-one

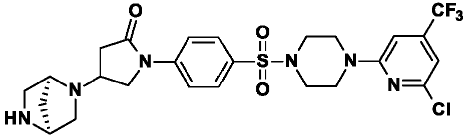

The title compound was prepared in analogy to the preparation of **Example 49** by using *(1S,* 4*S*)-2-Boc-2,5-diazabicyclo[2.2.1]heptane instead of *tert-butyl N*-(azetidin-3-ylmethyl)carbamate. **Example 55** (22 mg) was obtained as a white solid. MS obsd.: 589.0 (MH⁺). ¹H NMR (400 MHz, DMSO-*d₆*): δ ppm 9.31 - 10.01 (m, 2H), 7.93 (d, *J* = 8.8 Hz, 2H), 7.79 (d, *J =* 8.8 Hz, 2H), 7.09 (s, 1H), 6.97 (s, 1H), 4.34 - 4.61 (m, 2H), 3.96 - 4.29 (m, 3H), 3.57 - 3.75 (m, 6H), 2.86 - 3.07 (m, 6H), 1.92 - 2.37 (m, 2H).

### Example 56

### 4-[(3R,4R)-3-amino-4-hydroxy-pyrrolidin-1-yl]-1-[4-[4-[6-chloro-4-(trifluoromethyl)-2-pyridyl]piperazin-1-yl]sulfonylphenyl]pyrrolidin-2-one

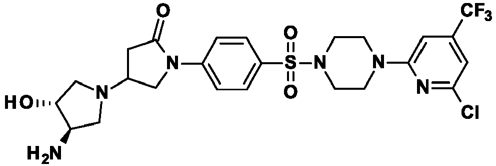

The title compound was prepared in analogy to the preparation of **Example 49** by using *tert-butyl N-[(3R,* 4*R*)-4-hydroxypyrrolidin-3-yl]carbamate instead of *tert-*butyl *N*-(azetidin-3-ylmethyl) carbamate. **Example 56** (31 mg) was obtained as a white solid. MS obsd.: 589.1 (MH⁺). ¹H NMR (400 MHz, DMSO-*d₆*): δ ppm 8.54 - 9.03 (m, 3H), 7.91 - 7.97 (m, 2H), 7.80 (s, 2H), 7.06 - 7.10 (m, 1H), 6.94 - 6.98 (m, 1H). 4.43 - 4.53 (m, 1H), 4.23 - 4.29 (m, 1H), 4.08 - 4.21 (m, 1H), 3.64 - 3.86 (m, 7H), 3.00 - 3.13 (m, 2H), 2.98 (br d, *J =* 4.4 Hz, 4H).

### Example 57

### 4-((3R,4R)-3-amino-4-hydroxypiperidin-1-yl)-1-(4-((4-(6-chloro-4-(trifluoromethyl)pyridin-2-yl)piperazin-1-yl)sulfonyl)phenyl)pyrrolidin-2-one

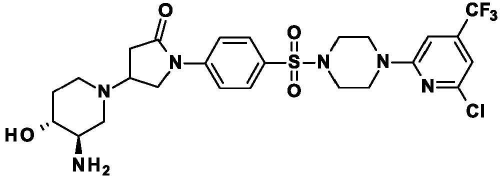

The title compound was prepared in analogy to the preparation of **Example 49** by using *tert-butyl N-[(3R,* 4*R*)-4-hydroxy-3-piperidyl]carbamate instead of *tert-*butyl *N*-(azetidin-3-ylmethyl) carbamate. **Example 57** (20.1 mg) was obtained as a white solid. MS obsd.: 603.2 (MH⁺). ¹H NMR (400 MHz, DMSO-*d₆*): δ ppm 8.28 - 8.65 (m, 3H), 7.93 (s, 2H), 7.80 (br d, *J =* 1.2 Hz, 2H), 7.08 (s, 1H), 6.96 (s, 1H), 3.89 - 4.42 (m, 4H), 3.58 - 3.87 (m, 7H), 3.18 - 3.25 (m, 1H), 2.94 - 3.01 (m, 1H), 2.98 (br d, *J =* 4.4 Hz, 7H), 1.97 - 2.12 (m, 1H), 1.70 - 1.93 (m, 1H).

### Example 58

### 4-(Azetidin-3-ylamino)-1-[4-[4-[6-chloro-4-(trifluoromethyl)-2-pyridyl]piperazin-1-yl]sulfonylphenyl]pyrrolidin-2-one

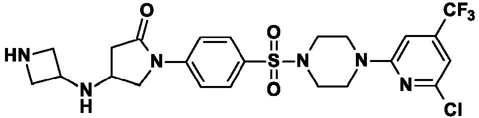

The title compound was prepared according to the following procedures:

### Step 1: tert-butyl 3-[[1-[4-[4-[6-chloro-4-(trifluoromethyl)-2-pyridyl]piperazin-1-yllsulfonyl phenyl]-5-oxo-pyrrolidin-3-yl]amino]azetidine-1-carboxylate (compound 58a)

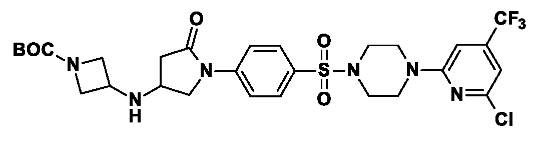

A solution of **Example 1** (120.0 mg, 0.24 mmol), *tert-butyl* 3-oxoazetidine-1-carboxylate (203.8 mg, 1.2 mmol) in methanol (0.42 mL) and THF (0.42 mL) was added anhydrous Na₂SO₄ (85.01 mg, 0.710 mmol). The mixture was stirred at 25 °C for 2 hrs and then NaBH₃CN (104.75 mg, 1.67 mmol) was added and the mixture was stirred at 45 °C for 6 hrs. The mixture was diluted with water (100 mL) and extracted with EA (100 mL) three times. The combined organic layer was washed with brine (100 mL) two times, dried over anhydrous Na₂SO₄, filtered and concentrated. The crude was purified by prep-HPLC (FA as additive) to give compound 58a (90 mg, 0.140 mmol, 57.34% yield) as a white solid. MS obsd.: 659.0 (MH⁺).

### Step 2: 4-(azetidin-3-ylamino)-1-[4-[4-[6-chloro-4-(trifluoromethyl)-2-pyridyl]piperazin-1-yl]sulfonylphenyl]pyrrolidin-2-one(Example 58)

To a solution of compound 58a (80.0 mg. 0.12 mmol) in DCM (2 mL) was added TFA (1.0 mL, 12.98 mmol). The mixture was stirred at 25°C for 2 hrs and then concentrated. The crude was dissolved in methanol (3.0 mL) and basified with ammonium hydroxide to pH 7.0. The solution was purified by prep-HPLC to give **Example 58** (29.6 mg, 0.05 mmol, 39.7% yield) as a white solid. MS obsd.: 559.2 (MH⁺). ¹H NMR (400 MHz, DMSO-*d₆*): δ ppm 8.87 - 9.87 (m, 2 H), 7.71 - 7.99 (m, 4 H), 7.06 - 7.14 (m, 1 H), 6.93 - 7.00 (m, 1 H), 3.66 - 4.42 (m, 12 H), 2.88 - 3.08 (m, 5 H), 2.56 - 2.75 (m, 1 H).

### Example 59

### 1-[4-[4-[6-Chloro-4-(trifluoromethyl)-2-pyridyl]piperazin-1-yl]sulfonylphenyl]-4-(pyrrolidin-3-ylamino)pyrrolidin-2-one

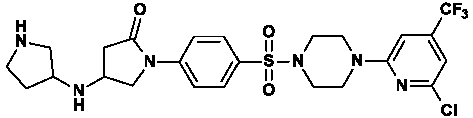

The title compound was prepared in analogy to the preparation of **Example 58** by using *tert-butyl* 3-oxopyrrolidine-1-carboxylate instead of *tert-butyl* 3-oxoazetidine-1-carboxylate. **Example 59** (33.5 mg) was obtained as a white solid. MS obsd.: 573.0 (MH⁺). ¹H NMR (400 MHz, DMSO-*d₆*): δ ppm 10.17 - 10.81 (m, 2 H), 9.29 - 9.92 (m, 2 H), 7.88 - 7.96 (m, 2 H), 7.77 - 7.86 (m, 2 H), 7.08 - 7.14 (m, 1 H), 6.95 - 7.01 (m, 1 H), 3.98 - 4.38 (m, 4 H), 3.68 - 3.81 (m, 4 H), 3.44 - 3.64 (m, 3 H), 2.91 - 3.28 (m, 7 H), 2.13 - 2.41 (m, 2 H).

### Example 60

### 1-[4-[4-[6-Chloro-4-(trifluoromethyl)-2-pyridyl]piperazin-1-yl]sulfonylphenyl]-4-(4-piperidylamino)pyrrolidin-2-one

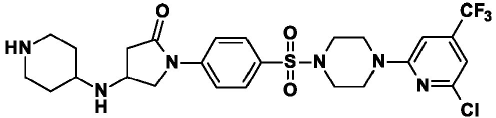

The title compound was prepared in analogy to the preparation of **Example 58** by using *tert-butyl* 4-oxopiperidine-1-carboxylate instead of *tert-butyl* 3-oxoazetidine-1-carboxylate. **Example 60** (58 mg) was obtained as a white solid. MS obsd.: 587.3 (MH⁺). ¹H NMR (400 MHz, DMSO-*d₆*): δ ppm 8.77 - 10.68 (m, 4 H), 7.75 - 7.96 (m, 4 H), 7.05 - 7.16 (m, 1 H), 6.93 - 7.01 (m, 1 H), 4.07 - 4.38 (m, 3 H), 3.65 - 3.81 (m, 4 H), 3.39 - 3.61 (m, 3 H), 2.82 - 3.10 (m, 8 H), 2.14 - 2.29 (m, 2 H), 1.82 - 2.02 (m, 2 H).

### Example 61

### 4-Amino-1-[4-[4-[6-chloro-4-(trifluoromethyl)-2-pyridyl]piperazin-1-yl]sulfonylphenyl]-5-methyl-pyrrolidin-2-one

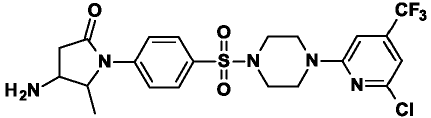

The title compound was prepared in analogy to the preparation of **Example 1** by using *tert-*butyl *N*-(2-methyl-5-oxo-pyrrolidin-3-yl)carbamate instead of *tert-butyl* (5-oxopyrrolidin-3-yl)carbamate. **Example 61** (20 mg) was obtained as a white solid. MS obsd.: 518.2 (MH⁺). ¹H NMR (400 MHz, DMSO-*d₆*): δ ppm 7.82 - 7.93 (m, 2H), 7.70 - 7.79 (m, 2H), 7.08 (s, 1H), 6.97 (s, 1H), 3.91 - 4.17 (m, 3H), 3.65 - 378 (m, 6H), 2.92 - 3.04 (m, 5H), 2.22 (dd, *J =* 2.8, 17.2 Hz, 1H), 1.08 - 1.24 (m, 3H).

### Example 62

### 4-Amino-1-[5-[4-[6-chloro-4-(trifluoromethyl)-2-pyridyl]piperazin-1-yl]sulfonyl-2-pyridyl]pyrrolidin-2-one

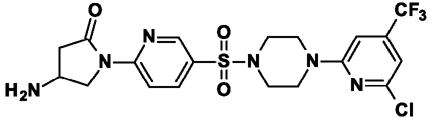

The title compound was prepared according to the following procedures:

### Step 1: 1-[(6-bromo-3-pyridyl)sulfonyl]-4-[6-chloro-4-(trifluoromethyl)-2-pyridyl] piperazine (compound 62a)

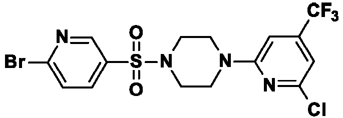

A mixture of intermediate 1a (207.14 mg, 0.780 mmol), 6-bromo-pyridine-3-sulfonyl chloride (200.0 mg, 0.780 mmol) and DIPEA (0.27 mL, 1.56 mmol) in DCM (6.67 mL) was stirred at 25 °C for 2 hrs. After the reaction was completed, the solvent was removed under vacuum and the crude was purified by column chromatography on silica gel (PE: EA = 5:2) to give compound 62a (300 mg, 75.25% yield) as a white solid. MS obsd.: 485.0 (MH⁺).

### Step 2 : tert-butyl N-[1-[5-[4-[6-chloro-4-(trifluoromethyl)-2-pyridyl]piperazin-1-yl]sulfonyl-2-pyridyl]-5-oxo-pyrrolidin-3-yl]carbamate (compound 62b)

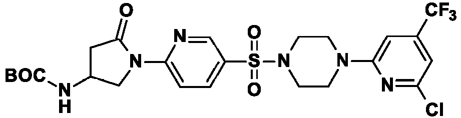

A mixture of *tert-*butyl *N*-(5-oxopyrrolidin-3-yl)carbamate (41.2 mg, 0.21 mmol), CuI (0.02 mmol), Cs₂CO₃ (134.2 mg, 0.41 mmol), compound 62a (100.0 mg, 0.21 mmol) in DMF (2 mL) was heated at 85 °C in a microwave reactor for 1 h. The mixture was poured into ice water, extracted with DCM (15 mL) twice, dried over Na₂SO₄, filtered and concentrated. The crude was purified by column chromatography on silica gel to give compound 62b (80 mg, 0.130 mmol, 61.01% yield) as a white solid. MS obsd.: 605.2 (MH⁺).

### Step 3: 4-amino-1-[5-[4-[6-chloro-4-(trifluoromethyl)-2-pyridyl]piperazin-1-yl]sulfonyl-2-pyridyl]pyrrolidin-2-one (Example 62)

A mixture of compound 62b (60.0 mg, 0.10 mmol) and HCl in EA (0.05 mL, 0.10 mmol) was stirred at 25 °C for 2 h. After the reaction was completed, the solvent was removed under vacuum and the crude was purified by prep-HPLC to give **Example 62** (6 mg, 0.01 mmol, 11.58% yield) as a white solid. MS obsd.: 505.1 (MH⁺). ¹H NMR (400 MHz, DMSO-*d₆*): δ ppm 8.73 (d, *J =* 2.4 Hz, 1H), 8.50 (d, *J =* 9.2 Hz, 1H), 8.18 - 8.25 (m, 3H), 7.09 (s, 1H), 6.96 (s, 1H), 4.23 (dd, *J =* 12.6, 7.2 Hz, 1H), 4.11 (dd, *J =* 12.6, 2.0 Hz, 1H), 4.03 (s, 1H), 3.66 - 3.76 (m, 4H), 3.15 (dd, *J =* 18.0, 8.0 Hz, 1H), 2.97 - 3.05 (m, 4H), 2.60 - 2.67 (m, 1H).

### Example 63

### 5-(Aminomethyl)-3-[4-[4-[6-chloro-4-(trifluoromethyl)-2-pyridyl]piperazin-1-yl]sulfonyl-2-fluoro-phenyl]oxazolidin-2-one

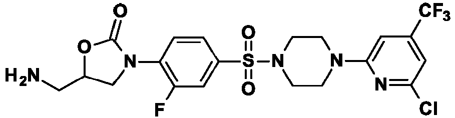

The title compound was prepared in analogy to the preparation of **Example 1** by using *tert-*butyl *N*-[(2-oxooxazolidin-5-yl)methyl]carbamate instead of *tert-butyl* (5-oxopyrrolidin-3-yl)carbamate and 4-bromo-3-fluorobenzenesulfonyl chloride instead of 4-iodobenzenesulfonyl chloride. **Example 63** (35 mg) was obtained as a white solid. MS obsd.: 538.1 (MH⁺). ¹H NMR (400 MHz, DMSO-*d₆*): δ ppm 8.17-8.31 (m, 2H), 7.77-7.83 (m, 1H), 7.72 (br dd, *J* = 13.2, 2.0 Hz, 1H), 7.55 (br dd, *J* = 8.8, 2.0 Hz, 1H), 7.10 (s, 1H), 6.98 (s, 1H), 4.62 - 4.72 (m, 2H), 4.11 (m, 1H), 3.90 (br dd, *J* = 9.2, 6.0 Hz, 1H), 3.67 - 3.77 (m, 4H), 3.06 - 3.16 (m, 4H).

### Example 64

### 5-(Aminomethyl)-3-[4-[4-[6-chloro-4-(trifluoromethyl)-2-pyridyl]piperazin-1-yl]sulfonyl-3-fluoro-phenyl]oxazolidin-2-one

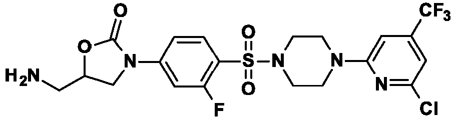

The title compound was prepared in analogy to the preparation of **Example 1** by using *tert-*butyl N-[(2-oxooxazolidin-5-yl)methyl]carbamate instead of *tert-butyl* (5-oxopyrrolidin-3-yl)carbamate and 4-bromo-2-fluorobenzenesulfonyl chloride instead of 4-iodobenzenesulfonyl chloride. **Example 64** (45 mg) was obtained as a white solid. MS obsd.: 538.1 (MH⁺). ¹H NMR (400 MHz, DMSO-*d₆*): δ ppm 7.82-7.92 (m, 1H), 7.74 (dd, 1H, *J =* 2.0, 10.8 Hz), 7.67 (dd, 1H, *J* = 2.0, 8.4 Hz), 7.11 (s, 1H), 6.98 (s, 1H), 4.83 - 4.93 (m, 1H), 4.11 - 4.34 (m, 1H), 3.93 - 4.02 (m, 1H), 3.61 - 3.85 (m, 4H), 3.11 (br d, 2H, *J =* 6.0 Hz), 3.03 - 3.14 (m, 4H).

### Example 65

### 4-Amino-1-[4-[4-[4-methyl-6-(trifluoromethyl)pyrimidin-2-yl]piperazin-1-yl]sulfonylphenyl]pyrrolidin-2-one

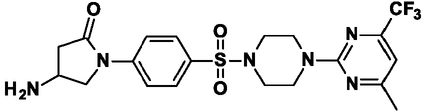

The title compound was prepared in analogy to the preparation of **Example 1** by using 2-chloro-4-methyl-6-(trifluoromethyl)pyrimidine instead of 2,6-dichloro-4-(trifluoromethyl) pyridine. **Example 65** (14 mg) was obtained as a white solid. MS obsd.: 484.8 (MH⁺). ¹H NMR (400 MHz, DMSO-*d₆*): δ ppm 8.23(br s, 2H), 7.86 - 7.99 (m, 2H), 7.72 - 7.83 (m, 2H), 6.85 - 7.04 (m, 1H), 4.21 - 4.30 (m, 1H), 4.03 - 4.10 (m, 1H), 3.74 - 3.90 (m, 5H), 3.02 - 3.13 (m, 1H), 2.97 (br t, *J* = 4.8 Hz, 4H), 2.53 - 2.61 (m, 1H), 2.33 - 2.40 (m, 3H).

### Example 66

### 4-Amino-1-[4-[4-[4-(trideuteriomethyl)-6-(trifluoromethyl)pyrimidin-2-yl]piperazin-1-yl]sulfonylphenyl]pyrrolidin-2-one

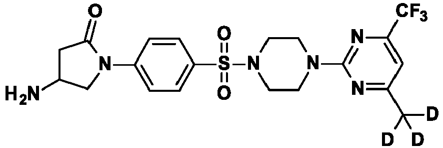

The title compound was prepared according to the following procedures:

### Step 1: tert-butyl N-[1-[4-[4-[4-chloro-6-(trifluoromethy])pyrimidin-2-yl]piperazin-1-yl]sulfonylphenyl]-5-oxo-pyrrolidin-3-yl]carbamate (compound 66a)

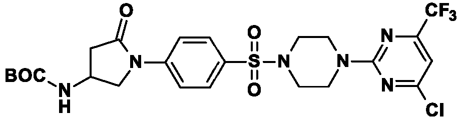

A mixture of intermediate C (0.320 g, 754 µmol), 2,4-dichloro-6-(trifluoromethyl) pyrimidine (164 mg, 754 µmol) and DIPEA (292 mg, 2.26 mmol) in THF (2 mL) was stirred at 90 °C overnight. The mixture was concentrated and the residue was diluted with EA, washed with 1 N HCl (10 mL). The organic layer was dried, concentrated and used directly in next step. MS obsd.: 605.4 (MH⁺).

### Step 2: tert-butyl N-[5-oxo-1-[4-[4-[4-(trideuteriomethyl)-6-(trifluoromethyl)pyrimidin-2-yl]piperazin-1-yl]sulfonylphenyl]pyrrolidin-3-yl]carbamate (compound 66b)

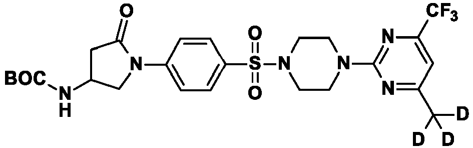

A mixture of compound 66a (0.15 g, 248 µmol), (methyl-d₃)boronic acid (31.2 mg, 496 µmol) and K₂CO₃ (103 mg, 744 µmol), Ag₂O(144 mg) and Pd(dppf)Cl₂(18.1 mg) in THF (0.5 mL) was stirred at 80 °C overnight. The organic layer was concentrated and used directly in next step. MS obsd.: 588.3 (MH⁺).

### Step 3: 4-amino-1-[4-[4-[4-(trideuteriomethyl)-6-(trifluoromethyl)pyrimidin-2-yl] piperazin-1-yl]sulfonylphenyl]pyrrolidin-2-one (Example 66)

A mixture of compound 66b (0.120 g, 204 µmol) and TFA (460 mg) in DCM (0.5 mL) was stirred at 25 °C 1h. The mixture was concentrated and the crude was purified by pre-HPLC to give 4.5 mg of **Example** 66. MS obsd.: 488.3 (MH⁺). ¹H NMR (400 MHz, CD₃OD): δ ppm 7.92 - 8.01 (m, 2H), 7.80 - 7.84 (m, 2H), 6.93 (s, 1H), 4.24 (dd, 1H, *J =* 6.4, 10.4 Hz), 3.94 (br d, 1H, J=3.2 Hz), 3.87 (br s, 4H), 3.74 (dd, 1H, *J* = 2.8, 10.4 Hz), 3.11 - 3.15 (m, 4H), 3.02 (dd, 1H, *J* = 7.6, 17.6 Hz), 2.51 (dd, 1H, J=3.6, 17.6 Hz).

### Example 67

### 1-[4-(4-Amino-2-oxo-pyrrolidin-1-yl)phenyl]sulfonyl-4-[6-chloro-4-(trifluoromethyl)-2-pyridyl]piperazine-2-carbonitrile

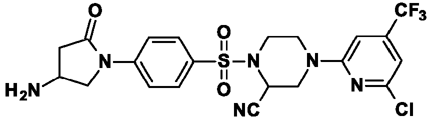

The title compound was prepared according to the following procedures:

### Step 1: tert-butyl N-[1-(4-benzylsulfanylphenyl)-5-oxo-pyrrolidin-3-yl]carbamate (compound 67a)

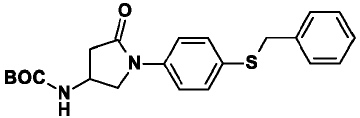

A mixture of *tert-butyl* N-(5-oxopyrrolidin-3-yl)carbamate (215.15 mg, 1.07 mmol), CuI (0.100 eq), Cs₂CO₃ (700.19 mg, 2.15 mmol), 1-benzylsulfanyl-4-bromo-benzene (300.0 mg, 1.07 mmol) in DMF (10.44 mL) was heated at 85 °C in a microwave reactor for 1h. The mixture was poured into ice water, extracted with DCM (15mL) two times, dried over Na₂SO₄, filtered and concentrated. The crude was purified by chromatography on silica gel to give compound 67a (300 mg, 0.750 mmol, 65.16% yield) as a white solid. MS obsd.: 399.2 (MH⁺).

### Step 2: tert-butyl N-[1-(4-chlorosulfonylphenyl)-5-oxo-pyrrolidin-3-yl]carbamate (compound 67b)

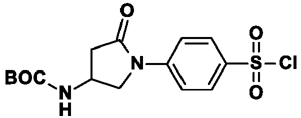

To a mixture of compound 67a (200.0 mg, 0.500 mmol) in ACN (7.53 mL), AcOH (0.753 mL) and water (0.753 mL) was added 1,3-dichloro-5,5-dimethylhydantoin (197.75 mg, 1 mmol) at 25 °C for 1 h. After completion, the mixture was poured into brine (25 mL) and extracted by EA (15mL) twice, washed with brine (15 mL) twice. The organic layer was concentrated and the crude was purified by chromatography on silica gel (PE: EA =13:3) to give compound 67b (42 mg, 0.110 mmol, 20.09% yield) as colorless oil. MS obsd.: 397 (MNa⁺).

### Step 3: tert-butyl 4-[6-chloro-4-(trifluoromethyl)-2-pyridyl]-2-cyano-piperazine-1-carboxylate (compound 67c)

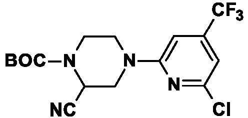

A mixture of 2,6-dichloro-4-(trifluoromethyl)pyridine (239.91 mg, 1.11 mmol) and *tert-*butyl 2-cyanopiperazine-1-carboxylate (200.0 mg, 1.11 mmol) was stirred at 100 °C for 12 h. Then water (30 mL) was added and the solution was extracted with EA, dried over Na₂SO₄, filtered and concentrated. The crude was purified by prep-HPLC to give compound 67c (150 mg, 0.380 mmol, 31.1% yield). MS obsd.: 391.1 (MH⁺).

### Step 4: 4-[6-chloro-4-(trifluoromethyl)-2-pyridyl]piperazine-2-carbonitrile (compound 67d)

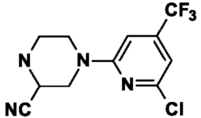

Compound 67c (150.0 mg, 0.380 mmol) was dissolved in 2M HCl/EA (10 mL) and the mixture was stirred at rt for 4 hrs. The mixture was concentrated and the crude was purified by prep-HPLC to give compound 67d (75 mg, 0.26 mmol, 60.5% yield). MS obsd.: 291.1 (MH⁺).

### Step 5: tert-butyl N-[1-[4-[4-[6-chloro-4-(trifluoromethyl)-2-pyridyl]-2-cyano-piperazin-1-yl]sulfonylphenyl]-5-oxo-pyrrolidin-3-yl]carbamate (compound 67e)

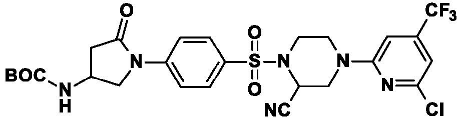

To a mixture of compound 67b (75.0 mg, 0.200 mmol) and pyridine (0.5 mL) in DCM (4.52 mL) was added compound 67d (42.0 mg, 0.140 mmol) and the reaction was stirred at rt for 8 hrs. Water (10 mL) was added and the mixture was extracted with EA, dried over Na₂SO₄, filtered and concentrated. The crude was purified by prep-HPLC to give compound 67e (50 mg, 0.080 mmol, 49.51% yield). MS obsd.:651.1 (MNa⁺).

### Step 6: 1-[4-(4-amino-2-oxo-pyrrolidin-1-yl)phenyl]sulfonyl-4-[6-chloro-4-(trifluoromethyl)-2-pyridyl]piperazine-2-carbonitrile (Example 67)

Compound 67e (49.76 mg, 0.080 mmol) was dissolved in 4M HCl/1,4-dioxane(10 mL) and the mixture was stirred at rt for 4 hrs. The mixture was concentrated and the crude was purified by prep-HPLC to give **Example 67** (25 mg, 0.050 mmol, 58.74% yield) as a white solid. MS obsd.: 529.1 (MH⁺). ¹H NMR (400 MHz, DMSO-*d₆*): δ ppm 7.95 (d, *J* = 8.8 Hz, 2H), 7.86 (d, *J* = 8.8 Hz, 2H), 7.25 (s, 1H), 7.10 (s, 1H), 5.25 (s, 1H), 4.78 (d, *J =* 13.6 Hz, 1H), 4.45 (d, *J* = 12.8 Hz, 1H), 4.06 (d, *J =* 9.6 Hz, 1H), 3.82 (d, *J =* 12.0 Hz, 1H), 3.75 (s, 1H), 3.57 (d, *J =* 9.6 Hz, 1H), 3.28 (dd, *J =* 13.6, 3.2 Hz, 1H), 3.09 (t, *J =* 11.2 Hz, 1H), 2.71 - 2.90 (m, 2H), 2.33 (d, *J* = 16.4 Hz, 1H).

### Example 68

### 4-Amino-1-[4-[4-[6-chloro-4-(difluoromethyl)-2-pyridyl]piperazin-1-yl]sulfonylphenyl] pyrrolidin-2-one

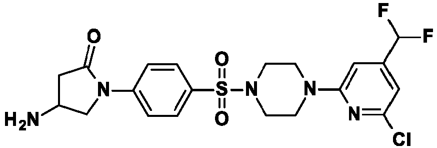

The title compound was prepared in analogy to the preparation of **Example 1** by using 2,6-dichloro-4-(difluoromethyl)pyridine instead of 2,6-dichloro-4-(trifluoromethyl)pyridine. **Example 68** (42.4 mg) was obtained as a white solid. MS obsd.: 486.1 (MH⁺). ¹H NMR (400 MHz, CD₃OD): δ ppm 7.90 - 7.94 (m, 2 H), 7.81 - 7.86 (m, 2 H), 6.79 (s, 1 H), 6.73 (s, 1 H), 6.56 (s, 1 H), 4.39 (dd, *J =* 11.2, 6.8 Hz, 1 H), 4.18 (m, 1 H), 3.93 (dd, *J =* 11.2, 2.4 Hz, 1 H), 3.65 - 3.75 (m, 4 H), 3.19 (dd, *J* =18.0, 8.4 Hz, 1 H), 3.03 - 3.11 (m, 4 H), 2.67 (dd, *J =* 18.0, 2.8 Hz, 1 H).

### Example 69

### 4-Amino-1-[4-[4-(6-chloro-4-cyclopropylsulfonyl-2-pyridyl)piperazin-1-yl]sulfonylphenyl] pyrrolidin-2-one

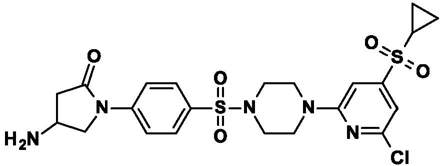

The title compound was prepared according to the following procedures:

### Step 1: 2,6-dichloro-4-cyclopropylsulfanyl-pyridine (compound 69a)

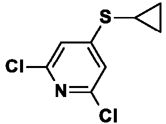

To a solution of 4-bromo-2,6-dichloro-pyridine (100.0 mg, 0.440 mmol), cyclopropanethiol (32.68 mg, 0.440 mmol) in DMF (2 mL) were added K₂CO₃ (0.25 mL, 1.32 mmol). The mixture was heated to 80 °C for 4 hrs, then diluted with water (40 mL) and extracted with EA (50 mL) three times. The combined organic layer was washed with brine (50 mL), dried over Na₂SO₄, filtered and concentrated. The crude was purified by prep-HPLC to give compound 69a (100 mg, 0.450 mmol, 82.46% yield) as yellow oil. MS obsd.: 220.0 (MH⁺).

### Step 2: 2,6-dichloro-4-(cyclopropylsulfonyl)pyridine (compound 69b)

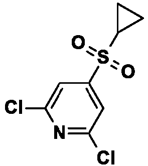

To a solution of compound 69a (80.0 mg, 0.360 mmol) in DCM (4 mL) were added m-CPBA (0.15 mL, 1.09 mmol) in one portion. The mixture was stirred at 20 °C for 2 hrs and then concentrated. The crude was purified by prep-HPLC to give compound 69b (60 mg, 0.240 mmol, 65.48% yield) as a yellow solid. MS obsd.: 252.9 (MH⁺).

### Step 3: tert-butyl (1-(4-((4-(6-chloro-4-(cyclopropylsulfonyl)pyridin-2-yl)piperazin-1-yl)sulfonyl)phenyl)-5-oxopyrrolidin-3-yl)carbamate (compound 69c)

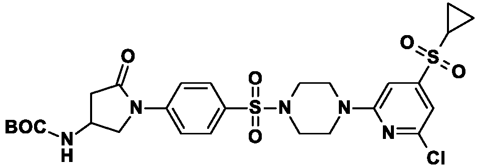

To a mixture of **Intermediate C** (80.0 mg, 0.190 mmol), Cs₂CO₃ (184.19 mg, 0.570 mmol) in DMSO (4 mL) was added compound 69b (61.77 mg, 0.240 mmol) and the mixture was stirred at 80 °C for 2 hrs. Solvent was evaporated and the residue was dissolved in water, extracted with EA (50 mL) three times. The combined organic layer was washed with brine, dried over Na₂SO₄, filtered and concentrated to give compound 69c (100 mg, 0.160 mmol, 66.31% yield) as a yellow solid MS obsd.: 640.1 (MH⁺).

### Step 4: 4-amino-1-(4-((4-(6-chloro-4-(cyclopropylsulfonyl)pyridin-2-yl)piperazin-1-yl)sulfonyl) phenyl)pyrrolidin-2-one (Example 69)

To a solution of compound 69b (80.0 mg, 0.120 mmol) in DCM (2 mL) was added TFA (1.0 mL, 0.370 mmol). The mixture was stirred at 20 °C for 1 h and then concentrated. The crude was purified by prep-HPLC to give **Example 69** (30.4 mg, 0.060 mmol, 43.67% yield) as a yellow solid. MS obsd.: 540.1 (MH⁺). ¹H NMR (400 MHz, DMSO-*d₆*): δ ppm 8.38 - 8.51 (m, 3H), 7.87 - 7.97 (m, 2H), 7.73 - 7.83 (m, 2H), 7.11 (d, *J =* 0.8 Hz, 1H), 7.03 (d, *J =* 0.8 Hz, 1H), 4.20 - 4.32 (m, 1H), 3.99 - 4.13 (m, 1H), 3.81 - 3.894 (m, 1H), 3.64 - 3.78 (m, 4H), 2.91 - 3.15 (m, 6H), 2.57 - 2.67 (m, 1H), 1.03 - 1.21 (m, 4H).

### Example 70

### 4-Amino-1-[4-[4-(6-chloro-4-tetrahydropyran-4-ylsulfonyl-2-pyridyl)piperazin-1-yl]sulfonylphenyl]pyrrolidin-2-one

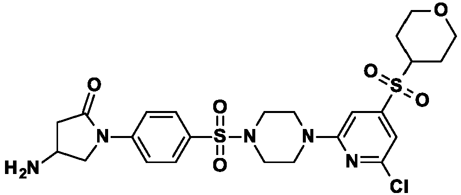

The title compound was prepared according to the following procedures:

### Step 1: 2,6-dichloro-4-tetrahydropyran-4-ylsulfanyl-pyridine(compound 70a)

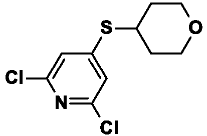

To a solution of 2,6-dichloropyridine-4-thiol (90.0 mg, 0.500 mmol), K₂CO₃ (0.08 mL, 1 mmol) in NMP (2 mL) were added 4-bromo-tetrahydropyran (82.49 mg, 0.500 mmol). The mixture was stirred at 70 °C for 15 hrs under N₂. The mixture was diluted with water (50 mL) and extracted with EA (50 mL) three times. The organic layer was washed with brine (50 mL) two times, dried over anhydrous Na₂SO₄, filtered and concentrated. The crude was purified by prep-HPLC to give compound 70a (120 mg, 0.450 mmol, 90.88% yield) as a yellow solid. MS obsd.: 490.9 (MH⁺).

### Step 2 : 4-Amino-1-[4-[4-(6-chloro-4-tetrahydropyran-4-ylsulfonyl-2-pyridyl)piperazin-1-yl]sulfonylphenyl]pyrrolidin-2-one (Example 70)

The title compound was prepared in analogy to the preparation of **Example 69** by using compound 70a instead of compound 69a. **Example 70** (33.2 mg) was obtained as a white solid. MS obsd.: 584.2 (MH⁺). ¹H NMR (400 MHz, DMSO-*d₆*): δ ppm 8.48 - 8.64 (m, 3H), 7.87 - 8.01 (m, 2H), 7.70 - 7.84 (m, 2H), 7.00 - 7.12 (m, 1H), 6.89 - 6.98 (m, 1H), 4.21 - 4.33 (m, 1H), 4.03 - 4.10 (m, 1H), 3.86 - 3.94 (m, 3H), 3.69 - 3.78 (m, 5H), 3.22 - 3.31 (m, 2H), 2.97 - 3.11(m, 5H), 2.67 (s, 1H), 1.65 - 1.74 (m, 2H), 1.51 (br s, 2H).

### Example 71

### 1-[2-[4-[4-(4-Amino-2-oxo-pyrrolidin-1-yl)phenyl]sulfonylpiperazin-1-yl]-6-chloro-4-pyridyl]cyclopropanecarbonitrile

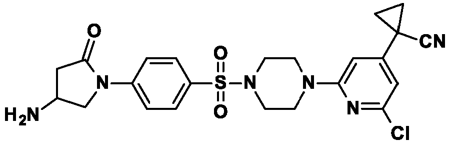

The title compound was prepared according to the following procedures:

### Step 1: tert-butyl 2-cyano-2-(2,6-dichloropyridin-4-yl)acetate (compound 71a)

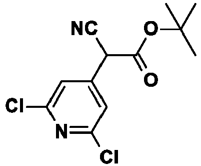

A mixture of 2,4,6-trichloropyridine (1.82 g, 10 mmol), *tert-butyl* 2-cyanoacetate (1.69 g, 12 mmol) and Cs₂CO₃ (4.9 g, 15 mmol) in DMF (20 mL) was stirred for 2 hrs at 90 °C. Then the mixture was poured into water (10 mL), extracted with EA. The organic layer was dried over anhydrous Na₂SO₄, filtered and concentrated. The crude was purified by column chromatography on silica gel (PE: EA= 20:1~10:1) to give compound 71a (2.01 g, 8.4 mmol, 70% yield) as a yellow oil. MS obsd.: 287.0 (MH⁺).

### Step 2: 2-(2,6-dichloropyridin-4-yl)acetonitrile (compound 71b)

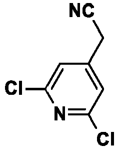

A mixture of intermediate 71a (2 g, 6.97 mmol) and TFA (7.94 g, 69.7 mmol) in DCM (3 mL) was stirred for 2 hrs at rt and then concentrated. The crude was poured into saturated NaHCO₃ solution (10 mL) and extracted with EA. The organic layer was dried over anhydrous Na₂SO₄, filtered and concentrated. The crude was purified by column chromatography on silica gel (PE: EA= 10:1~10:5) to give compound 71b (1.17 g, 6.27 mmol, 90% yield) as a yellow solid. MS obsd.: 187.0 (MH⁺).

### Step 3: 1-(2,6-dichloro-4-pyridyl)cyclopropanecarbonitrile (Intermediate 71c)

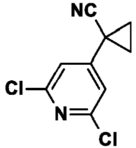

A solution of intermediate 71b (0.8 g, 4.28 mmol) and diphenyl(vinyl)sulfonium trifluoromethanesulfonate (1.86 g, 5.13 mmol) in DMF (10 mL) was added DBU (1.95 g, 12.8 mmol). The mixture was stirred for 2 hrs at rt and then poured into water, extracted with EA. The organic layer was dried over anhydrous Na₂SO₄, filtered and concentrated. The residue was purified by column chromatography on silica gel (PE: EA= 2:1-1:5) to give compound 71c (0.6 g, 2.81 mmol, 65.8% yield) as a white solid. MS obsd.: 214.1 (MH⁺).

### Step 4: 1-[2-[4-[4-(4-amino-2-oxo-pyrrolidin-1-yl)phenyl]sulfonylpiperazin-1-yl]-6-chloro-4-pyridyl]cyclopropanecarbonitrile (Example 71)

The title compound was prepared in analogy to the preparation of **Example 69** by using compound 71b instead of compound 69b. **Example 71** (6.0 mg) was obtained as a white solid. MS obsd.: 501.4 (MH⁺). ¹H NMR (400 MHz, CD₃OD): δ ppm 7.89 - 7.93 (m, 2 H), 7.77 - 7.82 (m, 2 H), 6.53 (d, *J = 1.2* Hz, 1 H), 6.50 (d, *J* =1.2 Hz, 1 H), 4.58 (br s, 2 H), 4.15 (dd, *J =10.0,* 6.4 Hz, 1 H), 3.78 (s, 1 H), 3.61 - 3.69 (m, 5 H), 3.03 - 3.08 (m, 4 H), 2.91 (dd, *J =* 17.2, 7.2 Hz, 1 H), 2.38 - 2.43 (m, 1 H), 1.74 - 1.78 (m, 2 H), 1.55 - 1.59 (m, 2 H).

### Example 72

### 4-Amino-1-[4-[4-(6-chloro-4-phenoxy-2-pyridyl)piperazin-1-yl]sulfonylphenyl]pyrrolidin-2-one

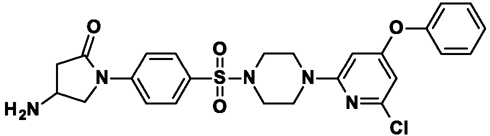

The title compound was prepared according to the following procedures:

### Step 1: 2,6-dichloro-4-phenoxy-pyridine (compound 72a)

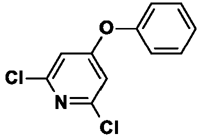

To a solution of 4-bromo-2,6-dichloro-pyridine (100.0 mg, 0.44 mmol) in DMF (1.0 mL) was added phenol (41.48 mg, 0.44 mmol), potassium (51.7 mg, 1.32 mmol) in one portion. The reaction was stirred at 100 °C for 1 h and diluted with water (20 mL), extracted with EA(10 mL) three times. The combined organic layer was washed with brine (50 mL), dried over anhydrous Na₂SO₄, filtered and concentrated to give compound 72a (100 mg, 0.42 mmol, 94.5% yield) as colorless oil.

### Step 2: 4-Amino-1-[4-[4-(6-chloro-4-phenoxy-2-pyridyl)piperazin-1-yl]sulfonylphenyl] pyrrolidin-2-one(Example 72)

The title compound was prepared in analogy to the preparation of **Example 69** by using compound 72a instead of compound 69a. **Example 72** (45 mg) was obtained as a white solid. MS obsd.: 528.2 (MH⁺). ¹H NMR (400 MHz, DMSO-*d₆*): δ ppm 8.47 - 8.70 (m, 3H), 8.45 - 8.72 (m, 4H), 7.88 - 7.91 (m, 2H), 7.78 (s, 2H), 7.42 - 7.48 (m, 2H), 7.24 - 7.30 (m, 1H), 7.09 - 7.13 (m, 2H), 6.25 - 6.28 (m, 1H), 6.27 (s, 1H), 6.11 - 6.14 (m, 1H), 4.24 - 4.29 (m, 1H), 4.05 - 4.11 (m, 1H), 3.95 - 3.99 (m, 1H), 3.53 - 3.58 (m, 1H), 3.01 - 3.06 (m, 5H), 2.69 - 2.75 (m, 1H).

### Example 73

### 4-Amino-1-[4-[4-[6-chloro-4-[difluoro(phenyl)methyl]-2-pyridyl]piperazin-1-yl]sulfonylphenyl] pyrrolidin-2-one

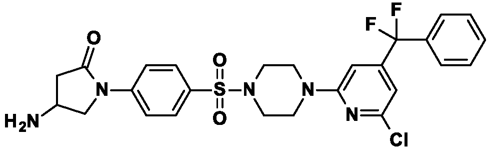

The title compound was prepared according to the following procedures:

### Step 1: 2,6-dichloro-N-methoxy-N-methyl-pyridine-4-carboxamide (compound 73a)

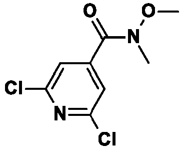

A mixture of 2,6-dichloroisonicotinic acid (30 g, 156.25 mmol) in SOCl₂ was stirred at 90 °C for 16 hrs and then concentrated. The crude was dissolved in DCM (300 mL) and a solution of O, N-dimethylhydroxylamine (18288.75 mg, 187.5 mmol) and DIPEA (20 mL) was added. The mixture was stirred at rt for 12 hrs and washed with brine (150 mL) three times. The organic layer was dried over Na₂SO₄, filtered and concentrated. The crude was purified by flash column on silica gel (PE: EA=100:0~0:100) to give compound 73a (30000 mg, 127.62 mmol, 73.51% yield) as a white solid. MS obsd.: 235.1 (MH⁺).

### Step 2: (2,6-dichloro-4-pyridyl)-phenyl-methanone (compound 73b)

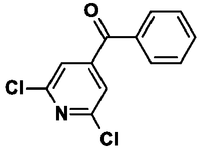

To a solution of compound 73a (500.0 mg, 2.13 mmol) in THF (15 mL) was added phenylmagnesium bromide (0.24 mL, 2.13 mmol) at -70 °C under N₂ and the mixture was stirred for 2 hrs. The mixture was quenched with saturated NH₄Cl (100 mL) and extracted with EA (50 mL) three times. The organic layers were dried over Na₂SO₄, filtered and concentrated. The crude was purified by flash chromatography on silica gel (EA in PE=0-30%) to give compound 73b (150 mg, 0.600 mmol, 25.2% yield) as a white solid. MS obsd.: 252 (MH⁺).

### Step 3: 2,6-dichloro-4-[difluoro(phenyl)methyl]pyridine (compound 73c)

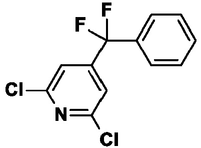

A solution of compound 73b (1300.0 mg, 5.16 mmol) and BAST (10.0 mL) was stirred at 25 °C for 12 hrs. The reaction was quenched with ice water (50 mL) and extracted with EA (30 mL) three times. The combined organic layer was dried over Na₂SO₄, filtered and concentrated. The crude was purified by flash column (PE: EA=1:0 to 1:1) to give compound 73c (800 mg, 2.92 mmol, 50.94% yield) as a light yellow solid. MS obsd.: 274(MH⁺).

### Step 4: tert-butyl N-[1-[4-[4-[6-chloro-4-[difluoro(phenyl)methyl]-2-pyridyl]piperazin-1-yl] sulfonylphenyl]-5-oxo-pyrrolidin-3-yl]carbamate (compound 73d)

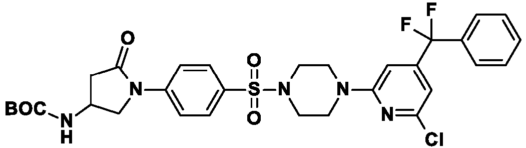

To a solution of intermediate C (309.76 mg, 0.730 mmol) and compound 73c (200.0 mg, 0.730 mmol) in DMSO (0.5 mL) was added DIPEA (2.0 mL) and the reaction was stirred at 115 °C for 12 hrs. The reaction was quenched with brine (50 mL) and extracted with DCM (30 mL) two times. The combined organic layer was dried over Na₂SO₄, filtered and concentrated. The crude was purified by flash column (EA in PE=0-70%) to give compound 73d (400 mg, 0.600 mmol, 82.79% yield) as a white solid. MS obsd.: 662(MH⁺).

### Step 5: 4-amino-1-[4-[4-[6-chloro-4-[difluoro(phenyl)methyl]-2-pyridyl]piperazin-1-yllsulfonyl phenyllpyrrolidin-2-one(Example 73)

Compound 73d (400.0 mg, 0.60 mmol) was dissolved in 2 M HCl/EA (10 mL) and the reaction was stirred at rt for 4 hrs. The mixture was concentrated and the crude was purified by prep-HPLC to give **Example 73** (300 mg, 0.530 mmol, 86.47% yield) as a white solid. MS obsd.: 562(MH⁺). ¹H NMR (400 MHz, DMSO-*d₆*): δ ppm 7.92 (d, *J =* 8.8 Hz, 2H), 7.77 (d, *J =* 8.8 Hz, 2H), 7.55 - 7.62 (m, 2H), 7.44 - 7.54 (m, 3H), 6.95 (s, 1H), 6.76 (s, 1H), 4.14 (dd, *J =* 10.4, 6.4 Hz, 1H), 3.88 (s, 1H), 3.64 -3.72 (m, 5H), 2.86 - 3.01 (m, 5H), 2.46 (dd, *J =* 17.4, 3.2 Hz, 1H).

### Example 74

### 4-Amino-1-[4-[4-(4-benzyl-6-chloro-2-pyridyl)piperazin-1-yl]sulfonylphenyl]pyrrolidin-2-one

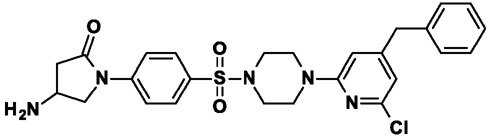

The title compound was prepared according to the following procedures:

### Step 1: 4-benzyl-2,6-dichloro-pyridine (compound 74a)

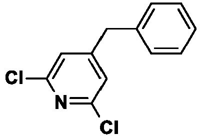

A mixture of methyltriphenylphosphonium bromide (1.7 g, 4.76 mmol), potassium t-Butoxide (0.45 g, 4.76 mmol) in THF (20 mL) was stirred for 0.5 h under N₂, then compound 73b (1 g, 3.97 mmol) was added. The resulted mixture was stirred for 12 hrs at rt and then poured into water, extracted with EA. The organic layer was dried over Na₂SO₄, filtered and concentrated. The crude was purified by column chromatography on silica gel (PE: EA= 20:1-10:1) to give compound 74a (0.23 g, 0.99 mmol, 25% yield) as yellow gum. MS obsd.: 236.0(MH⁺).

### Step 2: 4-amino-1-[4-[4-(4-benzyl-6-chloro-2-pyridyl)piperazin-1-yl]sulfonylphenyl] pyrrolidin-2-one (Example 74)

The title compound was prepared in analogy to the preparation of **Example 73** by using compound 74a instead of compound 73c. **Example 74** (3.6 mg) was obtained as a white solid. MS obsd.: 526 (MH⁺). ¹H NMR (400 MHz, CD₃OD): δ ppm 7.92 (d, *J* = 8.8 Hz, 2 H), 7.78 - 7.82 (m, 2 H), 7.24 - 7.31 (m, 2 H), 7.15 - 7.21 (m, 3 H), 6.52 (s, 1 H), 6.45 (s, 1 H), 4.26 (m, 1 H), 3.94 - 4.00 (m, 1 H), 3.83 (s, 2 H), 3.77 (dd, *J =* 10.8, 2.8 Hz, 1 H), 3.56 - 3.63 (m, 5 H), 2.93 - 3.15 (m, 8 H), 2.51 (m, 1 H).

### Example 75

### 4-Amino-1-[4-[4-[6-chloro-4-(1-phenylcyclopropyl)-2-pyridyl]piperazin-1-yl]sulfonylphenyl] pyrrolidin-2-one

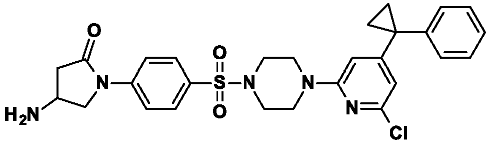

The title compound was prepared according to the following procedures:

### Step 1: 2,6-dichloro-4-(1-phenylvinyl)pyridine (compound 75a)

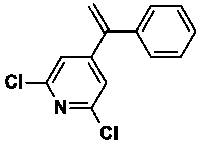

A mixture of methyltriphenylphosphonium bromide (1.7 g, 4.76 mmol), potassium t-Butoxide (0.45 g, 4.76 mmol) in THF (20 mL) was stirred for 0.5h under N₂, then compound 73b (1 g, 3.97 mmol) was added. The resulted mixture was stirred for 12 hrs at rt and then poured into water, extracted with EA. The organic layer was dried over Na₂SO₄, filtered and concentrated. The crude was purified by column chromatography on silica gel (PE: EA= 20:1~10:1) to give compound 75a (0.50 g, 2.38 mmol, 50% yield) as yellow gum. MS obsd.: 250.2(MH⁺).

### Step 2: 2,6-dichloro-4-(1-phenylcyclopropyl)pyridine (compound 75b)

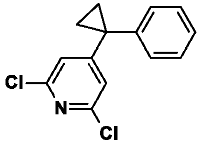

A mixture of trimethylsulfoxonium iodide (2.2 g, 10 mmol), sodium hydride (0.4 g, 10 mmol) in THF (5 mL) was stirred for 0.5h 30 at rt, then compound 75a (500 mg, 2 mmol) in THF (5 mL) was added dropwise. The mixture was stirred for 2 hrs at rt and then poured into water, extracted with EA. The organic layer was dried over Na₂SO₄, filtered and concentrated. The crude was purified by column chromatography on silica gel (PE: EA= 20:1~10:1) to compound 75b (0.32 g, 1.2 mmol, 60% yield) as a yellow gum. MS obsd.: 264.1(MH⁺).

### Step 3: 4-amino-1-[4-[4-[6-chloro-4-(1-phenylcyclopropyl)-2-pyridyl]piperazin-1-yllsulfonyl phenyl]pyrrolidin-2-one(Example 75)

The title compound was prepared in analogy to the preparation of **Example 73** by using compound 75b instead of compound 73c. **Example 75** (7.4 mg) was obtained as a white solid. MS obsd.: 552 (MH⁺). ¹H NMR (400 MHz, CD₃OD): δ ppm 7.92 (m, 2 H), 7.78 (m, 2 H), 7.23 - 7.31 (m, 5 H), 6.24 - 6.39 (m, 2 H), 4.18 - 4.33 (m, 1 H), 3.86 - 4.00 (m, 1 H), 3.69 - 3.80 (m, 1 H), 3.48 - 3.50 (m, 4 H), 2.99 - 3.03 (m, 5 H), 2.45 - 2.58 (m, 1 H), 1.31 (s, 4H).

### Example 76

### 4-Amino-1-[4-[4-[4-[difluoro(phenyl)methyl]-6-methyl-pyrimidin-2-yl]piperazin-1-yl]sulfonylphenyl]pyrrolidin-2-one

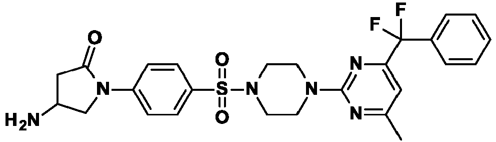

The title compound was prepared in analogy to the preparation of Example 73 by using 2-chloro-6-methylpyrimidine-4-carboxylate instead of 2,6-dichloro-N-methoxy-N-methylpyridine-4-carboxamide. **Example 76** (28 mg) was obtained as a white solid. MS obsd.: 543 (MH⁺). ¹H NMR (400 MHz, DMSO-*d₆*): δ ppm 8.18 (br s, 2H), 7.90 (d, *J =* 9.2 Hz, 2H), 7.77 (d, *J =* 9.2 Hz, 2H), 7.45 - 7.56 (m, 5H), 6.89 (s, 1H), 4.25 (dd, *J =* 6.8, 11.2 Hz, 1H), 4.07 (br s, 1H), 3.79 (br s, 5H), 3.43 - 3.63 (m, 1H), 3.21 - 3.31 (m, 1H), 3.17 (s, 1H), 3.07 (dd, *J* = 8.4, 17.6 Hz, 1H), 2.92 (br t, *J* = 4.8 Hz, 4H), 2.67 (s, 1H), 2.53 - 2.60 (m, 1H), 2.33 (s, 3H), 2.07 (s, 1H).

### Examples 77 and 78

### 4-Amino-1-[4-[4-[2-chloro-6-[difluoro(phenyl)methyl]pyrimidin-4-yl]piperazin-1-yl]sulfonylphenyl]pyrrolidin-2-one

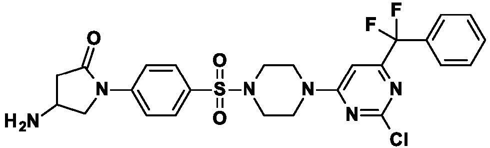

### 4-Amino-1-[4-[4-[4-chloro-6-[difluoro(phenyl)methyl]pyrimidin-2-yl]piperazin-1-yl]sulfonylphenyl]pyrrolidin-2-one

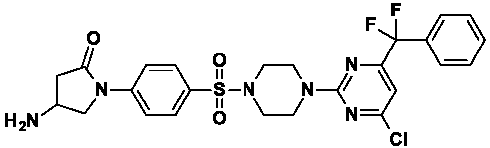

### The title compounds were prepared according to the following procedures:

### Step 1: (2,6-dichloropyrimidin-4-yl)-phenyl-methanone (compound 77a)

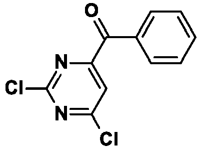

A solution of methyl 2,6-dichloropyrimidine-4-carboxylate (1.1 g, 5.31 mmol) in THF (1.5 mL) was cooled to -78 °C under N₂ and treated with PhMgBr (5.31 mL, 5.31 mmol) dropwise over a period of 0.5 h. The reaction was stirred at -78 °C for 30 min and then quenched with water (5 mL) and brine (5 mL). The mixture was extracted with EA (10 mL) twice and the organic phase was dried and concentrated to give compound 77a (500 mg, 37.2%). MS obsd.: 253.2 (MH⁺).

### Step 2: 2,4-dichloro-6-[difluoro(phenyl)methyl]pyrimidine (compound 77b)

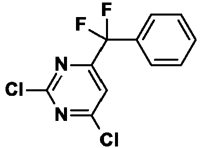

Compound 77a (0.500 g, 1.98 mmol) and DAST (15.9 g, 13 mL, 98.4 mmol) was stirred at rt for 4 hrs. The mixture was cooled at 0 °C and quenched with water (10 mL). The mixture was extracted with DCM (20 mL). The organic phase was dried and concentrated to give the crude product (545 mg, 100% yield).

### Step 3: tert-butyl N-[1-[4-[4-[2-chloro-6-[difluoro(phenyl)methyl]pyrimidin-4-yl] piperazin-1-yl]sulfonylphenyl]-5-oxo-pyrrolidin-3-yl]carbamate (compound 77c)

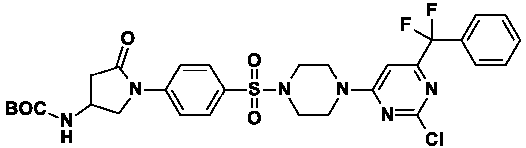

### tert-butyl N-[1-[4-[4-[4-chloro-6-[difluoro(phenyl)methyl]pyrimidin-2-yl]piperazin-1-yl] sulfonylphenyl]-5-oxo-pyrrolidin-3-yl]carbamate (compound 78c)

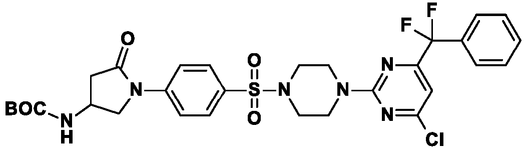

A mixture of intermediate C (23.1 mg, 54.5 µmol), compound 77a (0.015 g, 54.5 µmol) and DIPEA (21.1 mg, 29.2 µL, 164 µmol) in THF (200 µL) was stirred at 70 °C overnight. The mixture was concentrated to give the mixture of compounds 77c and 78c, which was used directly in next step. MS obsd.: 663.7 (MH⁺).

### Step 4: 4-amino-1-[4-[4-[2-chloro-6-[difluoro(phenyl)methyl]pyrimidin-4-yl]piperazin-1-vl] sulfonylphenyl]pyrrolidin-2-one (Example 77) and 4-amino-1-[4-[4-[4-chloro-6-[difluoro (phenyl)methyl]pyrimidin-2-yl]piperazin-1-yl]sulfonylphenyl]pyrrolidin-2-one (Example 78)

A mixture of compounds 77c and 78c (0.035 g, 52.8 µmol) and TFA (181 mg) in DCM (1 mL) was stirred at 25 °C for 1h. The mixture was concentrated and purified by pre-HPLC to give **Example 77** (24 mg, 80% yield) and **Example 78** (5 mg, 16.8% yield) as white solids.

**Example 77:** MS obsd.: 563.3 (MH⁺). ¹H NMR (400 MHz, DMSO-*d₆*): *δ* ppm 7.88 - 7.95 (m, *J* = 8.8 Hz, 2H), 7.69 - 7.79 (m, *J* = 8.8 Hz, 2H), 7.45 - 7.58 (m, 5H), 7.19 (s, 1H), 3.88 - 4.08 (m, 1H), 3.82 (br s, 4H), 3.56 - 3.74 (m, 1H), 3.45 - 3.50 (m, 1H), 3.18 (br s, 1H), 3.00 (br t, *J* = 4.8 Hz, 4H), 2.76 (dd, *J =* 6.8, 16.8 Hz, 1H), 2.25 (dd, *J =* 4.0, 16.8 Hz, 1H).

**Example 78:** MS obsd.: 563.6 (MH⁺). ¹H NMR (400 MHz, DMSO-*d₆*): δ ppm 7.85 - 7.93 (m, *J* = 8.8 Hz, 2H), 7.67 - 7.77 (m, *J* = 8.8 Hz, 2H), 7.43 - 7.62 (m, 5H), 7.08 (s, 1H), 3.97 (br dd, *J =* 6.0, 9.6 Hz, 1H), 3.72 - 3.84 (m, 4H), 3.57 - 3.71 (m, 1H), 3.44 - 3.49 (m, 1H), 2.94 (br t, *J* = 4.8 Hz, 4H), 2.54 - 2.581 (m, 1H), 2.24 (dd, *J* = 4.0, 16.8 Hz, 1H).

### Examples 79 and 80

### 4-Amino-1-[4-[4-[2-cyclopropyl-6-[difluoro(phenyl)methyl]pyrimidin-4-yl]piperazin-1-yl]sulfonylphenyl]pyrrolidin-2-one

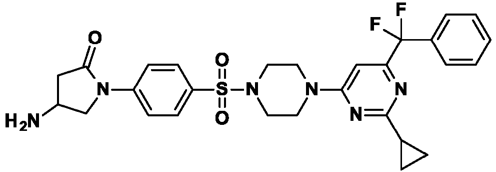

### 4-Amino-1-[4-[4-[4-cyclopropyl-6-[difluoro(phenyl)methyl]pyrimidin-2-yl]piperazin-1-yl]sulfonylphenyl]pyrrolidin-2-one

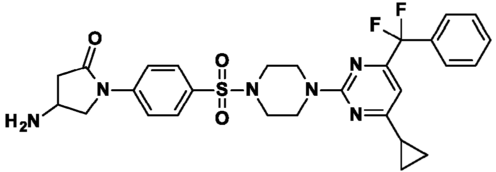

The title compounds were prepared according to the following procedures:

### Step 1: tert-butyl 4-[2-chloro-6-[difluoro(phenyl)methyl]pyrimidin-4-yl]piperazine-1-carboxylate (compound 79a)

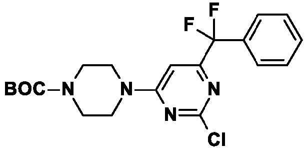

### tert-butyl 4-[4-chloro-6-[difluoro(phenyl)methyl]pyrimidin-2-yl]piperazine-1-carboxylate (compound 80a)

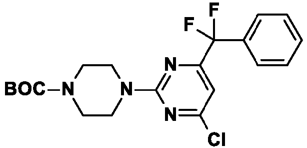

A mixture of *tert-butyl* piperazine-1-carboxylate (339 mg, 1.82 mmol), compound 77b (0.50 g, 1.82 mmol) and K₂CO₃ (502 mg, 3.64 mmol) in DMSO (10 mL) was stirred at 85 °C overnight. The mixture was diluted with EA (20 mL) and washed with water (20 mL). The organic was dried, concentrated to give the mixture of compound 79a and 80a (772 mg, 100% yield) which was used in next step without further purification. MS obsd.: 425.3 (MH⁺).

### Step 2: tert-butyl 4-[2-cyclopropyl-6-[difluoro(phenyl)methyl]pyrimidin-4-yl]piperazine-1-carboxylate (compound 79b)

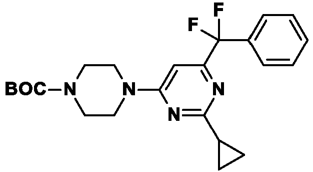

### tert-butyl 4-[4-cyclopropyl-6-[difluoro(phenyl)methyl]pyrimidin-2-yl]piperazine-1-carboxylate(compound 80b)

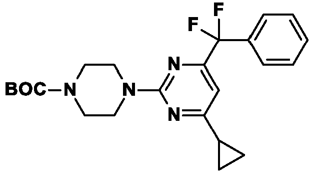

A mixture of compound 79a and 80a (0.10 g, 235 µmol), potassium cyclopropyltrifluoro-borate (104 mg, 706 µmol), Pd(dppf)Cl₂(17 mg, 23.5 µmol) and K₂CO₃ (97.4 mg, 706 µmol) in 1, 4-dioxane (1mL) and water (0.2 mL) was stirred at 70 °C overnight. The mixture was diluted with EA and washed with water. The organic was concentrated to give the crude product (100 mg, 100% yield), which was used directly in next step. MS obsd.: 431.3 (MH⁺).

### Step 3: 2-cyclopropyl-4-[difluoro(phenyl)methyl]-6-piperazin-1-yl-pyrimidine (compound 79c)

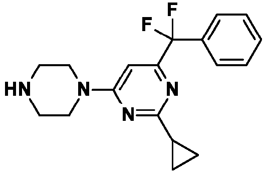

### 4-cyclopropyl-6-[difluoro(phenyl)methy]-2-piperazin-1-yl-pyrimidine (compound 80c)

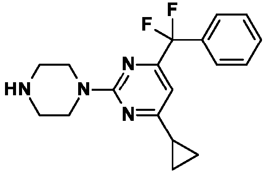

A mixture of compounds 79b and 80b (0.100 g, 232 µmol) and TFA (750 mg, 0.5 mL, 6.58 mmol) in DCM (1mL) was stirred at 25 °C for 1 h. The mixture was concentrated to give the crude product (76 mg, 100% yield), which was used directly in next step. MS obsd.: 331.2 (MH⁺).

### Step 4: 2-cyclopropyl-4-[difluoro(phenyl)methyl]-6-[4-(4-iodophenyl)sulfonylpiperazin-1-yl]pyrimidine (compound 79d)

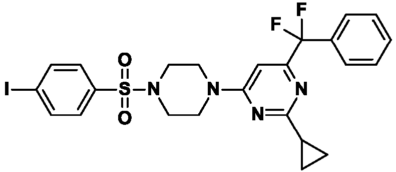

### 4-cyclopropyl-6-[difluoro(phenyl)methyl]-2-[4-(4-iodophenyl)sulfonylpiperazin-1-yllpyrimidine (compound 80d)

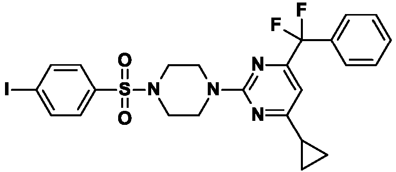

A solution of 4-iodobenzenesulfonyl chloride (104 mg, 345 µmol) and a mixture of compounds 79c and 80c (0.076 g, 230 µmol), TEA (140 mg, 192 µl, 1.38 mmol) was stirred at rt for 1h. The mixture was diluted with EA (20 mL) and washed with water (10 mL) two times. The organic was dried and concentrated to give the crude (137 mg, 100% yield). MS obsd.: 597.3 (MH⁺).

### Step 5: tert-butyl N-[1-[4-[4-[2-cyclopropyl-6-[difluoro(phenyl)methyl]pyrimidin-4-yl]piperazin -1-yl]sulfonylphenyl]-5-oxo-pyrrolidin-3-yl]carbamate (compound 79e)

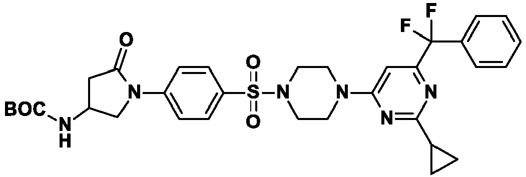

### tert-butyl N-[1-[4-[4-[4-cyclopropyl-6-[difluoro(phenyl)methyl]pyrimidin-2-yl]piperazin-1-yl]sulfonylphenyl]-5-oxo-pyrrolidin-3-yl]carbamate (compound 80e)

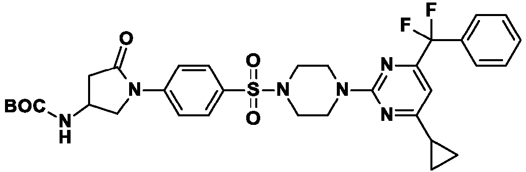

A mixture of compounds 79d and 80d (0.137 g, 230 µmol), *tert-butyl* (5-oxopyrrolidin-3-yl)carbamate (69 mg, 345 µmol), DMEDA (4.05 mg, 45.9 µmol), CuI (8.75 mg, 45.9 µmol) and K₃PO₄ (122 mg, 574 µmol) in DMF (1.5 mL) was heated at 90 °C overnight. This reaction was diluted with EA (20 mL), washed with water (10 mL) two times. The organic was dried, concentrated to give the crude products (150 mg, 98% yield) which was used directly in next step. MS obsd.: 669.4 (MH⁺).

### Step 6: 4-amino-1-[4-[4-[2-cyclopropyl-6-[difluoro(phenyl)methyl]pyrimidin-4-yl] piperazin-1-yll sulfonylphenyl]pyrrolidin-2-one(Example 79) and 4-amino-1-[4-[4-[4-cyclopropyl-6-[difluoro(phenyl)methyl]pyrimidin-2-yl]piperazin-1-yl]sulfonylphenyl]pyrrolidin-2 one(Example 80)

A mixture of compounds 79e and 80e (0.150 g, 224 µmol) and TFA (750 mg, 0.5 mL, 6.58 mmol) in DCM (2 mL) was stirred at 25 °C for 2 hrs. The mixture was concentrated and purified by pre-HPLC to give **Example 79** (15 mg, 11.8%) and **Example 80** (3.5 mg, 2.7%) as white solids.

**Example 79:** MS obsd.: 569.3 (MH⁺). ¹H NMR (400 MHz, DMSO-*d₆*): δ ppm 7.83 (d, *J* = 8.8 Hz, 2H), 7.67 (d, *J =* 8.8 Hz, 2H), 7.36 - 7.49 (m, 5H), 6.83 (s, 1H), 3.93 (br dd, *J =* 6.4, 9.6 Hz, 2H), 3.69 (br s, 4H), 2.87 (br t, *J* = 4.4 Hz, 4H), 2.71 (br dd, *J =* 7.2, 16.8 Hz, 1H), 2.57 - 2.64 (m, 1H), 2.212 - 2.29 (m, 1H), 1.80 - 1.86 (m, 1H), 0.71 - 0.84 (m, 4H).

**Example 80:** MS obsd.: 569.3 (MH⁺). ¹H NMR (400 MHz, DMSO-*d₆*): δ ppm 7.82 (d, *J* = 8.8 Hz, 2H), 7.65 (d, *J =* 8.8 Hz, 2H), 7.45 - 7.49 (m, 2H), 7.37 - 7.45 (m, 3H), 6.87 (s, 1H), 3.92 (br dd, *J =* 6.4, 9.6 Hz, 2H), 3.67 (br s, 4H), 2.78 - 2.83 (m, 4H), 2.71 (br dd, *J =* 7.2, 16.8 Hz, 2H), 2.14 - 2.26 (m, 1H), 1.91 - 2.05 (m, 1H), 0.81 - 1.03 (m, 4H).

### Examples 81 and 82

### 4-Amino-1-[4-[4-[6-[difluoro(phenyl)methyl]-2-methoxy-pyrimidin-4-yl]piperazin-1-yl]sulfonylphenyl]pyrrolidin-2-one

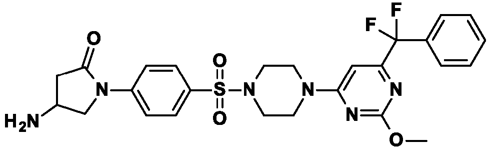

### 4-Amino-1-[4-[4-[4-[difluoro(phenyl)methyl]-6-methoxy-pyrimidin-2-yl]piperazin-1-yl]sulfonylphenyl]pyrrolidin-2-one

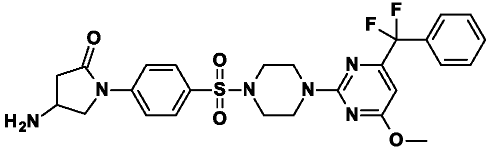

The title compounds were prepared according to the following procedures:

### Step 1: tert-butyl 4-[4-[difluoro(phenyl)methyl]-2-methoxy-pyrimidin-2-yl]piperazine-1-carboxylate(compound 81a)

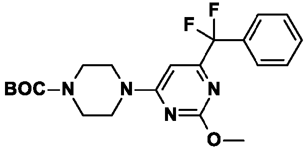

### tert-butyl 4-[6-[difluoro(phenyl)methyl]-6-methoxy-pyrimidin-4-yl]piperazine-1-carboxylate(Intermediate 82a)

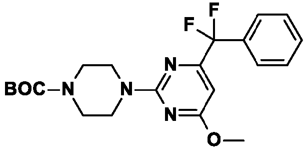

A mixture of compounds 79a and 80a (0.10 g, 235 µmol) and sodium methanolate (94.1 µL, 471 µmol) in MeOH (1 mL) was stirred at 80 °C overnight. This mixture was concentrated and diluted with EA (5 mL) and washed with water (10 mL). The organic layer was dried, concentrated to give the crude (99 mg, 100% yield) which was used directly in next step. MS obsd.: 421.5 (MH⁺).

### Step 2: 4-amino-1-[4-[4-[6-[difluoro(phenyl)methyl]-2-methoxy-pyrimidin-4-yl]piperazin-1-yl]sulfonylphenyl]pyrrolidin-2-one (Example 81) and 4-amino-1-[4-[4-[4 [difluoro(phenyl) methyl]-6-methoxy-pyrimidin-2-yl]piperazin-1-yl]sulfonylphenyl]pyrrolidin-2-one (Example 82)

The title compounds were prepared in analogy to the preparation of **Example 79** and **80** by using the mixture of compounds 81a and 82a instead of the mixture of compounds 79e and 80e. **Examples 81** (28 mg) and **82** (4.2 mg) were obtained as white solids.

**Example 81:** MS obsd.: 559.3 (MH⁺). ¹H NMR (400 MHz, DMSO-*d₆*): δ ppm 7.91 (d, *J* = 8.8 Hz, 2H), 7.74 (d, *J =* 8.8 Hz, 2H), 7.43 - 7.57 (m, 5H), 6.82 (s, 1H), 4.00 (br dd, *J =* 6.0, 9.6 Hz, 2H), 3.79 (br s, 4H), 3.73 (s, 3H), 3.67 - 3.68 (m, 1H), 2.96 (br t, *J =* 4.4 Hz, 4H), 2.79 (dd, *J =* 7.2, 16.8 Hz, 1H), 2.19 - 2.36 (m, 1H).

**Example 82:** MS obsd.: 559.4 (MH⁺). ¹H NMR (400 MHz, DMSO-*d₆*): δ ppm 7.82 (br d, *J* = 8.8 Hz, 2H), 7.65 (d, *J* = 8.8 Hz, 2H), 7.41 - 7.49 (m, 5H), 6.30 (s, 1H), 3.92 (br dd, *J* = 6.0, 9.6 Hz, 2H), 3.77 (s, 3H), 3.72 (br s, 4H), 2.83 (br s, 4H), 2.64 - 2.76 (m, 1H), 2.18 (br dd, *J* = 4.0, 16.8 Hz, 1H), 2.00 (s, 1H).

### Example 83

### (4R)-4-Amino-1-[4-[4-[6-[difluoro(phenyl)methyl]-2-methyl-pyrimidin-4-yl]piperazin-1-yl]sulfonylphenyl]pyrrolidin-2-one

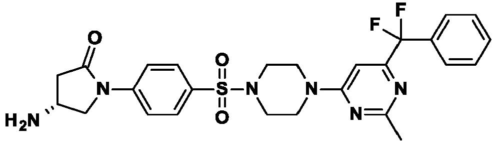

The title compound was prepared according to the following procedures:

### Step 1: tert-butyl 4-[6-[difluoro(phenyl)methyl]-2-methyl-pyrimidin-4-yl]piperazine-1-carboxylate (compound 83a)

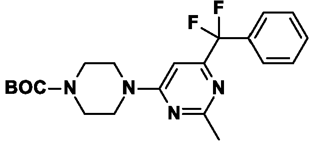

A mixture of compound 79a (0.100 g, 235 µmol), 2,4,6-trimethyl-1,3,5,2,4,6-trioxatriborinane (202 µL, 706 µmol), Na₂CO₃ (62.4 mg, 588 µmol) and Pd(PPh₃)₄ (27 mg) in 1,4-dioxane (0.5 mL) and water (0.1 mL) was stirred at 130 °C overnight. The mixture was concentrated and the residue was used directly in next step. MS obsd.: 405.3 (MH⁺).

### Step 2: (4R)-4-amino-1-[4-[4-[6-[difluoro(phenyl)methyl]-2-methyl-pyrimidin-4-yl] piperazin-1-yl]sulfonylphenyl]pyrrolidin-2-one(Example 83)

The title compound was prepared in analogy to the preparation of **Example 79** by using compound 83a instead of compound 79e and intermediate D instead of intermediate C. **Example 83** (12 mg) was obtained as a white solid. MS obsd.: 543.2 (MH⁺). ¹H NMR (400 MHz, DMSO-*d₆*): δ ppm 7.80 - 7.87 (m, *J =* 8.8 Hz, 2H), 7.62 - 7.72 (m, *J =* 8.8 Hz, 2H), 7.36 - 7.49 (m, 5H), 6.91 (s, 1H), 3.91 (br dd, *J =* 6.0, 9.6 Hz, 2H), 3.73 (br s, 4H), 3.57 (br s, 1H), 2.88 (br t, *J* = 4.4 Hz, 4H), 2.69 (dd, *J =* 6.8, 16.8 Hz, 1H), 2.24 (s, 3H), 2.17 (dd, *J =* 4.0, 16.8 Hz, 1H).

### Example 84

### 4-Amino-1-[4-[4-[6-[difluoro(phenyl)methyl]-2-vinyl-pyrimidin-4-yl]piperazin-1-yl]sulfonylphenyl]pyrrolidin-2-one

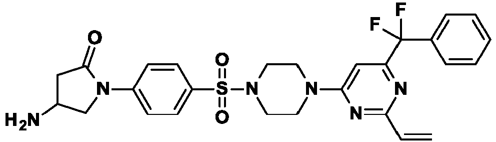

The title compound was prepared in analogy to the preparation of **Example 79** by using 4,4,5,5-tetramethyl-2-vinyl-1,3,2-dioxaborolane instead of potassium cyclopropyltrifluoro-borate. **Example 84** (2 mg) was obtained as a white solid. MS obsd.: 555.3 (MH⁺). ¹H NMR (400 MHz, CD₃OD): δ ppm 7.86 - 7.97 (m, 2H), 7.80 - 7.84 (m, 2H), 7.54 - 7.61 (m, 2H), 7.44 - 7.53 (m, 3H), 7.00 (s, 1H), 6.58 - 6.66 (m, 1H), 6.53 (d, *J* = 2.0 Hz, 1H), 5.66 (br dd, *J* = 2.0, 10.2 Hz, 1H), 4.40 (br dd, *J* = 7.2, 11.2 Hz, 1H), 4.21 (br t, *J* = 7.2 Hz, 1H), 3.97 (br dd, *J =* 2.0, 11.2 Hz, 1H), 3.90 (br s, 4H), 3.20 (br dd, *J =* 8.4, 18.0 Hz, 1H), 3.06 - 3.14 (m, 4H), 2.72 (br dd, *J =* 2.8, 18.0 Hz, 1H).

### Example 85

### 2-[4-[4-[6-Chloro-4-[difluoro(phenyl)methyl]-2-pyridyl]piperazin-1-yl]sulfonylphenyl]-2,6,9-triazaspiro[4.5]decan-3-one

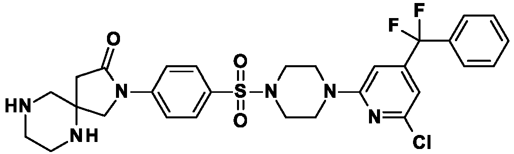

The title compound was prepared according to the following procedures:

### Step 1: tert-butyl 4-[6-chloro-4-[difluoro(phenyl)methyl]-2-pyridyl]piperazine-1-carboxylate(compound 85a)

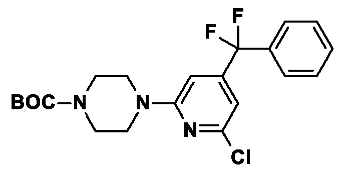

Compound 73c (150 mg, 547 µmol) and *tert-butyl* piperazine-1-carboxylate (204 mg, 1.09 mmol) were dissolved in DMSO (5 mL), followed by adding K₂CO₃ (227 mg, 1.64 mmol). The reaction was stirred at 90 °C overnight. The mixture was partitioned between EA (300 mL) and water (300 mL). The organic layer was combined, wash with water (100 mL) three times and brine (300 mL), dried over anhydrous Na₂SO₄, filtered and concentrated to give the crude (230 mg, 0.542 mmol, 99.1%) as yellow oil. MS obsd. 424.6: (MH⁺).

### Step 2: 1-[6-chloro-4-[difluoro(phenyl)methyl]-2-pyridyl]piperazine(compound 85b)

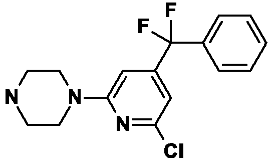

Compound 85a (0.23 g, 543 µmol) was dissolved in DCM (7.5 mL), followed by adding TFA (2.5 mL, 543 µmol). The mixture was stirred at rt for 2 hrs and then concentrated. The residue was re-dissolved in the EA (200 mL) and washed with 1M aq. K₂CO₃ solution (200 mL), brine (300 mL), dried over anhydrous Na₂SO₄, filtered and concentrated to give the crude (0.25 g, 543 µmol, 100 % yield) as a light yellow solid. MS obsd.: 324.5 (MH⁺).

### Step 3: 1-[6-chloro-4-[difluoro(phenyl)methyl]-2-pyridyl]-4-(4-iodophenyl)sulfonyl-piperazine (compound 85c)

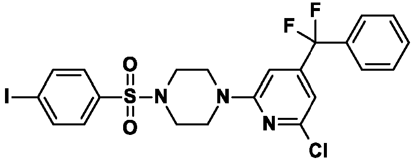

Compound 85b (50 mg, 154 µmol) was dissolved in DCM (5 mL), followed by adding TEA (64.6 µL, 463 µmol) and 4-iodobenzenesulfonyl chloride (93.4 mg, 309 µmol) and the reaction was stirred at rt for 2 hrs. The mixture was partitioned between EA (300 mL) and water (300 mL). The organic layer was combined, washed with brine (200 mL), dried over anhydrous Na₂SO₄, filtered and concentrated to give the crude (90 mg, 0.152 mmol, 99% yield) as a light yellow solid. MS obsd.: 590.1 (MH⁺).

### Step 4: di-tert-butyl 2-[4-[4-[6-chloro-4-[difluoro(phenyl)methyl]-2-pyridyl]piperazin-1-yl]sulfonylphenyl]-3-oxo-2,6,9-triazaspiro[4.5]decane-6,9-dicarboxylate (compound 85d)

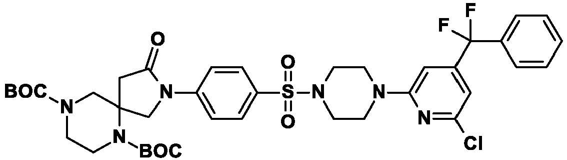

A mixture of compound 85c (0.07 g, 119 µmol), di-*tert*-butyl 3-oxo-2,6,9-triazaspiro[4.5] decane-6,9-dicarboxylate (65 mg, 183 µmol, WuXi AppTec), DMEDA (10.5 mg, 12.8 µL), CuI (6.78 mg, 35.6 µmol) and K₃PO₄ (78.7 mg, 356 µmol) in DMF (5 mL) was heated at 70 °C for 2 hrs. Then, the mixture was poured into water (250 mL), extracted with DCM (200 mL) three times. The combined organic layer was washed with brine (200 mL), dried over anhydrous Na₂SO₄, filtered and concentrated. The crude was purified by flash chromatography on silica gel (EA in PE=0-30%) to give compound 85d (90 mg, 110.2 µmol, 92.8% yield) as brown oil. MS obsd.: 817.5 (MH⁺).

### Step 5: 2-[4-[4-[6-Chloro-4-[difluoro(phenyl)methyl]-2-pyridyl]piperazin-1-yl] sulfonylphenyl]-2,6,9-triazaspiro[4.5]decan-3-one(Example 85)

Compound 85d (90 mg, 110 µmol) was dissolved in DCM (5 mL), followed by adding TFA (100 µl, 1.3 mmol). The reaction was stirred at rt for 1 h and concentrated. The crude was purified by prep-HPLC to give **Example 85** (4.15 mg, 6.72 µmol, 5% yield) as a light brown solid. MS obsd.: 617.5 (MH⁺). ¹H NMR (400 MHz, CD₃OD): δ ppm 7.90 (d, *J =* 8.8 Hz, 2 H), 7.81 (d, *J =* 8.8 Hz, 2 H), 7.42 - 7.52 (m, 5 H). 6.78 (s, 1 H), 6.66 (s, 1 H), 4.61 - 4.67 (m, 1 H), 4.04 (d, *J =* 10.4 Hz, 1 H), 3.92 (d, *J =* 10.4 Hz, 1 H), 3.63 - 3.72 (m, 4 H), 3.11 - 3.19 (m, 4 H), 3.03 - 3.10 (m, 4 H), 2.89 (d, *J =* 17.6 Hz, 1 H), 2.75 (d, *J* = 17.6 Hz, 1 H), 2.19 (t, *J*= 7.6 Hz, 1 H), 2.03 (br d, *J =* 5.6 Hz, 1 H), 1.60 (br s, 1 H).

### Example 86

### 5-(Aminomethyl)-1-[4-[4-[6-chloro-4-[difluoro(phenyl)methyl]-2-pyridyl]piperazin-1-yl]sulfonylphenyl]pyrrolidin-2-one

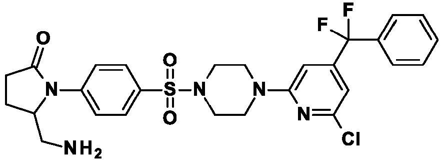

A mixture of compound 85c (91 mg, 154 µmol), 5-(aminomethyl)pyrrolidin-2-one (35.2 mg, 309 µmol), DMEDA (16.6 µL, 154 µmol), CuI (12 mg, 63 µmol) and K₃PO₄ (102 mg, 463 µmol) in DMF (5 mL) was heated at 70 °C for 2 hrs. Then, the mixture was poured into water (200 mL), extracted with DCM (200 mL) three times. The organic layer was combined, washed with brine (200 mL), dried over anhydrous Na₂SO₄, filtered and concentrated. The crude was purified by prep-HPLC to give **Example 86** (6 mg, 8.7 µmol, 5.35% yield) as a white solid. MS obsd.: 576.2 (MH⁺). ¹H NMR (400 MHz, DMSO-*d₆*): δ ppm 7.88 (br d, *J =* 9.2 Hz, 4 H), 7.75 - 7.83 (m, 2 H), 7.46 - 7.65 (m, 5 H), 6.93 - 6.97 (m, 1 H), 6.76 (s, 1 H), 4.61 - 4.67 (m, 1 H), 3.63 - 3.71 (m, 4 H), 2.93 - 3.06 (m, 4 H), 2.60 - 2.80 (m, 2 H), 2.52 - 2.52 (m, 1 H), 2.39 - 2.47 (m, 1 H), 2.26 - 2.36 (m, 1 H), 2.04 - 2.13 (m, 1 H).

### Example 87

### (3aR,7aS)-3-[4-[4-[6-chloro-4-[difluoro(phenyl)methyl]-2-pyridyl]piperazin-1-yl]sulfonyl phenyl]-3a,4,5,6,7,7a-hexahydrooxazolo[4,5-c]pyridin-2-one

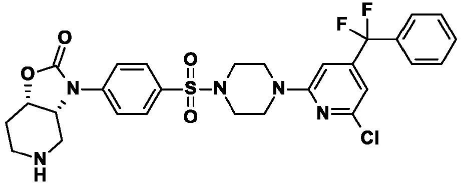

The title compound was prepared in analogy to the preparation of Example 85 by using *tert-butyl* (3aR,7aS)-2-oxohexahydrooxazolo[4,5-c]pyridine-5(4H)-carboxylate instead of di-tert-butyl 3-oxo-2,6,9-triazaspiro[4.5]decane-6,9-dicarboxylate. Example 87 (23 mg) was obtained as a white solid. MS obsd.: 585.3 (MH⁺). ¹H NMR (400 MHz, DMSO-*d₆*): *δ* ppm 7.78 (s, 4H), 7.52 - 7.56 (m, 2H), 7.45 - 7.52 (m, 2H), 7.37 - 7.60 (m, 1H), 6.90 (s, 1H), 4.99 (td, *J =* 4.0, 7.6 Hz, 1H), 4.84 - 4.94 (m, 1H), 3.81 (br s, 4H), 3.50 (br dd, *J* = 8.8, 13.2 Hz, 1H), 3.15 - 3.27 (m, 2H), 3.08 (br d, *J =* 9.6 Hz, 1H), 2.88 - 3.01 (m, 4H), 2.17 - 2.18 (m, 1H), 2.07 (s, 1H).

### Example 88

### 5-(Aminomethyl)-3-[4-[4-[6-chloro-4-[difluoro(phenyl)methyl]-2-pyridyl]piperazin-1-yl]sulfonylphenyl]oxazolidin-2-one

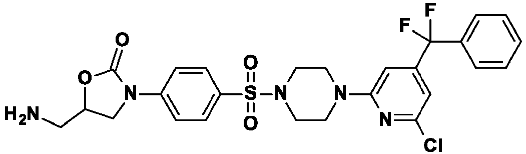

The title compound was prepared in analogy to the preparation of **Example 85** by using *tert-butyl* ((2-oxooxazolidin-5-yl)methyl)carbamate instead of di-tert-butyl 3-oxo-2,6,9-triazaspiro[4.5] decane-6,9-dicarboxylate. **Example 88** (21 mg) was obtained as a white solid. MS obsd.: 559.3 (MH⁺). ¹H NMR (400 MHz, DMSO-*d₆*): *δ* ppm 7.71 - 7.85 (m, 4H), 7.45 - 7.58 (m, 5H), 6.89 (s, 1H), 4.84 - 4.97 (m, 1H), 4.22 (t, *J =* 9.2 Hz, 1H), 3.86 - 3.88 (m, 1H), 3.78 - 3.89 (m, 4H), 3.13 - 3.29 (m, 2H), 2.91 (br t, *J =* 4.8 Hz, 4H), 2.33 (s, 3H).

### Example 89

### 4-Amino-1-[4-[4-[6-chloro-4-[difluoro(2-pyridyl)methyl]-2-pyridyl]piperazin-1-yl] sulfonylphenyl] pyrrolidin-2-one

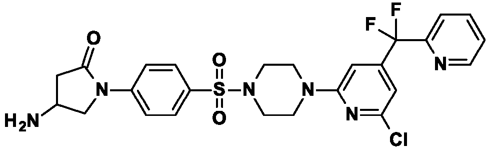

The title compound was prepared according to the following procedures:

### Step 1: (2,6-dichloro-4-pyridyl)-(2-pyridyl)methanone (compound 89a)

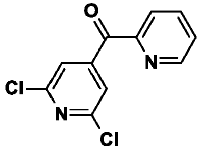

To a solution of 2-bromopyridine (0.41 mL, 4.25 mmol) and 2,6-dichloro-N-methoxy-N-methyl-pyridine-4-carboxamide (1000.0 mg, 4.25 mmol) in THF (20 mL) was added n-BuLi (1.77 mL, 4.25 mmol) at -70 °C for 40 min under N₂. The mixture was stirred at -70 °C for 4 hrs and then quenched with saturated NH₄Cl (50 mL), extracted with EA (30 mL × 3). The organic layer was dried over Na₂SO₄, filtered and concentrated. The crude was purified by flash chromatography on silica gel (EA in PE= 0~30%) to give compound 89a (450 mg, 1.78 mmol, 37.62% yield) as a white solid. MS obsd.: 253 (MH⁺).

### Step 2: 2,6-dichloro-4-[difluoro(2-pyridyl)methyl]pyridine (compound 89b)

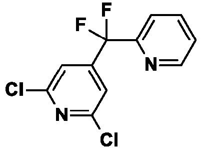

Compound 89a (150.0 mg, 0.590 mmol) was dissolved in BAST (2.0 mL) and the reaction was stirred at 25 °C for 12 hrs. The mixture was quenched with ice water (20 mL) and extracted with EA (20 mL) two times. The combined organic layer was dried over Na₂SO₄, filtered and concentrated. The crude was purified by flash chromatography on silica gel (EA in PE= 0-50%) to give compound 89b (85 mg, 0.310 mmol, 46.9% yield) as a yellow solid. MS obsd.: 275.1 (MH⁺).

### Step 3: tert-butyl N-[1-[4-[4-[6-chloro-4-[difluoro(2-pyridyl)methyl]-2-pyridyl]piperazin-1-yl]sulfonylphenyl]-5-oxo-pyrrolidin-3-yl]carbamate (compound 89c)

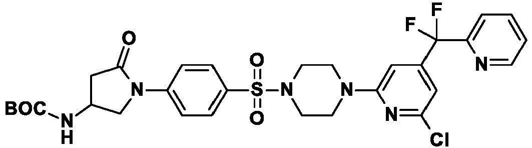

Compound 89b (85.0 mg, 0.310 mmol) and intermediate D (131.17 mg, 0.310 mmol) was dissolved in a mixed solution of DMSO (0.5 mL) and DIPEA (2.0 mL). The reaction was stirred at 115 °C for 12 h. The reaction was quenched with brine (20 mL) and extracted with DCM (20 mL) twice. The combined organic layer was dried over Na₂SO₄, filtered and concentrated. The crude was purified by flash chromatography on silica gel (EA in PE=0-70%) to give compound 89c (120 mg, 0.180 mmol, 52.71% yield) as a yellow solid. MS obsd.: 663.2 (MH⁺).

### Step 4: 4-amino-1-[4-[4-[6-chloro-4-[difluoro(2-pyridyl)methyl]-2-pyridyl]piperazin-1-yl]sulfonylphenyl]pyrrolidin-2-one (Example 89)

Compound 89c (100.0 mg, 0.150 mmol) was dissolved in 2 M HCl/EA (5 mL). The reaction mixture was stirred at rt for 12 hrs. The solvent was removed and the crude was purified by prep-HPLC to give **Example 89** (83 mg, 0.150 mmol, 96.8% yield) as a white solid. MS obsd.: 563.2 (MH⁺). ¹H NMR (400 MHz, DMSO-*d₆*): δ ppm 8.63 (d, *J =* 4.4 Hz, 1H), 8.02 (td, *J* = 7.6, 1.6 Hz, 1H), 7.92 (d, *J =* 8.8 Hz, 2H), 7.86 (d, *J =* 7.8 Hz, 1H), 7.76 (d, *J =* 8.8 Hz, 2H), 7.55 (dd, *J =* 7.2, 5.2 Hz, 1H), 6.92 (s, 1H), 6.76 (s, 1H), 4.11 (dd, *J =* 10.4, 6.4 Hz, 1H), 3.84 (dd, *J =* 8.4, 5.2 Hz, 1H), 3.66 (d, *J =* 5.2 Hz, 5H), 2.86 - 3.01 (m, 5H), 2.42 (dd, *J* = 17.2, 3.2 Hz, 1H).

### Example 90

### 4-Amino-1-[4-[4-[6-chloro-4-[difluoro(3-pyridyl)methyl]-2-pyridyl]piperazin-1-yl]sulfonylphenyl] pyrrolidin-2-one

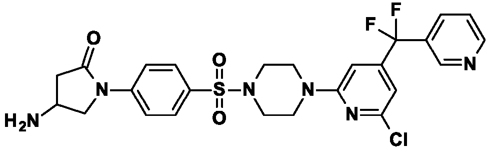

The title compound was prepared in analogy to the preparation of **Example 89** by using 3-bromopyridine instead of 2-bromopyridine. **Example 90** (60 mg) was obtained as a white solid. MS obsd.: 563.1 (MH⁺). ¹H NMR (400 MHz, DMSO-*d₆*): δ ppm 8.85 (s, 1H), 8.73 (d, *J =* 4.4 Hz, 1H), 8.30 (s, 2H), 8.06 (d, *J* = 8.0 Hz, 1H), 7.92 (d, *J* = 8.8 Hz, 2H), 7.80 (d, *J* = 8.8 Hz, 2H), 7.54 (dd, *J =* 8.0, 4.8 Hz, 1H), 7.01 (s, 1H), 6.87 (s, 1H), 4.27 (dd, *J =* 11.2, 6.9 Hz, 1H), 4.09 (s, 1H), 3.83 (d, *J =* 10.0 Hz, 1H), 3.68 (s, 4H), 3.08 (dd, *J* = 18.0, 8.0 Hz, 1H), 2.96 (s, 4H), 2.59 (dd, *J =* 18.0, 2.4 Hz, 1H).

### Example 91

### 4-Amino-1-[4-[4-[6-chloro-4-[difluoro(4-pyridyl)methyl]-2-pyridyl]piperazin-1-yl] sulfonylphenyl]pyrrolidin-2-one

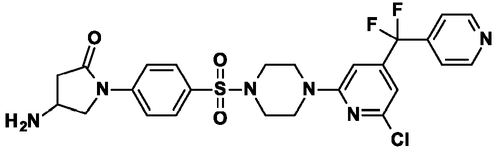

The title compound was prepared in analogy to the preparation of **Example 89** by using 4-bromopyridine instead of 2-bromopyridine. **Example 91** (22 mg) was obtained as a white solid. MS obsd. 563.2 (MH⁺). ¹H NMR (400 MHz, DMSO-*d₆*): δ ppm 8.78 (s, 2H), 8.37 (s, 2H), 7.91 (d, *J* = 8.8 Hz, 2H), 7.79 (d, *J =* 8.8 Hz, 2H), 7.67 (s, 2H), 6.99 (s, 1H), 6.85 (s, 1H), 4.26 (dd, *J* = 11.2, 6.8 Hz, 1H), 4.09 (s, 1H), 3.82 (s, 1H), 3.68 (d, *J =* 4.0 Hz, 4H), 3.08 (dd, *J =* 18.0, 8.2 Hz, 1H), 2.96 (s, 4H), 2.60 (dd, *J =* 18.0, 2.4 Hz, 1H).

### Example 92

### 4-Amino-1-[4-[4-[6-chloro-4-[difluoro(pyrimidin-5-yl)methyl]-2-pyridyl]piperazin-1-yl]sulfonylphenyl]pyrrolidin-2-one

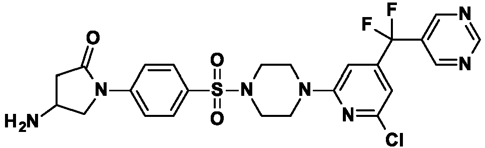

The title compound was prepared in analogy to the preparation of **Example 89** by using 4-bromopyridine instead of 5-bromopyrimidine. **Example 92** (19.5 mg) was obtained as a white solid. MS obsd. MS obsd.: 563.2 (MH⁺). ¹H NMR (400 MHz, DMSO-*d₆*): δ ppm ¹H NMR (400 MHz, DMSO) 9.36 (s, 1H), 9.12 (s, 2H), 7.92 (d, *J =* 8.8 Hz, 2H), 7.79 (d, *J =* 8.8 Hz, 2H), 7.03 (s, 1H), 6.95 (s, 1H), 4.25 (dd, *J =* 11.2, 6.8 Hz, 1H), 4.07 (br s, 1H), 3.82 (br s, 1H), 3.68 (br s, 4H), 3.31 (br s, 1H), 3.11 - 3.03 (m, 1H), 2.97 (d, *J =* 4.4 Hz, 4H).

### Example 93

### 4-Amino-1-[4-[4-[6-chloro-4-[difluoro(pyrazin-2-yl)methyl]-2-pyridyl]piperazin-1-yl]sulfonylphenyl]pyrrolidin-2-one

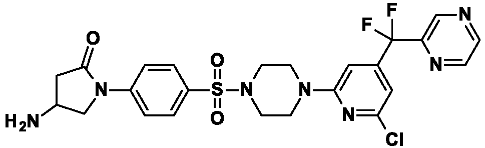

The title compound was prepared in analogy to the preparation of **Example 89** by using 2-bromopyrazine instead of 2-bromopyridine. **Example 92** (50 mg) was obtained as a white solid. MS obsd.: 564 (MH⁺). ¹H NMR (400 MHz, DMSO-*d₆*): δ ppm 9.17 (t, *J =* 10.0 Hz, 1H), 8.86 (d, *J =* 2.4 Hz, 1H), 8.74 (s, 1H), 7.91 (d, *J =* 8.8 Hz, 2H), 7.75 (d, *J =* 8.8 Hz, 2H), 6.97 (d, *J =* 14.0 Hz, 1H), 6.84 (s, 1H), 4.03 (dd, J = 10.0, 6.0 Hz, 1H), 3.62 - 3.68 (m, 5H), 3.52 - 3.56 (m, 1H), 2.88 - 3.03 (m, 4H), 2.81 (dd, *J =* 17.2, 7.2 Hz, 1H), 2.30 (dd, *J =* 17.2, 3.6 Hz, 1H).

### Example 94

### 4-Amino-1-[4-[4-[6-chloro-4-[difluoro(pyrimidin-2-yl)methyl]-2-pyridyl]piperazin-1-yl]sulfonylphenyl]pyrrolidin-2-one

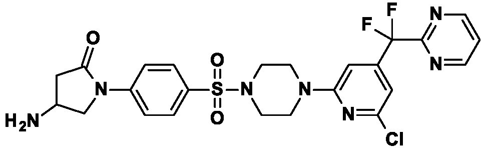

The title compound was prepared according to the following procedures:

### Step 1: 2,6-dichloro-4-pyridyl)-pyrimidin-2-yl-methanone (compound 93a)

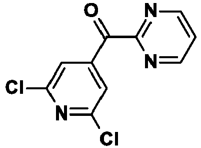

To a solution of n-BuLi (2.82 mL, 6.76 mmol) in THF (30 mL) was added 2,6-dichloropyridine (1.0 g, 6.76 mmol) at -78 °C and then stirred for 30 min under N₂. A solution of methyl pyrimidine-2-carboxylate (0.26 mL, 6.76 mmol) in THF (15 mL) was added dropwise. The reaction was stirred for further 5 hrs at -78 °C under N₂. The reaction mixture was quenched with aq. NH₄Cl (100 mL) and extracted with EA (40 mL) three times. The combined organic layer was washed with brine (80 mL), dried over Na₂SO₄, filtered and concentrated. The crude was purified by silica gel (EA in PE=0~15%) to give compound 93a (450 mg, 1.77 mmol, 23.59% yield) as a colorless oil. MS obsd.: 254 (MH⁺).

### Step 2: 4-amino-1-[4-[4-[6-chloro-4-[difluoro(pyrimidin-2-yl)methyl]-2-pyridyl]piperazin-1-yl]sulfonylphenyl]pyrrolidin-2-one (Example 93)

The title compound was prepared in analogy to the preparation of **Example 89** by using compound 93a instead of compound 89b. **Example 93** (7 mg) was obtained as a white solid. MS obsd.: 564.1 (MH⁺). ¹H NMR (400 MHz, DMSO-*d₆*): *δ* ppm 8.96 (d, *J =* 4.8 Hz, 2H), 7.91 (d, *J* = 8.8 Hz, 2H), 7.63 - 7.78 (m, 3H), 6.90 (s, 1H), 6.76 (s, 1H), 3.95 - 4.05 (m, 1H), 3.64 (s, 5H), 3.49 (d, *J* = 7.2 Hz, 1H), 2.94 (s, 4H), 2.77 (dd, *J =* 16.8, 6.8 Hz, 1H), 2.22 - 2.33 (m, 1H).

### Example 95

### 4-Amino-1-[4-[4-[6-chloro-4-[difluoro(tetrahydropyran-4-yl)methyl]-2-pyridyl]piperazin-1-yl]sulfonylphenyl]pyrrolidin-2-one

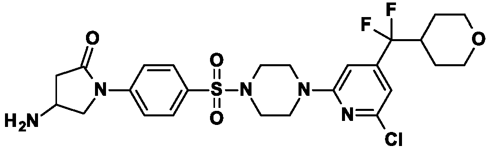

The title compound was prepared according to the following procedures:

### Step 1: N-methoxy-N-methyl-tetrahydropyran-4-carboxamide (compound 95a)

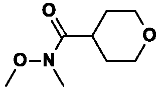

A mixture solution of tetrahydro-2H-pyran-4-carboxylic acid (2000.0 mg, 15.37 mmol) was added HATU (11688.3 mg, 30.74 mmol) and DIPEA (20 mL) in DCM (60 mL) was stirred at rt for 30 min. Then O, N-dimethylhydroxylamine (1798.8 mg, 18.44 mmol) was added and the reaction was stirred at rt for 12 hrs. The mixture was washed with brine (30 mL) three times, dried over Na₂SO₄, filtered and concentrated. The crude was purified by flash chromatography on silica gel (EA in PE=0~100%) to give compound 95a (2000 mg, 11.55 mmol, 67.62% yield) as a light yellow oil. MS obsd.: 174 (MH⁺).

### Step 2: (2,6-dichloro-4-pyridyl)-tetrahydropyran-4-yl-methanone (compound 95b)

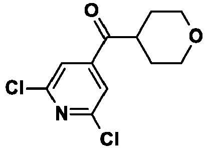

To a solution of n-BuLi (1.2 mL, 2.89 mmol) in THF (20 mL) was added 2,6-dichloropyridine (428 mg, 2.89 mmol) at -70 °C for 40 min under nitrogen and compound 95a (500.0 mg, 2.89 mmol) was added. The mixture was stirred at -70 °C for 4 hrs, quenched with saturated NH₄Cl (50 mL) and extracted with EA (30 mL × 3). The combined organic layer was dried over Na₂SO₄, filtered and concentrated. The crude was purified by flash chromatography on silica gel (EA in PE = 0-30%) to give compound 95b (200 mg, 0.770 mmol, 23.97% yield) as a white solid. MS obsd.: 260, 262 (MH⁺).

### Step 2: 2,6-dichloro-4-[difluoro(tetrahydropyran-4-yl)methyl]pyridine (compound 95c)

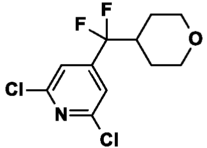

Compound 95b (200.0 mg, 0.770 mmol) was dissolved in BAST (3.0 mL) and the reaction was stirred at 25 °C for 12 hrs. The mixture was quenched with ice water (20 mL) and extracted with EA (20 mL) two times. The combined organic layer was dried over Na₂SO₄, filtered and concentrated. The crude was purified by flash chromatography on silica gel (EA in PE=0~50%) to give compound 95c (150 mg, 0.530 mmol, 62.24% yield) as a white solid. MS obsd.: 282, 284 (MH⁺).

### Step 3: tert-butyl N-[1-[4-[4-[6-chloro-4-[difluoro(tetrahydropyran-4-yl)methyl]-2-pyridyl]piperazin -1-yl]sulfonylphenyl]-5-oxo-pyrrolidin-3-yl]carbamate (compound 95d)

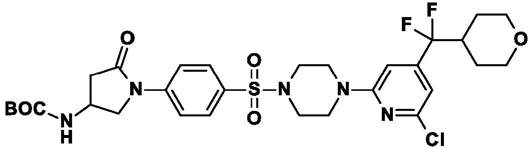

Compound 95c (150.0 mg, 0.530 mmol) and intermediate C (92.93 mg, 0.220 mmol) was dissolved in a mixed solution of DMSO (0.5 mL) and DIPEA (2.0 mL). The reaction was stirred at 115 °C for 12 h. The mixture was quenched with brine (50 mL) and extracted with DCM (30 mL) two times. The combined organic layer was dried over Na₂SO₄, filtered and concentrated. The crude was purified by flash chromatography on silica gel (EA in PE=10~70%) to give compound 95d (100 mg, 0.150 mmol, 25.26% yield) as a yellow solid. MS obsd.: 670 (MH⁺).

### Step 4: 4-amino-1-[4-[4-[6-chloro-4-[difluoro(tetrahydropyran-4-yl)methyl]-2-pyridyl] piperazin-1-yl]sulfonylphenyl]pyrrolidin-2-one (Example 95)

Compound 95d (100.0 mg, 0.150 mmol) was dissolved in 2 M HCl/EA (10 mL) and the reaction was stirred at rt for 4 hrs. The mixture was then concentrated and the crude was purified by prep-HPLC to give **Example 95** (28 mg, 0.05 mmol, 32.6% yield) as a white solid. MS obsd.: 570 (MH⁺). ¹H NMR (400 MHz, DMSO-*d₆*): *δ* ppm 7.92 (d, *J* = 8.8 Hz, 2H), 7.75 (d, *J* = 8.8 Hz, 2H), 6.79 (s, 1H), 6.71 (s, 1H), 3.98 (dd, *J =* 9.6, 6.0 Hz, 1H), 3.85 (dd, *J =* 11.2, 3.2 Hz, 2H), 3.65 (dd, *J =* 11.6, 7.2 Hz, 5H), 3.48 (dd, *J =* 9.6, 3.2 Hz, 1H), 3.24 (t, *J =* 10.8 Hz, 2H), 2.92 - 3.01 (m, 4H), 2.76 (dd, *J =* 16.8, 6.8 Hz, 1H), 2.45 (s, 1H), 2.24 (dd, *J =* 16.8, 4.0 Hz, 1H), 1.97 (s, 2H), 1.39 (dt, *J* = 12.4, 8.8 Hz, 4H).

### Example 96

### 4-Amino-1-[4-[4-[6-chloro-4-[difluoro(tetrahydrofuran-3-yl)methyl]-2-pyridyl]piperazin-1-yl]sulfonylphenyl]pyrrolidin-2-one

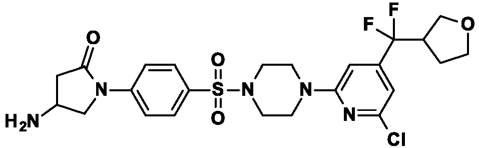

The title compound was prepared in analogy to the preparation of **Example 95** by using tetrahydrofuran-3-carboxylic acid instead of tetrahydro-2H-pyran-4-carboxylic acid. **Example 96** (160 mg) was obtained as a white solid. MS obsd.: 556 (MH⁺). ¹H NMR (400 MHz, CD₃OD): δ ppm 7.92 (d, *J* = 8.8 Hz, 2H), 7.81 (d, *J* = 8.8 Hz, 2H), 6.74 (d, *J* = 8.8 Hz, 2H), 4.35 (dd, *J* = 11.2, 6.8 Hz, 1H), 4.11 (s, 1H), 3.89 (dd, *J* = 11.2, 2.8 Hz, 1H), 3.73 - 3.84 (m, 3H), 3.60 - 3.73 (m, 5H), 2.99 - 3.18 (m, 6H), 2.63 (dd, *J* = 18.0, 3.2 Hz, 1H), 1.88 - 2.00 (m, 2H).

### Example 97

### 4-Amino-1-[4-[4-[6-chloro-4-[difluoro(oxetan-3-yl)methyl]-2-pyridyl]piperazin-1-yl]sulfonylphenyl] pyrrolidin-2-one

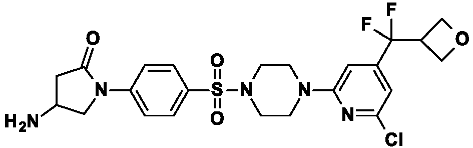

The title compound was prepared in analogy to the preparation of **Example 95** by using oxetane-3-carboxylic acid instead of tetrahydro-2H-pyran-4-carboxylic acid. **Example 97** (22 mg) was obtained as a white solid. MS obsd.: 542 (MH⁺). ¹H NMR (400 MHz, CD₃OD): δ ppm 7.91 (d, *J* = 8.8 Hz, 2H), 7.79 (d, *J =* 8.8 Hz, 2H), 6.73 (s, 1H), 6.67 (s, 1H), 5.60 (s, 1H), 5.50 (s, 1H), 4.15 (dd, *J =* 10.0, 6.4 Hz, 1H), 4.07 (s, 2H), 3.78 (td, *J =* 7.2, 3.6 Hz, 1H), 3.61 - 3.72 (m, 5H), 3.26 - 3.28 (m, 1H), 3.01 - 3.10 (m, 4H), 2.92 (dd, *J* = 17.2, 7.6 Hz, 1H), 2.41 (dd, *J* = 17.2, 4.0 Hz, 1H).

### Example 98

### 4-Amino-1-[4-[4-[6-chloro-4-[3,6-dihydro-2H-pyran-4-yl(difluoro)methyl]-2-pyridyl] piperazin-1-yl]sulfonylphenyl]pyrrolidin-2-one

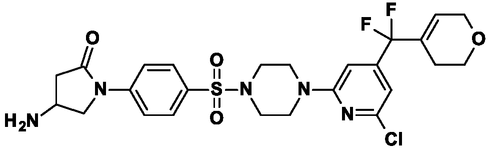

The title compound was prepared in analogy to the preparation of **Example 95** by using 3,6-dihydro-2H-pyran-4-carboxylic acid instead of tetrahydro-2H-pyran-4-carboxylic acid. **Example 98** (60 mg) was obtained as a white solid. MS obsd.: 568 (MH⁺). ¹H NMR (400 MHz, DMSO-*d₆*): *δ* ppm 7.92 (d, *J* = 8.8 Hz, 2H), 7.76 (d, *J* = 8.8 Hz, 2H), 6.78 (s, 1H), 6.67 (s, 1H), 6.09 (s, 1H), 4.09 (d, *J* = 16.0 Hz, 3H), 3.80 (s, 1H), 3.58 - 3.70 (m, 7H), 2.96 (s, 4H), 2.87 (dd, *J* = 17.2, 7.2 Hz, 1H), 2.38 (dd, *J* = 17.2, 2.8 Hz, 1H), 2.04 (s, 2H).

### Example 99

### 4-Amino-1-[4-[4-[6-chloro-4-[difluoro(tetrahydropyran-3-yl)methyl]-2-pyridyl]piperazin-1-yl]sulfonylphenyl]pyrrolidin-2-one

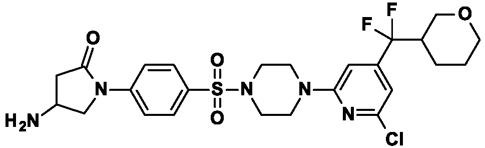

The title compound was prepared in analogy to the preparation of **Example 95** by using tetrahydro-2H-pyran-3-carboxylic acid instead of tetrahydro-2H-pyran-4-carboxylic acid. **Example 99** (200 mg) was obtained as a white solid. MS obsd.: 570.2 (MH⁺). ¹H NMR (400 MHz, DMSO-*d₆*): *δ* ppm 7.92 (d, *J* = 8.8 Hz, 2H), 7.77 (d, *J* = 8.8 Hz, 2H), 6.80 (s, 1H), 6.72 (s, 1H), 4.13 (dd, *J* = 10.4, 6.4 Hz, 1H), 3.73 - 3.88 (m, 3H), 3.59 - 3.73 (m, 5H), 3.21 (ddd, *J =* 11.2, 9.2, 4.4 Hz, 2H), 2.83 - 3.03 (m, 5H), 2.45 (dd, *J =* 17.4, 3.2 Hz, 2H), 1.61 (dd, *J =* 31.4, 12.4 Hz, 2H), 1.29 - 1.52 (m, 2H).

### Example 100

### 4-Amino-1-[4-[4-[6-chloro-4-[difluoro(8-oxabicyclo[3.2.1]octan-3-yl)methyl]-2-pyridyl] piperazin-1-yl]sulfonylphenyl]pyrrolidin-2-one

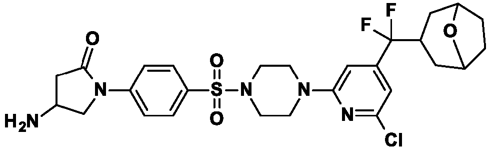

The title compound was prepared in analogy to the preparation of **Example 95** by using 8-oxabicyclo[3.2.1]octane-3-carboxylic acid instead of tetrahydro-2H-pyran-4-carboxylic acid. **Example 100** (70.4 mg) was obtained as a white solid. MS obsd.: 596.3 (MH⁺). ¹H NMR (400 MHz, CD₃OD): δ ppm 7.95 (d, *J* = 8.8 Hz, 2H), 7.85 (d, *J* = 8.8 Hz, 2H), 6.70 (d, *J* = 5.2 Hz, 2H), 4.42 (dd, *J* = 11.4, 6.8 Hz, 3H), 4.21 (t, *J* = 7.6 Hz, 1H), 3.97 (dd, *J* = 11.4, 2.0 Hz, 1H), 3.66 - 3.78 (m, 4H), 3.21 (dd, *J* = 18.0, 8.4 Hz, 1H), 3.14 - 3.03 (m, 4H), 2.71 (dd, *J =* 18.0, 2.8 Hz, 1H), 2.46 - 2.64 (m, 1H), 1.86 - 1.99 (m, 2H), 1.60 - 1.81 (m, 4H), 1.40 (dd, *J* = 13.2, 4.4 Hz, 2H).

### Example 101

### (4R)-4-amino-1-[4-[4-[6-chloro-4-[difluoro(3-oxabicyclo[3.1.0]hexan-6-yl)methyl]-2-pyridyl]piperazin-1-yl]sulfonylphenyl]pyrrolidin-2-one

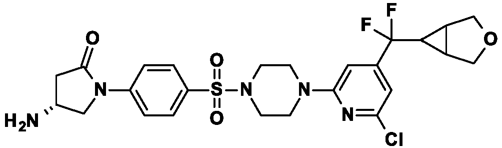

The title compound was prepared in analogy to the preparation of **Example 95** by using 8-oxabicyclo[3.2.1]octane-3-carboxylic acid instead of tetrahydro-2H-pyran-4-carboxylic acid and using intermediate D instead of intermediate C. **Example 101** (300 mg) was obtained as a white solid. MS obsd.: 568.3 (MH⁺). ¹H NMR (400 MHz, DMSO-*d₆*): *δ* ppm 7.92 (d, *J =* 8.8 Hz, 2 H), 7.75 (m, *J* = 8.8 Hz, 2 H), 6.85 (s, 1 H), 6.79 (s, 1 H), 3.99 (br dd, *J* = 9.6, 6.0 Hz, 1 H), 3.75 (d, *J* = 8.8 Hz, 2 H), 3.56 (br d, *J* = 8*.*4 Hz, 2 H), 3.48 (br dd, *J =* 9.6, 3.2 Hz, 2 H), 3.33 (br s, 4 H), 2.96 (br s, 4 H), 2.76 (dd, *J =* 16.8, 6.8 Hz, 1 H), 2.24 (br dd, *J =* 16.8, 4.0 Hz, 2 H), 2.03 (br s, 2 H), 1.43 - 1.56 (m, 1 H).

### Example 102 and Example 103

### (4R)-4-amino-1-[4-[4-[6-chloro-4-[[(2S)-1,4-dioxan-2-yl]-difluoro-methyl]-2-pyridyl] piperazin-1-yl]sulfonylphenyl]pyrrolidin-2-one

### (4R)-4-amino-1-[4-[4-[6-chloro-4-[[(2R)-1,4-dioxan-2-yl]-difluoro-methyl]-2-pyridyl] piperazin-1-yl]sulfonylphenyl]pyrrolidin-2-one

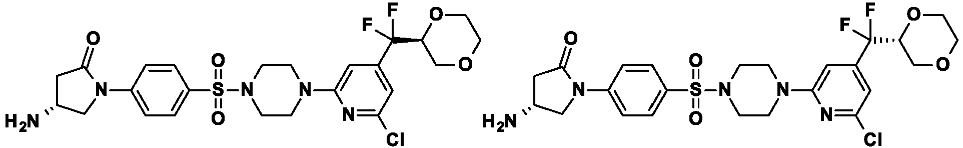

The title compounds were prepared in analogy to the preparation of **Example 95** by using intermediate E or intermediate F instead of compound 95b, and using intermediate D instead of intermediate C.

**Example 102** (40.4 mg) was obtained as a white solid. MS obsd.: 572 (MH⁺). ¹H NMR (400 MHz, DMSO-*d₆*): δ ppm 7.92 (d, *J* = 9.2 Hz, 2 H), 7.72 - 7.77 (m, 2 H), 6.84 (s, 1 H), 6.76 (s, 1 H), 4.11 - 4.27 (m, 1 H), 3.99 (dd, *J =* 9.6. 6.0 Hz, 1 H), 3.71 - 3.85 (m, 2 H), 3.41 - 3.68 (m, 8 H), 2.96 (br t, *J* = 4.8 Hz, 4 H), 2.76 (dd, *J* = 16.8, 6.8 Hz, 1 H), 2.11 - 2.38 (m, 2 H), 1.97 - 2.05 (m, 1 H), 1.23 (br s, 1 H).

**Example 103** (35.8 mg) was obtained as a white solid. MS obsd.: 572 (MH⁺). ¹H NMR (400 MHz, DMSO-*d*₆): δ ppm 7.92 (d, *J* = 9.2 Hz, 2 H), 7.72 - 7.77 (m, 2 H), 6.84 (s, 1 H), 6.76 (s, 1 H), 4.11 - 4.27 (m, 1 H), 3.99 (dd, *J* = 9.6. 6.0 Hz, 1 H), 3.71 - 3.85 (m, 2 H), 3.41 - 3.68 (m, 8 H), 2.96 (br t, *J* = 4.8 Hz, 4 H), 2.76 (dd, *J* = 16.8, 6.8 Hz, 1 H), 2.11 - 2.38 (m, 2 H), 1.97 - 2.05 (m, 1 H), 1.23 (br s, 1 H).

### Example 104

### (4R)-4-amino-1-[4-[4-[6-chloro-4-[difluoro-[(2S)-morpholin-2-yl]methyl]-2-pyridyl] piperazin-1-yl]sulfonylphenyl]pyrrolidin-2-one

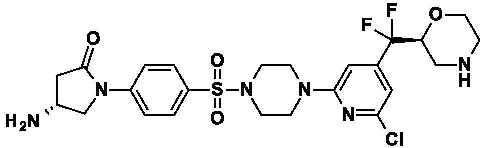

The title compound was prepared according to the following procedures:

### Step 1: tert-butyl (2S)-2-[[2-[4-[4-[(4R)-4-(tert-butoxycarbonylamino)-2-oxo-pyrrolidin-1-yl]phenyl]sulfonylpiperazin-1-yl]-6-chloro-4-pyridyl]-difluoro-methyl]morpholine-4-carboxylate (compound 104a)

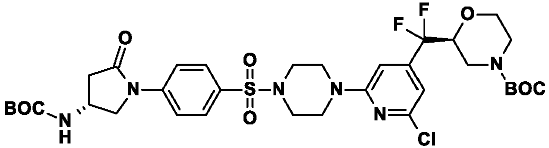

To a solution of intermediate G (59.58 mg, 0.160 mmol) and intermediate D (60.0 mg, 0.140 mmol) in DMSO (2 mL) was added DIPEA (36.47 mg, 0.280 mmol). The mixture was stirred at 100 °C for 12 hrs. The mixture was added water (50 mL) and EA (50 mL). The organic phase was washed with aq.CaCl₂ (30 mL), brine (30 mL), dried over Na₂SO₄ and concentrated. The crude was purified by prep-TLC to give compound 104a (90 mg, 0.120 mmol, 82.56% yield) as a white solid. MS obsd.: 771.1 (MH⁺).

### Step 2: (4R)-4-amino-1-[4-[4-[6-chloro-4-[difluoro-[(2S)-morpholin-2-yl]methyl]-2-pyridyl] piperazin-1-yl]sulfonylphenyl]pyrrolidin-2-one (Example 104)

A mixture of compound 104a (80.0 mg, 0.10 mmol) in TFA (1.33 mL, 18.01 mmol) was stirred at 15 °C for 2 hrs. The mixture was concentrated and the crude was purified by prep-HPLC to give **Example 104** (56 mg, 0.090 mmol, 83.54% yield) as a white solid. MS obsd.: 571.1 (MH⁺). ¹H NMR (400 MHz, DMSO-*d*₆): δ ppm 7.89 - 7.95 (m, 2 H), 7.80 (d, *J* = 8.8 Hz, 3 H), 6.85 (s, 1 H), 6.76 (s, 1 H), 4.33 - 4.44 (m, 1 H), 4.26 (dd, *J* = 11.2, 7.2 Hz, 1 H), 3.99 - 4.11 (m, 2 H), 3.87 (d, *J* =12.8 Hz, 1 H), 3.77 (t, *J =* 12.8 Hz, 1 H), 3.64 - 3.71 (m, 4 H), 3.18 (d, *J* = 12.4 Hz, 3 H), 3.02 - 3.12 (m, 2 H), 2.94 - 3.02 (m, 5 H).

### Example 105

### (4R)-4-amino-1-[4-[4-[6-chloro-4-[difluoro-[(2R)-morpholin-2-yl]methyl]-2-pyridyl] piperazin-1-yl]sulfonylphenyl]pyrrolidin-2-one

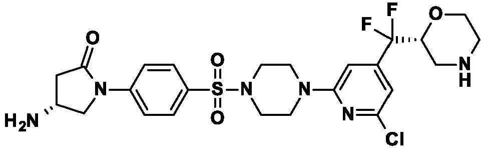

The title compound was prepared in analogy to the preparation of **Example 104** by using intermediate H instead of intermediate G. **Example 105** (35.5 mg) was obtained as a white solid. MS obsd.: 571.1 (MH⁺). ¹H NMR (400 MHz, DMSO-*d*₆): δ ppm 8.38 (br s, 3 H) 7.92 (d, *J* = 8.8 Hz, 2 H), 7.80 (d, *J =* 9.2 Hz, 2 H), 6.86 (s, 1 H), 6.76 (s, 1 H), 4.31 - 4.46 (m, 1 H), 4.27 (dd, *J =* 11.2, 6.8 Hz, 1 H), 4.03 (dd, *J =* 12.4, 2.4 Hz, 2 H), 3.86 (d, *J =* 10.4 Hz, 1 H), 3.64 - 3.82 (m, 5 H), 3.03 - 3.26 (m, 3 H), 2.95 - 3.03 (m, 5 H), 2.58 - 2.70 (m, 2 H).

### Example 106

### (4R)-4-amino-1-[4-[4-[6-chloro-4-[difluoro(1,2,3,6-tetrahydropyridin-4-yl)methyl]-2-pyridyl]piperazin-1-yl]sulfonylphenyl]pyrrolidin-2-one

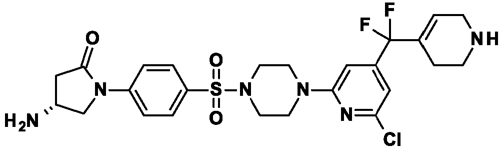

The title compound was prepared in analogy to the preparation of **Example 104** by using intermediate I instead of intermediate G. **Example 106** (11.8 mg) was obtained as a white solid. MS obsd.: 567.1 (MH⁺). ¹H NMR (400 MHz, DMSO-*d*₆): δ ppm 9.53 (br s, 2 H), 8.66 (s, 3 H), 7.91 (d, *J* = 8.8 Hz, 2 H), 7.79 (d, *J* = 8.8 Hz, 2 H), 6.80 (s, 1 H), 6.72 (s, 1 H), 6.17 ( s, 1 H), 4.27 (dd, *J* = 11.2, 6.8 Hz, 1 H), 4.06 ( s, 1 H), 3.92 (d, *J* = 9.6 Hz, 1 H), 3.68 (br s, 6 H), 3.16 (br s, 2 H), 2.97 (br s, 4 H), 2.67 (d, *J* = 18.0 Hz, 1 H), 2.26 (br s, 2 H).

### Example 107 and Example 108

### (4R)-4-amino-1-[4-[4-[6-chloro-4-[difluoro-[(3R)-3-piperidyl]methyl]-2-pyridyl]piperazin-1-yl]sulfonylphenyl]pyrrolidin-2-one

### (4R)-4-amino-1-[4-[4-[6-chloro-4-[difluoro-[(3S)-3-piperidyl]methyl]-2-pyridyl]piperazin-1-yl]sulfonylphenyl]pyrrolidin-2-one

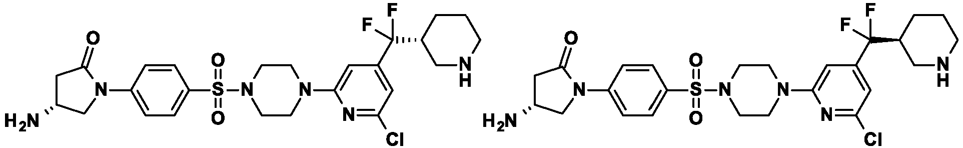

The title compounds were prepared according to the following procedures:

### Step 1: tert-butyl 3-[2-chloro-6-[4-[4-[(4R)-4-(benzyloxycarbonylamino)-2-oxo-pyrrolidin-1-yl]phenyl]sulfonylpiperazin-1-yl]pyridine-4-carbonyl]piperidine-1-carboxylate (compound 107a)

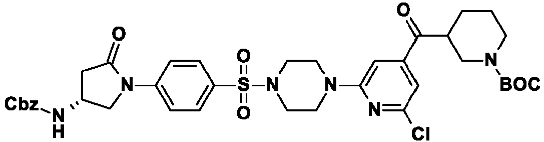

To a solution of benzyl *N*-[(3*R*)-5-oxo-1-(4-piperazin-1-ylsulfonylphenyl)pyrrolidin-3-yl]carbamate (280 mg, 557 µmol) and intermediate J (200 mg, 557 µmol) in DMSO (10 mL) was added DIPEA (216 mg, 292 µL, 1.67 mmol). The reaction was stirred at 100 °C for 2 days. After cooling to rt, the mixture was diluted with water (30 mL) and extracted with EA (30 mL) three times. The combined organic layer was washed with brine (100 mL), dried over anhydrous Na₂SO₄, filtered and concentrated. The crude was purified by flash column on silica gel (EA in PE=70~90%) to give **Intermediate 107a** (320 mg, 69.6% yield) as yellow oil. MS obsd.: 725.2 (MH⁺).

### Step 2: tert-butyl (3R)-3-[[2-[4-[4-[(4R)-4-(benzyloxycarbonylamino)-2-oxo-pyrrolidin-1-yl] phenyl]sulfonylpiperazin-1-yl]-6-chloro-4-pyridyl]-difluoro-methyl]piperidine-1-carboxylate and tert-butyl (3S)-3-[[2-[4-[4-[(4R)-4-(benzyloxycarbonylamino)-2-oxo-pyrrolidin-1-yl]phenyl]sulfonylpiperazin-1-yl]-6-chloro-4-pyridyl]-difluoro-methyl]piperidine-1-carboxylate(compounds 107b and 108b)

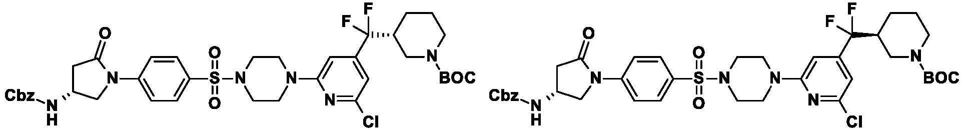

Compound 107a (280 mg, 358 µmol) was dissolved in the DAST (10 mL) and the mixture was stirred at rt for 2 days. The reaction was quenched by adding water dropwise in the ice/water bath and then extracted with DCM (50 mL) three times. The combined organic layer was washed with brine (100 mL), dried over anhydrous Na₂SO₄, filtered and concentrated. The crude was purified by flash chromatography on silica gel (70-100% EA in PE) and then chiral separation by SFC to give compound 107b (60 mg, 74.7 µmol, 20.8% yield, faster eluting) and compound 108b (56 mg, 69.7 µmol, 19.5% yield, slower eluting) as white solids. MS obsd.: 803.0 (MH⁺). SFC conditions: Column: AD, 250×30 mm I.D., 5µm; Mobile phase: A for CO₂ and B for Ethanol (0.1% NH₃H₂O); Gradient: B 50%; Flow rate: 50 mL/min; Backpressure: 100 bar; Column temperature: 35 °C.

### Step 3: (4R)-4-amino-1-[4-[4-[6-chloro-4-[difluoro-[(3R)-3-piperidyl]methyl]-2-pyridyl] piperazin-1-yl]sulfonylphenyl]pyrrolidin-2-one and (4R)-4-amino-1-[4-[4-[6-chloro-4-[difluoro-[(3S)-3-piperidyl]methyl]-2-pyridyl]piperazin-1-yl]sulfonylphenyl]pyrrolidin-2-one (Example 107 and Example 108)

A solution of compound 107b (50 mg, 62.2 µmol) and TFA was stirred at 65 °C for 2 hrs. The mixture was the concentrated and the crude was purified by prep-HPLC to give **Example 107** (20.8 mg, 36.4 µmol, 58.6% yield) as a white solid. MS obsd.: 569.2 (MH⁺). ¹H NMR (400 MHz, DMSO-*d*₆): δ ppm 8.29 (br s, 2 H), 7.92 (m, *J* = 8.8 Hz, 2 H), 7.80 (m, *J* = 8.8 Hz, 2 H), 6.81 (s, 1 H), 6.72 (s, 1 H), 4.26 (dd, *J =* 11.2, 6.8 Hz, 1 H), 4.08 (br s, 1 H), 3.74 - 3.92 (m, 1 H), 3.62 - 3.72 (m, 4 H), 3.18 - 3.29 (m, 2 H), 3.08 (dd, *J =* 18.0, 8.0 Hz, 2 H), 2.96 - 3.02 (m, 4 H), 2.77 (br d, *J =* 12.0 Hz, 2 H), 1.90 - 2.12 (m, 1 H), 1.69 - 1.87 (m, 2 H), 1.57 (q, *J* = 13.2 Hz, 1 H), 1.28 - 1.47 (m, 2 H), 1.24 (br s, 1 H).

**Example 108** (23.7 mg) was prepared in analogy to the preparation of **Example 107** by using compound 108b instead of compound 107b. MS obsd.: 569.2 (MH⁺). ¹H NMR (400 MHz, DMSO-*d*₆): δ ppm 7.92 (m, *J* = 8.98 Hz, 2 H), 7.80 (m, *J* = 8.8 Hz, 2 H), 6.80 (s, 1 H), 6.73 (s, 1 H), 4.26 (dd, *J* = 11.2, 6.98 Hz, 1 H), 4.08 (br s, 1 H), 3.81 (m, 1 H), 3.62 - 3.72 (m, 4 H), 3.15 - 3.28 (m, 2 H), 3.08 (dd, *J* = 18.0, 8.4 Hz, 2 H), 2.98 (m, 4 H), 2.77 (m, 2 H), 1.90 - 2.12 (m, 1 H), 1.69 - 1.87 (m, 2 H), 1.57 (q, *J* = 13.2 Hz, 1 H), 1.28 - 1.47 (m, 2 H)1.24 (br s, 1 H).

### Example 109

### (4R)-4-amino-1-[4-[4-[4-[azetidin-3-yl(difluoro)methyl]-6-chloro-2-pyridyl]piperazin-1-yl]sulfonylphenyl]pyrrolidin-2-one

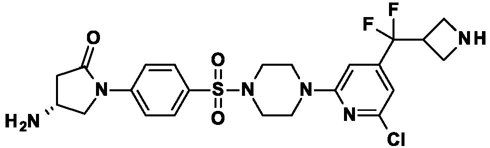

The title compound was prepared in analogy to the preparation of **Example 104** by using intermediate I instead of intermediate K. **Example 109** (30 mg) was obtained as a white solid. MS obsd.: 540.9 (MH⁺). ¹H NMR (400 MHz, DMSO-*d*₆): δ ppm 7.92 (d, *J* = 9.2 Hz, 2H), 7.77 (d, *J* = 9.2 Hz, 2H), 6.83 (s, 1H), 6.78 (s, 1H), 4.08 (dd, *J* = 6.4, 9.6 Hz, 1H), 3.80 (t, *J =* 9.2 Hz, 5H), 3.55 - 3.72 (m, 6H), 2.96 (t, *J =* 4.8 Hz, 4H), 2.87 (dd, *J* = 7.2, 16.8 Hz, 1H), 2.30 - 2.40 (m, 1H).

### Example 110

### 3-[4-[4-[6-Chloro-4-[difluoro-[(2S)-morpholin-2-yl]methyl]-2-pyridyl]piperazin-1-yl]sulfonylphenyl] -5-(hydroxymethyl)oxazolidin-2-one

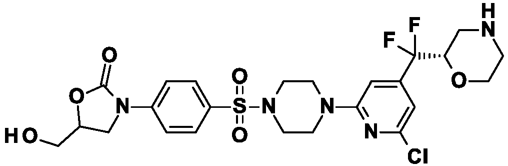

The title compound was prepared according to the following procedures:

### Step 1: tert-butyl 4-[4-[5-(hydroxymethyl)-2-oxo-oxazolidin-3-yl]phenyl]sulfonyl piperazine-1-carboxylate (compound 110a)

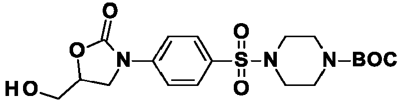

A mixture of *tert*-butyl 4-((4-iodophenyl)sulfonyl)piperazine-1-carboxylate (0.430 g, 951 µmol), 5-(hydroxymethyl)oxazolidin-2-one (111 mg, 951 µmol), DMEDA (29.3 mg, 333 µmol), CuI (63.4 mg, 333 µmol) and K₃PO₄ (404 mg, 1.9 mmol) in DMF (4 mL) was heated at 75 °C in a microwave for 1h. The mixture was poured into ice water, extracted with DCM, dried over Na₂SO₄, filtered and concentrated. The crude was purified by flash chromatography on silica gel to give compound 110a (310 mg, 702 µmol, 73.9 % yield) as a white solid. MS obsd.: 442 (MH⁺).

### Step 2: 5-(hydroxymethyl)-3-(4-piperazin-1-ylsulfonylphenyl)oxazolidin-2-one (compound 110b)

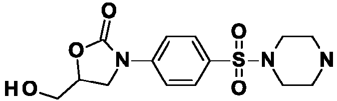

A mixture of compound 110a (0.060 g, 136 µmol) and TFA (877 mg) in CH₂Cl₂ (1 mL) was stirred at rt for 1h. The mixture was concentrated and used directly in next step. MS obsd.: 342 (MH⁺).

### Step 3: tert-butyl (2S)-2-[[2-chloro-6-[4-[4-[5-(hydroxymethyl)-2-oxo-oxazolidin-3-yl]phenyl]sulfonylpiperazin-1-yl]-4-pyridyl]-difluoro-methyl]morpholine-4-carboxylate (compound 110c)

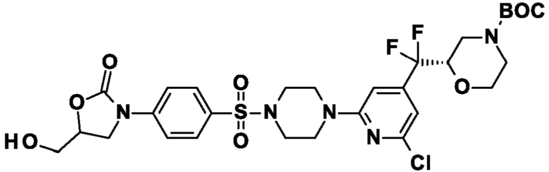

A mixture of compound 110b (0.045 g, 132 µmol), intermediate H (50.5 mg, 132 µmol) and DIPEA (85.1 mg, 115 µl, 659 µmol) in NMP (1.5 mL) was stirred at 100 °C overnight. The mixture was diluted with EA (10 mL), washed with water (10 mL) twice. The organic layer was dried, concentrated and used directly in next step. MS obsd.: 688 (MH⁺).

### Step 4: 3-[4-[4-[6-chloro-4-[difluoro-[(2S)-morpholin-2-yl]methyl]-2-pyridyl]piperazin-1-yl]sulfonylphenyl]-5-(hydroxymethyl)oxazolidin-2-one (Example 110)

A mixture of compound 110c (0.090 g, 131 µmol,) and TFA (844 mg) in CH₂Cl₂ (1.5 mL) was stirred at rt for 1h. The mixture was concentrated and isolated by pre-HPLC to give **Example 110** (24 mg, 29.6% yield) as a white solid. MS obsd.: 588 (MH⁺). ¹H NMR (400 MHz, DMSO-*d*₆): δ ppm 7.73 - 7.78 (m, 2H), 7.69 - 7.71 (d, *J =* 8.4 Hz, 2H), 6.75 (s, 1H), 6.67 (s, 1H), 5.16 (br s, 1H), 4.61 - 4.73 (m, 1H), 3.94 - 4.16 (m, 2H), 3.81 (br dd, *J* = 6.0, 9.2 Hz, 2H), 3.55 - 3.59 (m, 4H), 3.41- 3.53 (m, 2H), 2.99 (br d, *J* = 12.4 Hz, 1H), 2.90 - 2.91 (m, 4H), 2.79 - 2.84 (m, 1H), 2.62 - 2.77 (m, 2H).

### Example 111

### (4R)-4-amino-1-[4-[4-[6-chloro-4-[difluoro-(5-methylpyrazin-2-yl)methyl]-2-pyridyl]piperazin-1-yl]sulfonylphenyl]pyrrolidin-2-one

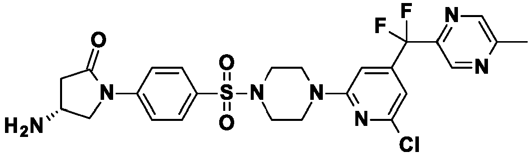

The title compound was prepared in analogy to the preparation of **Example 89** by using 2-bromo-5-methyl-pyrazine instead of 2-bromopyridine. **Example 111** (89 mg) was obtained as a white solid. MS obsd.: 578.0 (MH⁺). ¹H NMR (400 MHz, DMSO-*d*₆) δ ppm 8.87 - 9.07 (m, 1 H), 8.50 - 8.68 (m, 1 H), 7.66 - 8.06 (m, 3 H), 6.64 - 7.01 (m, 2 H), 3.92 - 4.15 (m, 2 H), 3.51 - 3.79 (m, 7 H), 2.89 - 3.08 (m, 4 H), 2.71 - 2.91 (m, 1 H), 2.51 (br s, 3 H), 2.21 - 2.39 (m, 1 H). ¹⁹F NMR (376 MHz, DMSO-*d*₆) δ ppm -97.90 - -96.24 (m, 2 F).

### Example 112

### (4R)-4-amino-1-[4-[4-[6-chloro-4-[difluoro-(6-methylpyrazin-2-yl)methyl]-2-pyridyl]piperazin-1-yl]sulfonylphenyl]pyrrolidin-2-one

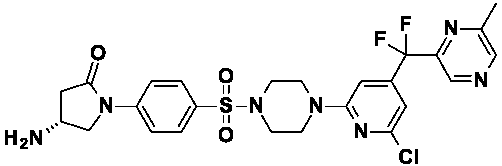

The title compound was prepared in analogy to the preparation of **Example 89** by using 2-bromo-6-methyl-pyrazine instead of 2-bromopyridine. **Example 112** (59 mg) was obtained as a white solid. MS obsd.: 578.2 (MH⁺). ¹H NMR (400 MHz, DMSO-*d*₆) δ ppm 8.82 - 8.94 (m, 1 H), 8.73 (s, 1 H), 7.91 (d, *J* =9.05 Hz, 2 H), 7.74 (d, *J* =9.05 Hz, 2 H), 6.93 (s, 1 H), 6.81 (s, 1 H), 4.00 (dd, *J* =10.03, 6.11 Hz, 1 H), 3.60 - 3.71 (m, 5 H), 3.49 (br dd, *J* =9.66, 3.30 Hz, 1 H), 3.33 - 3.34 (m, 3 H), 2.89 - 3.01 (m, 4 H), 2.78 (dd, *J* =16.87, 7.09 Hz, 1 H), 2.26 (dd, *J* =16.63, 3.91 Hz, 1 H). ¹⁹F NMR (376 MHz, DMSO-*d*₆) δ ppm -97.18 (s, 2 F).

### Example 113

### (4R)-4-amino-1-[4-[4-[6-chloro-4-[(5-chloro-6-methyl-pyrazin-2-yl)-difluoro-methyl]-2-pyridyl]piperazin-1-yl]sulfonylphenyl]pyrrolidin-2-one

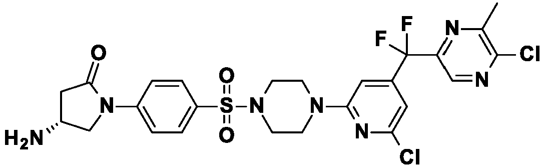

The title compound was prepared in analogy to the preparation of **Example 94 by** using methyl 5-chloro-6-methylpyrazine-2-carboxylate instead of methyl pyrimidine-2-carboxylate. **Example 113** (20 mg) was obtained as a white solid. MS obsd.: 612.3 (MH⁺). ¹H NMR (400 MHz, DMSO-*d*₆) δ ppm 8.79 (s, 1 H), 7.91 (d, *J* = 8.9 Hz, 2 H), 7.74 (d, *J* = 8.9 Hz, 2 H), 6.92 (s, 1 H), 6.82 (s, 1 H), 4.0-4.0 (m, 1 H), 3.6-3.7 (m, 5 H), 3.50 (br dd, *J* = 3.1, 9.8 Hz, 1 H), 2.95 (t, *J* = 4.7 Hz, 4 H), 2.7-2.8 (m, 1 H), 2.56 (s, 3 H), 2.26 (dd, *J* = 3.8, 16.9 Hz, 1 H). ¹⁹F NMR (376 MHz, DMSO-*d*₆) δ ppm -96.54 (s, 2 F).

### Example 114

### (4R)-4-amino-1-[4-[4-[6-chloro-4-[difluoro-[cis-6-methylmorpholin-2-yl]methyl]-2-pyridyl]piperazin-1-yl]sulfonylphenyl]pyrrolidin-2-one

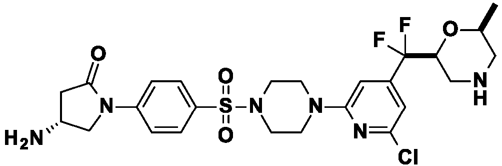

The title compound was prepared according to the following procedures:

### Step 1: Tert-butyl cis-2-[(2,6-dichloro-4-pyridyl)-difluoro-methyl]-6-methyl-morpholine-4-carboxylate (compound 114a)

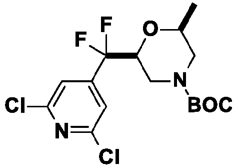

Compound 114a was prepared in analogy to the preparation of intermediate G by using (*cis*-6-methylmorpholin-2-yl)methanol instead of (*R*)-*N*-Boc-2-hydroxymethylmorpholine. Compound 114a (1.2 g) was obtained as a white solid. MS obsd.: 341.0 (MH⁺-56).

### Step 2: (4R)-4-amino-1-[4-[4-[6-chloro-4-[difluoro-[cis-6-methylmorpholin-2-yl]methyl]-2-pyridyl]piperazin-1-yl]sulfonylphenyl]pyrrolidin-2-one (Example 114)

The title compound was prepared in analogy to the preparation of **Example 104** by using compound 114a instead of Intermediate G. **Example 114** (23.6 mg) was obtained as a white solid. MS obsd.: 585.4 (MH⁺). ¹H NMR (400 MHz, DMSO-*d*₆) *δ* ppm 8.59 (br d, *J =* 2.57 Hz, 3 H), 7.90 (d, *J =* 8.93 Hz, 2 H), 7.79 (d, *J =* 8.93 Hz, 2 H), 6.85 (s, 1 H), 6.74 (s, 1 H), 4.49 (q, *J =* 10.43 Hz, 1 H), 4.26 (dd, *J =* 11.07, 6.91 Hz, 1 H), 3.84 - 4.12 (m, 3 H), 3.67 (br s, 4 H), 3.17 - 3.28 (m, 2 H), 3.06 (dd, *J =* 17.85, 8.19 Hz, 1 H), 2.97 (br d, *J* = 4.16 Hz, 4 H), 2.72 - 2.85 (m, 1 H), 2.65 (br dd, *J* = 17.79, 2.75 Hz, 2 H), 1.09 (d, *J* = 6.24 Hz, 3 H). ¹⁹F NMR (376 MHz, DMSO-*d₆*) δ ppm -109.50 - -105.5 (m, 2 F).

### Example 115

### ((4R)-4-amino-1-[4-[4-[6-chloro-4-[difluoro-(5-methylmorpholin-2-yl)methyl]-2-pyridyl]piperazin-1-yl]sulfonylphenyl]pyrrolidin-2-one

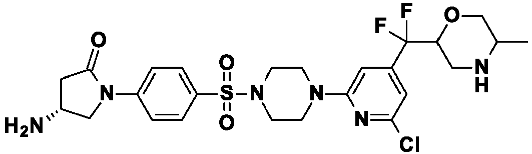

The title compound was prepared according to the following procedures:

### Step 1: Tert-butyl 2-[(2,6-dichloro-4-pyridyl)-difluoro-methyl]-5-methyl-morpholine-4-carboxylate (compound 115a)

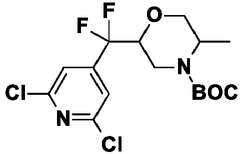

Compound 115a was prepared in analogy to the preparation of intermediate G by using (5-methylmorpholin-2-yl)methanol instead of (*R*)-*N*-Boc-2-hydroxymethylmorpholine. Compound 115a (580 mg) was obtained as a white solid. MS obsd.: 341.2 (MH⁺-56).

### Step 2: ((4R)-4-amino-1-[4-[4-[6-chloro-4-[difluoro-(5-methylmorpholin-2-yl)methyl]-2-pyridyl]piperazin-1-yl]sulfonylphenyl]pyrrolidin-2-one (Example 115)

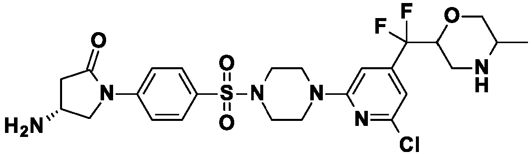

The title compound was prepared in analogy to the preparation of **Example 104** by using compound 115a instead of Intermediate G. **Example 115** (25.4 mg) was obtained as a white solid. MS obsd.: 585.2 (MH⁺). ¹H NMR (400 MHz, DMSO-*d₆*) *δ* ppm 8.66 ( s, 3 H), 7.91 (d, *J* = 8.93 Hz, 2 H), 7.79 (d, *J* = 8.93 Hz, 2 H), 6.89 (s, 1 H), 6.77 (s, 1 H), 4.37 - 4.54 (m, 1 H), 4.27 (dd, *J* = 11.07, 6.91 Hz, 1 H), 4.06 ( s, 1 H), 3.92 ( d, *J* = 11.25 Hz, 2 H), 3.76 ( d, *J* = 12.10 Hz, 1 H), 3.68 ( s, 4 H), 3.52 ( d, *J* = 7.09 Hz, 1 H). 3.02 - 3.15 (m, 3 H), 2.97 ( s, 4 H), 2.67 (dd, *J* = 17.85, 2.81 Hz, 1 H), 1.29 (d, *J* = 6.85 Hz, 3 H).

### Example 116

### (4R)-4-amino-1-[4-[4-[4-[[cis-3-azabicyclo[3.1.0]hexan-1-yl]-difluoro-methyl]-6-chloro-2-pyridyl]piperazin-1-yl]sulfonylphenyl]pyrrolidin-2-one

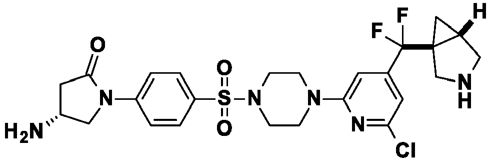

The title compound was prepared according to the following procedures:

### Step 1: Tert-butyl cis-1-[(2,6-dichloro-4-pyridyl)-difluoro-methyl]-3-azabicyclo[3.1.0]hexane-3-carboxylate (compound 116a)

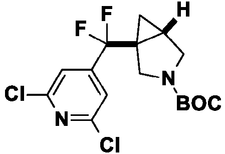

Compound 116a was prepared in analogy to the preparation of intermediate G by using *tert*-butyl cis-1-(hydroxymethyl)-3-azabicyclo[3.1.0]hexane-3-carboxylate instead of (*R*)-*N*-Boc-2-hydroxymethylmorpholine. Compound 116a (2.6 g) was obtained as a white solid. MS obsd.: 323.1 (MH⁺-56).

### Step 2: (4R)-4-amino-1-[4-[4-[4-[[cis-3-azabicyclo[3.1.0]hexan-1-yl]-difluoro-methyl]-6-chloro-2-pyridyl]piperazin-1-yl]sulfonylphenyl]pyrrolidin-2-one

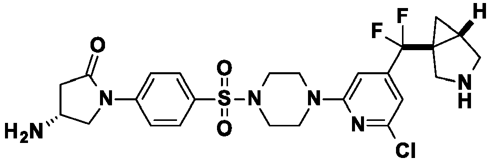

The title compound was prepared in analogy to the preparation of **Example 104** by using compound 116a instead of Intermediate G. **Example 115** (80.2 mg) was obtained as a white solid. MS obsd.: 567.2 (MH⁺). ¹H NMR (400 MHz, DMSO-*d₆*) *δ* ppm 8.55 - 8.35 (m, 3 H), 7.95 - 7.88 (m, 2 H), 7.82 - 7.74 (m, 2 H), 6.84 - 6.80 (m, 1 H), 6.80 - 6.77 (m, 1 H), 4.30 - 4.22 (m, 1 H), 4.11 - 4.02 (m, 1 H), 3.91 - 3.84 (m, 1 H), 3.71 - 3.62 (m, 4 H), 3.28 - 3.21 (m, 2 H), 3.12 - 3.01 (m, 1 H), 3.01 - 2.93 (m, 4 H), 2.66 - 2.58 (m, 1 H), 2.24 - 2.16 (m, 1 H), 1.27 - 1.15 (m, 2 H). ¹⁹F NMR (376 MHz, DMSO-*d6*) δ -97.642 (s, 2 F).

### Example 117

### (4R)-4-amino-1-[4-[4-[6-chloro-4-[trans-difluoro-[2-(hydroxymethyl)cyclopropyl]methyl]-2-pyridyl]piperazin-1-yl]sulfonylphenyl]pyrrolidin-2-one

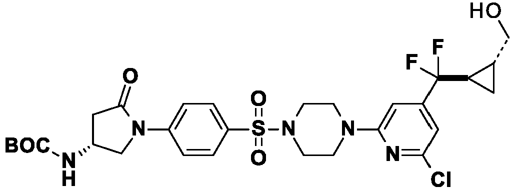

The title compound was prepared according to the following procedures:

### Step 1: 2-(2,6-dichloro-4-pyridyl)-2,2-difluoro-acetaldehyde (117a)

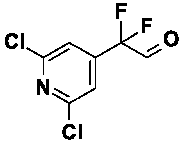

To a solution of 2-(2,6-dichloro-4-pyridyl)-2,2-difluoro-ethanol (2.5 g, 10.96 mmol) in DCM (20 mL) was added Dess-Martin periodinane (5.12 g, 12.06 mmol) at 0 °C under N₂. The mixture was stirred at 25 °C for 2 hours. After the starting material was consumed, the mixture was poured into water (100 mL) at 0 °C, and extracted with EA (150 mL × 3). The organic layer was washed with aq. NH₄Cl solution (100 mL × 3) and brine (100 mL), dried over anhydrous Na₂SO₄, filtered and concentrated to dryness to give crude product. The residue was purified by column chromatography on silica gel (EA in PE = 1:100 - 2:1) to give compound 117a (1.8 g, 72.64% yield) as colorless oil. MS obsd.: 243.7 (MH+18⁺).

### Step 2: Ethyl (E)-4-(2,6-dichloro-4-pyridyl)-4,4-difluoro-but-2-enoate (117b)

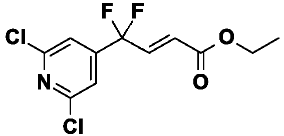

To a solution of compound 117a (1.8 g, 7.96 mmol) in THF (15 mL) was added (carbethoxymethylene)triphenylphosphorane (5.55 g, 15.93 mmol) under N₂, and the reaction mixture was stirred at 70 °C for 5 hours. After the starting material was consumed, the mixture was poured into water (100 mL). Then, the mixture was extracted with EA (150 mL × 3). The combined organic layer was washed with aq. NH₄Cl (100 mL × 3) and brine (100 mL × 2), dried over anhydrous Na₂SO₄, filtered and concentrated to dryness to give crude product. The crude product was purified by column chromatography on silica gel (EA in PE = 1:100 - 1:5 to give compound 117b (1.5 g, 60.34% yield) as colorless oil. MS obsd.: 295.8 (MH⁺).

### Step 3: Trans-ethyl 2-[(2,6-dichloro-4-pyridyl)-difluoro-methyl]cyclopropanecarboxylate ( compound 117c)

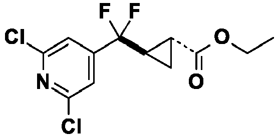

To a solution of sodium hydride (303.95 mg, 7.6 mmol) in THF (15 mL) and DMSO (15 mL) were added trimethylsulfoxonium iodide (2.23 g, 10.13 mmol) at 0 °C under N₂ and compound 117b (1.5 g, 5.07 mmol). The reaction was stirred at 15 °C for 1 hour. After the starting material was consumed, the mixture was poured into water (100 mL) at 0 °C. The resulting mixture was extracted with EA (150 mL × 3). The combined organic layer was washed with aq. NH₄Cl solution (100 mL × 3) and brine (100 mL × 2), dried over anhydrous Na₂SO₄, filtered and concentrated to dryness to give crude product. The crude product was purified by column chromatography on silica gel (EA:PE=1:10 - 1:1) to give 117c (900 mg, 57.29% yield) as colorless oil. MS obsd.: 310.2 (MH⁺).

### Step 4: [Tran-2-[(2,6-dichloro-4-pyridyl)-difluoro-methyl]cyclopropyl]methanol (117d)

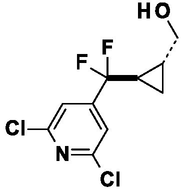

To a solution of compound 117d (600.0 mg, 1.93 mmol) in ethanol (4 mL) was added sodium borohydride (146.39 mg, 3.87 mmol) slowly at 0 °C. The mixture was stirred at 45 °C for 2 hours. After the starting material was consumed, the mixture was poured into water (100 mL) at 0 °C, and extracted with EA (100 mL × 3). The combined organic layer was washed with aq NH₄Cl solution (100 mL × 3) and brine (100 mL × 2), dried over anhydrous Na₂SO₄, filtered and concentrated to dryness to give the crude product, which was purified by prep-HPLC to give 117d (400 mg, 77.12% yield) as a colorless oil. MS obsd.: 267.8 (MH⁺).

### Step 4: Tert-butyl N-[(3R)-1-[4-[4-[6-chloro-4-[trans-difluoro-[2-(hydroxymethyl)cyclopropyl]methyl]-2-pyridyl]piperazin-1-yl]sulfonylphenyl]-5-oxopyrrolidin-3-yl]carbamate (117e)

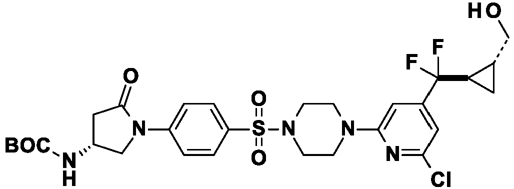

To a solution of compound 117d (100.0 mg, 0.370 mmol) in DMSO (0.5 mL) were added Intermediate D (158.35 mg, 0.370 mmol) and *N,N*-diisopropylethylamine (0.13 mL, 0.750 mmol) in one portion. The reaction was stirred at 100 °C for 16 hours. After completion, the mixture was diluted with water (20 mL) and extracted with EA (20 mL × 3). The combined organic layer was washed with brine (50 mL), dried over anhydrous Na₂SO₄, filtered and concentrated *in vacuo* to give a crude product. The crude was purified by prep-HPLC to give 117e (205 mg, 83.76% yield) as a yellow oil. MS obsd.: 656.14 (MH⁺).

### Step 5: (4R)-4-amino- 1-[4-[4-[6-chloro-4-[trans-difluoro-[2-(hydroxymethyl)cyclopropyl]methyl]-2-pyridyl]piperazin-1-yl]sulfonylphenyl]pyrrolidin-2-one

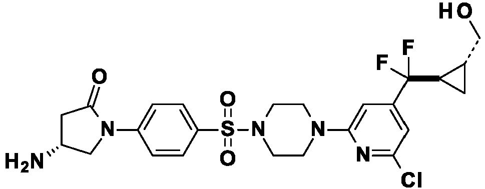

To a solution of 117e (190.0 mg, 0.290 mmol) in DCM (2 mL) was added hydrochloric acid methanol solution (3.62 mL, 14.48 mmol) in one portion. The reaction was stirred at 25 °C for 1 hour. After completion, the mixture was concentrated *in vacuo* to give a crude residue, which was purified by prep-HPLC to give **Example 117** (98 mg, 59.67% yield) as a white solid. MS obsd.: 556.1 (MH⁺). ¹H NMR (400 MHz, DMSO-*d*₆) δ ppm 8.56 - 8.46 (m, 3 H), 7.94 - 7.89 (m, 2 H), 7.80 (s, 2 H), 6.98 - 6.94 (m, 1 H), 6.80 (s, 1 H), 4.78 - 4.68 (m, 1 H), 4.29 - 4.21 (m, 1 H), 4.09 - 4.02 (m, 1 H), 3.92 - 3.86 (m, 1 H), 3.71 - 3.62 (m, 4 H), 3.47 - 3.40 (m, 1 H), 3.24 - 3.17 (m, 1 H), 3.10 - 3.01 (m, 1 H), 3.00 - 2.91 (m, 4 H), 2.67 - 2.60 (m, 1 H), 1.63 - 1.49 (m, 1 H), 1.36 - 1.26 (m, 1 H), 0.86 - 0.78 (m, 1 H). ¹⁹F NMR (376 MHz, DMSO-*d*₆) δ -103.881 (s, 2 F).

### Example 118

### (4R)-4-amino-1-[4-[4-[4-[[trans-4-(aminomethyl)cyclohexyl]-difluoro-methyl]-6-chloro-2-pyridyl]piperazin-1-yl]sulfonylphenyl]pyrrolidin-2-one

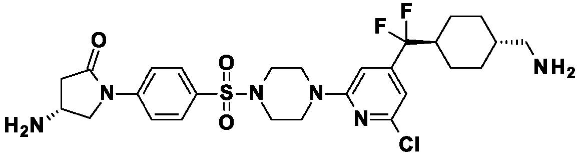

The title compound was prepared according to the following procedures:

### Step 1: Trans-methyl 4-(hydroxymethyl)cyclohexanecarboxylate (118a)

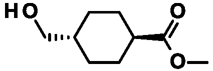

To a solution of *trans*-4-methoxycarbonylcyclohexanecarboxylic acid (50.0 g, 268.51 mmol) in THF (150 mL) was added BH₃•Me₂S (53.7 mL, 537.03 mmol) dropwise at 0-10°C for 1 hour. The mixture was stirred at 25 °C for 12 hours. After completion, the reaction was quenched by adding methanol (100 mL) dropwise at 0 °C. The mixture was concentrated *in vacuo* at 40 °C to give crude residue, which was purified by silica gel chromatography (EA:PE = 0-50%) to give compound 118a (28 g, 60.55% yield) as colorless oil.

### Step 2: Trans-methyl 4-formylcyclohexanecarboxylate (118b)

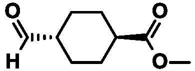

To a solution of oxalyl chloride (30.95 g, 243.87 mmol) in DCM (100 mL) was added dimethyl sulfoxide (25.4 g, 325.17 mmol) at -60 °C and stirred at -60 °C for 0.5 hour under N₂. The solution of compound 118b (28.0 g, 162.58 mmol) in DCM (100 mL) was added into the reaction at -60 °C and kept stirring for additional 1 hour. Then, DIPEA (84.05 g, 650.33 mmol) was added at -60 °C. The reaction was allowed to warm to 0 °C and kept stirring for 2 hours. After completion, the reaction mixture was diluted with EA (600 mL) and washed with aq. CaCl₂ solution (100 mL × 3) and brine (50 mL × 2). The combined organic layer was dried over anhydrous Na₂SO₄, filtered, and concentrated *in vacuo* to give the crude residue, which was purified by flash chromatography on silica gel (EA:PE = 3-50%) to give compound 118b (24 g, 86.73% yield) as light yellow oil.

### Step 3: Trans-methyl 4-[(2,6-dichloro-4-pyridyl)-hydroxy-methyl]cyclohexanecarboxylate (118c)

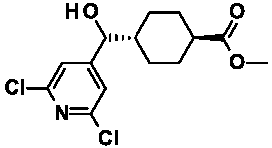

To a solution of 2,6-dichloro-4-iodo-pyridine (42.48 g, 155 mmol) in THF (50 mL) was added isopropylmagnesium chloride lithium chloride complex in THF (130.16 mL, 169.2 mmol) at -40°C under N₂. The mixture was stirred at 25 °C for 1 hour. The reaction mixture was then added into a solution of compound 118b (24.0 g, 141 mmol) in THF (100 mL) dropwise at -40°C. Upon addition, the mixture was stirred at 25 °C for 3 hours. After completion, the reaction mixture was poured into aq. NH₄Cl solution (200 mL) and extracted with EA (300 mL × 3). The combined organic layer was washed with brine (100 mL × 2), dried over anhydrous Na₂SO₄, filtered, and concentrated *in vacuo* to give the crude residue, which was purified by flash chromatography on silica gel (EA:PE = 0-30%) to give compound 118c (33 g, 73.55% yield) as a white solid. MS obsd. (ESI⁺) [(M+Cl)⁺]: 317.8.

### Step 4: Trans-methyl 4-(2,6-dichloropyridine-4-carbonyl)cyclohexanecarboxylate (118d)

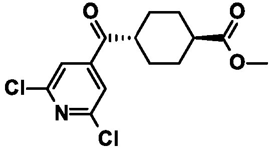

To a solution of compound 118c (33.0 g, 103.71 mmol) in THF (150 mL) was added Dess-Martin periodinane (48.39 g, 114 mmol) slowly at 0 °C under N₂. The reaction was stirred at 25 °C for 3 hours. After completion, the mixture was diluted with EA (500 mL), and poured into aq. K₂CO₃ solution (200 mL). The mixture was kept stirring at room temperature for 0.5 hour. The combined organic layer was washed with brine (100 mL × 2), dried over anhydrous Na₂SO₄, filtered, and concentrated *in vacuo* to give crude compound 118d (30 g, 91.5% yield) as a white solid, which was used in the next step directly. MS obsd. (ESI⁺) [(M+H)⁺]: 315.8.

### Step 5: Trans-methyl 4-[(2,6-dichloro-4-pyridyl)-difluoro-methyl]cyclohexanecarboxylate (118e)

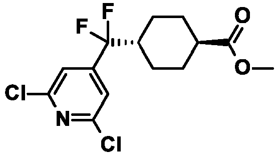

To a solution of compound 118d (29.5 g, 93.3 mmol) in DCM (20 mL) was added DAST (135.35 g, 839.71 mmol), and the reaction was stirred at 45°C for 12 hours. After completion, the reaction mixture was poured into the ice-water and extracted with EA (200 mL × 3). The combined organic layer was washed with aq. K₂CO₃ solution (100 mL × 2) and brine (100 mL × 2), dried over anhydrous Na₂SO₄, filtered, and concentrated *in vacuo* to give a crude residue. The residue was recrystallized with methanol (150 mL) to give compound 118e (25.6 g, 81.13% yield) as a white solid. MS obsd. (ESI⁺) [(M+H)⁺]: 337.8.

### Step 6: Trans-[4-[(2,6-dichloro-4-pyridyl)-difluoro-methyl]cyclohexyl]methanol (118f)

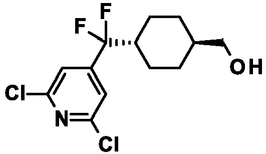

To a solution of 118e (250.0 mg, 0.740 mmol) in THF (5 mL) was added LiAlH₄ (42.14 mg, 1.11 mmol) at -10 °C and the reaction was stirred at -10 °C for 2 hours. After completion, the mixture was allowed to warm to 0 °C, and quenched by addition of 0.1 mL H₂O, followed by 0.15 mL of 15% aq. NaOH solution. After stirring for 0.5 hour, the mixture was filtered through a Celite pad, and the filtrate was concentrated *in vacuo* to give crude product. The residue was purified by prep-HPLC to give 118f (210 mg, 91.59% yield) as a white solid. MS obsd. (ESI⁺) [(M+H)⁺]: 310.1.

### Step 7: tert-butyl N-[(3R)-1-[4-[4-[4-[[trans-4-(aminomethyl)cyclohexyl]-difluoro-methyl]-6-chloro-2-pyridyl]piperazin-1-yl]sulfonylphenyl]-5-oxo-pyrrolidin-3-yl]carbamate (compound 118g)

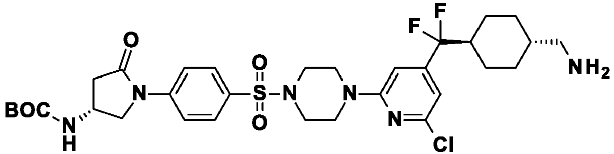

To a solution of compound 118f (380.0 mg, 0.540 mmol) in DCM (5 mL) was added trifluoromethanesulfonic anhydride (0.18 mL, 1.09 mmol) at -20 °C, and the reaction was stirred at -20 °C for 0.5 hour. To the mixture was added ammonia gas in THF (92.68 mg, 5.44 mmol) and stirred for 1 hour at 45 °C. After completion, the mixture was quenched by slow addition of saturated aqueous ammonium chloride (20 mL). The resulting mixture was extracted with EA (20 mL × 3). The combined organic layer was washed with brine (50 mL), dried over anhydrous Na₂SO₄, filtered, and concentrated under reduced pressure to afford the crude product, which was purified by prep-HPLC to give 118g (120 mg, 0.170 mmol, 31.62% yield) as white solid. MS obsd. (ESI⁺) [(M+H)⁺]: 697.2.

### Step 8: (4R)-4-amino-1-[4-[4-[4-[[trans-4-(aminomethyl)cyclohexyl]-difluoro-methyl]-6-chloro-2-pyridyl]piperazin-1-yl]sulfonylphenyl]pyrrolidin-2-one

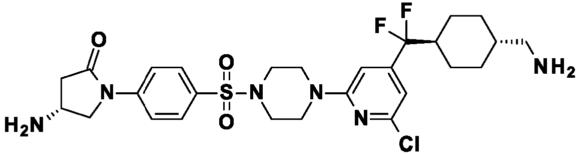

To a solution of compound 118 (40.0 mg, 0.060 mmol) in DCM (1 mL) was added TFA (65.4 mg, 0.570 mmol) at 25 °C. The reaction was stirred at 25 °C for 15 minutes. After completion, the mixture was concentrated under reduced pressure to afford the crude product, which was purified by prep-HPLC to give **Example 118** (24.7 mg, 59.1% yield) as a white solid. MS obsd. (ESI⁺) [(M+H)⁺]: 597.2. ¹H NMR (400 MHz, DMSO-*d₆*) δ ppm 8.74 - 8.26 (m, 3 H), 7.95 - 7.91 (m, 2 H), 7.85 - 7.75 (m, 2 H), 6.86 - 6.76 (m, 1 H), 6.76 - 6.68 (m, 1 H), 4.35 - 4.21 (m, 1 H), 4.13 - 4.00 (m, 1 H), 3.97 - 3.82 (m, 1 H), 3.75 - 3.60 (m, 4 H), 3.15 - 2.88 (m, 5 H), 2.71 - 2.60 (m, 3 H), 2.22 - 2.04 (m, 1 H), 1.90 - 1.74 (m, 2 H), 1.73 - 1.61 (m, 2 H), 1.56 - 1.42 (m, 1 H), 1.18 - 1.01 (m, 2 H), 0.99 - 0.83 (m, 2 H). ¹⁹F NMR (376 MHz, DMSO-*d₆*) δ -103.881 (s, 2 F).

### Example 119

### (4R)-4-amino-1-[4-[4-[6-chloro-4-[trans-difluoro-(3-hydroxycyclobutyl)methyl]-2-pyridyl]piperazin-1-yl]sulfonylphenyl]pyrrolidin-2-one

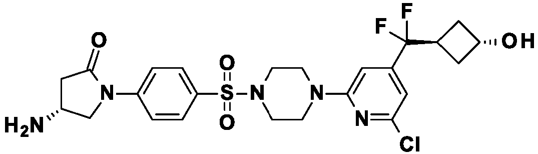

The title compound was prepared according to the following procedures:

### Step 1: Trans-(3-benzyloxycyclobutyl)methanol (compound 119a)

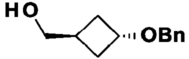

To a solution of 3-benzyloxycyclobutanecarboxylic acid (1.2 g, 5.82 mmol) in THF (5 mL) was added borane-methyl sulfide complex (1.16 mL, 11.64 mmol) slowly. The reaction was stirred at 20 °C for 2 hours. After completion, the reaction was quenched with methanol (5 mL), and diluted with aq. NH₄Cl solution (20 mL). The aqueous phase was extracted with EA (50 mL × 2). The combined organic layer was washed with brine (100 mL), dried over anhydrous Na₂SO₄, filtered and concentrated *in vacuo* to give compound 119a (1.05 g, 93.87% yield) as colorless oil.

### Step 2: Trans-3-benzyloxycyclobutanecarbaldehyde (compound 119b)

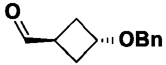

To a solution of DMSO (0.85 mL, 11.96 mmol) in DCM (20 mL) was added 119a (1000 mg, 5.2 mmol) dropwise at -78 °C. After being stirred for 10 minutes, oxalyl chloride (1056 mg, 8.32 mmol) in DCM (1 mL) was added and the reaction was stirred at -78 °C for 0.5 hour. Then triethylamine (2.9 mL, 20.81 mmol) was added and the mixture was stirred at -78 °C for 3 hours. After completion, the reaction was diluted with water (30 mL) and then extracted with DCM (20 mL × 2). The combined organic layer was washed with brine (50 mL), dried over anhydrous Na₂SO₄, filtered, and concentrated *in vacuo* to give a crude residue, which was purified by column chromatography on silica gel (EA:PE = 1:100 - 1:10) to afford 119b (980 mg, 99.04% yield) as light yellow oil.

### Step 3: Trans-(3-Benzyloxycyclobutyl)-(2,6-dichloro-4-pyridyl)methanol (compound 119c)

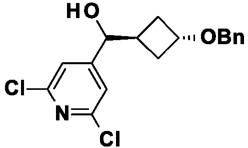

To a solution of 2,6-dichloro-4-iodopyridine (1.55 g, 5.67 mmol) in THF (10 mL) was added isopropylmagnesium chloride lithium chloride complex in THF (4.76 mL, 6.18 mmol) dropwise at -78 °C. After addition, the reaction was warmed up to 25 °C and stirred for 1 hour. Then, compound 119b (980.0 mg, 5.15 mmol) in THF (5 mL) was added at 0 °C. The reaction was stirred at 25 °C for 1 hour. The reaction was quenched with aq. NH₄Cl solution (50 mL), extracted with EA (100 mL × 2). The combined organic layer was washed with brine (100 mL), dried over anhydrous Na₂SO₄, filtered and concentrated *in vacuo* to give the crude product, which was purified by prep-HPLC to afford 119c (760 mg, 43.62% yield) as a yellow oil. MS obsd.: 338.1 (MH⁺).

### Step 4: Trans-(3-Benzyloxycyclobutyl)-(2,6-dichloro-4-pyridyl)methanone (compound 119d)

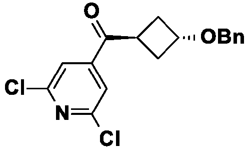

To a solution of compound 119c (660 mg, 1.95 mmol) in DCM (5 mL) was added Dess-Martin periodinane (993 mg, 2.34 mmol) in one portion, and the reaction was stirred at 25 °C for 2 hours. After completion, the reaction was poured into aq. K₂CO₃ solution (100 mL) and stirred for 30 minutes. The aqueous phase was extracted with EA (50 mL × 3). The combined organic layer was washed with brine (100 mL), dried over anhydrous Na₂SO₄, filtered, and concentrated *in vacuo* to give 119d (650 mg, 99.08% yield) as colorless oil. MS obsd.: 336.1 (MH⁺).

### Step 5: trans-4-[(3-Benzyloxycyclobutyl)-difluoro-methyl]-2,6-dichloro-pyridine (compound 119e)

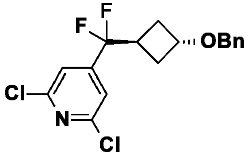

To a solution of 119d (600 mg, 1.78 mmol) in DCM (5 mL) was added DAST (2.88 g, 17.85 mmol) in one portion, and the reaction was stirred at 25 °C for 16 hours. After completion, the reaction mixture was poured into ice water and the pH was adjusted to 7~8 with aq. NaHCO₃ solution. The aqueous phase was extracted with EA (30 mL × 3). The combined organic layer was washed with brine (100 mL), dried over anhydrous Na₂SO₄, filtered and concentrated *in vacuo* to give the crude product, which was purified by flash column on silica gel (EA:PE = 1:100-1:10) to afford 119e (590 mg, 92.29% yield) as a yellow solid. MS obsd.: 358.1 (MH⁺).

### Step 6: Trans-3-[(2,6-dichloro-4-pyridyl)-difluoro-methyl]cyclobutanol (compound 119f)

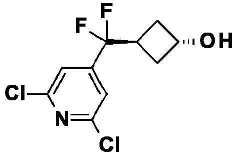

A solution of compound 119e (550 mg, 1.54 mmol) in DCM (5 mL) was added boron trichloride (4.61 mL, 4.61 mmol) and the reaction was stirred at -78 °C for 1 hour. After completion, the mixture was quenched with water (20 mL) and extracted with EA (30 mL × 2). The combined organic layer was washed with brine (50 mL), dried over anhydrous Na₂SO₄, filtered, and concentrated *in vacuo* to give the crude product, which was purified by chromatography on silica gel (EA:PE = 1:100-1:10) to afford 119f (340 mg, 82.6% yield) as yellow oil. MS obsd.: 267.9 (MH⁺).

### Step 7: tert-butyl N-[(3R)-1-[4-[4-[6-chloro-4-[trans-difluoro-(3-hydroxycyclobutyl)methyl]-2-pyridyl]piperazin-1-yl]sulfonylphenyl]-5-oxo-pyrrolidin-3-yl]carbamate (compound 119g)

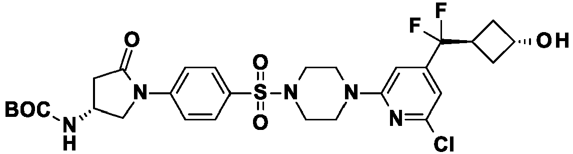

To a solution of compound 119f (40 mg, 0.150 mmol) in DMSO (1 mL) were added **Intermediate D** (63.34 mg, 0.150 mmol), *N,N*-diisopropylethylamine (0.05 mL, 0.300 mmol) in one portion. The reaction was stirred at 100 °C for 16 hours. After completion, the reaction was poured into water (10 mL) and the aqueous phase was extracted with EA (10 mL × 3). The combined organic layer was washed with brine (50 mL), dried over anhydrous Na₂SO₄, filtered, and concentrated *in vacuo* to give crude product, which was purified by prep-HPLC to give 119g (60 mg, 61.29% yield) as a white solid. MS obsd.: 656.1 (MH⁺).

### Step 7: (4R)-4-amino-1-[4-[4-[6-chloro-4-[trans-difluoro-(3-hydroxycyclobutyl)methyl]-2-pyridyl]piperazin-1-yl]sulfonylphenyl]pyrrolidin-2-one

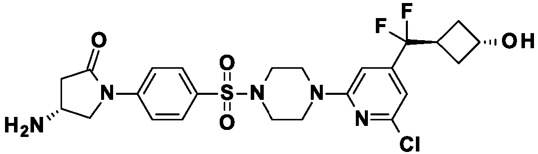

To solution of compound 119g (60 mg, 0.090 mmol) in DCM (0.5 mL) was added TFA (0.5 mL, 6.49 mmol) in one portion. The reaction was stirred at 25 °C for 1 hour. After completion, the reaction was concentrated *in vacuo* to give crude product, which was purified by prep-HPLC to afford **Example 119** (32 mg, 57.63% yield) as a white solid. MS obsd.: 556.1 (MH⁺). ¹H NMR (400 MHz, DMSO-*d₆*) δ ppm 8.48 - 8.33 (m, 3 H), 7.91 (br d, *J* = 8.8 Hz, 2 H), 7.79 (br d, *J =* 8.6 Hz, 2 H), 6.84 - 6.78 (m, 1 H), 6.76 - 6.68 (m, 1 H), 5.25 - 5.15 (m, 1 H), 4.32 - 4.21 (m, 1 H), 4.19 - 4.10 (m, 1 H), 4.09 - 4.00 (m, 1 H), 3.88 (br s, 1 H), 3.65 (br s, 4 H), 3.11 - 3.03 (m, 1 H), 2.96 (br s, 4 H), 2.64 (br s, 1 H), 2.23 (br d, *J* = 4.9 Hz, 2 H), 2.02 - 1.88 (m, 2 H), 1.32 - 1.14 (m, 1 H). ¹⁹F NMR (376 MHz, DMSO-d₆) δ -103.606 (s, 2 F).

### Example 120

### Trans-4-[[2-[4-[4-[(4R)-4-amino-2-oxo-pyrrolidin-1-yl]phenyl]sulfonylpiperazin-1-yl]-6-chloro-4-pyridyl]-difluoro-methyl]cyclohexanecarboxamide

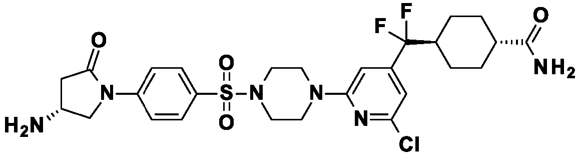

The title compound was prepared according to the following procedures:

### Step 1: Trans-methyl 4-[[2-chloro-6-[4-[4-[(4R)-4-(tert-butoxycarbonylamino)-2-oxo-pyrrolidin-1-yl]phenyl]sulfonylpiperazin-1-yl]-4-pyridyl]-difluoro-methyl]cyclohexanecarboxylate (compound 120a)

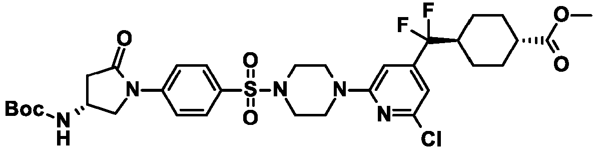

To a solution of compound Intermediate D (50 mg, 0.118 mmol) and compound 118d (60 mg, 0.177 mmol) in DMSO (1 mL) was added K₂CO₃ (48 mg, 0.353 mmol). The mixture was stirred at 90 °C for 12 hours. After completion, the mixture was diluted with EA (50 mL) and water (30 mL). The organic phase was washed with brine (50 mL), dried over anhydrous Na₂SO₄, concentrated *in vacuo* to give the crude compound 120a (60 mg, 70.73% yield) as a yellow solid, which was used directly in the next step. MS obsd. (ESI⁺) [(M+H)⁺]: 726.4.

### Step 2: Trans-4-[[2-chloro-6-[4-[4-[(4R)-4-(tert-butoxycarbonylamino)-2-oxo-pyrrolidin-1-yl]phenyl]sulfonylpiperazin-1-yl]-4-pyridyl]-difluoro-methyl]cyclohexanecarboxylic acid (compound 120b)

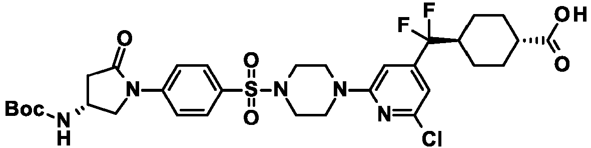

To a solution of compound 120a (2.4 g, 3.3 mmol) in MeCN (30 mL) and water (10 mL) were added LiBr (2.87 g, 33.06 mmol) and TEA (2.3 mL, 16.52 mmol). The mixture was stirred at 50 °C for 5 hours. After completion, the mixture was diluted with EA (200 ml) and adjusted to pH=5 with 1M HCl solution. The organic layer was washed with brine (200 mL), dried over anhydrous Na₂SO₄, filtered, and concentrated *in vacuo* to give the crude product, which was purified by flash column on silica gel (EA:PE = 1:5-3:1) to afford compound 120b (640 mg, 27.19% yield) as a yellow solid. MS obsd. (ESI⁺) [(M+H)⁺]: 712.2.

### Step 3: Trans-tert-butyl N-[(3R)-1-[4-[4-[4-[(4-carbamoylcyclohexyl)-difluoro-methyl]-6-chloro-2-pyridyl]piperazin-1-yl]sulfonylphenyl]-5-oxo-pyrrolidin-3-yl]carbamate (compound 120c)

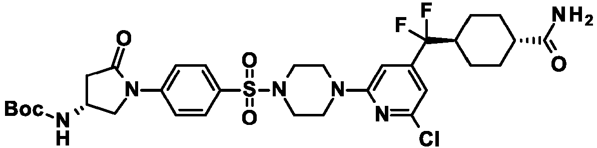

A mixture of ammonium chloride (20 mg, 0.374 mmol), compound 120b (100 mg, 0.140 mmol), HATU (106.71 mg, 0.281 mmol) and DIPEA (54.38 mg, 73.49 µL, 0.421 mmol) in DMF (5 mL) was stirred at room temperature for 2 hours. After completion, the mixture was diluted with EA, washed with water (100 mL) and brine (100 mL). The combined organic layer was dried over anhydrous Na₂SO₄, filtered, concentrated *in vacuo* to give crude product 120c (50 mg, 50.07%) as a white solid, which was used directly in the next step. MS obsd. (ESI⁺) [(M+H)⁺]: 710.9.

### Step 4: Trans-4-[[2-chloro-6-[4-[4-[(4R)-4-amino-2-oxo-pyrrolidin-1-yl]phenyl]sulfonylpiperazin-1-yl]-4-pyridyl]-difluoro-methyl]cyclohexanecarboxamide

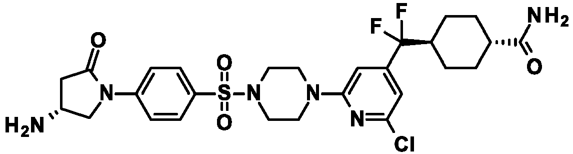

Compound 120c (90 mg, 0.127 mmol) and TFA (1 mL) were dissolved in the DCM (5 mL). The reaction was stirred at room temperature for 2 hours. After completion, the reaction was concentrated *in vacuo* to give crude product, which was purified by prep-HPLC to give **Example 120** (50.8 mg, 62.41% yield) as a white solid. MS obsd.: 611.1 (MH⁺). ¹H NMR (400 MHz, METHANOL-*d*₆) δ ppm 7.88 - 7.98 (m, 2 H), 7.70 - 7.79 (m, 2 H), 6.75 - 6.80 (m, 1 H), 6.69 - 6.72 (m, 1 H), 3.90 - 4.07 (m, 1 H), 3.59 - 3.78 (m, 5 H), 3.46 - 3.59 (m, 1 H), 2.88 - 3.03 (m, 4 H), 2.74 - 2.89 (m, 1 H), 2.26 - 2.40 (m, 1 H), 2.10 - 2.25 (m, 1 H), 1.92 - 2.07 (m, 2 H), 1.70 - 1.81 (m, 2 H), 1.58 - 1.70 (m, 2 H), 1.18 - 1.37 (m, 4 H), 0.96 - 1.18 (m, 3 H). ¹⁹F NMR (376 MHz, METHANOL-*d*₄) δ ppm -107.25 (br d, *J* = 14.99 Hz, 2 F).

### BIOLOGICAL EXAMPLES

### Example 121: 50% Inhibitory Concentration (IC₅₀) to UDP-2,3-diacylglucosamine hydrolase (LpxH)

The inhibitory potency of molecules to UDP-2,3-diacylglucosamine hydrolase (LpxH) was determined using the UMP/CMP-Glo^{™} Glycosyltransferase Assay kit. LpxH hydrolyzes the pyrophosphate bond of UDP-2,3-diacylglucosamine (UDP-DAG) to yield 2,3-diacylglucosamine 1-phosphate (lipid X) and UMP. The assay kit could quantify the UMP production by convert the UMP to ATP and generate light in a luciferase reaction. The compound's effect on the LpxH activity is detected by measuring the light using a luminometer (Envision).

**Table 1: Enzymatic IC₅₀ values of the compounds of this invention against Escherichia coli LpxH**

| **Example No.** | IC₅₀ (µg/mL) | **Example No.** | IC₅₀ (µg/mL) |
|---|---|---|---|
| **1** | 0.131 | **53** | 0.004 |
| **2** | 0.044 | **54** | 0.012 |
| **3** | 0.239 | **55** | 0.030 |
| **4** | 0.016 | **56** | 0.008 |
| **5** | 0.082 | **57** | 0.021 |
| **6** | 0.023 | **58** | 0.016 |
| **7** | 0.031 | **59** | 0.055 |
| **8** | 0.059 | **60** | 0.005 |
| **9** | 0.013 | **61** | 0.026 |
| **10** | 0.011 | **63** | 0.110 |
| **11** | 0.013 | **64** | 0.142 |
| **12** | 0.037 | **67** | 0.079 |
| **13** | 0.068 | **68** | 0.126 |
| **14** | 0.054 | **69** | 0.403 |
| **15** | 0.051 | **70** | 0.028 |
| **16** | 0.021 | **71** | 0.296 |
| **17** | 0.263 | **72** | 0.215 |
| **18** | 0.083 | **73** | 0.018 |
| **19** | 0.019 | **76** | 0.015 |
| **20** | 0.033 | **77** | 0.004 |
| **21** | 0.006 | **79** | 0.102 |
| **22** | <0.002 | **82** | 0.138 |
| **24** | 0.009 | **83** | 0.002 |
| **25** | 0.015 | **84** | 0.008 |
| **26** | 0.020 | **87** | 0.007 |
| **27** | 0.012 | **88** | 0.008 |
| **28** | 0.007 | **89** | 0.034 |
| **29** | 0.015 | **90** | 0.007 |
| **30** | 0.009 | **91** | 0.006 |
| **31** | 0.004 | **92** | 0.054 |
| **32** | 0.015 | **93** | 0.007 |
| **33** | 0.009 | **94** | 0.105 |
| **34** | 0.016 | **95** | 0.033 |
| **35** | <0.002 | **96** | 0.002 |
| **36** | 0.016 | **97** | 0.048 |
| **37** | 0.030 | **98** | 0.027 |
| **38** | 0.028 | **99** | <0.002 |
| **39** | 0.008 | **100** | <0.002 |
| **40** | 0.012 | **101** | 0.055 |
| **41** | 0.006 | **102** | 0.006 |
| **42** | 0.009 | **103** | 0.002 |
| **43** | 0.009 | **104** | 0.005 |
| **44** | 0.102 | **105** | 0.003 |
| **45** | 0.025 | **106** | 0.113 |
| **46** | 0.005 | **107** | 0.011 |
| **48** | 0.004 | **108** | 0.020 |
| **49** | 0.047 | **109** | 0.037 |
| **51** | 0.030 | **110** | 0.002 |
| **52** | 0.020 | **120** | <0.002 |

### Example 122: Minimal Inhibitory Concentration Protocol (MIC) Assay:

The in vitro potency of compounds to inhibit *E. coli* (ATCC 25922) and *K. pneumonia* (ATCC 43816) growth was assessed by MIC (Minimal Inhibitory Concentration) assay. Samples were prepared from 10 mM DMSO stock solutions. After serial 2-fold dilution in DMSO in a master plate (Greiner, Cat No: 651201), 180 µL sterile distilled water was added to 20 µL each aliquot of the samples. Then 10µL diluted compounds were transferred into a new assay plate (Costar, 3599).

The growth medium Cation-Adjusted Mueller Hinton Broth (CAMHB) was prepared by adding the 20 mg per liter CaCl₂ and 20 mg per liter MgCl₂ into the MHB medium (Jianglai Company, sterilized).

Vials of each of the test microorganisms were maintained frozen in the vapor phase of a liquid nitrogen freezer. The bacteria *E.coli* ATCC 25922 (KWIKSTIK, 0335K) and *K. pneumonia* ATCC 43816 were taken out from liquid nitrogen freezer, then thawed in room temperature, and diluted in the CAMHB medium to achieve a final inoculum of 5 × 105 CFU/ mL. 90 µL CAMHB with bacteria was dispensed to the assay plate and pipetted 5 times.

Then the assay plates were incubated for 20 hours at 35°C in ambient air. Following incubation, the MIC (µg/mL), the lowest concentration of drug that inhibits visible growth of the microorganism was read and recorded throw the microscope.

**Table 2: MIC values of the compounds of this invention against E. coli and K. pneumonia**

| **Example No.** | MIC (µg/mL) | | **Example No.** | MIC (µg/mL) | |
|---|---|---|---|---|---|
| | *E. coli* ATCC 25922 | *K. pneumoniae* ATCC43816 | | *E. coli* ATCC 25922 | *K. pneumoniae* ATCC43816 |
| **1** | 1.45 | 1.93 | **61** | 6.47 | 6.47 |
| **2** | 1.98 | 3.95 | **62** | 7.74 | 15.47 |
| **3** | 3.96 | 1.98 | **63** | 1.83 | 7.30 |
| **4** | 3.51 | 7.03 | **64** | 3.65 | 14.60 |
| **5** | 8.08 | 16.15 | **65** | 3.74 | 7.48 |
| **6** | 1.98 | 1.98 | **66** | 3.76 | 7.52 |
| **7** | 3.78 | 5.66 | **67** | 6.61 | 13.22 |
| **8** | 3.86 | 7.72 | **68** | 2.59 | 10.35 |
| **9** | 1.78 | 3.57 | **69** | 13.50 | 6.75 |
| **10** | 1.05 | 4.20 | **70** | 15.51 | 15.51 |
| **11** | 2.10 | 4.20 | **71** | 12.53 | 12.53 |
| **12** | 2.01 | 4.03 | **72** | 7.06 | 7.06 |
| **13** | 0.83 | 2.49 | **73** | 0.44 | 0.44 |
| **14** | 1.75 | 3.50 | **74** | 1.18 | 1.18 |
| **15** | 1.01 | 2.02 | **75** | 1.22 | 1.22 |
| **16** | 1.03 | 1.03 | **76** | 0.51 | 1.03 |
| **17** | 1.82 | 3.64 | **77** | 0.44 | 0.44 |
| **18** | 0.91 | 0.91 | **78** | 0.22 | 1.76 |
| **19** | 1.05 | 1.05 | **79** | 14.22 | >28.432 |
| **20** | 1.77 | 3.54 | **80** | >28.432 | >28.432 |
| **21** | 0.91 | 1.81 | **81** | 4.36 | 6.98 |
| **22** | 1.99 | 1.99 | **82** | 6.98 | 6.98 |
| **23** | 30.45 | 60.90 | **83** | 1.70 | 1.70 |
| **24** | 2.24 | 2.24 | **84** | 4.18 | 16.72 |
| **25** | 2.60 | 2.60 | **85** | 2.29 | 2.29 |
| **26** | 2.64 | 5.28 | **86** | 1.26 | 1.26 |
| **27** | 1.78 | 3.57 | **87** | 0.55 | 1.09 |
| **28** | 1.83 | 1.83 | **88** | 0.26 | 0.53 |
| **29** | 1.96 | 1.96 | **89** | 0.88 | 0.88 |
| **30** | 2.01 | 4.02 | **90** | 1.76 | 1.76 |
| **31** | 2.15 | 4.31 | **91** | 1.76 | 1.76 |
| **32** | 8.94 | 8.94 | **92** | 2.12 | 4.24 |
| **33** | 1.10 | 2.20 | **93** | 1.76 | 1.76 |
| **34** | 4.39 | 4.39 | **94** | 3.53 | 3.53 |
| **35** | 0.64 | 1.27 | **95** | 0.89 | 0.89 |
| **36** | 1.88 | 3.76 | **96** | 3.48 | 1.74 |
| **37** | 2.64 | 5.27 | **97** | 0.85 | 1.27 |
| **38** | 5.09 | 5.09 | **98** | 0.89 | 0.89 |
| **39** | 0.96 | 1.92 | **99** | 4.01 | 0.89 |
| **40** | 1.79 | 1.79 | **100** | 1.86 | 0.93 |
| **41** | 1.27 | 2.55 | **101** | 0.44 | 0.89 |
| **42** | 10.36 | 10.36 | **102** | 0.89 | 0.89 |
| **43** | 3.82 | 5.09 | **103** | 0.89 | 0.89 |
| **44** | 1.73 | 3.47 | **104** | 1.01 | 2.01 |
| **45** | 3.65 | 7.29 | **105** | 1.79 | 1.79 |
| **46** | 1.83 | 3.65 | **106** | 1.77 | 1.77 |
| **47** | 1.91 | 5.71 | **107** | 2.85 | 5.70 |
| **48** | 1.84 | 3.69 | **108** | 4.98 | 4.98 |
| **49** | 3.87 | 7.74 | **109** | 16.38 | 16.38 |
| **50** | 1.91 | 3.81 | **110** | 0.99 | 0.99 |
| **51** | 3.78 | 15.13 | **111** | 0.90 | 1.80 |
| **52** | 17.48 | 17.48 | **112** | 0.45 | 0.45 |
| **53** | 2.00 | 3.00 | **113** | 0.26 | 0.51 |
| **54** | 2.00 | 4.00 | **114** | 0.91 | 1.80 |
| **55** | 3.88 | 7.77 | **115** | 0.97 | 1.90 |
| **56** | 1.96 | 3.91 | **116** | 0.47 | 0.94 |
| **57** | 2.00 | 2.00 | **117** | 0.93 | 1.90 |
| **58** | 7.44 | 14.89 | **118** | 0.50 | 0.99 |
| **59** | 3.81 | 7.62 | **119** | 0.46 | 0.46 |
| **60** | 1.95 | 3.90 | **120** | 0.96 | 0.48 |

## Claims

1. A compound of formula (I), 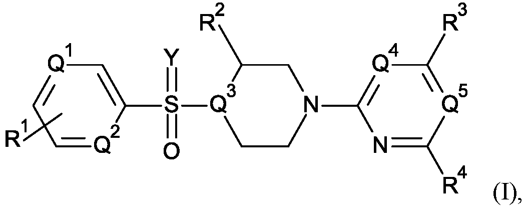 wherein
R¹ is 2-oxo-1-oxa-3,8-diazaspiro[4.5]decanyl unsubstituted or substituted by aminoC₁₋₆alkyl,
2-oxo-3a,4,5,6,7,7a-hexahydrooxazolo[4,5-c]pyridinyl,
2-oxo-3a,4,5,6,7,7a-hexahydrooxazolo[5,4-c]pyridinyl,
2-oxo-3a,4,6,7,8,8a-hexahydrooxazolo[4,5-c]azepinyl substituted by aminoC₁₋₆alkyl or C₁₋₆alkylaminoC₁₋₆alkyl,
2-oxo-3a,5,6,6a-tetrahydrofuro[2,3-d]oxazolyl substituted twice by substituents independently selected from hydroxy and hydroxyC₁₋₆alkyl,
2-oxo-4,5,6,6a-tetrahydro-3aH-pyrrolo[3,4-d]oxazolyl,
2-oxo-4,5,6,7,8,8a-hexahydro-3aH-oxazolo[4,5-c]azepinyl,
2-oxo-5,6,7,7a-tetrahydro-3aH-pyrano[2,3-d]oxazolyl substituted three times by substituents independently selected from hydroxy and hydroxyC₁₋₆alkyl,
3-oxo-2,6,9-triazaspiro[4.5]decanyl,
3-oxo-2,6-diazaspiro[4.5]decanyl,
3-oxo-2,8-diazaspiro[4.5]decanyl,
5-oxo-1,2,3,3a,6,6a-hexahydropyrrolo[3,2-b]pyrrolyl,
6-oxo-5-oxa-2,7-diazaspiro[3.4]octanyl,
7-oxo-2,6-diazaspiro[3 .4]octanyl,
oxoazetidinyl substituted by amino,
oxooxazolidinyl substituted once or twice by substituents independently selected from ((aminoC₁₋₆alkyl)azetidinyl)C₁₋₆alkyl, (C₁₋₆alkyl)₂aminoC₁₋₆alkyl, (dihydroxypyrrolidinyl)C₁₋₆alkyl, (pyrrolidinylamino)C₁₋₆alkyl, 2,6-diazaspiro[3.3]heptanylC₁₋₆alkyl, dihydroxy(C₁₋₆alkoxy)tetrahydrofuranyl, aminoazetidinylC₁₋₆alkyl, aminoC₁₋₆alkyl, C₁₋₆alkyl, C₁₋₆alkylaminoC₁₋₆alkyl, C₁₋₆alkylcarbonylaminoC₁₋₆alkyl, hydroxyC₁₋₆alkyl and piperazinylC₁₋₆alkyl, or
oxopyrrolidinyl substituted once, twice or three times by substituents independently selected from (((C₁₋₆alkyl)₂aminoC₁₋₆alkyl)azetidinyl)C₁₋₆alkyl, ((aminoC₁₋₆alkyl)azetidinyl)C₁₋₆alkyl, ((aminoC₃₋₇cycloalkyl)azetidinyl)C₁₋₆alkyl, ((C₁₋₆alkylamino)azetidinyl)C₁₋₆alkyl, (aminoazetidinyl)C₁₋₆alkyl, (aminoC₁₋₆alkyl)azetidinyl, (azetidinylC₁₋₆alkylamino)C₁₋₆alkyl, (C₁₋₆alkylpiperazinyl)C₁₋₆alkyl, (hydroxyC₁₋₆alkyl)piperazinyl, 2,5-diazabicyclo[2.2.1]heptanyl, 2,6-diazaspiro[3.3]heptanyl, amino, amino(hydroxy)piperidinyl, amino(hydroxy)pyrrolidinyl, aminoazetidinyl, aminoC₁₋₆alkyl, (aminoC₁₋₆alkyl)amino, aminopyrrolidinyl, azetidinylamino, C₁₋₆alkyl, C₁₋₆alkylamino, dihydroxypyrrolidinyl, (hydroxyC₁₋₆alkyl)amino, piperazinyl, piperazinylC₁₋₆alkyl, piperidinylamino and pyrrolidinylamino;
R² is H or cyano;
R³ is benzyl,
C₃₋₇cycloalkylsulfonyl,
cyanoC₃₋₇cycloalkyl,
phenoxy,
phenylC₃₋₇cycloalkyl,
tetrahydropyranylsulfonyl, or
C₁₋₆alkyl substituted twice or three times by substituents independently selected from halogen, (aminoC₁₋₆alkyl)C₃₋₇cycloalkyl, (hydroxyC₁₋₆alkyl)C₃₋₇cycloalkyl, 1,2,3,6-tetrahydropyridinyl, 1,4-dioxanyl, 3,6-dihydro-2H-pyranyl, 3-azabicyclo[3.1.0]hexanyl, 3-oxabicyclo[3.1.0]hexanyl, 8-oxabicyclo[3.2.1]octanyl, azetidinyl, C₁₋₆alkylhalopyrazinyl, C₁₋₆alkylmorpholinyl, C₁₋₆alkylpyrazinyl, carbamoylC₃₋₇cycloalkyl, hydroxyC₃₋₇cycloalkyl, morpholinyl, oxetanyl, piperidinyl, pyrazinyl, pyridinyl, pyrimidinyl, tetrahydrofuranyl, tetrahydropyranyl, phenyl and pyridinyl;
R⁴ is halogen, C₁₋₆alkyl, C₃₋₇cycloalkyl, C₁₋₆alkoxy or C₂₋₆alkynyl;
Q¹ is N or CR^{a}, wherein R^{a} is H or halogen;
Q² is N or CR^{b}, wherein R^{b} is H or halogen;
Q³ is N or CH;
Q⁴ is N or CH;
Q⁵ is N or CH;
Y is O or NH;
or a pharmaceutically acceptable salt thereof.

2. A compound according to claim 1 of formula (Ia), 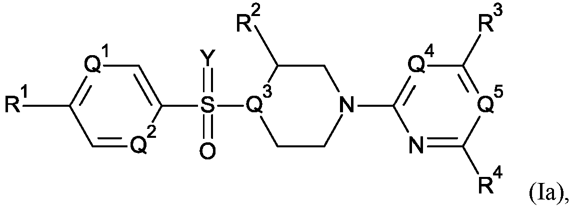 wherein
R¹ is 2-oxo-1-oxa-3,8-diazaspiro[4.5]decanyl unsubstituted or substituted by aminoC₁₋₆alkyl,
2-oxo-3a,4,5,6,7,7a-hexahydrooxazolo[4,5-c]pyridinyl,
2-oxo-3a,4,5,6,7,7a-hexahydrooxazolo[5,4-c]pyridinyl,
2-oxo-3a,4,6,7,8,8a-hexahydrooxazolo[4,5-c]azepinyl substituted by aminoC₁₋₆alkyl or C₁₋₆alkylaminoC₁₋₆alkyl,
2-oxo-3a,5,6,6a-tetrahydrofuro[2,3-d]oxazolyl substituted twice by substituents independently selected from hydroxy and hydroxyC₁₋₆alkyl,
2-oxo-4,5,6,6a-tetrahydro-3aH-pyrrolo[3,4-d]oxazolyl,
2-oxo-4,5,6,7,8,8a-hexahydro-3aH-oxazolo[4,5-c]azepinyl,
2-oxo-5,6,7,7a-tetrahydro-3aH-pyrano[2,3-d]oxazolyl substituted three times by substituents independently selected from hydroxy and hydroxyC₁₋₆alkyl,
3-oxo-2,6,9-triazaspiro[4.5]decanyl,
3-oxo-2,6-diazaspiro[4.5]decanyl,
3-oxo-2,8-diazaspiro[4.5]decanyl,
5-oxo-1,2,3,3a,6,6a-hexahydropyrrolo[3,2-b]pyrrolyl,
6-oxo-5-oxa-2,7-diazaspiro[3.4]octanyl,
7-oxo-2,6-diazaspiro[3.4]octanyl,
oxoazetidinyl substituted by amino,
oxooxazolidinyl substituted once or twice by substituents independently selected from ((aminoC₁₋₆alkyl)azetidinyl)C₁₋₆alkyl, (C₁₋₆alkyl)₂aminoC₁₋₆alkyl, (dihydroxypyrrolidinyl)C₁₋₆alkyl, (pyrrolidinylamino)C₁₋₆alkyl, 2,6-diazaspiro[3.3]heptanylC₁₋₆alkyl, dihydroxy(C₁₋₆alkoxy)tetrahydrofuranyl, aminoazetidinylC₁₋₆alkyl, aminoC₁₋₆alkyl, C₁₋₆alkyl, C₁₋₆alkylaminoC₁₋₆alkyl, C₁₋₆alkylcarbonylaminoC₁₋₆alkyl, hydroxyC₁₋₆alkyl and piperazinylC₁₋₆alkyl, or
oxopyrrolidinyl substituted once, twice or three times by substituents independently selected from (((C₁₋₆alkyl)₂aminoC₁₋₆alkyl)azetidinyl)C₁₋₆alkyl, ((aminoC₁₋₆alkyl)azetidinyl)C₁₋₆alkyl, ((aminoC₃₋₇cycloalkyl)azetidinyl)C₁₋₆alkyl, ((C₁₋₆alkylamino)azetidinyl)C₁₋₆alkyl, (aminoazetidinyl)C₁₋₆alkyl, (aminoC₁₋₆alkyl)azetidinyl, (azetidinylC₁₋₆alkylamino)C₁₋₆alkyl, (C₁₋₆alkylpiperazinyl)C₁₋₆alkyl, (hydroxyC₁₋₆alkyl)piperazinyl, 2,5-diazabicyclo[2.2.1]heptanyl, 2,6-diazaspiro[3.3]heptanyl, amino, amino(hydroxy)piperidinyl, amino(hydroxy)pyrrolidinyl, aminoazetidinyl, aminoC₁₋₆alkyl, (aminoC₁₋₆alkyl)amino, aminopyrrolidinyl, azetidinylamino, C₁₋₆alkyl, C₁₋₆alkylamino, dihydroxypyrrolidinyl, (hydroxyC₁₋₆alkyl)amino, piperazinyl, piperazinylC₁₋₆alkyl, piperidinylamino and pyrrolidinylamino;
R² is H or cyano;
R³ is benzyl,
C₃₋₇cycloalkylsulfonyl,
cyanoC₃₋₇cycloalkyl,
phenoxy,
phenylC₃₋₇cycloalkyl,
tetrahydropyranylsulfonyl, or
C₁₋₆alkyl substituted twice or three times by substituents independently selected from halogen, (aminoC₁₋₆alkyl)C₃₋₇cycloalkyl, (hydroxyC₁₋₆alkyl)C₃₋₇cycloalkyl, 1,2,3,6-tetrahydropyridinyl, 1,4-dioxanyl, 3,6-dihydro-2H-pyranyl, 3-azabicyclo[3.1.0]hexanyl, 3-oxabicyclo[3.1.0]hexanyl, 8-oxabicyclo[3.2.1]octanyl, azetidinyl, C₁₋₆alkylhalopyrazinyl, C₁₋₆alkylmorpholinyl, C₁₋₆alkylpyrazinyl, carbamoylC₃₋₇cycloalkyl, hydroxyC₃₋₇cycloalkyl, morpholinyl, oxetanyl, piperidinyl, pyrazinyl, pyridinyl, pyrimidinyl, tetrahydrofuranyl, tetrahydropyranyl, phenyl and pyridinyl;
R⁴ is halogen, C₁₋₆alkyl, C₃₋₇cycloalkyl, C₁₋₆alkoxy or C₂₋₆alkynyl;
Q¹ is N or CR^{a}, wherein R^{a} is H or halogen;
Q² is CR^{b}, wherein R^{b} is H or halogen;
Q³ is N;
Q⁴ is N or CH;
Q⁵ is N or CH;
Y is O;
or a pharmaceutically acceptable salt thereof.

3. A compound according to claim 1 or 2, wherein
R¹ is 2-oxo-1-oxa-3,8-diazaspiro[4.5]decanyl,
2-oxo-3a,4,5,6,7,7a-hexahydrooxazolo[4,5-c]pyridinyl,
2-oxo-3a,4,5,6,7,7a-hexahydrooxazolo[5,4-c]pyridinyl,
2-oxo-3a,5,6,6a-tetrahydrofuro[2,3-d]oxazolyl substituted twice by substituents independently selected from hydroxy and hydroxyC₁₋₆alkyl,
2-oxo-4,5,6,6a-tetrahydro-3aH-pyrrolo[3,4-d]oxazolyl,
2-oxo-4,5,6,7,8,8a-hexahydro-3aH-oxazolo[4,5-c]azepinyl,
3-oxo-2,6-diazaspiro[4.5]decanyl,
5-oxo-1,2,3,3a,6,6a-hexahydropyrrolo[3,2-b]pyrrolyl,
6-oxo-5-oxa-2,7-diazaspiro[3.4]octanyl,
oxooxazolidinyl substituted once or twice by substituents independently selected from ((aminoC₁₋₆alkyl)azetidinyl)C₁₋₆alkyl, (C₁₋₆alkyl)₂aminoC₁₋₆alkyl, dihydroxy(C₁₋₆alkoxy)tetrahydrofuranyl, aminoC₁₋₆alkyl, C₁₋₆alkyl, C₁₋₆alkylaminoC₁₋₆alkyl, hydroxyC₁₋₆alkyl and piperazinylC₁₋₆alkyl, or
oxopyrrolidinyl substituted by substituent selected from ((aminoC₁₋₆alkyl)azetidinyl)C₁₋₆alkyl, ((aminoC₃₋₇cycloalkyl)azetidinyl)C₁₋₆alkyl, (aminoazetidinyl)C₁₋₆alkyl, (C₁₋₆alkylpiperazinyl)C₁₋₆alkyl, (hydroxyC₁₋₆alkyl)piperazinyl, amino, amino(hydroxy)piperidinyl, amino(hydroxy)pyrrolidinyl, aminoC₁₋₆alkyl, aminopyrrolidinyl, C₁₋₆alkylamino, dihydroxypyrrolidinyl, (hydroxyC₁₋₆alkyl)amino, piperazinyl, piperazinylC₁₋₆alkyl and piperidinylamino.

4. A compound according to any one of claims 1 to 3, wherein R³ is benzyl, phenylC₃₋₇cycloalkyl, or C₁₋₆alkyl substituted three times by substituents independently selected from halogen, (hydroxyC₁₋₆alkyl)C₃₋₇cycloalkyl, 1,2,3,6-tetrahydropyridinyl, 1,4-dioxanyl, 3,6-dihydro-2H-pyranyl, 3-azabicyclo[3.1.0]hexanyl, 3-oxabicyclo[3.1.0]hexanyl, 8-oxabicyclo[3.2.1]octanyl, C₁₋₆alkylhalopyrazinyl, C₁₋₆alkylpyrazinyl, carbamoylC₃₋₇cycloalkyl, hydroxyC₃₋₇cycloalkyl, morpholinyl, oxetanyl, pyrazinyl, pyridinyl, tetrahydropyranyl and phenyl.

5. A compound according to any one of claims 1 to 4, wherein R² is H.

6. A compound according to any one of claims 1 to 5, wherein R⁴ is halogen or C₁₋₆alkyl, in particular wherein R⁴ is chloro or methyl.

7. A compound according to any one of claims 1 to 6, wherein Q¹ is CR^{a}, wherein R^{a} is H or halogen, in particular wherein Q¹ is CR¹, wherein R¹ is H or fluoro.

8. A compound according to any one of claims 1 to 7, wherein Q² is CH.

9. A compound according to claim 1 or 2, wherein
R¹ is oxopyrrolidinyl substituted by amino or aminoC₁₋₆alkyl;
R² is H;
R³ is C₁₋₆alkyl substituted three times by substituents independently selected from halogen, (hydroxyC₁₋₆alkyl)C₃₋₇cycloalkyl, 1,4-dioxanyl, 3-azabicyclo[3.1.0]hexanyl, 3-oxabicyclo[3.1.0]hexanyl, C₁₋₆alkylhalopyrazinyl, C₁₋₆alkylpyrazinyl, hydroxyC₃₋₇cycloalkyl, morpholinyl, oxetanyl, phenyl, pyridinyl and tetrahydropyranyl;
R⁴ is halogen or C₁₋₆alkyl;
Q¹ is CR^{a}, wherein R^{a} is H or halogen;
Q² is CH;
Q³ is N;
Q⁴ is N or CH;
Q⁵ is N or CH;
Y is O;
or a pharmaceutically acceptable salt thereof.

10. A compound according to claim 9, wherein
R¹ is oxopyrrolidinyl substituted by amino or aminomethyl;
R² is H;
R³ is difluoro(1,4-dioxan-2-yl)methyl, difluoro(2-(hydroxymethyl)cyclopropyl)methyl, difluoro(2-pyridinyl)methyl, difluoro(3-hydroxycyclobutyl)methyl, difluoro(3-oxabicyclo[3.1.0]hexan-6-yl)methyl, difluoro(5-chloro-6-methyl-pyrazin-2-yl)methyl, difluoro(6-methylpyrazin-2-yl)methyl, difluoro(morpholin-2-yl)methyl, difluoro(oxetan-3-yl)methyl, difluoro(phenyl)methyl, difluoro(tetrahydropyran-4-yl)methyl or difluoro[3-azabicyclo[3.1.0]hexan-1-yl]methyl;
R⁴ is chloro or methyl;
Q¹ is CR^{a}, wherein R^{a} is H or fluoro;
Q² is CH;
Q³ is N;
Q⁴ is N or CH;
Q⁵ is N or CH;
Y is O;
or a pharmaceutically acceptable salt thereof.

11. A compound according to claim 1 selected from:
4-Amino-1-[4-[4-[6-chloro-4-(trifluoromethyl)-2-pyridyl]piperazin-1-yl]sulfonylphenyl] pyrrolidin-2-one;
4-(Aminomethyl)-1-[4-[4-[6-chloro-4-(trifluoromethyl)-2-pyridyl]piperazin-1-yl]sulfonylphenyl]pyrrolidin-2-one;
5-(Aminomethyl)-3-[4-[4-[6-chloro-4-(trifluoromethyl)-2-pyridyl]piperazin-1-yl]sulfonylphenyl]oxazolidin-2-one;
*N*-[[3-[4-[4-[6-chloro-4-(trifluoromethyl)-2-pyridyl]piperazin-1-yl]sulfonylphenyl]-2-oxo-oxazolidin-5-yl]methyl]acetamide;
4-Amino-1-[4-[4-[6-chloro-4-(trifluoromethyl)-2-pyridyl]piperazin-1-yl]sulfonylphenyl]-3,3-dimethyl-pyrrolidin-2-one;
4-(Aminomethyl)-3-[4-[4-[6-chloro-4-(trifluoromethyl)-2-pyridyl]piperazin-1-yl]sulfonylphenyl]oxazolidin-2-one;
(3*S*)-3-amino-1-[4-[4-[6-chloro-4-(trifluoromethyl)-2-pyridyl]piperazin-1-yl]sulfonylphenyl] azetidin-2-one;
3-Amino-1-[4-[4-[6-chloro-4-(trifluoromethyl)-2-pyridyl]piperazin-1-yl]sulfonylphenyl] pyrrolidin-2-one;
5-(2-Aminoethyl)-3-[4-[4-[6-chloro-4-(trifluoromethyl)-2-pyridyl]piperazin-1-yl]sulfonylphenyl]oxazolidin-2-one;
2-[4-[4-[6-Chloro-4-(trifluoromethyl)-2-pyridyl]piperazin-1-yl]sulfonylphenyl]-2,6-diazaspiro[4.5]decan-3-one;
2-[4-[4-[6-Chloro-4-(trifluoromethyl)-2-pyridyl]piperazin-1-yl]sulfonylphenyl]-2,8-diazaspiro[4.5]decan-3-one;
6-[4-[4-[6-Chloro-4-(trifluoromethyl)-2-pyridyl]piperazin-1-yl]sulfonylphenyl]-2,6-diazaspiro[3.4]octan-7-one;
7-[4-[4-[6-Chloro-4-(trifluoromethyl)-2-pyridyl]piperazin-1-yl]sulfonylphenyl]-5-oxa-2,7-diazaspiro[3.4]octan-6-one;
3-[4-[4-[6-Chloro-4-(trifluoromethyl)-2-pyridyl]piperazin-1-yl]sulfonylphenyl]-1-oxa-3,8-diazaspiro[4.5]decan-2-one;
(3a*R*,6a*S*)-3-[4-[4-[6-chloro-4-(trifluoromethyl)-2-pyridyl]piperazin-1-yl]sulfonylphenyl]-4,5,6,6a-tetrahydro-3a*H*-pyrrolo[3,4-d]oxazol-2-one;
(3a*R*,7a*S*)-3-[4-[4-[6-chloro-4-(trifluoromethyl)-2-pyridyl]piperazin-1-yl]sulfonylphenyl]-3a,4,5,6,7,7a-hexahydrooxazolo[4,5-c]pyridin-2-one;
(3a*R*,7a*S*)-1-[4-[4-[6-chloro-4-(trifluoromethyl)-2-pyridyl]piperazin-1-yl]sulfonylphenyl]-3a,4,5,6,7,7a-hexahydrooxazolo[5,4-c]pyridin-2-one;
(3a*S*,7a*R*)-3-[4-[4-[6-chloro-4-(trifluoromethyl)-2-pyridyl]piperazin-1-yl]sulfonylphenyl]-3a,4,5,6,7,7a-hexahydrooxazolo[4,5-c]pyridin-2-one;
(3a*S*,8a*R*)-3-[4-[4-[6-chloro-4-(trifluoromethyl)-2-pyridyl]piperazin-1-yl]sulfonylphenyl]-4,5,6,7,8,8a-hexahydro-3aH-oxazolo[4,5-c]azepin-2-one;
4-[4-[4-[6-Chloro-4-(trifluoromethyl)-2-pyridyl]piperazin-1-yl] sulfonylphenyl]-1,2,3,3 a, 6,6a-hexahydropyrrolo[3,2-b]pyrrol-5-one;
3-[4-[4-[6-Chloro-4-(trifluoromethyl)-2-pyridyl]piperazin-1-yl]sulfonylphenyl]-6-hydroxy-5-(hydroxymethyl)-3a,5,6,6a-tetrahydrofuro[2,3-d]oxazol-2-one;
3-[4-[4-[6-Chloro-4-(trifluoromethyl)-2-pyridyl]piperazin-1-yl] sulfonylphenyl]-5-[(3*S*,4*R*,5*R*)-3,4-dihydroxy-5-methoxy-tetrahydrofuran-2-yl]-4-methyl-oxazolidin-2-one;
(3a*S*,5*S*,6*S*,7*S*,7a*R*)-3-[4-[4-[6-chloro-4-(trifluoromethyl)-2-pyridyl]piperazin-1-yl]sulfonyl phenyl]-5,7-dihydroxy-6-(hydroxymethyl)-5,6,7,7a-tetrahydro-3aH-pyrano[2,3-d]oxazol-2-one;
8-(2-Aminoethyl)-3-[4-[4-[6-chloro-4-(trifluoromethyl)-2-pyridyl]piperazin-1-yl]sulfonylphenyl]-1-oxa-3,8-diazaspiro[4.5]decan-2-one;
(3a*S*,8a*R*)-5-(2-aminoethyl)-3-[4-[4-[6-chloro-4-(trifluoromethyl)-2-pyridyl]piperazin-1-yl]sulfonylphenyl]-3a,4,6,7,8,8a-hexahydrooxazolo[4,5-c]azepin-2-one;
(3a*S*,8a*R*)-3-[4-[4-[6-chloro-4-(trifluoromethyl)-2-pyridyl]piperazin-1-yl]sulfonylphenyl]-5-[2-(methylamino)ethyl]-3a,4,6,7,8,8a-hexahydrooxazolo[4,5-c]azepin-2-one;
3-[4-[4-[6-Chloro-4-(trifluoromethyl)-2-pyridyl]piperazin-1-yl] sulfonylphenyl]-5-(methylaminomethyl)oxazolidin-2-one;
3-[4-[4-[6-Chloro-4-(trifluoromethyl)-2-pyridyl]piperazin-1-yl] sulfonylphenyl]-5-[(dimethylamino)methyl]oxazolidin-2-one;
3-[4-[4-[6-Chloro-4-(trifluoromethyl)-2-pyridyl]piperazin-1-yl] sulfonylphenyl]-5-(piperazin-1-ylmethyl)oxazolidin-2-one;
3-[4-[4-[6-Chloro-4-(trifluoromethyl)-2-pyridyl]piperazin-1-yl]sulfonylphenyl]-5-[[cis-3,4-dihydroxypyrrolidin-1-yl]methyl]oxazolidin-2-one;
5-[(3-Aminoazetidin-1-yl)methyl]-3-[4-[4-[6-chloro-4-(trifluoromethyl)-2-pyridyl]piperazin-1-yl]sulfonylphenyl]oxazolidin-2-one;
3-[4-[4-[6-Chloro-4-(trifluoromethyl)-2-pyridyl]piperazin-1-yl]sulfonylphenyl]-5-(2,6-diazaspiro[3.3]heptan-2-ylmethyl)oxazolidin-2-one;
5-[[3-(Aminomethyl)azetidin-1-yl]methyl]-3-[4-[4-[6-chloro-4-(trifluoromethyl)-2-pyridyl]piperazin-1-yl]sulfonylphenyl]oxazolidin-2-one;
3-[4-[4-[6-Chloro-4-(trifluoromethyl)-2-pyridyl]piperazin-1-yl] sulfonylphenyl]-5-[(pyrrolidin-3-ylamino)methyl]oxazolidin-2-one;
4-[[3-(Aminomethyl)azetidin-1-yl]methyl]-1-[4-[4-[6-chloro-4-(trifluoromethyl)-2-pyridyl]piperazin-1-yl]sulfonylphenyl]pyrrolidin-2-one;
1-[4-[4-[6-Chloro-4-(trifluoromethyl)-2-pyridyl]piperazin-1-yl] sulfonylphenyl]-4-[(4-methylpiperazin-1-yl)methyl]pyrrolidin-2-one;
1-[4-[4-[6-Chloro-4-(trifluoromethyl)-2-pyridyl]piperazin-1-yl]sulfonylphenyl]-4-[[3-[(dimethylamino)methyl]azetidin-1-yl]methyl]pyrrolidin-2-one;
4-[(Azetidin-3-ylmethylamino)methyl]-1-[4-[4-[6-chloro-4-(trifluoromethyl)-2-pyridyl]piperazin-1-yl]sulfonylphenyl]pyrrolidin-2-one;
4-[[3-(1-Aminocyclopropyl)azetidin-1-yl]methyl]-1-[4-[4-[6-chloro-4-(trifluoromethyl)-2-pyridyl]piperazin-1-yl]sulfonylphenyl]pyrrolidin-2-one;
4-[(3-Aminoazetidin-1-yl)methyl]-1-[4-[4-[6-chloro-4-(trifluoromethyl)-2-pyridyl] piperazin-1-yl]sulfonylphenyl]pyrrolidin-2-one;
1-[4-[4-[6-Chloro-4-(trifluoromethyl)-2-pyridyl]piperazin-1-yl] sulfonylphenyl]-4-(piperazin-1-ylmethyl)pyrrolidin-2-one;
1-[4-[4-[6-Chloro-4-(trifluoromethyl)-2-pyridyl]piperazin-1-yl]sulfonylphenyl]-4-[[3-(dimethylamino)azetidin-1-yl]methyl]pyrrolidin-2-one;
1-[4-[4-[6-Chloro-4-(trifluoromethyl)-2-pyridyl]piperazin-1-yl]sulfonylphenyl]-4-[[3-(methylamino)azetidin-1-yl]methyl]pyrrolidin-2-one;
1-[4-[4-[6-chloro-4-(trifluoromethyl)-2-pyridyl]piperazin-1-yl]sulfonylphenyl]-4-(methylamino)pyrrolidin-2-one;
4-(2-Aminoethylamino)-1-[4-[4-[6-chloro-4-(trifluoromethyl)-2-pyridyl]piperazin-1-yl]sulfonylphenyl]pyrrolidin-2-one;
1-[4-[4-[6-Chloro-4-(trifluoromethyl)-2-pyridyl]piperazin-1-yl] sulfonylphenyl]-4-(2-hydroxyethylamino)pyrrolidin-2-one;
1-[4-[4-[6-Chloro-4-(trifluoromethyl)-2-pyridyl]piperazin-1-yl] sulfonylphenyl]-4-piperazin-1-yl-pyrrolidin-2-one;
1-[4-[4-[6-Chloro-4-(trifluoromethyl)-2-pyridyl]piperazin-1-yl] sulfonylphenyl]-4-[**(3S,4R)**-3,4-dihydroxypyrrolidin-1-yl]pyrrolidin-2-one;
4-[3-(Aminomethyl)azetidin-1-yl]-1-[4-[4-[6-chloro-4-(trifluoromethyl)-2-pyridyl]piperazin-1-yl]sulfonylphenyl]pyrrolidin-2-one;
4-(3-Aminopyrrolidin-1-yl)-1-[4-[4-[6-chloro-4-(trifluoromethyl)-2-pyridyl]piperazin-1-yl]sulfonylphenyl]pyrrolidin-2-one;
4-(3-Aminoazetidin-1-yl)-1-[4-[4-[6-chloro-4-(trifluoromethyl)-2-pyridyl]piperazin-1-yl]sulfonylphenyl]pyrrolidin-2-one;
1-[4-[4-[6-Chloro-4-(trifluoromethyl)-2-pyridyl]piperazin-1-yl]sulfonylphenyl]-4-(2,6-diazaspiro[3.3]heptan-2-yl)pyrrolidin-2-one;
1-[4-[4-[6-Chloro-4-(trifluoromethyl)-2-pyridyl]piperazin-1-yl]sulfonylphenyl]-4-[(3*S*)-3-(hydroxymethyl)piperazin-1-yl]pyrrolidin-2-one;
1-[4-[4-[6-Chloro-4-(trifluoromethyl)-2-pyridyl]piperazin-1-yl] sulfonylphenyl]-4-[(3R)-3-(hydroxymethyl)piperazin-1-yl]pyrrolidin-2-one;
1-[4-[4-[6-Chloro-4-(trifluoromethyl)-2-pyridyl]piperazin-1-yl]sulfonylphenyl]-4-[(1*S*,4*S*)-2,5-diazabicyclo[2.2.1]heptan-2-yl]pyrrolidin-2-one;
4-[(3*R*,4*R*)-3-amino-4-hydroxy-pyrrolidin-1-yl]-1-[4-[4-[6-chloro-4-(trifluoromethyl)-2-pyridyl]piperazin-1-yl]sulfonylphenyl]pyrrolidin-2-one;
4-((3*R*,4*R*)-3-amino-4-hydroxypiperidin-1-yl)-1-(4-((4-(6-chloro-4-(trifluoromethyl)pyridin-2-yl)piperazin-1-yl)sulfonyl)phenyl)pyrrolidin-2-one;
4-(Azetidin-3-ylamino)-1-[4-[4-[6-chloro-4-(trifluoromethyl)-2-pyridyl]piperazin-1-yl]sulfonylphenyl]pyrrolidin-2-one;
1-[4-[4-[6-Chloro-4-(trifluoromethyl)-2-pyridyl]piperazin-1-yl]sulfonylphenyl]-4-(pyrrolidin-3-ylamino)pyrrolidin-2-one;
1-[4-[4-[6-Chloro-4-(trifluoromethyl)-2-pyridyl]piperazin-1-yl]sulfonylphenyl]-4-(4-piperidylamino)pyrrolidin-2-one;
4-Amino-1-[4-[4-[6-chloro-4-(trifluoromethyl)-2-pyridyl]piperazin-1-yl]sulfonylphenyl]-5-methyl-pyrrolidin-2-one;
4-Amino-1-[5-[4-[6-chloro-4-(trifluoromethyl)-2-pyridyl]piperazin-1-yl]sulfonyl-2-pyridyl]pyrrolidin-2-one;
5-(Aminomethyl)-3-[4-[4-[6-chloro-4-(trifluoromethyl)-2-pyridyl]piperazin-1-yl]sulfonyl-2-fluoro-phenyl]oxazolidin-2-one;
5-(Aminomethyl)-3-[4-[4-[6-chloro-4-(trifluoromethyl)-2-pyridyl]piperazin-1-yl]sulfonyl-3-fluoro-phenyl]oxazolidin-2-one;
4-Amino-1-[4-[4-[4-methyl-6-(trifluoromethyl)pyrimidin-2-yl]piperazin-1-yl]sulfonylphenyl]pyrrolidin-2-one;
4-Amino-1-[4-[4-[4-(trideuteriomethyl)-6-(trifluoromethyl)pyrimidin-2-yl]piperazin-1-yl]sulfonylphenyl]pyrrolidin-2-one;
1-[4-(4-Amino-2-oxo-pyrrolidin-1-yl)phenyl] sulfonyl-4-[6-chloro-4-(trifluoromethyl)-2-pyridyl]piperazine-2-carbonitrile;
4-Amino-1-[4-[4-[6-chloro-4-(difluoromethyl)-2-pyridyl]piperazin-1-yl] sulfonylphenyl] pyrrolidin-2-one;
4-Amino-1-[4-[4-(6-chloro-4-cyclopropylsulfonyl-2-pyridyl)piperazin-1-yl] sulfonylphenyl] pyrrolidin-2-one;
4-Amino-1-[4-[4-(6-chloro-4-tetrahydropyran-4-ylsulfonyl-2-pyridyl)piperazin-1-yl]sulfonylphenyl]pyrrolidin-2-one;
1-[2-[4-[4-(4-Amino-2-oxo-pyrrolidin-1-yl)phenyl] sulfonylpiperazin-1-yl]-6-chloro-4-pyridyl]cyclopropanecarbonitrile;
4-Amino-1-[4-[4-(6-chloro-4-phenoxy-2-pyridyl)piperazin-1-yl]sulfonylphenyl]pyrrolidin-2-one;
4-Amino-1-[4-[4-[6-chloro-4-[difluoro(phenyl)methyl]-2-pyridyl]piperazin-1-yl]sulfonylphenyl] pyrrolidin-2-one;
4-Amino-1-[4-[4-(4-benzyl-6-chloro-2-pyridyl)piperazin-1-yl] sulfonylphenyl]pyrrolidin-2-one;
4-Amino-1-[4-[4-[6-chloro-4-(1-phenylcyclopropyl)-2-pyridyl]piperazin-1-yl]sulfonylphenyl] pyrrolidin-2-one;
4-Amino-1-[4-[4-[4-[difluoro(phenyl)methyl]-6-methyl-pyrimidin-2-yl]piperazin-1-yl]sulfonylphenyl]pyrrolidin-2-one;
4-Amino-1-[4-[4-[2-chloro-6-[difluoro(phenyl)methyl]pyrimidin-4-yl]piperazin-1-yl]sulfonylphenyl]pyrrolidin-2-one;
4-Amino-1-[4-[4-[4-chloro-6-[difluoro(phenyl)methyl]pyrimidin-2-yl]piperazin-1-yl]sulfonylphenyl]pyrrolidin-2-one;
4-Amino-1-[4-[4-[2-cyclopropyl-6-[difluoro(phenyl)methyl]pyrimidin-4-yl]piperazin-1-yl]sulfonylphenyl]pyrrolidin-2-one;
4-Amino-1-[4-[4-[4-cyclopropyl-6-[difluoro(phenyl)methyl]pyrimidin-2-yl]piperazin-1-yl]sulfonylphenyl]pyrrolidin-2-one;
4-Amino-1-[4-[4-[6-[difluoro(phenyl)methyl]-2-methoxy-pyrimidin-4-yl]piperazin-1-yl]sulfonylphenyl]pyrrolidin-2-one;
4-Amino-1-[4-[4-[4-[difluoro(phenyl)methyl]-6-methoxy-pyrimidin-2-yl]piperazin-1-yl]sulfonylphenyl]pyrrolidin-2-one;
(4*R*)-4-Amino-1-[4-[4-[6-[difluoro(phenyl)methyl]-2-methyl-pyrimidin-4-yl]piperazin-1-yl]sulfonylphenyl]pyrrolidin-2-one;
4-Amino-1-[4-[4-[6-[difluoro(phenyl)methyl]-2-vinyl-pyrimidin-4-yl]piperazin-1-yl]sulfonylphenyl]pyrrolidin-2-one;
2-[4-[4-[6-Chloro-4-[difluoro(phenyl)methyl]-2-pyridyl]piperazin-1-yl]sulfonylphenyl]-2,6,9-triazaspiro[4.5]decan-3-one;
5-(Aminomethyl)-1-[4-[4-[6-chloro-4-[difluoro(phenyl)methyl]-2-pyridyl]piperazin-1-yl]sulfonylphenyl]pyrrolidin-2-one;
(3a*R*,7a*S*)-3-[4-[4-[6-chloro-4-[difluoro(phenyl)methyl]-2-pyridyl]piperazin-1-yl]sulfonyl phenyl]-3a,4,5,6,7,7a-hexahydrooxazolo[4,5-c]pyridin-2-one;
5-(Aminomethyl)-3-[4-[4-[6-chloro-4-[difluoro(phenyl)methyl]-2-pyridyl]piperazin-1-yl]sulfonylphenyl]oxazolidin-2-one;
4-Amino-1-[4-[4-[6-chloro-4-[difluoro(2-pyridyl)methyl]-2-pyridyl]piperazin-1-yl] sulfonylphenyl] pyrrolidin-2-one;
4-Amino-1-[4-[4-[6-chloro-4-[difluoro(3-pyridyl)methyl]-2-pyridyl]piperazin-1-yl]sulfonylphenyl] pyrrolidin-2-one;
4-Amino-1-[4-[4-[6-chloro-4-[difluoro(4-pyridyl)methyl]-2-pyridyl]piperazin-1-yl] sulfonylphenyl]pyrrolidin-2-one;
4-Amino-1-[4-[4-[6-chloro-4-[difluoro(pyrimidin-5-yl)methyl]-2-pyridyl]piperazin-1-yl]sulfonylphenyl]pyrrolidin-2-one;
4-Amino-1-[4-[4-[6-chloro-4-[difluoro(pyrazin-2-yl)methyl]-2-pyridyl]piperazin-1-yl]sulfonylphenyl]pyrrolidin-2-one;
4-Amino-1-[4-[4-[6-chloro-4-[difluoro(pyrimidin-2-yl)methyl]-2-pyridyl]piperazin-1-yl]sulfonylphenyl]pyrrolidin-2-one;
4-Amino-1-[4-[4-[6-chloro-4-[difluoro(tetrahydropyran-4-yl)methyl]-2-pyridyl]piperazin-1-yl]sulfonylphenyl]pyrrolidin-2-one;
4-Amino-1-[4-[4-[6-chloro-4-[difluoro(tetrahydrofuran-3-yl)methyl]-2-pyridyl]piperazin-1-yl]sulfonylphenyl]pyrrolidin-2-one;
4-Amino-1-[4-[4-[6-chloro-4-[difluoro(oxetan-3-yl)methyl]-2-pyridyl]piperazin-1-yl]sulfonylphenyl] pyrrolidin-2-one;
4-Amino-1-[4-[4-[6-chloro-4-[3,6-dihydro-2H-pyran-4-yl(difluoro)methyl]-2-pyridyl] piperazin-1-yl]sulfonylphenyl]pyrrolidin-2-one;
4-Amino-1-[4-[4-[6-chloro-4-[difluoro(tetrahydropyran-3-yl)methyl]-2-pyridyl]piperazin-1-yl]sulfonylphenyl]pyrrolidin-2-one;
4-Amino-1-[4-[4-[6-chloro-4-[difluoro(8-oxabicyclo[3.2.1]octan-3-yl)methyl]-2-pyridyl] piperazin-1-yl]sulfonylphenyl]pyrrolidin-2-one;
(4*R*)-4-amino-1-[4-[4-[6-chloro-4-[difluoro(3-oxabicyclo[3.1.0]hexan-6-yl)methyl]-2-pyridyl]piperazin-1-yl]sulfonylphenyl]pyrrolidin-2-one;
(4*R*)-4-amino-1-[4-[4-[6-chloro-4-[[(2*S*)-1,4-dioxan-2-yl]-difluoro-methyl]-2-pyridyl] piperazin-1-yl]sulfonylphenyl]pyrrolidin-2-one;
(4*R*)-4-amino-1-[4-[4-[6-chloro-4-[[(2*R*)-1,4-dioxan-2-yl]-difluoro-methyl]-2-pyridyl] piperazin-1-yl]sulfonylphenyl]pyrrolidin-2-one;
(4*R*)-4-amino-1-[4-[4-[6-chloro-4-[difluoro-[(2*S*)-morpholin-2-yl]methyl]-2-pyridyl] piperazin-1-yl]sulfonylphenyl]pyrrolidin-2-one;
(4*R*)-4-amino-1-[4-[4-[6-chloro-4-[difluoro-[(2*R*)-morpholin-2-yl]methyl]-2-pyridyl] piperazin-1-yl]sulfonylphenyl]pyrrolidin-2-one;
(4*R*)-4-amino-1-[4-[4-[6-chloro-4-[difluoro(1,2,3,6-tetrahydropyridin-4-yl)methyl]-2-pyridyl]piperazin-1-yl]sulfonylphenyl]pyrrolidin-2-one;
(4*R*)-4-amino-1-[4-[4-[6-chloro-4-[difluoro-[(3*R*)-3-piperidyl]methyl]-2-pyridyl]piperazin-1-yl]sulfonylphenyl]pyrrolidin-2-one;
(4*R*)-4-amino-1-[4-[4-[6-chloro-4-[difluoro-[(3*S*)-3-piperidyl]methyl]-2-pyridyl]piperazin-1-yl]sulfonylphenyl]pyrrolidin-2-one;
(4*R*)-4-amino-1-[4-[4-[4-[azetidin-3-yl(difluoro)methyl]-6-chloro-2-pyridyl]piperazin-1-yl]sulfonylphenyl]pyrrolidin-2-one;
3-[4-[4-[6-Chloro-4-[difluoro-[(2*S*)-morpholin-2-yl]methyl]-2-pyridyl]piperazin-1-yl]sulfonylphenyl] -5-(hydroxymethyl)oxazolidin-2-one;
(4*R*)-4-amino-1-[4-[4-[6-chloro-4-[difluoro-(5-methylpyrazin-2-yl)methyl]-2-pyridyl]piperazin-1-yl]sulfonylphenyl]pyrrolidin-2-one;
(4*R*)-4-amino-1-[4-[4-[6-chloro-4-[difluoro-(6-methylpyrazin-2-yl)methyl]-2-pyridyl]piperazin-1-yl]sulfonylphenyl]pyrrolidin-2-one;
(4*R*)-4-amino-1-[4-[4-[6-chloro-4-[(5-chloro-6-methyl-pyrazin-2-yl)-difluoro-methyl]-2-pyridyl]piperazin-1-yl]sulfonylphenyl]pyrrolidin-2-one;
(4*R*)-4-amino-1-[4-[4-[6-chloro-4-[difluoro-[cis-6-methylmorpholin-2-yl]methyl]-2-pyridyl]piperazin-1-yl]sulfonylphenyl]pyrrolidin-2-one;
((4*R*)-4-amino-1-[4-[4-[6-chloro-4-[difluoro-(5-methylmorpholin-2-yl)methyl]-2-pyridyl]piperazin-1-yl]sulfonylphenyl]pyrrolidin-2-one;
(4*R*)-4-amino-1-[4-[4-[4-[[*cis*-3-azabicyclo[3.1.0]hexan-1-yl]-difluoro-methyl]-6-chloro-2-pyridyl]piperazin-1-yl]sulfonylphenyl]pyrrolidin-2-one;
(4R)-4-amino-1-[4-[4-[6-chloro-4-[*trans*-difluoro-[2-(hydroxymethyl)cyclopropyl]methyl]-2-pyridyl]piperazin-1-yl]sulfonylphenyl]pyrrolidin-2-one;
(4*R*)-4-amino-1-[4-[4-[4-[[*trans*-4-(aminomethyl)cyclohexyl]-difluoro-methyl]-6-chloro-2-pyridyl]piperazin-1-yl]sulfonylphenyl]pyrrolidin-2-one;
(4*R*)-4-amino-1-[4-[4-[6-chloro-4-[*trans*-difluoro-(3-hydroxycyclobutyl)methyl]-2-pyridyl]piperazin-1-yl]sulfonylphenyl]pyrrolidin-2-one; and
*Trans*-4-[[2-[4-[4-[(4R)-4-amino-2-oxo-pyrrolidin-1-yl]phenyl]sulfonylpiperazin-1-yl]-6-chloro-4-pyridyl]-difluoro-methyl]cyclohexanecarboxamide; or a pharmaceutically acceptable salt thereof.

12. A process for the preparation of a compound according to any one of claims 1 to 11 comprising the following step:
a) Coupling reaction between compound of formula (VII), 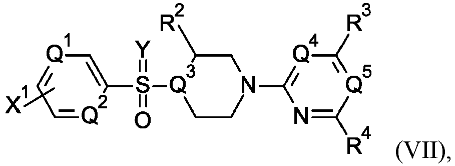 with lactam or carbamate, R¹H, in the presence of catalyst and base;
wherein
in step a), the catalyst is CuI, the base is Cs₂CO₃;
R¹, R², R³, R⁴, Y, Q¹, Q², Q³, Q⁴ and Q⁵ are defined as in any one of claims 1 to 10.

13. A compound or pharmaceutically acceptable salt according to any one of claims 1 to 11 for use as therapeutically active substance.

14. A pharmaceutical composition comprising a compound in accordance with any one of claims 1 to 11 and a therapeutically inert carrier.

15. A compound or pharmaceutically acceptable salt according to any one of claims 1 to 11 for use in the treatment or prophylaxis of bacterial infection, particularly the bacteria is gram-negative bacteria, more particularly the gram-negative bacteria is selected from *Enterobacteriaceae, Neisseria gonorrhoeae, Haemophilus influenzae, Helicobacter pylorus, Acinetobacter baumannii* and *Pseudomonas aeruginosa.*

## Patentansprüche

1. Verbindung der Formel (I), 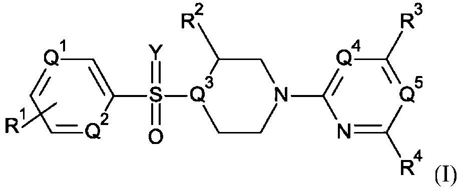 wobei
R¹ 2-Oxo-1-oxa-3,8-diazaspiro[4.5]decanyl, unsubstituiert oder substituiert mit Amino-C₁₋₆-alkyl,
2-Oxo-3a,4,5,6,7,7a-hexahydrooxazolo[4,5-c]pyridinyl,
2-Oxo-3a,4,5,6,7,7a-hexahydrooxazolo[5,4-c]pyridinyl,
2-Oxo-3a,4,6,7,8,8a-hexahydrooxazolo[4,5-c]azepinyl, substituiert mit Amino-C₁₋₆-alkyl oder C₁₋₆-Alkylamino-C₁₋₆-alkyl,
2-Oxo-3a,5,6,6a-tetrahydrofuro[2,3-d]oxazolyl, zweifach substituiert mit Substituenten, unabhängig ausgewählt aus Hydroxy und Hydroxy-C₁₋₆-alkyl,
2-Oxo-4,5,6,6a-tetrahydro-3aH-pyrrolo[3,4-d]oxazolyl,
2-Oxo-4,5,6,7,8,8a-hexahydro-3aH-oxazolo[4,5-c]azepinyl,
2-Oxo-5,6,7,7a-tetrahydro-3aH-pyrano[2,3-d]oxazolyl, dreifach substituiert mit Substituenten, unabhängig ausgewählt aus Hydroxy und Hydroxy-C₁₋₆-alkyl,
3-Oxo-2,6,9-triazaspiro[4.5]decanyl,
3-Oxo-2,6-diazaspiro[4.5]decanyl,
3-Oxo-2,8-diazaspiro[4.5]decanyl,
5-Oxo-1,2,3,3a,6,6a-hexahydropyrrolo[3,2-b]pyrrolyl,
6-Oxo-5-oxa-2,7-diazaspiro[3.4]octanyl,
7-Oxo-2,6-diazaspiro[3.4]octanyl,
Oxoazetidinyl, substituiert mit Amino,
Oxooxazolidinyl, ein- oder zweifach substituiert mit Substituenten, unabhängig voneinander ausgewählt aus ((Amino-C₁₋₆-alkyl)azetidinyl)-C₁₋₆-alkyl, (C₁₋₆-Alkyl)₂-amino-C1₋₆-alkyl, (Dihydroxypyrrolidinyl)-C₁₋₆-alkyl, (pyrrolidinylamino)-C₁₋₆-alkyl, 2,6-Diazaspiro[3.3]heptanyl-C₁₋₆-alkyl, Dihydroxy-(C₁₋₆-alkoxy)tetrahydrofuranyl, Aminoazetidinyl-C₁₋₆-alkyl, Amino-C₁₋₆-alkyl, C₁₋₆-Alkyl, C₁₋₆-Alkylamino-C₁₋₆-alkyl, C₁₋₆-Alkylcarbonylamino-C₁₋₆-alkyl, Hydroxy-C₁₋₆-alkyl und Piperazinyl-C₁₋₆-alkyl oder
Oxopyrrolidinyl ist, einfach, zweifach oder dreifach mit Substituenten substituiert, unabhängig voneinander ausgewählt aus (((C₁₋₆-Alkyl)₂ -amino-C₁₋₆-alkyl)azetidinyl)-C₁₋₆-alkyl, ((Amino-C₁₋₆-alkyl)azetidinyl)-C₁₋₆-alkyl, ((Amino-C₃₋₇-cycloalkyl)azetidinyl)-C₁₋₆-alkyl, ((C₁₋₆-Alkylamino)azetidinyl)-C₁₋₆-alkyl, (Aminoazetidinyl)-C₁₋₆-alkyl, (Amino-C₁₋₆-alkyl)azetidinyl, (Azetidinyl-C₁₋₆-alkylamino)-C₁₋₆-alkyl, (C₁₋₆-Alkylpiperazinyl)-C₁₋₆-alkyl, (Hydroxy-C₁₋₆-alkyl)piperazinyl, 2,5-Diazabicyclo[2.2.1]heptanyl, 2,6-Diazaspiro[3.3]heptanyl, Amino, Amino(hydroxy)piperidinyl, Amino(hydroxy)pyrrolidinyl, Aminoazetidinyl, Amino-C₁₋₆-alkyl, (Amino-C₁₋₆-alkyl)amino, Aminopyrrolidinyl, Azetidinylamino, C₁₋₆-Alkyl, C₁₋₆-Alkylamino, Dihydroxypyrrolidinyl, (Hydroxy-C₁₋₆-alkyl)amino, Piperazinyl, Piperazinyl-C₁₋₆-alkyl, Piperidinylamino und Pyrrolidinylamino;
R² H oder Cyano ist;
R³ Benzyl,
C₃₋₇-Cycloalkylsulfonyl,
Cyano-C₃₋₇-cycloalkyl,
Phenoxy,
Phenyl-C₃₋₇-cycloalkyl,
Tetrahydropyranylsulfonyl oder
C₁₋₆-Alkyl ist, zweifach oder dreifach mit Substituenten substituiert, unabhängig voneinander ausgewählt aus Halogen, (Amino-C₁₋₆-alkyl)-C₃₋₇-cycloalkyl, (Hydroxy-C₁₋₆-alkyl)-C₃₋₇-cycloalkyl, 1,2,3,6-Tetrahydropyridinyl, 1,4-Dioxanyl, 3,6-Dihydro-2H-pyranyl, 3-Azabicyclo[3.1.0]hexanyl, 3-Oxabicyclo[3.1.0]hexanyl, 8-Oxabicyclo[3.2.1]octanyl, Azetidinyl, C₁₋₆-Alkylhalogenpyrazinyl, C₁₋₆-Alkylmorpholinyl, C₁₋₆-Alkylpyrazinyl, Carbamoyl-C₃₋₇-cycloalkyl, Hydroxy-C₃₋₇-cycloalkyl, Morpholinyl, Oxetanyl, Piperidinyl, Pyrazinyl, Pyridinyl, Pyrimidinyl, Tetrahydrofuranyl, Tetrahydropyranyl, Phenyl und Pyridinyl;
R⁴ Halogen, C₁₋₆-Alkyl, C₃₋₇-Cycloalkyl, C₁₋₆-Alkoxy oder C₂₋₆-Alkinyl ist;
Q¹ N oder CR^{a} ist, wobei R^{a} H oder Halogen ist;
Q² N oder CR^{b} ist, wobei R^{b} H oder Halogen ist;
Q³ N oder CH ist;
Q⁴ N oder CH ist;
Q⁵ N oder CH ist;
Y O oder NH ist;
oder ein pharmazeutisch verträgliches Salz davon.

2. Verbindung nach Anspruch 1 der Formel (Ia), 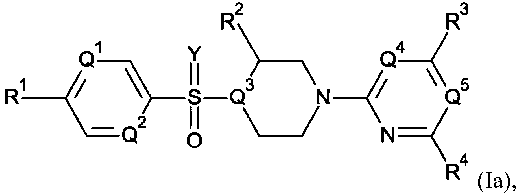 wobei
R¹ 2-Oxo-1-oxa-3,8-diazaspiro[4.5]decanyl, unsubstituiert oder substituiert mit Amino-C₁₋₆-alkyl,
2-Oxo-3a,4,5,6,7,7a-hexahydrooxazolo[4,5-c]pyridinyl,
2-Oxo-3a,4,5,6,7,7a-hexahydrooxazolo[5,4-c]pyridinyl,
2-Oxo-3a,4,6,7,8,8a-hexahydrooxazolo[4,5-c]azepinyl, substituiert mit Amino-C₁₋₆-alkyl oder C₁₋₆-Alkylamino-C₁₋₆-alkyl,
2-Oxo-3a,5,6,6a-tetrahydrofuro[2,3-d]oxazolyl, zweifach substituiert mit Substituenten, unabhängig ausgewählt aus Hydroxy und Hydroxy-C₁₋₆-alkyl,
2-Oxo-4,5,6,6a-tetrahydro-3aH-pyrrolo[3,4-d]oxazolyl,
2-Oxo-4,5,6,7,8,8a-hexahydro-3aH-oxazolo[4,5-c]azepinyl,
2-Oxo-5,6,7,7a-tetrahydro-3aH-pyrano[2,3-d]oxazolyl, dreifach substituiert mit Substituenten, unabhängig voneinander ausgewählt aus Hydroxy und Hydroxy-C₁₋₆-alkyl,
3-Oxo-2,6,9-triazaspiro[4.5]decanyl,
3-Oxo-2,6-diazaspiro[4.5]decanyl,
3-Oxo-2,8-diazaspiro[4.5]decanyl,
5-Oxo-1,2,3,3a,6,6a-hexahydropyrrolo[3,2-b]pyrrolyl,
6-Oxo-5-oxa-2,7-diazaspiro[3.4]octanyl,
7-Oxo-2,6-diazaspiro[3.4]octanyl,
Oxoazetidinyl, substituiert mit Amino,
Oxooxazolidinyl, ein- oder zweifach mit Substituenten substituiert, unabhängig voneinander ausgewählt aus ((Amino-C₁₋₆-alkyl)azetidinyl)-C₁₋₆-alkyl, (C₁₋₆-Alkyl)₂-amino-C₁₋₆-alkyl, (Dihydroxypyrrolidinyl)-C₁₋₆-alkyl, (Pyrrolidinylamino)-C₁₋₆-alkyl, 2,6-Diazaspiro[3.3]heptanyl-C₁₋₆-alkyl, Dihydroxy-(C₁₋₆-alkoxy)tetrahydrofuranyl,
Aminoazetidinyl-C₁₋₆-alkyl, Amino-C₁₋₆-alkyl, C₁₋₆-Alkyl, C₁₋₆-Alkylamino-C₁₋₆-alkyl, C₁₋₆-Alkylcarbonylamino-C₁₋₆-alkyl, Hydroxy-C₁₋₆-alkyl und Piperazinyl-C₁₋₆-alkyl, oder
Oxopyrrolidinyl ist, einfach, zweifach oder dreifach mit Substituenten substituiert, unabhängig voneinander ausgewählt aus (((C₁₋₆-Alkyl)₂-amino-C₁₋₆-alkyl)azetidinyl)-C₁₋₆-alkyl, ((Amino-C₁₋₆-alkyl)azetidinyl)-C₁₋₆-alkyl, ((Amino-C₃₋₇-cycloalkyl)azetidinyl)-C₁₋₆-alkyl, ((C₁₋₆-Alkylamino)azetidinyl)-C₁₋₆-alkyl, (Aminoazetidinyl)-C₁₋₆-alkyl, (Amino-C₁₋₆-alkyl)azetidinyl, (Azetidinyl-C₁₋₆-alkylamino)-C₁₋₆-alkyl, (C₁₋₆-Alkylpiperazinyl)-C₁₋₆-alkyl, (Hydroxy-C₁₋₆-alkyl)piperazinyl, 2,5-Diazabicyclo[2.2.1]heptanyl, 2,6-Diazaspiro[3.3]heptanyl, Amino, Amino(hydroxy)piperidinyl, Amino(hydroxy)pyrrolidinyl, Aminoazetidinyl, Amino-C₁₋₆-alkyl, (Amino-C₁₋₆-alkyl)amino, Aminopyrrolidinyl, Azetidinylamino, C₁₋₆-Alkyl, C₁₋₆-Alkylamino, Dihydroxypyrrolidinyl, (Hydroxy-C₁₋₆-alkyl)amino, Piperazinyl, Piperazinyl-C₁₋₆-alkyl, Piperidinylamino und Pyrrolidinylamino;
R² H oder Cyano ist;
R³ Benzyl,
C₃₋₇-Cycloalkylsulfonyl,
Cyano-C₃₋₇-cycloalkyl,
Phenoxy,
Phenyl-C₃₋₇-cycloalkyl,
Tetrahydropyranylsulfonyl oder
C₁₋₆-Alkyl ist, zweifach oder dreifach substituiert mit Substituenten, unabhängig voneinander ausgewählt aus Halogen, (Amino-C₁₋₆-alkyl)-C₃₋₇-cycloalkyl, (Hydroxy-C₁₋₆-alkyl)-C₃₋₇-cycloalkyl, 1,2,3,6-Tetrahydropyridinyl, 1,4-Dioxanyl, 3,6-Dihydro-2H-pyranyl, 3-Azabicyclo[3.1.0]hexanyl, 3-Oxabicyclo[3.1.0]hexanyl, 8-Oxabicyclo[3.2.1]octanyl, Azetidinyl, C₁₋₆-Alkylhalogenpyrazinyl, C₁₋₆-Alkylmorpholinyl, C₁₋₆-Alkylpyrazinyl, Carbamoyl-C₃₋₇-cycloalkyl, Hydroxy-C₃₋₇-cycloalkyl, Morpholinyl, Oxetanyl, Piperidinyl, Pyrazinyl, Pyridinyl, Pyrimidinyl, Tetrahydrofuranyl, Tetrahydropyranyl, Phenyl und Pyridinyl;
R⁴ Halogen, C₁₋₆-Alkyl, C₃₋₇-Cycloalkyl, C₁₋₆-Alkoxy oder C₂₋₆-Alkinyl ist;
Q¹ N oder CR^{a} ist, wobei R^{a} H oder Halogen ist;
Q² CR^{b} ist, wobei R^{b} H oder Halogen ist;
Q³ N ist;
Q⁴ N oder CH ist;
Q⁵ N oder CH ist;
Y O ist;
oder ein pharmazeutisch verträgliches Salz davon.

3. Verbindung nach Anspruch 1 oder 2, wobei
R¹ 2-Oxo-1-oxa-3,8-diazaspiro[4.5]decanyl,
2-Oxo-3a,4,5,6,7,7a-hexahydrooxazolo[4,5-c]pyridinyl,
2-Oxo-3a,4,5,6,7,7a-hexahydrooxazolo[5,4-c]pyridinyl,
2-Oxo-3a,5,6,6a-tetrahydrofuro[2,3-d]oxazolyl, zweifach substituiert mit Substituenten, unabhängig voneinander ausgewählt aus Hydroxy und Hydroxy-C₁₋₆-alkyl,
2-Oxo-4,5,6,6a-tetrahydro-3aH-pyrrolo[3,4-d]oxazolyl,
2-Oxo-4,5,6,7,8,8a-hexahydro-3aH-oxazolo[4,5-c]azepinyl,
3-Oxo-2,6-diazaspiro[4.5]decanyl,
5-Oxo-1,2,3,3a,6,6a-hexahydropyrrolo[3,2-b]pyrrolyl,
6-Oxo-5-oxa-2,7-diazaspiro[3.4]octanyl,
Oxooxazolidinyl, einfach oder zweifach substituiert mit Substituenten, unabhängig voneinander ausgewählt aus ((Amino-C₁₋₆-alkyl)azetidinyl)-C₁₋₆-alkyl, (C₁₋₆-Alkyl)₂-amino-C₁₋₆-alkyl, Dihydroxy-(C₁₋₆-alkoxy)tetrahydrofuranyl, Amino-C₁₋₆-alkyl, C₁₋₆-Alkyl, C₁₋₆-Alkylamino-C₁₋₆-alkyl, Hydroxy-C₁₋₆-alkyl und Piperazinyl-C₁₋₆-alkyl oder
Oxopyrrolidinyl ist, substituiert mit einem Substituenten, ausgewählt aus ((Amino-C₁₋₆-alkyl)azetidinyl)-C₁₋₆-alkyl, ((Amino-C₃₋₇-cycloalkyl)azetidinyl)-C₁₋₆-alkyl, (Aminoazetidinyl)-C₁₋₆-alkyl, (C₁₋₆-Alkylpiperazinyl)-C₁₋₆-alkyl, (Hydroxy-C₁₋₆-alkyl)piperazinyl, Amino, Amino(hydroxy)piperidinyl, Amino(hydroxy)pyrrolidinyl, Amino-C₁₋₆-alkyl, Aminopyrrolidinyl, C₁₋₆-Alkylamino, Dihydroxypyrrolidinyl, (Hydroxy-C₁₋₆-alkyl)amino, Piperazinyl, Piperazinyl-C₁₋₆-alkyl und Piperidinylamino.

4. Verbindung nach einem der Ansprüche 1 bis 3, wobei R³ Benzyl, Phenyl-C₃₋₇-cycloalkyl oder C₁₋₆-Alkyl ist, dreifach substituiert mit Substituenten, unabhängig voneinander ausgewählt aus Halogen, (Hydroxy-C₁₋₆-alkyl)-C₃₋₇-cycloalkyl, 1,2,3,6-Tetrahydropyridinyl, 1,4-Dioxanyl, 3,6-Dihydro-2H-pyranyl, 3-Azabicyclo[3.1.0]hexanyl, 3-Oxabicyclo[3.1.0]hexanyl,
8-Oxabicyclo[3.2.1]octanyl, C₁₋₆Alkylhalogenpyrazinyl, C₁₋₆Alkylpyrazinyl, CarbamoylC₃₋₇Cycloalkyl, HydroxyC₃₋₇Cycloalkyl, Morpholinyl, Oxetanyl, Pyrazinyl, Pyridinyl, Tetrahydropyranyl und Phenyl.

5. Verbindung nach einem der Ansprüche 1 bis 4, wobei R² H ist.

6. Verbindung nach einem der Ansprüche 1 bis 5, wobei R⁴ Halogen oder C₁₋₆-Alkyl ist, insbesondere wobei R⁴ Chlor oder Methyl ist.

7. Verbindung nach einem der Ansprüche 1 bis 6, wobei Q¹ CR^{a} ist, wobei R^{a} H oder Halogen ist, insbesondere wobei Q¹ CR¹ ist, wobei R¹ H oder Fluor ist.

8. Verbindung nach einem der Ansprüche 1 bis 7, wobei Q² CH ist.

9. Verbindung nach Anspruch 1 oder 2, wobei
R¹ Oxopyrrolidinyl ist, substituiert mit Amino oder Amino-C₁₋₆-alkyl;
R² H ist;
R³ C₁₋₆-Alkyl ist, das dreifach mit Substituenten substituiert ist, unabhängig voneinander ausgewählt aus Halogen, (Hydroxy-C₁₋₆-alkyl)-C₃₋₇-cycloalkyl, 1,4-Dioxanyl, 3-Azabicyclo[3.1.0]hexanyl, 3-Oxabicyclo[3.1.0]hexanyl, C₁₋₆-Alkylhalogenpyrazinyl, C₁₋₆-Alkylpyrazinyl, Hydroxy-C₃₋₇-cycloalkyl, Morpholinyl, Oxetanyl, Phenyl, Pyridinyl und Tetrahydropyranyl;
R⁴ Halogen oder C₁₋₆-Alkyl ist;
Q¹ CR^{a} ist, wobei R^{a} H oder Halogen ist;
Q² CH ist;
Q³ N ist;
Q⁴ N oder CH ist;
Q⁵ N oder CH ist;
Y O ist;
oder ein pharmazeutisch verträgliches Salz davon.

10. Verbindung nach Anspruch 9, wobei
R¹ Oxopyrrolidinyl ist, substituiert mit Amino oder Aminomethyl;
R² H ist;
R³ Difluor(1,4-dioxan-2-yl)methyl, Difluor(2-(hydroxymethyl)cyclopropyl)methyl, Difluor(2-pyridinyl)methyl, Difluor(3-hydroxycyclobutyl)methyl, Difluor(3-oxabicyclo[3.1.0]hexan-6-yl)methyl, Difluor(5-chlor-6-methylpyrazin-2-yl)methyl, Difluor(6-methylpyrazin-2-yl)methyl, Difluor(morpholin-2-yl)methyl, Difluor(oxetan-3-yl)methyl, Difluor(phenyl)methyl, Difluor(tetrahydropyran-4-yl)methyl oder Difluor[3-azabicyclo[3.1.0]hexan-1-yl]methyl ist;
R⁴ Chlor oder Methyl ist;
Q¹ CR^{a} ist, wobei R^{a} H oder Fluor ist;
Q² CH ist;
Q³ N ist;
Q⁴ N oder CH ist;
Q⁵ N oder CH ist;
Y O ist;
oder ein pharmazeutisch verträgliches Salz davon.

11. Verbindung nach Anspruch 1, ausgewählt aus:
4-Amino-1-[4-[4-[6-chlor-4-(trifluormethyl)-2-pyridyl]piperazin-1-yl]sulfonylphenyl]pyrrolidin-2-on;
4-(Aminomethyl)-1-[4-[4-[6-chlor-4-(trifluormethyl)-2-pyridyl]piperazin-1-yl]sulfonyl-phenyl]pyrrolidin-2-on;
5-(Aminomethyl)-3-[4-[4-[6-chlor-4-(trifluormethyl)-2-pyridyl]piperazin-1-yl]sulfonylphenyl]oxazolidin-2-on;
4-Amino-1-[4-[4-[6-chlor-4-(trifluormethyl)-2-pyridyl]piperazin-1-yl]sulfonylphenyl]-3,3-dimethyl-pyrrolidin-2-on;
4-(Aminomethyl)-3-[4-[4-[6-chlor-4-(trifluormethyl)-2-pyridyl]piperazin-1-yl]sulfonylphenyl]oxazolidin-2-on;
3-Amino-1-[4-[4-[6-chlor-4-(trifluormethyl)-2-pyridyl]piperazin-1-yl]sulfonylphenyl]pyrrolidin-2-on;
5-(2-Aminoethyl)-3-[4-[4-[6-chlor-4-(trifluormethyl)-2-pyridyl]piperazin-1-yl]sulfonylphenyl]oxazolidin-2-on;
2-[4-[4-[6-Chlor-4-(trifluormethyl)-2-pyridyl]piperazin-1-yl]sulfonylphenyl]-2,6-diazaspiro[4.5]decan-3-on;
2-[4-[4-[6-Chlor-4-(trifluormethyl)-2-pyridyl]piperazin-1-yl]sulfonylphenyl]-2,8-diazaspiro[4.5]decan-3-on;
6-[4-[4-[6-Chlor-4-(trifluormethyl)-2-pyridyl]piperazin-1-yl]sulfonylphenyl]-2,6-diazaspiro[3.4]octan-7-on;
7-[4-[4-[6-Chlor-4-(trifluormethyl)-2-pyridyl]piperazin-1-yl]sulfonylphenyl]-5-oxa-2,7-diazaspiro[3.4]octan-6-on;
3-[4-[4-[6-Chlor-4-(trifluormethyl)-2-pyridyl]piperazin-1-yl]sulfonylphenyl]-1-oxa-3,8-diazaspiro[4.5]decan-2-on;
4-[4-[4-[6-Chlor-4-(trifluormethyl)-2-pyridyl]piperazin-1-yl]sulfonylphenyl]-1,2,3,3a,6,6a-hexahydropyrrolo[3,2-b]pyrrol-5-on;
3-[4-[4-[6-Chlor-4-(trifluormethyl)-2-pyridyl]piperazin-1-yl]sulfonylphenyl]-6-hydroxy-5-(hydroxymethyl)-3a,5,6,6a-tetrahydrofuro[2,3-d]oxazol-2-on;
3-[4-[4-[6-Chlor-4-(trifluormethyl)-2-pyridyl]piperazin-1-yl]sulfonylphenyl]-5-[(3S,4R,5R)-3,4-dihydroxy-5-methoxy-tetrahydrofuran-2-yl]-4-methyl-oxazolidin-2-on;
(3aS,5S,6S,7S,7aR)-3-[4-[4-[6-Chlor-4-(trifluormethyl)-2-pyridyl]piperazin-1-yl]sulfonylphenyl]-5,7-dihydroxy-6-(hydroxymethyl)-5,6,7,7a-tetrahydro-3aH-pyrano[2,3-d]oxazol-2-on;
8-(2-Aminoethyl)-3-[4-[4-[6-chlor-4-(trifluormethyl)-2-pyridyl]piperazin-1-yl]sulfonylphenyl]-1-oxa-3,8-diazaspiro[4.5]decan-2-on;
(3aS,8aR)-5-(2-Aminoethyl)-3-[4-[4-[6-chlor-4-(trifluormethyl)-2-pyridyl]piperazin-1-yl]sulfonylphenyl]-3a,4,6,7,8,8a-hexahydrooxazolo[4,5-c]azepin-2-on;
(3aS,8aR)-3-[4-[4-[6-Chlor-4-(trifluormethyl)-2-pyridyl]piperazin-1-yl]sulfonylphenyl]-5-[2-(methylamino)ethyl]-3a,4,6,7,8,8a-hexahydrooxazolo[4,5-c]azepin-2-on;
3-[4-[4-[6-Chlor-4-(trifluormethyl)-2-pyridyl]piperazin-1-yl]sulfonylphenyl]-5-(methylaminomethyl)oxazolidin-2-on;
3-[4-[4-[6-Chlor-4-(trifluormethyl)-2-pyridyl]piperazin-1-yl]sulfonylphenyl]-5-[(dimethylamino)methyl]oxazolidin-2-on;
3-[4-[4-[6-Chlor-4-(trifluormethyl)-2-pyridyl]piperazin-1-yl]sulfonylphenyl]-5-(piperazin-1-ylmethyl)oxazolidin-2-on;
3-[4-[4-[6-Chlor-4-(trifluormethyl)-2-pyridyl]piperazin-1-yl]sulfonylphenyl]-5-[[cis-3,4-dihydroxypyrrolidin-1-yl]methyl]oxazolidin-2-on;
5-[(3-Aminoazetidin-1-yl)methyl]-3-[4-[4-[6-chlor-4-(trifluormethyl)-2-pyridyl]piperazin-1-yl]sulfonylphenyl]oxazolidin-2-on;
3-[4-[4-[6-Chlor-4-(trifluormethyl)-2-pyridyl]piperazin-1-yl]sulfonylphenyl]-5-(2,6-diazaspiro[3.3]heptan-2-ylmethyl)oxazolidin-2-on;
5-[[3-(Aminomethyl)azetidin-1-yl]methyl]-3-[4-[4-[6-chlor-4-(trifluormethyl)-2-pyridyl]piperazin-1-yl]sulfonylphenyl]oxazolidin-2-on;
3-[4-[4-[6-Chlor-4-(trifluormethyl)-2-pyridyl]piperazin-1-yl]sulfonylphenyl]-5-[(pyrrolidin-3-ylamino)methyl]oxazolidin-2-on;
4-[[3-(Aminomethyl)azetidin-1-yl]methyl]-1-[4-[4-[6-chlor-4-(trifluormethyl)-2-pyridyl]piperazin-1-yl]sulfonylphenyl]pyrrolidin-2-on;
1-[4-[4-[6-Chlor-4-(trifluormethyl)-2-pyridyl]piperazin-1-yl]sulfonylphenyl]-4-[(4-methylpiperazin-1-yl)methyl]pyrrolidin-2-on;
1-[4-[4-[6-Chlor-4-(trifluormethyl)-2-pyridyl]piperazin-1-yl]sulfonylphenyl]-4-[[3-[(dimethylamino)methyl]azetidin-1-yl]methyl]pyrrolidin-2-on;
4-[(Azetidin-3-ylmethylamino)methyl]-1-[4-[4-[6-chlor-4-(trifluormethyl)-2-pyridyl]piperazin-1-yl]sulfonylphenyl]pyrrolidin-2-on;
4-[[3-(1-Aminocyclopropyl)azetidin-1-yl]methyl]-1-[4-[4-[6-chlor-4-(trifluormethyl)-2-pyridyl]piperazin-1-yl]sulfonylphenyl]pyrrolidin-2-on;
4-[(3-Aminoazetidin-1-yl)methyl]-1-[4-[4-[6-chlor-4-(trifluormethyl)-2-pyridyl]piperazin-1-yl]sulfonylphenyl]pyrrolidin-2-on;
1-[4-[4-[6-Chlor-4-(trifluormethyl)-2-pyridyl]piperazin-1-yl]sulfonylphenyl]-4-(piperazin-1-ylmethyl)pyrrolidin-2-on;
1-[4-[4-[6-Chlor-4-(trifluormethyl)-2-pyridyl]piperazin-1-yl]sulfonylphenyl]-4-[[3-(dimethylamino)azetidin-1-yl]methyl]pyrrolidin-2-on;
1-[4-[4-[6-Chlor-4-(trifluormethyl)-2-pyridyl]piperazin-1-yl]sulfonylphenyl]-4-[[3-(methylamino)azetidin-1-yl]methyl]pyrrolidin-2-on;
1-[4-[4-[6-Chlor-4-(trifluormethyl)-2-pyridyl]piperazin-1-yl] sulfonylphenyl]-4-(methylamino)pyrrolidin-2-on;
4-(2-Aminoethylamino)-1-[4-[4-[6-chlor-4-(trifluormethyl)-2-pyridyl]piperazin-1-yl]sulfonylphenyl]pyrrolidin-2-on;
1-[4-[4-[6-Chlor-4-(trifluormethyl)-2-pyridyl]piperazin-1-yl]sulfonylphenyl]-4-(2-hydroxyethylamino)pyrrolidin-2-on;
1-[4-[4-[6-Chlor-4-(trifluormethyl)-2-pyridyl]piperazin-1-yl]sulfonylphenyl]-4-piperazin-1-yl-pyrrolidin-2-on;
4-[3-(Aminomethyl)azetidin-1-yl]-1-[4-[4-[6-chlor-4-(trifluormethyl)-2-pyridyl]piperazin-1-yl]sulfonylphenyl]pyrrolidin-2-on;
4-(3-Aminopyrrolidin-1-yl)-1-[4-[4-[6-chlor-4-(trifluormethyl)-2-pyridyl]piperazin-1-yl]sulfonylphenyl]pyrrolidin-2-on;
4-(3-Aminoazetidin-1-yl)-1-[4-[4-[6-chlor-4-(trifluormethyl)-2-pyridyl]piperazin-1-yl]sulfonylphenyl]pyrrolidin-2-on;
1-[4-[4-[6-Chlor-4-(trifluormethyl)-2-pyridyl]piperazin-1-yl]sulfonylphenyl]-4-(2,6-diazaspiro[3.3]heptan-2-yl)pyrrolidin-2-on;
1-[4-[4-[6-Chlor-4-(trifluormethyl)-2-pyridyl]piperazin-1-yl]sulfonylphenyl]-4-[(3S)-3-(hydroxymethyl)piperazin-1-yl]pyrrolidin-2-on;
1-[4-[4-[6-Chlor-4-(trifluormethyl)-2-pyridyl]piperazin-1-yl]sulfonylphenyl]-4-[(3R)-3-(hydroxymethyl)piperazin-1-yl]pyrrolidin-2-on;
4-((3R,4R)-3-Amino-4-hydroxypiperidin-1-yl)-1-(4-((4-(6-chlor-4-(trifluormethyl)pyridin-2-yl)piperazin-1-yl)sulfonyl)phenyl)pyrrolidin-2-on;
4-(Azetidin-3-ylamino)-1-[4-[4-[6-chlor-4-(trifluormethyl)-2-pyridyl]piperazin-1-yl]sulfonylphenyl]pyrrolidin-2-on;
1-[4-[4-[6-Chlor-4-(trifluormethyl)-2-pyridyl]piperazin-1-yl]sulfonylphenyl]-4-(pyrrolidin-3-ylamino)pyrrolidin-2-on;
1-[4-[4-[6-Chlor-4-(trifluormethyl)-2-pyridyl]piperazin-1-yl]sulfonylphenyl]-4-(4-piperidylamino)pyrrolidin-2-on;
4-Amino-1-[4-[4-[6-chlor-4-(trifluormethyl)-2-pyridyl]piperazin-1-yl]sulfonylphenyl]-5-methyl-pyrrolidin-2-on;
4-Amino-1-[5-[4-[6-chlor-4-(trifluormethyl)-2-pyridyl]piperazin-1-yl]sulfonyl-2-pyridyl]pyrrolidin-2-on;
5-(Aminomethyl)-3-[4-[4-[6-chlor-4-(trifluormethyl)-2-pyridyl]piperazin-1-yl]sulfonyl-2-fluor-phenyl]oxazolidin-2-on;
5-(Aminomethyl)-3-[4-[4-[6-chlor-4-(trifluormethyl)-2-pyridyl]piperazin-1-yl]sulfonyl-3-fluor-phenyl]oxazolidin-2-on;
4-Amino-1-[4-[4-[4-methyl-6-(trifluormethyl)pyrimidin-2-yl]piperazin-1-yl]sulfonylphenyl]pyrrolidin-2-on;
4-Amino-1-[4-[4-[4-(trideuteriomethyl)-6-(trifluormethyl)pyrimidin-2-yl]piperazin-1-yl]sulfonylphenyl]pyrrolidin-2-on;
1-[4-(4-Amino-2-oxo-pyrrolidin-1-yl)phenyl]sulfonyl-4-[6-chlor-4-(trifluormethyl)-2-pyridyl]piperazin-2-carbonitril;
4-Amino-1-[4-[4-[6-chlor-4-(difluormethyl)-2-pyridyl]piperazin-1-yl]sulfonylphenyl]pyrrolidin-2-on;
4-Amino-1-[4-[4-(6-chlor-4-cyclopropylsulfonyl-2-pyridyl)piperazin-1-yl]sulfonylphenyl]pyrrolidin-2-on;
4-Amino-1-[4-[4-(6-chlor-4-tetrahydropyran-4-ylsulfonyl-2-pyridyl)piperazin-1-yl]sulfonylphenyl]pyrrolidin-2-on;
1-[2-[4-[4-(4-Amino-2-oxo-pyrrolidin-1-yl)phenyl]sulfonylpiperazin-1-yl]-6-chlor-4-pyridyl]cyclopropancarbonitril;
4-Amino-1-[4-[4-(6-chlor-4-phenoxy-2-pyridyl)piperazin-1-yl]sulfonylphenyl]pyrrolidin-2-on;
4-Amino-1-[4-[4-[6-chlor-4-[difluor(phenyl)methyl]-2-pyridyl]piperazin-1-yl]sulfonylphenyl]pyrrolidin-2-on;
4-Amino-1-[4-[4-(4-benzyl-6-chlor-2-pyridyl)piperazin-1-yl]sulfonylphenyl]pyrrolidin-2-on;
4-Amino-1-[4-[4-[6-chlor-4-(1-phenylcyclopropyl)-2-pyridyl]piperazin-1-yl]sulfonylphenyl]pyrrolidin-2-on;
4-Amino-1-[4-[4-[4-[difluor(phenyl)methyl]-6-methyl-pyrimidin-2-yl]piperazin-1-yl]sulfonylphenyl]pyrrolidin-2-on;
4-Amino-1-[4-[4-[2-chlor-6-[difluor(phenyl)methyl]pyrimidin-4-yl]piperazin-1-yl]sulfonylphenyl]pyrrolidin-2-on;
4-Amino-1-[4-[4-[4-chlor-6-[difluor(phenyl)methyl]pyrimidin-2-yl]piperazin-1-yl]sulfonylphenyl]pyrrolidin-2-on;
4-Amino-1-[4-[4-[2-cyclopropyl-6-[difluor(phenyl)methyl]pyrimidin-4-yl]piperazin-1-yl]sulfonylphenyl]pyrrolidin-2-on;
4-Amino-1-[4-[4-[4-cyclopropyl-6-[difluor(phenyl)methyl]pyrimidin-2-yl]piperazin-1-yl]sulfonylphenyl]pyrrolidin-2-on;
4-Amino-1-[4-[4-[6-[difluor(phenyl)methyl]-2-methoxy-pyrimidin-4-yl]piperazin-1-yl]sulfonylphenyl]pyrrolidin-2-on;
4-Amino-1-[4-[4-[4-[difluor(phenyl)methyl]-6-methoxy-pyrimidin-2-yl]piperazin-1-yl]sulfonylphenyl]pyrrolidin-2-on;
(*4R*)-4-Amino-1-[4-[4-[6-[difluor(phenyl)methyl]-2-methyl-pyrimidin-4-yl]piperazin-1-yl]sulfonylphenyl]pyrrolidin-2-on;
4-Amino-1-[4-[4-[6-[difluor(phenyl)methyl]-2-vinyl-pyrimidin-4-yl]piperazin-1-yl]sulfonylphenyl]pyrrolidin-2-on;
2-[4-[4-[6-Chlor-4-[difluor(phenyl)methyl]-2-pyridyl]piperazin-1-yl]sulfonylphenyl]-2,6,9-triazaspiro[4.5]decan-3-on;
5-(Aminomethyl)-1-[4-[4-[6-chlor-4-[difluor(phenyl)methyl]-2-pyridyl]piperazin-1-yl]sulfonylphenyl]pyrrolidin-2-on;
5-(Aminomethyl)-3-[4-[4-[6-chlor-4-[difluor(phenyl)methyl]-2-pyridyl]piperazin-1-yl]sulfonylphenyl]oxazolidin-2-on;
4-Amino-1-[4-[4-[6-chlor-4-[difluor(2-pyridyl)methyl]-2-pyridyl]piperazin-1-yl]sulfonylphenyl]pyrrolidin-2-on;
4-Amino-1-[4-[4-[6-chlor-4-[difluor(3-pyridyl)methyl]-2-pyridyl]piperazin-1-yl]sulfonylphenyl]pyrrolidin-2-on;
4-Amino-1-[4-[4-[6-chlor-4-[difluor(4-pyridyl)methyl]-2-pyridyl]piperazin-1-yl]sulfonylphenyl]pyrrolidin-2-on;
4-Amino-1-[4-[4-[6-chlor-4-[difluor(pyrimidin-5-yl)methyl]-2-pyridyl]piperazin-1-yl]sulfonylphenyl]pyrrolidin-2-on;
4-Amino-1-[4-[4-[6-chlor-4-[difluor(pyrazin-2-yl)methyl]-2-pyridyl]piperazin-1-yl]sulfonylphenyl]pyrrolidin-2-on;
4-Amino-1-[4-[4-[6-chlor-4-[difluor(pyrimidin-2-yl)methyl]-2-pyridyl]piperazin-1-yl]sulfonylphenyl]pyrrolidin-2-on;
4-Amino-1-[4-[4-[6-chlor-4-[difluor(tetrahydropyran-4-yl)methyl]-2-pyridyl]piperazin-1-yl]sulfonylphenyl]pyrrolidin-2-on;
4-Amino-1-[4-[4-[6-chlor-4-[difluor(tetrahydrofuran-3-yl)methyl]-2-pyridyl]piperazin-1-yl]sulfonylphenyl]pyrrolidin-2-on;
4-Amino-1-[4-[4-[6-chlor-4-[difluor(oxetan-3-yl)methyl]-2-pyridyl]piperazin-1-yl]sulfonylphenyl]pyrrolidin-2-on;
4-Amino-1-[4-[4-[6-chlor-4-[3,6-dihydro-2H-pyran-4-yl(difluor)methyl]-2-pyridyl]piperazin-1-yl]sulfonylphenyl]pyrrolidin-2-on;
4-Amino-1-[4-[4-[6-chlor-4-[difluor(tetrahydropyran-3-yl)methyl]-2-pyridyl]piperazin-1-yl]sulfonylphenyl]pyrrolidin-2-on;
4-Amino-1-[4-[4-[6-chlor-4-[difluor(8-oxabicyclo[3.2.1]octan-3-yl)methyl]-2-pyridyl]piperazin-1-yl]sulfonylphenyl]pyrrolidin-2-on;
(*4R*)-4-Amino-1-[4-[4-[6-chlor-4-[trans-difluor-[2-(hydroxymethyl)cyclopropyl]methyl]-2-pyridyl]piperazin-1-yl]sulfonylphenyl]pyrrolidin-2-on;
(*4R*)-4-Amino-1-[4-[4-[4-[[trans-4-(aminomethyl)cyclohexyl]-difluormethyl]-6-chlor-2-pyridyl]piperazin-1-yl]sulfonylphenyl]pyrrolidin-2-on;
(*4R*)-4-Amino-1-[4-[4-[6-chlor-4-[trans-difluor-(3-hydroxycyclobutyl)methyl]-2-pyridyl]piperazin-1-yl]sulfonylphenyl]pyrrolidin-2-on; und
trans-4-[[2-[4-[4-[(4R)-4-Amino-2-oxo-pyrrolidin-1-yl]phenyl]sulfonylpiperazin-1-yl]-6-chlor-4-pyridyl]-difluormethyl]cyclohexancarboxamid;
oder ein pharmazeutisch verträgliches Salz davon.

12. Verfahren zur Herstellung einer Verbindung nach einem der Ansprüche 1 bis 11, umfassend den folgenden Schritt:
a) Kupplungsreaktion zwischen einer Verbindung der Formel (VII), 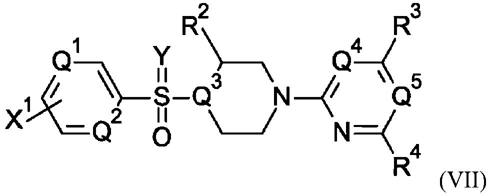 mit Lactam oder Carbamat, R¹ H, in Gegenwart eines Katalysators und einer Base;
wobei
in Schritt a) der Katalysator CuI ist, die Base Cs₂CO₃ ist;
R¹, R², R³, R⁴, Y, Q¹, Q², Q³, Q⁴ und Q⁵ wie in einem der Ansprüche 1 bis 10 definiert sind.

13. Verbindung oder ein pharmazeutisch verträgliches Salz nach einem der Ansprüche 1 bis 11 zur Verwendung als therapeutisch wirksame Substanz.

14. Pharmazeutische Zusammensetzung, umfassend eine Verbindung nach einem der Ansprüche 1 bis 11 und einen therapeutisch inerten Träger.

15. Verbindung oder pharmazeutisch verträgliches Salz nach einem der Ansprüche 1 bis 11 zur Verwendung bei der Behandlung oder Vorbeugung einer bakteriellen Infektion, wobei es sich bei den Bakterien insbesondere um gramnegative Bakterien handelt, insbesondere wobei die gramnegativen Bakterien ausgewählt sind aus *Enterobacteriaceae, Neisseria gonorrhoeae, Haemophilus influenzae, Helicobacter pylorus, Acinetobacter baumannii* und *Pseudomonas aeruginosa.*

## Revendications

1. Composé de formule (I), 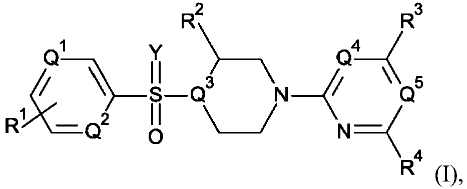 dans lequel
R¹ représente un 2-oxo-1-oxa-3,8-diazaspiro[4.5]décanyle non substitué ou substitué par un aminoalkyle en C₁₋₆,
un 2-oxo-3a,4,5,6,7,7a-hexahydrooxazolo[4,5-c]pyridinyle,
un 2-oxo-3a,4,5,6,7,7a-hexahydrooxazolo[5,4-c]pyridinyle,
un 2-oxo-3a,4,6,7,8,8a-hexahydrooxazolo[4,5-c]azépinyle substitué par un aminoalkyle en C₁₋₆ ou un alkyle en C₁₋₆-aminoalkyle en C₁₋₆,
un 2-oxo-3a,5,6,6a-tétrahydrofuro[2,3-d]oxazolyle substitué deux fois par des substituants indépendamment choisis parmi un hydroxy et un hydroxyalkyle en C₁₋₆,
un 2-oxo-4,5,6,6a-tétrahydro-3aH-pyrrolo[3,4-d]oxazolyle,
un 2-oxo-4,5,6,7,8,8a-hexahydro-3aH-oxazolo[4,5-c]azépinyle,
un 2-oxo-5,6,7,7a-tétrahydro-3aH-pyrano[2,3-d]oxazolyle substitué trois fois par des substituants indépendamment choisis parmi un hydroxy et un hydroxyalkyle en C₁₋₆,
un 3-oxo-2,6,9-triazaspiro[4.5]décanyle,
un 3-oxo-2,6-diazaspiro[4.5]décanyle,
un 3-oxo-2,8-diazaspiro[4.5]décanyle,
un 5-oxo-1,2,3,3a,6,6a-hexahydropyrrolo[3,2-b]pyrrolyle,
un 6-oxo-5-oxa-2,7-diazaspiro[3.4]octanyle,
un 7-oxo-2,6-diazaspiro[3.4]octanyle,
un oxoazétidinyle substitué par un amino,
un oxooxazolidinyle substitué une ou deux fois par des substituants indépendamment choisis parmi un ((aminoalkyle en C₁₋₆)azétidinyl)alkyle en C₁₋₆, un (alkyle en C₁₋₆)₂aminoalkyle en C₁₋₆, un (dihydroxypyrrolidinyl)alkyle en C₁₋₆, un (pyrrolidinylamino)alkyle en C₁₋₆, un 2,6-diazaspiro[3.3]heptanylalkyle en C₁₋₆, un dihydroxy(alcoxy en C₁₋₆)tétrahydrofuranyle, un aminoazétidinylalkyle en C₁₋₆, un aminoalkyle en C₁₋₆, un alkyle en C₁₋₆, un alkyle en C₁₋₆-aminoalkyle en C₁₋₆, un alkyle en C₁₋₆-carbonylaminoalkyle en C₁₋₆, un hydroxyalkyle en C₁₋₆ et un pipérazinylalkyle en C₁₋₆, ou
un oxopyrrolidinyle substitué une, deux ou trois fois par des substituants indépendamment choisis parmi un (((alkyle en C₁₋₆)₂aminoalkyle en C₁₋₆)azétidinyl)alkyle en C₁₋₆, un ((aminoalkyle en C₁₋₆)azétidinyl)alkyle en C₁₋₆, un ((aminocycloalkyle en C₃₋₇)azétidinyl)alkyle en C₁₋₆, un ((alkyle en C₁₋₆-amino)azétidinyl)alkyle en C₁₋₆, un (aminoazétidinyl)alkyle en C₁₋₆, un (aminoalkyle en C₁₋₆)azétidinyle, un (azétidinylalkyle en C₁₋₆-amino)alkyle en C₁₋₆, un (alkyle en C₁₋₆-pipérazinyl)alkyle en C₁₋₆, un (hydroxyalkyle en C₁₋₆)pipérazinyle, un 2,5-diazabicyclo[2.2.1]heptanyle, un 2,6-diazaspiro[3.3]heptanyle, un amino, un amino(hydroxy)pipéridinyle, un amino(hydroxy)pyrrolidinyle, un aminoazétidinyle, un aminoalkyle en C₁₋₆, un (aminoalkyle en C₁₋₆)amino, un aminopyrrolidinyle, un azétidinylamino, un alkyle en C₁₋₆, un alkyle en C₁₋₆-amino, un dihydroxypyrrolidinyle, un (hydroxyalkyle en C₁₋₆)amino, un pipérazinyle, un pipérazinylalkyle en C₁₋₆, un pipéridinylamino et un pyrrolidinylamino ;
R² représente H ou un cyano ;
R³ représente un benzyle,
un cycloalkyle en C₃₋₇-sulfonyle,
un cyanocycloalkyle en C₃₋₇,
un phénoxy,
un phénylcycloalkyle en C₃₋₇,
un tétrahydropyranylsulfonyle, ou
un alkyle en C₁₋₆ substitué deux ou trois fois par des substituants indépendamment choisis parmi un halogène, un (aminoalkyle en C₁₋₆)cycloalkyle en C₃₋₇, un (hydroxyalkyle en C₁₋₆)cycloalkyle en C₃₋₇, un 1,2,3,6-tétrahydropyridinyle, un 1,4-dioxanyle, un 3,6-dihydro-2H-pyranyle, un 3-azabicyclo[3.1.0]hexanyle, un 3-oxabicyclo[3.1.0]hexanyle, un 8-oxabicyclo[3.2.1]octanyle, un azétidinyle, un alkyle en C₁₋₆-halogénopyrazinyle, un alkyle en C₁₋₆-morpholinyle, un alkyle en C₁₋₆-pyrazinyle, un carbamoylcycloalkyle en C₃₋₇, un hydroxycycloalkyle en C₃₋₇, un morpholinyle, un oxétanyle, un pipéridinyle, un pyrazinyle, un pyridinyle, un pyrimidinyle, un tétrahydrofuranyle, un tétrahydropyranyle, un phényle et un pyridinyle ;
R⁴ représente un halogène, un alkyle en C₁₋₆, un cycloalkyle en C₃₋₇, un alcoxy en C₁₋₆ ou un alcynyle en C₂₋₆ ;
Q¹ représente N ou CR^{a}, dans lequel R^{a} représente H ou un halogène ;
Q² représente N ou CR^{b}, dans lequel R^{b} représente H ou un halogène ;
Q³ représente N ou CH ;
Q⁴ représente N ou CH ;
Q⁵ représente N ou CH ;
Y représente O ou NH ;
ou un sel pharmaceutiquement acceptable de celui-ci.

2. Composé selon la revendication 1 de formule (Ia), 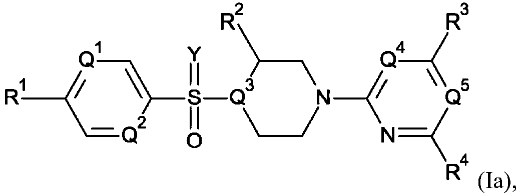 dans lequel
R¹ représente un 2-oxo-1-oxa-3,8-diazaspiro[4.5]décanyle non substitué ou substitué par un aminoalkyle en C₁₋₆,
un 2-oxo-3a,4,5,6,7,7a-hexahydrooxazolo[4,5-c]pyridinyle,
un 2-oxo-3a,4,5,6,7,7a-hexahydrooxazolo[5,4-c]pyridinyle,
un 2-oxo-3a,4,6,7,8,8a-hexahydrooxazolo[4,5-c]azépinyle substitué par un aminoalkyle en C₁₋₆ ou un alkyle en C₁₋₆-aminoalkyle en C₁₋₆,
un 2-oxo-3a,5,6,6a-tétrahydrofuro[2,3-d]oxazolyle substitué deux fois par des substituants indépendamment choisis parmi un hydroxy et un hydroxyalkyle en C₁₋₆,
un 2-oxo-4,5,6,6a-tétrahydro-3aH-pyrrolo[3,4-d]oxazolyle,
un 2-oxo-4,5,6,7,8,8a-hexahydro-3aH-oxazolo[4,5-c]azépinyle,
un 2-oxo-5,6,7,7a-tétrahydro-3aH-pyrano[2,3-d]oxazolyle substitué trois fois par des substituants indépendamment choisis parmi un hydroxy et un hydroxyalkyle en C₁₋₆,
un 3-oxo-2,6,9-triazaspiro[4.5]décanyle,
un 3-oxo-2,6-diazaspiro[4.5]décanyle,
un 3-oxo-2,8-diazaspiro[4.5]décanyle,
un 5-oxo-1,2,3,3a,6,6a-hexahydropyrrolo[3,2-b]pyrrolyle,
un 6-oxo-5-oxa-2,7-diazaspiro[3.4]octanyle,
un 7-oxo-2,6-diazaspiro[3.4]octanyle,
un oxoazétidinyle substitué par un amino,
un oxooxazolidinyle substitué une ou deux fois par des substituants indépendamment choisis parmi un ((aminoalkyle en C₁₋₆)azétidinyl)alkyle en C₁₋₆, un (alkyle en C₁₋₆)₂aminoalkyle en C₁₋₆, un (dihydroxypyrrolidinyl)alkyle en C₁₋₆, un (pyrrolidinylamino)alkyle en C₁₋₆, un 2,6-diazaspiro[3.3]heptanylalkyle en C₁₋₆, un dihydroxy(alcoxy en C₁₋₆)tétrahydrofuranyle, un aminoazétidinylalkyle en C₁₋₆, un aminoalkyle en C₁₋₆, un alkyle en C₁₋₆, un alkyle en C₁₋₆-aminoalkyle en C₁₋₆, un alkyle en C₁₋₆-carbonylaminoalkyle en C₁₋₆, un hydroxyalkyle en C₁₋₆ et un pipérazinylalkyle en C₁₋₆, ou
un oxopyrrolidinyle substitué une, deux ou trois fois par des substituants indépendamment choisis parmi un (((alkyle en C₁₋₆)₂aminoalkyle en C₁₋₆)azétidinyl)alkyle en C₁₋₆, un ((aminoalkyle en C₁₋₆)azétidinyl)alkyle en C₁₋₆, un ((aminocycloalkyle en C₃₋₇)azétidinyl)alkyle en C₁₋₆, un ((alkyle en C₁₋₆-amino)azétidinyl)alkyle en C₁₋₆, un (aminoazétidinyl)alkyle en C₁₋₆, un (aminoalkyle en C₁₋₆)azétidinyle, un (azétidinylalkyle en C₁₋₆-amino)alkyle en C₁₋₆, un (alkyle en C₁₋₆-pipérazinyl)alkyle en C₁₋₆, un (hydroxyalkyle en C₁₋₆)pipérazinyle, un 2,5-diazabicyclo[2.2.1]heptanyle, un 2,6-diazaspiro[3.3]heptanyle, un amino, un amino(hydroxy)pipéridinyle, un amino(hydroxy)pyrrolidinyle, un aminoazétidinyle, un aminoalkyle en C₁₋₆, un (aminoalkyle en C₁₋₆)amino, un aminopyrrolidinyle, un azétidinylamino, un alkyle en C₁₋₆, un alkyle en C₁₋₆-amino, un dihydroxypyrrolidinyle, un (hydroxyalkyle en C₁₋₆)amino, un pipérazinyle, un pipérazinylalkyle en C₁₋₆, un pipéridinylamino et un pyrrolidinylamino ;
R² représente H ou un cyano ;
R³ représente un benzyle,
un cycloalkyle en C₃₋₇-sulfonyle,
un cyanocycloalkyle en C₃₋₇,
un phénoxy,
un phénylcycloalkyle en C₃₋₇,
un tétrahydropyranylsulfonyle, ou
un alkyle en C₁₋₆ substitué deux ou trois fois par des substituants indépendamment choisis parmi un halogène, un (aminoalkyle en C₁₋₆)cycloalkyle en C₃₋₇, un (hydroxyalkyle en C₁₋₆)cycloalkyle en C₃₋₇, un 1,2,3,6-tétrahydropyridinyle, un 1,4-dioxanyle, un 3,6-dihydro-2H-pyranyle, un 3-azabicyclo[3.1.0]hexanyle, un 3-oxabicyclo[3.1.0]hexanyle, un 8-oxabicyclo[3.2.1]octanyle, un azétidinyle, un alkyle en C₁₋₆-halogénopyrazinyle, un alkyle en C₁₋₆-morpholinyle, un alkyle en C₁₋₆-pyrazinyle, un carbamoylcycloalkyle en C₃₋₇, un hydroxycycloalkyle en C₃₋₇, un morpholinyle, un oxétanyle, un pipéridinyle, un pyrazinyle, un pyridinyle, un pyrimidinyle, un tétrahydrofuranyle, un tétrahydropyranyle, un phényle et un pyridinyle ;
R⁴ représente un halogène, un alkyle en C₁₋₆, un cycloalkyle en C₃₋₇, un alcoxy en C₁₋₆ ou un alcynyle en C₂₋₆ ;
Q¹ représente N ou CR^{a}, dans lequel R^{a} représente H ou un halogène ;
Q² représente CR^{b}, dans lequel R^{b} représente H ou un halogène ;
Q³ représente N ;
Q⁴ représente N ou CH ;
Q⁵ représente N ou CH ;
Y représente O ;
ou un sel pharmaceutiquement acceptable de celui-ci.

3. Composé selon la revendication 1 ou 2, dans lequel
R¹ représente un 2-oxo-1-oxa-3,8-diazaspiro[4.5]décanyle,
un 2-oxo-3a,4,5,6,7,7a-hexahydrooxazolo[4,5-c]pyridinyle,
un 2-oxo-3a,4,5,6,7,7a-hexahydrooxazolo[5,4-c]pyridinyle,
un 2-oxo-3a,5,6,6a-tétrahydrofuro[2,3-d]oxazolyle substitué deux fois par des substituants indépendamment choisis parmi un hydroxy et un hydroxyalkyle en C₁₋₆,
un 2-oxo-4,5,6,6a-tétrahydro-3aH-pyrrolo[3,4-d]oxazolyle,
un 2-oxo-4,5,6,7,8,8a-hexahydro-3aH-oxazolo[4,5-c]azépinyle,
un 3-oxo-2,6-diazaspiro[4.5]décanyle,
un 5-oxo-1,2,3,3a,6,6a-hexahydropyrrolo[3,2-b]pyrrolyle,
un 6-oxo-5-oxa-2,7-diazaspiro[3.4]octanyle,
un oxooxazolidinyle substitué une ou deux fois par des substituants indépendamment choisis parmi un ((aminoalkyle en C₁₋₆)azétidinyl)alkyle en C₁₋₆, un (alkyle en C₁₋₆)₂aminoalkyle en C₁₋₆, un dihydroxy(alcoxy en C₁₋₆)tétrahydrofuranyle, un aminoalkyle en C₁₋₆, un alkyle en C₁₋₆, un alkyle en C₁₋₆-aminoalkyle en C₁₋₆, un hydroxyalkyle en C₁₋₆ et un pipérazinylalkyle en C₁₋₆, ou
un oxopyrrolidinyle substitué par un substituant choisi parmi un ((aminoalkyle en C₁₋₆)azétidinyl)alkyle en C₁₋₆, un ((aminocycloalkyle en C₃₋₇)azétidinyl)alkyle en C₁₋₆, un (aminoazétidinyl)alkyle en C₁₋₆, un (alkyle en C₁₋₆-pipérazinyl)alkyle en C₁₋₆, un (hydroxyalkyle en C₁₋₆)pipérazinyle, un amino, un amino(hydroxy)pipéridinyle, un amino(hydroxy)pyrrolidinyle, un aminoalkyle en C₁₋₆, un aminopyrrolidinyle, un alkyle en C₁₋₆-amino, un dihydroxypyrrolidinyle, un (hydroxyalkyle en C₁₋₆)amino, un pipérazinyle, un pipérazinylalkyle en C₁₋₆ et un pipéridinylamino.

4. Composé selon l'une quelconque des revendications 1 à 3, dans lequel R³ représente un benzyle, un phénylcycloalkyle en C₃₋₇ ou un alkyle en C₁₋₆ substitué trois fois par des substituants indépendamment choisis parmi un halogène, un (hydroxyalkyle en C₁₋₆)cycloalkyle en C₃₋₇, un 1,2,3,6-tétrahydropyridinyle, un 1,4-dioxanyle, un 3,6-dihydro-2H-pyranyle, un 3-azabicyclo[3.1.0]hexanyle, un 3-oxabicyclo[3.1.0]hexanyle, un 8-oxabicyclo[3.2.1]octanyle, un alkyle en C₁₋₆-halogénopyrazinyle, un alkyle en C₁₋₆-pyrazinyle, un carbamoylcycloalkyle en C₃₋₇, un hydroxycycloalkyle en C₃₋₇, un morpholinyle, un oxétanyle, un pyrazinyle, un pyridinyle, un tétrahydropyranyle et un phényle.

5. Composé selon l'une quelconque des revendications 1 à 4, dans lequel R² représente H.

6. Composé selon l'une quelconque des revendications 1 à 5, dans lequel R⁴ représente un halogène ou un alkyle en C₁₋₆, en particulier dans lequel R⁴ représente un chlore ou un méthyle.

7. Composé selon l'une quelconque des revendications 1 à 6, dans lequel Q¹ représente CR^{a}, dans lequel R^{a} représente H ou un halogène, en particulier dans lequel Q¹ représente CR¹, dans lequel R¹ représente H ou un fluor.

8. Composé selon l'une quelconque des revendications 1 à 7, dans lequel Q² représente CH.

9. Composé selon la revendication 1 ou 2, dans lequel
R¹ représente un oxopyrrolidinyle substitué par un amino ou un aminoalkyle en C₁₋₆ ;
R² représente H ;
R³ représente un alkyle en C₁₋₆ substitué trois fois par des substituants indépendamment choisis parmi un halogène, un (hydroxyalkyle en C₁₋₆)cycloalkyle en C₃₋₇, un 1,4-dioxanyle, un 3-azabicyclo[3.1.0]hexanyle, un 3-oxabicyclo[3.1.0]hexanyle, un alkyle en C₁₋₆-halogénopyrazinyle, un alkyle en C₁₋₆-pyrazinyle, un hydroxycycloalkyle en C₃₋₇, un morpholinyle, un oxétanyle, un phényle, un pyridinyle et un tétrahydropyranyle ;
R⁴ représente un halogène ou un alkyle en C₁₋₆ ;
Q¹ représente CR^{a}, dans lequel R^{a} représente H ou un halogène ;
Q² représente CH ;
Q³ représente N ;
Q⁴ représente N ou CH ;
Q⁵ représente N ou CH ;
Y représente O ;
ou un sel pharmaceutiquement acceptable de celui-ci.

10. Composé selon la revendication 9, dans lequel
R¹ représente un oxopyrrolidinyle substitué par un amino ou un aminométhyle ;
R² représente H ;
R³ représente un difluoro(1,4-dioxan-2-yl)méthyle, un difluoro(2-(hydroxyméthyl)cyclopropyl)méthyle, un difluoro(2-pyridinyl)méthyle, un difluoro(3-hydroxycyclobutyl)méthyle, un difluoro(3-oxabicyclo[3.1.0]hexan-6-yl)méthyle, un difluoro(5-chloro-6-méthyl-pyrazin-2-yl)méthyle, un difluoro(6-méthylpyrazin-2-yl)méthyle, un difluoro(morpholin-2-yl)méthyle, un difluoro(oxétan-3-yl)méthyle, un difluoro(phényl)méthyle, un difluoro(tétrahydropyran-4-yl)méthyle ou un difluoro[3-azabicyclo[3.1.0]hexan-1-yl]méthyle ;
R⁴ représente un chlore ou un méthyle ;
Q¹ représente CR^{a}, dans lequel R^{a} représente H ou un fluor ;
Q² représente CH ;
Q³ représente N ;
Q⁴ représente N ou CH ;
Q⁵ représente N ou CH ;
Y représente O ;
ou un sel pharmaceutiquement acceptable de celui-ci.

11. Composé selon la revendication 1 choisi parmi :
la 4-amino-1-[4-[4-[6-chloro-4-(trifluorométhyl)-2-pyridyl]pipérazin-1-yl]sulfonylphényl]pyrrolidin-2-one ;
la 4-(aminométhyl)-1-[4-[4-[6-chloro-4-(trifluorométhyl)-2-pyridyl]pipérazin-1-yl]sulfonyl-phényl]pyrrolidin-2-one ;
la 5-(aminométhyl)-3-[4-[4-[6-chloro-4-(trifluorométhyl)-2-pyridyl]pipérazin-1-yl]sulfonylphényl]oxazolidin-2-one ;
le *N*-[[3-[4-[4-[6-chloro-4-(trifluorométhyl)-2-pyridyl]pipérazin-1-yl]sulfonylphényl]-2-oxo-oxazolidin-5-yl]méthyl]acétamide ;
la 4-amino-1-[4-[4-[6-chloro-4-(trifluorométhyl)-2-pyridyl]pipérazin-1-yl]sulfonylphényl]-3,3-diméthyl-pyrrolidin-2-one ;
la 4-(aminométhyl)-3-[4-[4-[6-chloro-4-(trifluorométhyl)-2-pyridyl]pipérazin-1-yl]sulfonylphényl]oxazolidin-2-one ;
la (3*S*)-3-amino-1-[4-[4-[6-chloro-4-(trifluorométhyl)-2-pyridyl]pipérazin-1-yl]sulfonylphényl]azétidin-2-one ;
la 3-amino-1-[4-[4-[6-chloro-4-(trifluorométhyl)-2-pyridyl]pipérazin-1-yl]sulfonylphényl]pyrrolidin-2-one ;
la 5-(2-aminoéthyl)-3-[4-[4-[6-chloro-4-(trifluorométhyl)-2-pyridyl]pipérazin-1-yl]sulfonylphényl]oxazolidin-2-one ;
la 2-[4-[4-[6-chloro-4-(trifluorométhyl)-2-pyridyl]pipérazin-1-yl]sulfonylphényl]-2,6-diazaspiro[4.5]décan-3-one ;
la 2-[4-[4-[6-chloro-4-(trifluorométhyl)-2-pyridyl]pipérazin-1-yl]sulfonylphényl]-2,8-diazaspiro[4.5]décan-3-one ;
la 6-[4-[4-[6-chloro-4-(trifluorométhyl)-2-pyridyl]pipérazin-1-yl]sulfonylphényl]-2,6-diazaspiro[3.4]octan-7-one ;
la 7-[4-[4-[6-chloro-4-(trifluorométhyl)-2-pyridyl]pipérazin-1-yl]sulfonylphényl]-5-oxa-2,7-diazaspiro[3.4]octan-6-one ;
la 3-[4-[4-[6-chloro-4-(trifluorométhyl)-2-pyridyl]pipérazin-1-yl]sulfonylphényl]-1-oxa-3,8-diazaspiro[4.5]décan-2-one ;
la (3a*R*,6a*S*)-3-[4-[4-[6-chloro-4-(trifluorométhyl)-2-pyridyl]pipérazin-1-yl]sulfonylphényl]-4,5,6,6a-tétrahydro-3a*H*-pyrrolo[3,4-d]oxazol-2-one ;
la (3a*R*,7a*S*)-3-[4-[4-[6-chloro-4-(trifluorométhyl)-2-pyridyl]pipérazin-1-yl]sulfonylphényl]-3a,4,5,6,7,7a-hexahydrooxazolo[4,5-c]pyridin-2-one ;
la (3a*R*,7a*S*)-1-[4-[4-[6-chloro-4-(trifluorométhyl)-2-pyridyl]pipérazin-1-yl]sulfonylphényl]-3a,4,5,6,7,7a-hexahydrooxazolo[5,4-c]pyridin-2-one ;
la (3a*S*,7a*R*)-3-[4-[4-[6-chloro-4-(trifluorométhyl)-2-pyridyl]pipérazin-1-yl]sulfonylphényl]-3a,4,5,6,7,7a-hexahydrooxazolo[4,5-c]pyridin-2-one ;
la (3a*S*,8a*R*)-3-[4-[4-[6-chloro-4-(trifluorométhyl)-2-pyridyl]pipérazin-1-yl]sulfonylphényl]-4,5,6,7,8,8a-hexahydro-3aH-oxazolo[4,5-c]azépin-2-one ;
la 4-[4-[4-[6-chloro-4-(trifluorométhyl)-2-pyridyl]pipérazin-1-yl]sulfonylphényl]-1,2,3,3a,6,6a-hexahydropyrrolo[3,2-b]pyrrol-5-one ;
la 3-[4-[4-[6-chloro-4-(trifluorométhyl)-2-pyridyl]pipérazin-1-yl]sulfonylphényl]-6-hydroxy-5-(hydroxyméthyl)-3a,5,6,6a-tétrahydrofuro[2,3-d]oxazol-2-one ;
la 3-[4-[4-[6-chloro-4-(trifluorométhyl)-2-pyridyl]pipérazin-1-yl]sulfonylphényl]-5-[(3*S*,4*R*,5*R*)-3,4-dihydroxy-5-méthoxy-tétrahydrofuran-2-yl]-4-méthyl-oxazolidin-2 ;
la (3a*S*,5*S*,6*S*,7*S*,7a*R*)-3-[4-[4-[6-chloro-4-(trifluorométhyl)-2-pyridyl]pipérazin-1-yl]sulfonylphényl]-5,7-dihydroxy-6-(hydroxyméthyl)-5,6,7,7a-tétrahydro-3aH-pyrano[2,3-d]oxazol-2-one ;
la 8-(2-aminoéthyl)-3-[4-[4-[6-chloro-4-(trifluorométhyl)-2-pyridyl]pipérazin-1-yl]sulfonylphényl]-1-oxa-3,8-diazaspiro[4.5]décan-2-one ;
la (3a*S*,8a*R*)-5-(2-aminoéthyl)-3-[4-[4-[6-chloro-4-(trifluorométhyl)-2-pyridyl]pipérazin-1-yl]sulfonylphényl]-3a,4,6,7,8,8a-hexahydrooxazolo[4,5-c]azépin-2-one ;
la (3a*S*,8a*R*)-3-[4-[4-[6-chloro-4-(trifluorométhyl)-2-pyridyl]pipérazin-1-yl]sulfonylphényl]-5-[2-(méthylamino)éthyl]-3a,4,6,7,8,8a-hexahydrooxazolo[4,5-c]azépin-2-one ;
la 3-[4-[4-[6-chloro-4-(trifluorométhyl)-2-pyridyl]pipérazin-1-yl]sulfonylphényl]-5-(méthylaminométhyl)oxazolidin-2-one ;
la 3-[4-[4-[6-chloro-4-(trifluorométhyl)-2-pyridyl]pipérazin-1-yl]sulfonylphényl]-5-[(diméthylamino)méthyl]oxazolidin-2-one ;
la 3-[4-[4-[6-chloro-4-(trifluorométhyl)-2-pyridyl]pipérazin-1-yl]sulfonylphényl]-5-(pipérazin-1-ylméthyl)oxazolidin-2-one ;
la 3-[4-[4-[6-chloro-4-(trifluorométhyl)-2-pyridyl]pipérazin-1-yl]sulfonylphényl]-5-[[cis-3,4-dihydroxypyrrolidin-1-yl]méthyl]oxazolidin-2-one ;
la 5-[(3-aminoazétidin-1-yl)méthyl]-3-[4-[4-[6-chloro-4-(trifluorométhyl)-2-pyridyl]pipérazin-1-yl]sulfonylphényl]oxazolidin-2-one ;
la 3-[4-[4-[6-chloro-4-(trifluorométhyl)-2-pyridyl]pipérazin-1-yl]sulfonylphényl]-5-(2,6-diazaspiro[3.3]heptan-2-ylméthyl)oxazolidin-2-one ;
la 5-[[3-(aminométhyl)azétidin-1-yl]méthyl]-3-[4-[4-[6-chloro-4-(trifluorométhyl)-2-pyridyl]pipérazin-1-yl]sulfonylphényl]oxazolidin-2-one ;
la 3-[4-[4-[6-chloro-4-(trifluorométhyl)-2-pyridyl]pipérazin-1-yl]sulfonylphényl]-5-[(pyrrolidin-3-ylamino)méthyl]oxazolidin-2-one ;
la 4-[[3-(aminométhyl)azétidin-1-yl]méthyl]-1-[4-[4-[6-chloro-4-(trifluorométhyl)-2-pyridyl]pipérazin-1-yl]sulfonylphényl]pyrrolidin-2-one ;
la 1-[4-[4-[6-chloro-4-(trifluorométhyl)-2-pyridyl]pipérazin-1-yl]sulfonylphényl]-4-[(4-méthylpipérazin-1-yl)méthyl]pyrrolidin-2-one ;
la 1-[4-[4-[6-chloro-4-(trifluorométhyl)-2-pyridyl]pipérazin-1-yl]sulfonylphényl]-4-[[3-[(diméthylamino)méthyl]azétidin-1-yl]méthyl]pyrrolidin-2-one ;
la 4-[(azétidin-3-ylméthylamino)méthyl]-1-[4-[4-[6-chloro-4-(trifluorométhyl)-2-pyridyl]pipérazin-1-yl]sulfonylphényl]pyrrolidin-2-one ;
la 4-[[3-(1-aminocyclopropyl)azétidin-1-yl]méthyl]-1-[4-[4-[6-chloro-4-(trifluorométhyl)-2-pyridyl]pipérazin-1-yl]sulfonylphényl]pyrrolidin-2-one ;
la 4-[(3-aminoazétidin-1-yl)méthyl]-1-[4-[4-[6-chloro-4-(trifluorométhyl)-2-pyridyl]pipérazin-1-yl]sulfonylphényl]pyrrolidin-2-one ;
la 1-[4-[4-[6-chloro-4-(trifluorométhyl)-2-pyridyl]pipérazin-1-yl]sulfonylphényl]-4-(pipérazin-1-ylméthyl)pyrrolidin-2-one ;
la 1-[4-[4-[6-chloro-4-(trifluorométhyl)-2-pyridyl]pipérazin-1-yl]sulfonylphényl]-4-[[3-(diméthylamino)azétidin-1-yl]méthyl]pyrrolidin-2-one ;
la 1-[4-[4-[6-chloro-4-(trifluorométhyl)-2-pyridyl]pipérazin-1-yl]sulfonylphényl]-4-[[3-(méthylamino)azétidin-1-yl]méthyl]pyrrolidin-2-one ;
la 1-[4-[4-[6-chloro-4-(trifluorométhyl)-2-pyridyl]pipérazin-1-yl]sulfonylphényl]-4-(méthylamino)pyrrolidin-2-one ;
la 4-(2-aminoéthylamino)-1-[4-[4-[6-chloro-4-(trifluorométhyl)-2-pyridyl]pipérazin-1-yl]sulfonylphényl]pyrrolidin-2-one ;
la 1-[4-[4-[6-chloro-4-(trifluorométhyl)-2-pyridyl]pipérazin-1-yl]sulfonylphényl]-4-(2-hydroxyéthylamino)pyrrolidin-2-one ;
la 1-[4-[4-[6-chloro-4-(trifluorométhyl)-2-pyridyl]pipérazin-1-yl]sulfonylphényl]-4-pipérazin-1-yl-pyrrolidin-2-one ;
la 1-[4-[4-[6-chloro-4-(trifluorométhyl)-2-pyridyl]pipérazin-1-yl]sulfonylphényl]-4-**[(3S,4R)**-3,4-dihydroxypyrrolidin-1-yl]pyrrolidin-2-one ;
la **4**-[3-(aminométhyl)azétidin-1-yl]-1-[4-[4-[6-chloro-4-(trifluorométhyl)-2-pyridyl]pipérazin-1-yl]sulfonylphényl]pyrrolidin-2-one ;
la 4-(3-aminopyrrolidin-1-yl)-1-[4-[4-[6-chloro-4-(trifluorométhyl)-2-pyridyl]pipérazin-1-yl]sulfonylphényl]pyrrolidin-2-one ;
la 4-(3-aminoazétidin-1-yl)-1-[4-[4-[6-chloro-4-(trifluorométhyl)-2-pyridyl]pipérazin-1-yl]sulfonylphényl]pyrrolidin-2-one ;
la 1-[4-[4-[6-chloro-4-(trifluorométhyl)-2-pyridyl]pipérazin-1-yl]sulfonylphényl]-4-(2,6-diazaspiro[3.3]heptan-2-yl)pyrrolidin-2-one ;
la 1-[4-[4-[6-chloro-4-(trifluorométhyl)-2-pyridyl]pipérazin-1-yl]sulfonylphényl]-4-[(3*S*)-3-(hydroxyméthyl)pipérazin-1-yl]pyrrolidin-2-one ;
la 1-[4-[4-[6-chloro-4-(trifluorométhyl)-2-pyridyl]pipérazin-1-yl]sulfonylphényl]-4-[(3*R*)-3-(hydroxyméthyl)pipérazin-1-yl]pyrrolidin-2-one ;
la 1-[4-[4-[6-chloro-4-(trifluorométhyl)-2-pyridyl]pipérazin-1-yl]sulfonylphényl]-4-[(1S,4S)-2,5-diazabicyclo[2.2.1]heptan-2-yl]pyrrolidin-2-one ;
la 4-[(3R,4R)-3-amino-4-hydroxy-pyrrolidin-1-yl]-1-[4-[4-[6-chloro-4-(trifluorométhyl)-2-pyridyl]pipérazin-1-yl]sulfonylphényl]pyrrolidin-2-one ;
la 4-((3*R*,4*R*)-3-amino-4-hydroxypipéridin-1-yl)-1-(4-((4-(6-chloro-4-(trifluorométhyl)pyridin-2-yl)pipérazin-1-yl)sulfonyl)phényl)pyrrolidin-2-one ;
la 4-(azétidin-3-ylamino)-1-[4-[4-[6-chloro-4-(trifluorométhyl)-2-pyridyl]pipérazin-1-yl]sulfonylphényl]pyrrolidin-2-one ;
la 1-[4-[4-[6-chloro-4-(trifluorométhyl)-2-pyridyl]pipérazin-1-yl]sulfonylphényl]-4-(pyrrolidin-3-ylamino)pyrrolidin-2-one ;
la 1-[4-[4-[6-chloro-4-(trifluorométhyl)-2-pyridyl]pipérazin-1-yl]sulfonylphényl]-4-(4-pipéridylamino)pyrrolidin-2-one ;
la 4-amino-1-[4-[4-[6-chloro-4-(trifluorométhyl)-2-pyridyl]pipérazin-1-yl]sulfonylphényl]-5-méthyl-pyrrolidin-2-one ;
la 4-amino-1-[5-[4-[6-chloro-4-(trifluorométhyl)-2-pyridyl]pipérazin-1-yl]sulfonyl-2-pyridyl]pyrrolidin-2-one ;
la 5-(aminométhyl)-3-[4-[4-[6-chloro-4-(trifluorométhyl)-2-pyridyl]pipérazin-1-yl]sulfonyl-2-fluoro-phényl]oxazolidin-2-one ;
la 5-(aminométhyl)-3-[4-[4-[6-chloro-4-(trifluorométhyl)-2-pyridyl]pipérazin-1-yl]sulfonyl-3-fluoro-phényl]oxazolidin-2-one ;
la 4-amino-1-[4-[4-[4-méthyl-6-(trifluorométhyl)pyrimidin-2-yl]pipérazin-1-yl]sulfonylphényl]pyrrolidin-2-one ;
la 4-amino-1-[4-[4-[4-(trideutériométhyl)-6-(trifluorométhyl)pyrimidin-2-yl]pipérazin-1-yl]sulfonylphényl]pyrrolidin-2-one ;
le 1-[4-(4-amino-2-oxo-pyrrolidin-1-yl)phényl]sulfonyl-4-[6-chloro-4-(trifluorométhyl)-2-pyridyl]pipérazine-2-carbonitrile ;
la 4-amino-1-[4-[4-[6-chloro-4-(difluorométhyl)-2-pyridyl]pipérazin-1-yl]sulfonylphényl]pyrrolidin-2-one ;
la 4-amino-1-[4-[4-(6-chloro-4-cyclopropylsulfonyl-2-pyridyl)pipérazin-1-yl]sulfonylphényl]pyrrolidin-2-one ;
la 4-amino-1-[4-[4-(6-chloro-4-tétrahydropyran-4-ylsulfonyl-2-pyridyl)pipérazin-1-yl]sulfonylphényl]pyrrolidin-2-one ;
le 1-[2-[4-[4-(4-amino-2-oxo-pyrrolidin-1-yl)phényl]sulfonylpipérazin-1-yl]-6-chloro-4-pyridyl]cyclopropanecarbonitrile ;
la 4-amino-1-[4-[4-(6-chloro-4-phénoxy-2-pyridyl)pipérazin-1-yl]sulfonylphényl]pyrrolidin-2-one ;
la 4-amino-1-[4-[4-[6-chloro-4-[difluoro(phényl)méthyl]-2-pyridyl]pipérazin-1-yl]sulfonylphényl]pyrrolidin-2-one ;
la 4-amino-1-[4-[4-(4-benzyl-6-chloro-2-pyridyl)pipérazin-1-yl]sulfonylphényl]pyrrolidin-2-one ;
la 4-amino-1-[4-[4-[6-chloro-4-(1-phénylcyclopropyl)-2-pyridyl]pipérazin-1-yl]sulfonylphényl]pyrrolidin-2-one ;
la 4-amino-1-[4-[4-[4-[difluoro(phényl)méthyl]-6-méthyl-pyrimidin-2-yl]pipérazin-1-yl]sulfonylphényl]pyrrolidin-2-one ;
la 4-amino-1-[4-[4-[2-chloro-6-[difluoro(phényl)méthyl]pyrimidin-4-yl]pipérazin-1-yl]sulfonylphényl]pyrrolidin-2-one ;
la 4-amino-1-[4-[4-[4-chloro-6-[difluoro(phényl)méthyl]pyrimidin-2-yl]pipérazin-1-yl]sulfonylphényl]pyrrolidin-2-one ;
la 4-amino-1-[4-[4-[2-cyclopropyl-6-[difluoro(phényl)méthyl]pyrimidin-4-yl]pipérazin-1-yl]sulfonylphényl]pyrrolidin-2-one ;
la 4-amino-1-[4-[4-[4-cyclopropyl-6-[difluoro(phényl)méthyl]pyrimidin-2-yl]pipérazin-1-yl]sulfonylphényl]pyrrolidin-2-one ;
la 4-amino-1-[4-[4-[6-[difluoro(phényl)méthyl]-2-méthoxy-pyrimidin-4-yl]pipérazin-1-yl]sulfonylphényl]pyrrolidin-2-one ;
la 4-amino-1-[4-[4-[4-[difluoro(phényl)méthyl]-6-méthoxy-pyrimidin-2-yl]pipérazin-1-yl]sulfonylphényl]pyrrolidin-2-one ;
la (4*R*)-4-amino-1-[4-[4-[6-[difluoro(phényl)méthyl]-2-méthyl-pyrimidin-4-yl]pipérazin-1-yl]sulfonylphényl]pyrrolidin-2-one ;
la 4-amino-1-[4-[4-[6-[difluoro(phényl)méthyl]-2-vinyl-pyrimidin-4-yl]pipérazin-1-yl]sulfonylphényl]pyrrolidin-2-one ;
la 2-[4-[4-[6-chloro-4-[difluoro(phényl)méthyl]-2-pyridyl]pipérazin-1-yl]sulfonylphényl]-2,6,9-triazaspiro[4.5]décan-3-one ;
la 5-(aminométhyl)-1-[4-[4-[6-chloro-4-[difluoro(phényl)méthyl]-2-pyridyl]pipérazin-1-yl]sulfonylphényl]pyrrolidin-2-one ;
la (3a*R*,7a*S*)-3-[4-[4-[6-chloro-4-[difluoro(phényl)méthyl]-2-pyridyl]pipérazin-1-yl]sulfonylphényl]-3a,4,5,6,7,7a-hexahydrooxazolo[4,5-c]pyridin-2-one ;
la 5-(aminométhyl)-3-[4-[4-[6-chloro-4-[difluoro(phényl)méthyl]-2-pyridyl]pipérazin-1-yl]sulfonylphényl]oxazolidin-2-one ;
la 4-amino-1-[4-[4-[6-chloro-4-[difluoro(2-pyridyl)méthyl]-2-pyridyl]pipérazin-1-yl]sulfonylphényl]pyrrolidin-2-one ;
la 4-amino-1-[4-[4-[6-chloro-4-[difluoro(3-pyridyl)méthyl]-2-pyridyl]pipérazin-1-yl]sulfonylphényl]pyrrolidin-2-one ;
la 4-amino-1-[4-[4-[6-chloro-4-[difluoro(4-pyridyl)méthyl]-2-pyridyl]pipérazin-1-yl]sulfonylphényl]pyrrolidin-2-one ;
la 4-amino-1-[4-[4-[6-chloro-4-[difluoro(pyrimidin-5-yl)méthyl]-2-pyridyl]pipérazin-1-yl]sulfonylphényl]pyrrolidin-2-one ;
la 4-amino-1-[4-[4-[6-chloro-4-[difluoro(pyrazin-2-yl)méthyl]-2-pyridyl]pipérazin-1-yl]sulfonylphényl]pyrrolidin-2-one ;
la 4-amino-1-[4-[4-[6-chloro-4-[difluoro(pyrimidin-2-yl)méthyl]-2-pyridyl]pipérazin-1-yl]sulfonylphényl]pyrrolidin-2-one ;
la 4-amino-1-[4-[4-[6-chloro-4-[difluoro(tétrahydropyran-4-yl)méthyl]-2-pyridyl]pipérazin-1-yl]sulfonylphényl]pyrrolidin-2-one ;
la 4-amino-1-[4-[4-[6-chloro-4-[difluoro(tétrahydrofuran-3-yl)méthyl]-2-pyridyl]pipérazin-1-yl]sulfonylphényl]pyrrolidin-2-one ;
la 4-amino-1-[4-[4-[6-chloro-4-[difluoro(oxétan-3-yl)méthyl]-2-pyridyl]pipérazin-1-yl]sulfonylphényl]pyrrolidin-2-one ;
la 4-amino-1-[4-[4-[6-chloro-4-[3,6-dihydro-2H-pyran-4-yl(difluoro)méthyl]-2-pyridyl]pipérazin-1-yl]sulfonylphényl]pyrrolidin-2-one ;
la 4-amino-1-[4-[4-[6-chloro-4-[difluoro(tétrahydropyran-3-yl)méthyl]-2-pyridyl]pipérazin-1-yl]sulfonylphényl]pyrrolidin-2-one ;
la 4-amino-1-[4-[4-[6-chloro-4-[difluoro(8-oxabicyclo[3.2.1]octan-3-yl)méthyl]-2-pyridyl]pipérazin-1-yl]sulfonylphényl]pyrrolidin-2-one ;
la (4*R*)-4-amino-1-[4-[4-[6-chloro-4-[difluoro(3-oxabicyclo[3.1.0]hexan-6-yl)méthyl]-2-pyridyl]pipérazin-1-yl]sulfonylphényl]pyrrolidin-2-one ;
la (4*R*)-4-amino-1-[4-[4-[6-chloro-4-[[(25)-1,4-dioxan-2-yl]-difluoro-méthyl]-2-pyridyl]pipérazin-1-yl]sulfonylphényl]pyrrolidin-2-one ;
la (4*R*)-4-amino-1-[4-[4-[6-chloro-4-[[(2*R*)-1,4-dioxan-2-yl]-difluoro-méthyl]-2-pyridyl]pipérazin-1-yl]sulfonylphényl]pyrrolidin-2-one ;
la (4*R*)-4-amino-1-[4-[4-[6-chloro-4-[difluoro-[(25)-morpholin-2-yl]méthyl]-2-pyridyl]pipérazin-1-yl]sulfonylphényl]pyrrolidin-2-one ;
la (4*R*)-4-amino-1-[4-[4-[6-chloro-4-[difluoro-[(2R)-morpholin-2-yl]méthyl]-2-pyridyl]pipérazin-1-yl]sulfonylphényl]pyrrolidin-2-one ;
la (4*R*)-4-amino-1-[4-[4-[6-chloro-4-[difluoro(1,2,3,6-tétrahydropyridin-4-yl)méthyl]-2-pyridyl]pipérazin-1-yl]sulfonylphényl]pyrrolidin-2-one ;
la (4*R*)-4-amino-1-[4-[4-[6-chloro-4-[difluoro-[(3*R*)-3-pipéridyl]méthyl]-2-pyridyl]pipérazin-1-yl]sulfonylphényl]pyrrolidin-2-one ;
la (4*R*)-4-amino-1-[4-[4-[6-chloro-4-[difluoro-[(3*S*)-3-pipéridyl]méthy1]-2-pyridyl]pipérazin-1-yl]sulfonylphényl]pyrrolidin-2-one ;
la (4*R*)-4-amino-1-[4-[4-[4-[azétidin-3-yl(difluoro)méthyl]-6-chloro-2-pyridyl]pipérazin-1-yl]sulfonylphényl]pyrrolidin-2-one ;
la 3-[4-[4-[6-chloro-4-[difluoro-[(2*S*)-morpholin-2-yl]méthyl]-2-pyridyl]pipérazin-1-yl]sulfonylphényl]-5-(hydroxyméthyl)oxazolidin-2-one ;
la (4*R*)-4-amino-1-[4-[4-[6-chloro-4-[difluoro-(5-méthylpyrazin-2-yl)méthyl]-2-pyridyl]pipérazin-1-yl]sulfonylphényl]pyrrolidin-2-one ;
la (4*R*)-4-amino-1-[4-[4-[6-chloro-4-[difluoro-(6-méthylpyrazin-2-yl)méthyl]-2-pyridyl]pipérazin-1-yl]sulfonylphényl]pyrrolidin-2-one ;
la (4*R*)-4-amino-1-[4-[4-[6-chloro-4-[(5-chloro-6-méthyl-pyrazin-2-yl)-difluoro-méthyl]-2-pyridyl]pipérazin-1-yl]sulfonylphényl]pyrrolidin-2-one ;
la (4*R*)-4-amino-1-[4-[4-[6-chloro-4-[difluoro-[cis-6-méthylmorpholin-2-yl]méthyl]-2-pyridyl]pipérazin-1-yl]sulfonylphényl]pyrrolidin-2-one ;
la ((4*R*)-4-amino-1-[4-[4-[6-chloro-4-[difluoro-(5-méthylmorpholin-2-yl)méthyl]-2-pyridyl]pipérazin-1-yl]sulfonylphényl]pyrrolidin-2-one ;
la (4*R*)-4-amino-1-[4-[4-[4-[[*cis*-3-azabicyclo[3.1.0]hexan-1-yl]-difluoro-méthyl]-6-chloro-2-pyridyl]pipérazin-1-yl]sulfonylphényl]pyrrolidin-2-one ;
la (4R)-4-amino-1-[4-[4-[6-chloro-4-[trans-difluoro-[2-(hydroxyméthyl)cyclopropyl]méthyl]-2-pyridyl]pipérazin-1-yl]sulfonylphényl]pyrrolidin-2-one ;
la (4*R*)-4-amino-1-[4-[4-[4-[[*trans*-4-(aminométhyl)cyclohexyl]-difluoro-méthyl]-6-chloro-2-pyridyl]pipérazin-1-yl]sulfonylphényl]pyrrolidin-2-one ;
la (4*R*)-4-amino-1-[4-[4-[6-chloro-4-[*trans*-difluoro-(3-hydroxycyclobutyl)méthyl]-2-pyridyl]pipérazin-1-yl]sulfonylphényl]pyrrolidin-2-one ; et
le *trans*-4-[[2-[4-[4-[(4R)-4-amino-2-oxo-pyrrolidin-1-yl]phényl]sulfonylpipérazin-1-yl]-6-chloro-4-pyridyl]-difluoro-méthyl]cyclohexanecarboxamide ;
ou un sel pharmaceutiquement acceptable de ceux-ci.

12. Procédé de préparation d'un composé selon l'une quelconque des revendications 1 à 11 comprenant l'étape suivante :
a) réaction de couplage entre le composé de formule (VII), 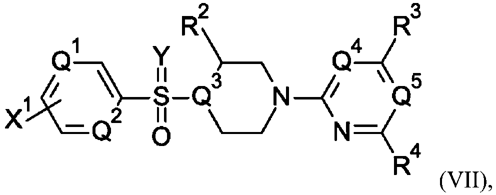 et un lactame ou un carbamate, R¹H, en présence d'un catalyseur et d'une base ;
dans lequel
dans l'étape a), le catalyseur est CuI, la base est Cs₂CO₃ ;
R¹, R², R³, R⁴, Y, Q¹, Q², Q³, Q⁴ et Q⁵ sont tels que définis dans l'une quelconque des revendications 1 à 10.

13. Composé ou sel pharmaceutiquement acceptable selon l'une quelconque des revendications 1 à 11 pour une utilisation comme substance thérapeutiquement active.

14. Composition pharmaceutique comprenant un composé selon l'une quelconque des revendications 1 à 11 et un véhicule thérapeutiquement inerte.

15. Composé ou sel pharmaceutiquement acceptable selon l'une quelconque des revendications 1 à 11 pour une utilisation dans le traitement ou la prophylaxie d'une infection bactérienne, en particulier la bactérie est une bactérie à Gram négatif, plus particulièrement la bactérie à Gram négatif est choisie parmi *Enterobacteriaceae, Neisseria gonorrhoeae, Haemophilus influenzae, Helicobacter pylorus, Acinetobacter baumannii* et *Pseudomonas aeruginosa.*
